# EUROPEAN PATENT SPECIFICATION

(11) **EP 2 714 915 B1**
(45) Date of publication and mention of the grant of the patent: **18.10.2017**
(21) Application number: 12725353.2
(22) Date of filing: 25.05.2012
(51) Int. Cl.: C12N 15/82, C12N 9/22

(54) **MEANS AND METHODS TO INDUCE APOMIXIS IN PLANTS**
MITTEL UND VERFAHREN ZUR EINFÜHRUNG VON APOMIXIS IN PFLANZEN
MOYENS ET PROCÉDÉS POUR INDUIRE L'APOMIXIE CHEZ LES PLANTES

(30) Priority: 30.05.2011 EP 11168075
(43) Date of publication of application: 09.04.2014
(73) Proprietor: Leibniz-Institut für Pflanzengenetik und Kulturpflanzenforschung Gatersleben (IPK), 06466 Gatersleben (DE)
(72) Inventor: CORRAL, José M., 06484 Quedlinburg (DE); SHARBEL, Timothy, 06484 Quedlinburg (DE)
(74) Representative: Desaix, Anne
(86) International application number: PCT/EP2012/059808
(87) International publication number: WO 2012/163818

(56) References cited:
- WO-A1-2011/020746
- DATABASE EMBASE [Online] ELSEVIER SCIENCE PUBLISHERS, AMSTERDAM, NL; 24 October 2002 (2002-10-24), Nguyen et al.: "Arabidopsis thaliana putative exonuclease (At1g74390) mRNA", XP002658227, retrieved from EBI Database accession no. BT000288
- LI HAIYING ET AL: "Proteomic analysis of sugar beet apomictic monosomic addition line M14.", JOURNAL OF PROTEOMICS 1 DEC 2009 LNKD- PUBMED:19782777, vol. 73, no. 2, 1 December 2009 (2009-12-01), pages 297-308, XP002658228, ISSN: 1876-7737

### SEQUENCE LISTING

<110> Leibniz-Institut für Pflanzengenetik und Kulturpflanzenforschung Gatersleben (IPK)
<120> Means and methods to induce apomixis in plants
<130> 203840
<160> 65
<170> PatentIn version 3.5
<210> 1
   <211> 165
   <212> PRT
   <213> Boechera
<220>
   <221> misc_feature
   <222> (10) .. (10)
   <223> Xaa can be any naturally occurring amino acid
<220>
   <221> misc_feature
   <222> (12) .. (12)
   <223> Xaa can be any naturally occurring amino acid
<220>
   <221> misc_feature
   <222> (17) .. (17)
   <223> Xaa can be any naturally occurring amino acid
<220>
   <221> misc_feature
   <222> (33) .. (33)
   <223> Xaa can be any naturally occurring amino acid
<220>
   <221> misc_feature
   <222> (39) .. (39)
   <223> Xaa can be any naturally occurring amino acid
<220>
   <221> misc_feature
   <222> (42) .. (42)
   <223> Xaa can be any naturally occurring amino acid
<220>
   <221> misc_feature
   <222> (51) .. (51)
   <223> Xaa can be any naturally occurring amino acid
<220>
   <221> misc_feature
   <222> (78) .. (78)
   <223> Xaa can be any naturally occurring amino acid
<220>
   <221> misc_feature
   <222> (82) .. (83)
   <223> Xaa can be any naturally occurring amino acid
<220>
   <221> misc_feature
   <222> (88) .. (88)
   <223> Xaa can be any naturally occurring amino acid
<220>
   <221> misc_feature
   <222> (100) .. (101)
   <223> Xaa can be any naturally occurring amino acid
<220>
   <221> misc_feature
   <222> (106) .. (106)
   <223> Xaa can be any naturally occurring amino acid
<220>
   <221> misc_feature
   <222> (109) .. (110)
   <223> Xaa can be any naturally occurring amino acid
<220>
   <221> misc_feature
   <222> (112) .. (112)
   <223> Xaa can be any naturally occurring amino acid
<220>
   <221> misc_feature
   <222> (114) .. (114)
   <223> Xaa can be any naturally occurring amino acid
<220>
   <221> misc_feature
   <222> (116) .. (117)
   <223> Xaa can be any naturally occurring amino acid
<220>
   <221> misc_feature
   <222> (120) .. (120)
   <223> Xaa can be any naturally occurring amino acid
<220>
   <221> misc_feature
   <222> (123) .. (124)
   <223> Xaa can be any naturally occurring amino acid
<220>
   <221> misc_feature
   <222> (131) .. (131)
   <223> Xaa can be any naturally occurring amino acid
<220>
   <221> misc_feature
   <222> (135) .. (135)
   <223> Xaa can be any naturally occurring amino acid
<220>
   <221> misc_feature
   <222> (139) .. (140)
   <223> Xaa can be any naturally occurring amino acid
<220>
   <221> misc_feature
   <222> (145) .. (145)
   <223> Xaa can be any naturally occurring amino acid
<220>
   <221> misc_feature
   <222> (164) .. (164)
   <223> Xaa can be any naturally occurring amino acid
<400> 1
   Val Phe Phe Asp Leu Glu Thr Ala Val Xaa Thr Xaa Ser Gly Gln Pro
   1               5                   10                  15
   Xaa Ala Ile Leu Glu Phe Gly Ala Ile Leu Val Cys Pro Met Lys Leu
               20                  25                  30
   Xaa Glu Leu Tyr Ser Tyr Xaa Thr Leu Xaa Arg Pro Thr Asp Leu Ser
           35                  40                  45
   Leu Ile Xaa Thr Leu Thr Lys Arg Arg Ser Gly Ile Thr Arg Asp Gly
       50                  55                  60
   Val Leu Ser Ala Pro Thr Phe Ser Glu Ile Ala Asp Glu Xaa Tyr Asp
   65                  70                  75                  80
   Ile Xaa Xaa Gly Arg Ile Trp Xaa Gly His Asn Ile Lys Arg Phe Asp
                   85                  90                  95
   Cys Val Arg Xaa Xaa Asp Ala Phe Ala Xaa Ile Gly Xaa Xaa Pro Xaa
               100                 105                 110
   Glu Xaa Lys Xaa Xaa Ile Asp Xaa Leu Ser Xaa Xaa Ser Gln Lys Phe
           115                 120                 125
   Gly Lys Xaa Ala Gly Asp Xaa Lys Met Ala Xaa Xaa Ala Thr Tyr Phe
       130                 135                 140
   Xaa Leu Gly Asp Gln Ala His Arg Ser Leu Asp Asp Val Arg Met Asn
   145                 150                 155                 160
   Leu Glu Val Xaa Lys
                   165
<210> 2
   <211> 165
   <212> PRT
   <213> Boechera
<220>
   <221> misc_feature
   <222> (17) .. (17)
   <223> Xaa can be any naturally occurring amino acid
<220>
   <221> misc_feature
   <222> (83) .. (83)
   <223> Xaa can be any naturally occurring amino acid
<220>
   <221> misc_feature
   <222> (88) .. (88)
   <223> Xaa can be any naturally occurring amino acid
<220>
   <221> misc_feature
   <222> (101) .. (101)
   <223> Xaa can be any naturally occurring amino acid
<220>
   <221> misc_feature
   <222> (106) .. (106)
   <223> Xaa can be any naturally occurring amino acid
<220>
   <221> misc_feature
   <222> (110) .. (110)
   <223> Xaa can be any naturally occurring amino acid
<220>
   <221> misc_feature
   <222> (124) .. (124)
   <223> Xaa can be any naturally occurring amino acid
<220>
   <221> misc_feature
   <222> (140) .. (140)
   <223> Xaa can be any naturally occurring amino acid
<220>
   <221> misc_feature
   <222> (145) .. (145)
   <223> Xaa can be any naturally occurring amino acid
<220>
   <221> misc_feature
   <222> (164) .. (164)
   <223> Xaa can be any naturally occurring amino acid
<400> 2
   Val Phe Phe Asp Leu Glu Thr Ala Val Pro Thr Lys Ser Gly Gln Pro
   1               5                   10                  15
   Xaa Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
           35                  40                  45
   Leu Ile Ser Thr Leu Thr Lys Arg Arg Ser Gly Ile Thr Arg Asp Gly
       50                  55                  60
   Val Leu Ser Ala Pro Thr Phe Ser Glu Ile Ala Asp Glu Val Tyr Asp
   65 70 75 80
   Ile Leu Xaa Gly Arg Ile Trp Xaa Gly His Asn Ile Lys Arg Phe Asp
   85 90 95
   Cys Val Arg Ile Xaa Asp Ala Phe Ala Xaa Ile Gly Leu Xaa Pro Pro
   100 105 110
   Glu Pro Lys Ala Thr Ile Asp Ser Leu Ser Leu Xaa Ser Gln Lys Phe
   115 120 125
   Gly Lys Arg Ala Gly Asp Met Lys Met Ala Ser Xaa Ala Thr Tyr Phe
   130 135 140
   Xaa Leu Gly Asp Gln Ala His Arg Ser Leu Asp Asp Val Arg Met Asn
   145 150 155 160
   Leu Glu Val Xaa Lys
   165
<210> 3
   <211> 165
   <212> PRT
   <213> Boechera
<220>
   <221> misc_feature
   <222> (10) .. (10)
   <223> Xaa can be any naturally occurring amino acid
<220>
   <221> misc_feature
   <222> (12) .. (12)
   <223> Xaa can be any naturally occurring amino acid
<220>
   <221> misc_feature
   <222> (33) .. (33)
   <223> Xaa can be any naturally occurring amino acid
<220>
   <221> misc_feature
   <222> (39) .. (39)
   <223> Xaa can be any naturally occurring amino acid
<220>
   <221> misc_feature
   <222> (42) .. (42)
   <223> Xaa can be any naturally occurring amino acid
<220>
   <221> misc_feature
   <222> (51) .. (51)
   <223> Xaa can be any naturally occurring amino acid
<220>
   <221> misc_feature
   <222> (78) .. (78)
   <223> Xaa can be any naturally occurring amino acid
<220>
   <221> misc_feature
   <222> (82) .. (82)
   <223> Xaa can be any naturally occurring amino acid
<220>
   <221> misc_feature
   <222> (100) .. (101)
   <223> Xaa can be any naturally occurring amino acid
<220>
   <221> misc_feature
   <222> (106) .. (106)
   <223> Xaa can be any naturally occurring amino acid
<220>
   <221> misc_feature
   <222> (109) .. (110)
   <223> Xaa can be any naturally occurring amino acid
<220>
   <221> misc_feature
   <222> (112) .. (112)
   <223> Xaa can be any naturally occurring amino acid
<220>
   <221> misc_feature
   <222> (114) .. (114)
   <223> Xaa can be any naturally occurring amino acid
<220>
   <221> misc_feature
   <222> (116) .. (117)
   <223> Xaa can be any naturally occurring amino acid
<220>
   <221> misc_feature
   <222> (120) .. (120)
   <223> Xaa can be any naturally occurring amino acid
<220>
   <221> misc_feature
   <222> (123) .. (124)
   <223> Xaa can be any naturally occurring amino acid
<220>
   <221> misc_feature
   <222> (131) .. (131)
   <223> Xaa can be any naturally occurring amino acid
<220>
   <221> misc_feature
   <222> (135) .. (135)
   <223> Xaa can be any naturally occurring amino acid
<220>
   <221> misc_feature
   <222> (139) .. (140)
   <223> Xaa can be any naturally occurring amino acid
<220>
   <221> misc_feature
   <222> (145) .. (145)
   <223> Xaa can be any naturally occurring amino acid
<220>
   <221> misc_feature
   <222> (164) .. (164)
   <223> Xaa can be any naturally occurring amino acid
<400> 3
   Val Phe Phe Asp Leu Glu Thr Ala Val Xaa Thr Xaa Ser Gly Gln Pro
   1               5                   10                  15
   Phe Ala Ile Leu Glu Phe Gly Ala Ile Leu Val Cys Pro Met Lys Leu
               20                  25                  30
   Xaa Glu Leu Tyr Ser Tyr Xaa Thr Leu Xaa Arg Pro Thr Asp Leu Ser
           35                  40                  45
   Leu Ile Xaa Thr Leu Thr Lys Arg Arg Ser Gly Ile Thr Arg Asp Gly
       50                  55                  60
   Val Leu Ser Ala Pro Thr Phe Ser Glu Ile Ala Asp Glu Xaa Tyr Asp
   65                  70                  75                  80
   Ile Xaa His Gly Arg Ile Trp Ala Gly His Asn Ile Lys Arg Phe Asp
                   85                  90                  95
   Cys Val Arg Xaa Xaa Asp Ala Phe Ala Xaa Ile Gly Xaa Xaa Pro Xaa
               100                 105                 110
   Glu Xaa Lys Xaa Xaa Ile Asp Xaa Leu Ser Xaa Xaa Ser Gln Lys Phe
           115                 120                 125
   Gly Lys Xaa Ala Gly Asp Xaa Lys Met Ala Xaa Xaa Ala Thr Tyr Phe
       130                 135                 140
   Xaa Leu Gly Asp Gln Ala His Arg Ser Leu Asp Asp Val Arg Met Asn
   145                 150                 155                 160
   Leu Glu Val Xaa Lys
                   165
<210> 4
   <211> 506
   <212> PRT
   <213> Boechera
<220>
   <221> misc_feature
   <222> (7) .. (7)
   <223> Xaa can be any naturally occurring amino acid
<220>
   <221> misc_feature
   <222> (9) .. (9)
   <223> Xaa can be any naturally occurring amino acid
<220>
   <221> misc_feature
   <222> (11) .. (11)
   <223> Xaa can be any naturally occurring amino acid
<220>
   <221> misc_feature
   <222> (23) .. (23)
   <223> Xaa can be any naturally occurring amino acid
<220>
   <221> misc_feature
   <222> (25) .. (25)
   <223> Xaa can be any naturally occurring amino acid
<220>
   <221> misc_feature
   <222> (30) .. (30)
   <223> Xaa can be any naturally occurring amino acid
<220>
   <221> misc_feature
   <222> (46) .. (46)
   <223> Xaa can be any naturally occurring amino acid
<220>
   <221> misc_feature
   <222> (52) .. (52)
   <223> Xaa can be any naturally occurring amino acid
<220>
   <221> misc_feature
   <222> (55) .. (55)
   <223> Xaa can be any naturally occurring amino acid
<220>
   <221> misc_feature
   <222> (64) .. (64)
   <223> Xaa can be any naturally occurring amino acid
<220>
   <221> misc_feature
   <222> (91) .. (91)
   <223> Xaa can be any naturally occurring amino acid
<220>
   <221> misc_feature
   <222> (95) .. (96)
   <223> Xaa can be any naturally occurring amino acid
<220>
   <221> misc_feature
   <222> (101) .. (101)
   <223> Xaa can be any naturally occurring amino acid
<220>
   <221> misc_feature
   <222> (113) .. (114)
   <223> Xaa can be any naturally occurring amino acid
<220>
   <221> misc_feature
   <222> (119) .. (119)
   <223> Xaa can be any naturally occurring amino acid
<220>
   <221> misc_feature
   <222> (122) .. (123)
   <223> Xaa can be any naturally occurring amino acid
<220>
   <221> misc_feature
   <222> (125) .. (125)
   <223> Xaa can be any naturally occurring amino acid
<220>
   <221> misc_feature
   <222> (127) .. (127)
   <223> Xaa can be any naturally occurring amino acid
<220>
   <221> misc_feature
   <222> (129) .. (130)
   <223> Xaa can be any naturally occurring amino acid
<220>
   <221> misc_feature
   <222> (133) .. (133)
   <223> Xaa can be any naturally occurring amino acid
<220>
   <221> misc_feature
   <222> (136) .. (137)
   <223> Xaa can be any naturally occurring amino acid
<220>
   <221> misc_feature
   <222> (144) .. (144)
   <223> Xaa can be any naturally occurring amino acid
<220>
   <221> misc_feature
   <222> (148) .. (148)
   <223> Xaa can be any naturally occurring amino acid
<220>
   <221> misc_feature
   <222> (152) .. (153)
   <223> Xaa can be any naturally occurring amino acid
<220>
   <221> misc_feature
   <222> (158) .. (158)
   <223> Xaa can be any naturally occurring amino acid
<220>
   <221> misc_feature
   <222> (177) .. (177)
   <223> Xaa can be any naturally occurring amino acid
<220>
   <221> misc_feature
   <222> (179) .. (179)
   <223> Xaa can be any naturally occurring amino acid
<220>
   <221> misc_feature
   <222> (181) .. (181)
   <223> Xaa can be any naturally occurring amino acid
<220>
   <221> misc_feature
   <222> (183) .. (183)
   <223> Xaa can be any naturally occurring amino acid
<220>
   <221> misc_feature
   <222> (195) .. (197)
   <223> Xaa can be any naturally occurring amino acid
<220>
   <221> misc_feature
   <222> (200) .. (202)
   <223> Xaa can be any naturally occurring amino acid
<220>
   <221> misc_feature
   <222> (206) .. (207)
   <223> Xaa can be any naturally occurring amino acid
<220>
   <221> misc_feature
   <222> (217) .. (217)
   <223> Xaa can be any naturally occurring amino acid
<220>
   <221> misc_feature
   <222> (228) .. (228)
   <223> Xaa can be any naturally occurring amino acid
<220>
   <221> misc_feature
   <222> (231) .. (232)
   <223> Xaa can be any naturally occurring amino acid
<220>
   <221> misc_feature
   <222> (240) .. (240)
   <223> Xaa can be any naturally occurring amino acid
<220>
   <221> misc_feature
   <222> (243) .. (243)
   <223> Xaa can be any naturally occurring amino acid
<220>
   <221> misc_feature
   <222> (258) .. (259)
   <223> Xaa can be any naturally occurring amino acid
<220>
   <221> misc_feature
   <222> (264) .. (264)
   <223> Xaa can be any naturally occurring amino acid
<220>
   <221> misc_feature
   <222> (285) .. (285)
   <223> Xaa can be any naturally occurring amino acid
<220>
   <221> misc_feature
   <222> (299) .. (299)
   <223> Xaa can be any naturally occurring amino acid
<220>
   <221> misc_feature
   <222> (303) .. (303)
   <223> Xaa can be any naturally occurring amino acid
<220>
   <221> misc_feature
   <222> (321) .. (321)
   <223> Xaa can be any naturally occurring amino acid
<220>
   <221> misc_feature
   <222> (328) .. (328)
   <223> Xaa can be any naturally occurring amino acid
<220>
   <221> misc_feature
   <222> (330) .. (331)
   <223> Xaa can be any naturally occurring amino acid
<220>
   <221> misc_feature
   <222> (336) .. (336)
   <223> Xaa can be any naturally occurring amino acid
<220>
   <221> misc_feature
   <222> (341) .. (342)
   <223> Xaa can be any naturally occurring amino acid
<220>
   <221> misc_feature
   <222> (348) .. (348)
   <223> Xaa can be any naturally occurring amino acid
<220>
   <221> misc_feature
   <222> (351) .. (352)
   <223> Xaa can be any naturally occurring amino acid
<220>
   <221> misc_feature
   <222> (354) .. (354)
   <223> Xaa can be any naturally occurring amino acid
<220>
   <221> misc_feature
   <222> (357) .. (357)
   <223> Xaa can be any naturally occurring amino acid
<220>
   <221> misc_feature
   <222> (360) .. (361)
   <223> Xaa can be any naturally occurring amino acid
<220>
   <221> misc_feature
   <222> (371) .. (371)
   <223> Xaa can be any naturally occurring amino acid
<220>
   <221> misc_feature
   <222> (387) .. (387)
   <223> Xaa can be any naturally occurring amino acid
<220>
   <221> misc_feature
   <222> (397) .. (398)
   <223> Xaa can be any naturally occurring amino acid
<220>
   <221> misc_feature
   <222> (400) .. (400)
   <223> Xaa can be any naturally occurring amino acid
<220>
   <221> misc_feature
   <222> (411) .. (411)
   <223> Xaa can be any naturally occurring amino acid
<220>
   <221> misc_feature
   <222> (413) .. (414)
   <223> Xaa can be any naturally occurring amino acid
<220>
   <221> misc_feature
   <222> (420) .. (420)
   <223> Xaa can be any naturally occurring amino acid
<220>
   <221> misc_feature
   <222> (422) .. (422)
   <223> Xaa can be any naturally occurring amino acid
<220>
   <221> misc_feature
   <222> (425) .. (425)
   <223> Xaa can be any naturally occurring amino acid
<220>
   <221> misc_feature
   <222> (431) .. (431)
   <223> Xaa can be any naturally occurring amino acid
<220>
   <221> misc_feature
   <222> (440) .. (440)
   <223> Xaa can be any naturally occurring amino acid
<220>
   <221> misc_feature
   <222> (442) .. (442)
   <223> Xaa can be any naturally occurring amino acid
<220>
   <221> misc_feature
   <222> (444) .. (444)
   <223> Xaa can be any naturally occurring amino acid
<220>
   <221> misc_feature
   <222> (448) .. (448)
   <223> Xaa can be any naturally occurring amino acid
<220>
   <221> misc_feature
   <222> (471) .. (471)
   <223> Xaa can be any naturally occurring amino acid
<220>
   <221> misc_feature
   <222> (474) .. (474)
   <223> Xaa can be any naturally occurring amino acid
<220>
   <221> misc_feature
   <222> (478) .. (478)
   <223> Xaa can be any naturally occurring amino acid
<220>
   <221> misc_feature
   <222> (482) .. (482)
   <223> Xaa can be any naturally occurring amino acid
<220>
   <221> misc_feature
   <222> (484) .. (484)
   <223> Xaa can be any naturally occurring amino acid
<220>
   <221> misc_feature
   <222> (488) .. (488)
   <223> Xaa can be any naturally occurring amino acid
<220>
   <221> misc_feature
   <222> (494) .. (494)
   <223> Xaa can be any naturally occurring amino acid
<220>
   <221> misc_feature
   <222> (503) .. (503)
   <223> Xaa can be any naturally occurring amino acid
<400> 4
   Met Ala Ser Thr Leu Gly Xaa Asp Xaa Arg Xaa Glu Ile Val Phe Phe
   1               5                   10                  15
   Asp Leu Glu Thr Ala Val Xaa Thr Xaa Ser Gly Gln Pro Xaa Ala Ile
               20                  25                  30
   Leu Glu Phe Gly Ala Ile Leu Val Cys Pro Met Lys Leu Xaa Glu Leu
           35                  40                  45
   Tyr Ser Tyr Xaa Thr Leu Xaa Arg Pro Thr Asp Leu Ser Leu Ile Xaa
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Xaa Tyr Asp Ile Xaa Xaa
                   85                  90                  95
   Gly Arg Ile Trp Xaa Gly His Asn Ile Lys Arg Phe Asp Cys Val Arg
               100                 105                 110
   Xaa Xaa Asp Ala Phe Ala Xaa Ile Gly Xaa Xaa Pro Xaa Glu Xaa Lys
           115                 120                 125
   Xaa Xaa Ile Asp Xaa Leu Ser Xaa Xaa Ser Gln Lys Phe Gly Lys Xaa
       130                 135                 140
   Ala Gly Asp Xaa Lys Met Ala Xaa Xaa Ala Thr Tyr Phe Xaa Leu Gly
   145                 150                 155                 160
   Asp Gln Ala His Arg Ser Leu Asp Asp Val Arg Met Asn Leu Glu Val
                   165                 170                 175
   Xaa Lys Xaa Cys Xaa Thr Xaa Leu Phe Leu Glu Ser Ser Val Pro Asp
               180                 185                 190
   Ile Leu Xaa Xaa Xaa Ser Trp Xaa Xaa Xaa Arg Lys Ser Xaa Xaa Thr
           195                 200                 205
   Arg Ser Asn Glu Lys Ser Leu Pro Xaa Gly Val Arg Glu Ser Pro Thr
       210                 215                 220
   Ser Ser Ser Xaa Ser Pro Xaa Xaa Asp Pro Ser Ser Ser Ser Val Xaa
   225                 230                 235                 240
   Ala Thr Xaa Val Lys Asn His Pro Ile Ile Ser Leu Leu Thr Glu Cys
                   245                 250                 255
   Ser Xaa Xaa Asp Thr Ser Ser Xaa Glu Ile Asp Pro Ser Asp Ile Thr
               260                 265                 270
   Thr Leu Ile Ser Lys Leu His Ile Gly Thr Leu Lys Xaa Asp Ala Ala
           275                 280                 285
   Asp Glu Ala Lys Thr Val Arg Asp Ala Ala Xaa Glu Ala Lys Xaa Val
       290                 295                 300
   Arg Gln Gln Gly Glu Ser Thr Asp Pro Asn Ala Lys Asp Glu Ser Phe
   305                 310                 315                 320
   Xaa Gly Val Asn Glu Val Ser Xaa Ser Xaa Xaa Arg Ala Ser Leu Xaa
                   325                 330                 335
   Pro Leu Tyr Arg Xaa Xaa Leu Arg Met Glu Leu Xaa His Asn Xaa Xaa
               340                 345                 350
   Pro Xaa His Leu Xaa Trp Tyr Xaa Xaa Lys Ile Arg Phe Gly Ile Ser
           355                 360                 365
   Arg Lys Xaa Val Asp His Val Gly Arg Pro Lys Met Asn Ile Val Val
       370                 375                 380
   Asp Ile Xaa Pro Asp Leu Cys Lys Ile Leu Asp Ala Xaa Xaa Ala Xaa
   385                 390                 395                 400
   Ala His Asn Leu Leu Ile Asp Ser Ser Thr Xaa Ser Xaa Xaa Arg Pro
                   405                 410                 415
   Thr Val Met Xaa Lys Xaa Gly Phe Xaa Asn Tyr Pro Thr Ala Xaa Leu
               420                 425                 430
   Gln Ile Ser Ser Glu Ser Asn Xaa Thr Xaa Val Xaa Gln Lys Glu Xaa
           435                 440                 445
   Pro Leu Gly Thr Asn Gln Lys Leu Asp Phe Ser Ser Asp Asn Phe Glu
       450                 455                 460
   Lys Leu Glu Ser Ala Leu Xaa Pro Gly Xaa Leu Val Asp Xaa Phe Phe
   465                 470                 475                 480
   Ser Xaa Glu Xaa Tyr Asp Tyr Xaa Lys Met Val Gly Ile Xaa Leu Ala
                   485                 490                 495
   Ala Arg Lys Leu Val Ile Xaa Leu Lys Lys
               500                 505
<210> 5
   <211> 506
   <212> PRT
   <213> Boechera
<220>
   <221> misc_feature
   <222> (9) .. (9)
   <223> Xaa can be any naturally occurring amino acid
<220>
   <221> misc_feature
   <222> (30) .. (30)
   <223> Xaa can be any naturally occurring amino acid
<220>
   <221> misc_feature
   <222> (96) .. (96)
   <223> Xaa can be any naturally occurring amino acid
<220>
   <221> misc_feature
   <222> (101) .. (101)
   <223> Xaa can be any naturally occurring amino acid
<220>
   <221> misc_feature
   <222> (114) .. (114)
   <223> Xaa can be any naturally occurring amino acid
<220>
   <221> misc_feature
   <222> (119) .. (119)
   <223> Xaa can be any naturally occurring amino acid
<220>
   <221> misc_feature
   <222> (123) .. (123)
   <223> Xaa can be any naturally occurring amino acid
<220>
   <221> misc_feature
   <222> (137) .. (137)
   <223> Xaa can be any naturally occurring amino acid
<220>
   <221> misc_feature
   <222> (153) .. (153)
   <223> Xaa can be any naturally occurring amino acid
<220>
   <221> misc_feature
   <222> (158) .. (158)
   <223> Xaa can be any naturally occurring amino acid
<220>
   <221> misc_feature
   <222> (177) .. (177)
   <223> Xaa can be any naturally occurring amino acid
<220>
   <221> misc_feature
   <222> (179) .. (179)
   <223> Xaa can be any naturally occurring amino acid
<220>
   <221> misc_feature
   <222> (195) .. (197)
   <223> Xaa can be any naturally occurring amino acid
<220>
   <221> misc_feature
   <222> (200) .. (201)
   <223> Xaa can be any naturally occurring amino acid
<220>
   <221> misc_feature
   <222> (207) .. (207)
   <223> Xaa can be any naturally occurring amino acid
<220>
   <221> misc_feature
   <222> (217) .. (217)
   <223> Xaa can be any naturally occurring amino acid
<220>
   <221> misc_feature
   <222> (228) .. (228)
   <223> Xaa can be any naturally occurring amino acid
<220>
   <221> misc_feature
   <222> (231) .. (231)
   <223> Xaa can be any naturally occurring amino acid
<220>
   <221> misc_feature
   <222> (243) .. (243)
   <223> Xaa can be any naturally occurring amino acid
<220>
   <221> misc_feature
   <222> (258) .. (258)
   <223> Xaa can be any naturally occurring amino acid
<220>
   <221> misc_feature
   <222> (285) .. (285)
   <223> Xaa can be any naturally occurring amino acid
<220>
   <221> misc_feature
   <222> (303) .. (303)
   <223> Xaa can be any naturally occurring amino acid
<220>
   <221> misc_feature
   <222> (330) .. (330)
   <223> Xaa can be any naturally occurring amino acid
<220>
   <221> misc_feature
   <222> (341) .. (342)
   <223> Xaa can be any naturally occurring amino acid
<220>
   <221> misc_feature
   <222> (348) .. (348)
   <223> Xaa can be any naturally occurring amino acid
<220>
   <221> misc_feature
   <222> (352) .. (352)
   <223> Xaa can be any naturally occurring amino acid
<220>
   <221> misc_feature
   <222> (354) .. (354)
   <223> Xaa can be any naturally occurring amino acid
<220>
   <221> misc_feature
   <222> (387) .. (387)
   <223> Xaa can be any naturally occurring amino acid
<220>
   <221> misc_feature
   <222> (397) .. (397)
   <223> Xaa can be any naturally occurring amino acid
<220>
   <221> misc_feature
   <222> (411) .. (411)
   <223> Xaa can be any naturally occurring amino acid
<220>
   <221> misc_feature
   <222> (414) .. (414)
   <223> Xaa can be any naturally occurring amino acid
<220>
   <221> misc_feature
   <222> (420) .. (420)
   <223> Xaa can be any naturally occurring amino acid
<220>
   <221> misc_feature
   <222> (422) .. (422)
   <223> Xaa can be any naturally occurring amino acid
<220>
   <221> misc_feature
   <222> (425) .. (425)
   <223> Xaa can be any naturally occurring amino acid
<220>
   <221> misc_feature
   <222> (444) .. (444)
   <223> Xaa can be any naturally occurring amino acid
<220>
   <221> misc_feature
   <222> (478) .. (478)
   <223> Xaa can be any naturally occurring amino acid
<220>
   <221> misc_feature
   <222> (482) .. (482)
   <223> Xaa can be any naturally occurring amino acid
<220>
   <221> misc_feature
   <222> (484) .. (484)
   <223> Xaa can be any naturally occurring amino acid
<220>
   <221> misc_feature
   <222> (494) .. (494)
   <223> Xaa can be any naturally occurring amino acid
<400> 5
   Met Ala Ser Thr Leu Gly Gly Asp Xaa Arg Asn Glu Ile Val Phe Phe
   1               5                   10                  15
   Asp Leu Glu Thr Ala Val Pro Thr Lys Ser Gly Gln Pro Xaa Ala Ile
               20                  25                  30
   Leu Glu Phe Gly Ala Ile Leu Val Cys Pro Met Lys Leu Val Glu Leu
           35                  40                  45
   Tyr Ser Tyr Ser Thr Leu Val Arg Pro Thr Asp Leu Ser Leu Ile Ser
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Val Tyr Asp Ile Leu Xaa
                   85                  90                  95
   Gly Arg Ile Trp Xaa Gly His Asn Ile Lys Arg Phe Asp Cys Val Arg
               100                 105                 110
   Ile Xaa Asp Ala Phe Ala Xaa Ile Gly Leu Xaa Pro Pro Glu Pro Lys
           115                 120                 125
   Ala Thr Ile Asp Ser Leu Ser Leu Xaa Ser Gln Lys Phe Gly Lys Arg
       130                 135                 140
   Ala Gly Asp Met Lys Met Ala Ser Xaa Ala Thr Tyr Phe Xaa Leu Gly
   145                 150                 155                 160
   Asp Gln Ala His Arg Ser Leu Asp Asp Val Arg Met Asn Leu Glu Val
                   165                 170                 175
   Xaa Lys Xaa Cys Ser Thr Val Leu Phe Leu Glu Ser Ser Val Pro Asp
               180                 185                 190
   Ile Leu Xaa Xaa Xaa Ser Trp Xaa Xaa Pro Arg Lys Ser Pro Xaa Thr
   195 200 205
   Arg Ser Asn Glu Lys Ser Leu Pro Xaa Gly Val Arg Glu Ser Pro Thr
   210 215 220
   Ser Ser Ser Xaa Ser Pro Xaa Thr Asp Pro Ser Ser Ser Ser Val Asp
   225 230 235 240
   Ala Thr Xaa Val Lys Asn His Pro Ile Ile Ser Leu Leu Thr Glu Cys
   245 250 255
   Ser Xaa Ser Asp Thr Ser Ser Cys Glu Ile Asp Pro Ser Asp Ile Thr
   260 265 270
   Thr Leu Ile Ser Lys Leu His Ile Gly Thr Leu Lys Xaa Asp Ala Ala
   275 280 285
   Asp Glu Ala Lys Thr Val Arg Asp Ala Ala Asp Glu Ala Lys Xaa Val
   290 295 300
   Arg Gln Gln Gly Glu Ser Thr Asp Pro Asn Ala Lys Asp Glu Ser Phe
   305 310 315 320
   Leu Gly Val Asn Glu Val Ser Val Ser Xaa Ile Arg Ala Ser Leu Ile
   325 330 335
   Pro Leu Tyr Arg Xaa Xaa Leu Arg Met Glu Leu Xaa His Asn Asp Xaa
   340 345 350
   Pro Xaa His Leu Cys Trp Tyr Ser Leu Lys Ile Arg Phe Gly Ile Ser
   355 360 365
   Arg Lys Tyr Val Asp His Val Gly Arg Pro Lys Met Asn Ile Val Val
   370 375 380
   Asp Ile Xaa Pro Asp Leu Cys Lys Ile Leu Asp Ala Xaa Asp Ala Ala
   385 390 395 400
   Ala His Asn Leu Leu Ile Asp Ser Ser Thr Xaa Ser Asp Xaa Arg Pro
   405 410 415
   Thr Val Met Xaa Lys Xaa Gly Phe Xaa Asn Tyr Pro Thr Ala Arg Leu
   420 425 430
   Gln Ile Ser Ser Glu Ser Asn Gly Thr Gln Val Xaa Gln Lys Glu Glu
   435 440 445
   Pro Leu Gly Thr Asn Gln Lys Leu Asp Phe Ser Ser Asp Asn Phe Glu
       450                 455                 460
   Lys Leu Glu Ser Ala Leu Leu Pro Gly Thr Leu Val Asp Xaa Phe Phe
   465                 470                 475                 480
   Ser Xaa Glu Xaa Tyr Asp Tyr Lys Lys Met Val Gly Ile Xaa Leu Ala
                   485                 490                 495
   Ala Arg Lys Leu Val Ile Gln Leu Lys Lys
               500                 505
<210> 6
   <211> 506
   <212> PRT
   <213> Boechera
<220>
   <221> misc_feature
   <222> (7) .. (7)
   <223> Xaa can be any naturally occurring amino acid
<220>
   <221> misc_feature
   <222> (9) .. (9)
   <223> Xaa can be any naturally occurring amino acid
<220>
   <221> misc_feature
   <222> (11) .. (11)
   <223> Xaa can be any naturally occurring amino acid
<220>
   <221> misc_feature
   <222> (23) .. (23)
   <223> Xaa can be any naturally occurring amino acid
<220>
   <221> misc_feature
   <222> (25) .. (25)
   <223> Xaa can be any naturally occurring amino acid
<220>
   <221> misc_feature
   <222> (46) .. (46)
   <223> Xaa can be any naturally occurring amino acid
<220>
   <221> misc_feature
   <222> (52) .. (52)
   <223> Xaa can be any naturally occurring amino acid
<220>
   <221> misc_feature
   <222> (55) .. (55)
   <223> Xaa can be any naturally occurring amino acid
<220>
   <221> misc_feature
   <222> (64) .. (64)
   <223> Xaa can be any naturally occurring amino acid
<220>
   <221> misc_feature
   <222> (91) .. (91)
   <223> Xaa can be any naturally occurring amino acid
<220>
   <221> misc_feature
   <222> (95) .. (95)
   <223> Xaa can be any naturally occurring amino acid
<220>
   <221> misc_feature
   <222> (113) .. (114)
   <223> Xaa can be any naturally occurring amino acid
<220>
   <221> misc_feature
   <222> (119) .. (119)
   <223> Xaa can be any naturally occurring amino acid
<220>
   <221> misc_feature
   <222> (122) .. (123)
   <223> Xaa can be any naturally occurring amino acid
<220>
   <221> misc_feature
   <222> (125) .. (125)
   <223> Xaa can be any naturally occurring amino acid
<220>
   <221> misc_feature
   <222> (127) .. (127)
   <223> Xaa can be any naturally occurring amino acid
<220>
   <221> misc_feature
   <222> (129) .. (130)
   <223> Xaa can be any naturally occurring amino acid
<220>
   <221> misc_feature
   <222> (133) .. (133)
   <223> Xaa can be any naturally occurring amino acid
<220>
   <221> misc_feature
   <222> (136) .. (137)
   <223> Xaa can be any naturally occurring amino acid
<220>
   <221> misc_feature
   <222> (144) .. (144)
   <223> Xaa can be any naturally occurring amino acid
<220>
   <221> misc_feature
   <222> (148) .. (148)
   <223> Xaa can be any naturally occurring amino acid
<220>
   <221> misc_feature
   <222> (152) .. (153)
   <223> Xaa can be any naturally occurring amino acid
<220>
   <221> misc_feature
   <222> (158) .. (158)
   <223> Xaa can be any naturally occurring amino acid
<220>
   <221> misc_feature
   <222> (177) .. (177)
   <223> Xaa can be any naturally occurring amino acid
<220>
   <221> misc_feature
   <222> (179) .. (179)
   <223> Xaa can be any naturally occurring amino acid
<220>
   <221> misc_feature
   <222> (181) .. (181)
   <223> Xaa can be any naturally occurring amino acid
<220>
   <221> misc_feature
   <222> (183) .. (183)
   <223> Xaa can be any naturally occurring amino acid
<220>
   <221> misc_feature
   <222> (195) .. (196)
   <223> Xaa can be any naturally occurring amino acid
<220>
   <221> misc_feature
   <222> (200) .. (202)
   <223> Xaa can be any naturally occurring amino acid
<220>
   <221> misc_feature
   <222> (206) .. (206)
   <223> Xaa can be any naturally occurring amino acid
<220>
   <221> misc_feature
   <222> (232) .. (232)
   <223> Xaa can be any naturally occurring amino acid
<220>
   <221> misc_feature
   <222> (240) .. (240)
   <223> Xaa can be any naturally occurring amino acid
<220>
   <221> misc_feature
   <222> (243) .. (243)
   <223> Xaa can be any naturally occurring amino acid
<220>
   <221> misc_feature
   <222> (259) .. (259)
   <223> Xaa can be any naturally occurring amino acid
<220>
   <221> misc_feature
   <222> (264) .. (264)
   <223> Xaa can be any naturally occurring amino acid
<220>
   <221> misc_feature
   <222> (285) .. (285)
   <223> Xaa can be any naturally occurring amino acid
<220>
   <221> misc_feature
   <222> (299) .. (299)
   <223> Xaa can be any naturally occurring amino acid
<220>
   <221> misc_feature
   <222> (303) .. (303)
   <223> Xaa can be any naturally occurring amino acid
<220>
   <221> misc_feature
   <222> (321) .. (321)
   <223> Xaa can be any naturally occurring amino acid
<220>
   <221> misc_feature
   <222> (328) .. (328)
   <223> Xaa can be any naturally occurring amino acid
<220>
   <221> misc_feature
   <222> (331) .. (331)
   <223> Xaa can be any naturally occurring amino acid
<220>
   <221> misc_feature
   <222> (336) .. (336)
   <223> Xaa can be any naturally occurring amino acid
<220>
   <221> misc_feature
   <222> (341) .. (342)
   <223> Xaa can be any naturally occurring amino acid
<220>
   <221> misc_feature
   <222> (348) .. (348)
   <223> Xaa can be any naturally occurring amino acid
<220>
   <221> misc_feature
   <222> (351) .. (351)
   <223> Xaa can be any naturally occurring amino acid
<220>
   <221> misc_feature
   <222> (357) .. (357)
   <223> Xaa can be any naturally occurring amino acid
<220>
   <221> misc_feature
   <222> (360) .. (361)
   <223> Xaa can be any naturally occurring amino acid
<220>
   <221> misc_feature
   <222> (371) .. (371)
   <223> Xaa can be any naturally occurring amino acid
<220>
   <221> misc_feature
   <222> (397) .. (398)
   <223> Xaa can be any naturally occurring amino acid
<220>
   <221> misc_feature
   <222> (400) .. (400)
   <223> Xaa can be any naturally occurring amino acid
<220>
   <221> misc_feature
   <222> (411) .. (411)
   <223> Xaa can be any naturally occurring amino acid
<220>
   <221> misc_feature
   <222> (413) .. (414)
   <223> Xaa can be any naturally occurring amino acid
<220>
   <221> misc_feature
   <222> (420) .. (420)
   <223> Xaa can be any naturally occurring amino acid
<220>
   <221> misc_feature
   <222> (422) .. (422)
   <223> Xaa can be any naturally occurring amino acid
<220>
   <221> misc_feature
   <222> (431) .. (431)
   <223> Xaa can be any naturally occurring amino acid
<220>
   <221> misc_feature
   <222> (440) .. (440)
   <223> Xaa can be any naturally occurring amino acid
<220>
   <221> misc_feature
   <222> (442) .. (442)
   <223> Xaa can be any naturally occurring amino acid
<220>
   <221> misc_feature
   <222> (444) .. (444)
   <223> Xaa can be any naturally occurring amino acid
<220>
   <221> misc_feature
   <222> (448) .. (448)
   <223> Xaa can be any naturally occurring amino acid
<220>
   <221> misc_feature
   <222> (471) .. (471)
   <223> Xaa can be any naturally occurring amino acid
<220>
   <221> misc_feature
   <222> (474) .. (474)
   <223> Xaa can be any naturally occurring amino acid
<220>
   <221> misc_feature
   <222> (478) .. (478)
   <223> Xaa can be any naturally occurring amino acid
<220>
   <221> misc_feature
   <222> (482) .. (482)
   <223> Xaa can be any naturally occurring amino acid
<220>
   <221> misc_feature
   <222> (484) .. (484)
   <223> Xaa can be any naturally occurring amino acid
<220>
   <221> misc_feature
   <222> (488) .. (488)
   <223> Xaa can be any naturally occurring amino acid
<220>
   <221> misc_feature
   <222> (503) .. (503)
   <223> Xaa can be any naturally occurring amino acid
<400> 6
   Met Ala Ser Thr Leu Gly Xaa Asp Xaa Arg Xaa Glu Ile Val Phe Phe
   1               5                   10                  15
   Asp Leu Glu Thr Ala Val Xaa Thr Xaa Ser Gly Gln Pro Phe Ala Ile
               20                  25                  30
   Leu Glu Phe Gly Ala Ile Leu Val Cys Pro Met Lys Leu Xaa Glu Leu
           35                  40                  45
   Tyr Ser Tyr Xaa Thr Leu Xaa Arg Pro Thr Asp Leu Ser Leu Ile Xaa
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Xaa Tyr Asp Ile Xaa His
                   85                  90                  95
   Gly Arg Ile Trp Ala Gly His Asn Ile Lys Arg Phe Asp Cys Val Arg
   100 105 110
   Xaa Xaa Asp Ala Phe Ala Xaa Ile Gly Xaa Xaa Pro Xaa Glu Xaa Lys
   115 120 125
   Xaa Xaa Ile Asp Xaa Leu Ser Xaa Xaa Ser Gln Lys Phe Gly Lys Xaa
   130 135 140
   Ala Gly Asp Xaa Lys Met Ala Xaa Xaa Ala Thr Tyr Phe Xaa Leu Gly
   145 150 155 160
   Asp Gln Ala His Arg Ser Leu Asp Asp Val Arg Met Asn Leu Glu Val
   165 170 175
   Xaa Lys Xaa Cys Xaa Thr Xaa Leu Phe Leu Glu Ser Ser Val Pro Asp
   180 185 190
   Ile Leu Xaa Xaa Met Ser Trp Xaa Xaa Xaa Arg Lys Ser Xaa Arg Thr
   195 200 205
   Arg Ser Asn Glu Lys Ser Leu Pro Asn Gly Val Arg Glu Ser Pro Thr
   210 215 220
   Ser Ser Ser Ser Ser Pro Lys Xaa Asp Pro Ser Ser Ser Ser Val Xaa
   225 230 235 240
   Ala Thr Xaa Val Lys Asn His Pro Ile Ile Ser Leu Leu Thr Glu Cys
   245 250 255
   Ser Glu Xaa Asp Thr Ser Ser Xaa Glu Ile Asp Pro Ser Asp Ile Thr
   260 265 270
   Thr Leu Ile Ser Lys Leu His Ile Gly Thr Leu Lys Xaa Asp Ala Ala
   275 280 285
   Asp Glu Ala Lys Thr Val Arg Asp Ala Ala Xaa Glu Ala Lys Xaa Val
   290 295 300
   Arg Gln Gln Gly Glu Ser Thr Asp Pro Asn Ala Lys Asp Glu Ser Phe
   305 310 315 320
   Xaa Gly Val Asn Glu Val Ser Xaa Ser Ser Xaa Arg Ala Ser Leu Xaa
   325 330 335
   Pro Leu Tyr Arg Xaa Xaa Leu Arg Met Glu Leu Xaa His Asn Xaa Thr
   340 345 350
   Pro Leu His Leu Xaa Trp Tyr Xaa Xaa Lys Ile Arg Phe Gly Ile Ser
           355                 360                 365
   Arg Lys Xaa Val Asp His Val Gly Arg Pro Lys Met Asn Ile Val Val
       370                 375                 380
   Asp Ile Pro Pro Asp Leu Cys Lys Ile Leu Asp Ala Xaa Xaa Ala Xaa
   385                 390                 395                 400
   Ala His Asn Leu Leu Ile Asp Ser Ser Thr Xaa Ser Xaa Xaa Arg Pro
                   405                 410                 415
   Thr Val Met Xaa Lys Xaa Gly Phe Ala Asn Tyr Pro Thr Ala Xaa Leu
               420                 425                 430
   Gln Ile Ser Ser Glu Ser Asn Xaa Thr Xaa Val Xaa Gln Lys Glu Xaa
           435                 440                 445
   Pro Leu Gly Thr Asn Gln Lys Leu Asp Phe Ser Ser Asp Asn Phe Glu
       450                 455                 460
   Lys Leu Glu Ser Ala Leu Xaa Pro Gly Xaa Leu Val Asp Xaa Phe Phe
   465                 470                 475                 480
   Ser Xaa Glu Xaa Tyr Asp Tyr Xaa Lys Met Val Gly Ile Arg Leu Ala
                   485                 490                 495
   Ala Arg Lys Leu Val Ile Xaa Leu Lys Lys
               500                 505
<210> 7
   <211> 496
   <212> PRT
   <213> Boechera
<220>
   <221> misc_feature
   <222> (7) .. (7)
   <223> Xaa can be any naturally occurring amino acid
<220>
   <221> misc_feature
   <222> (9) .. (9)
   <223> Xaa can be any naturally occurring amino acid
<220>
   <221> misc_feature
   <222> (11) .. (11)
   <223> Xaa can be any naturally occurring amino acid
<220>
   <221> misc_feature
   <222> (23) .. (23)
   <223> Xaa can be any naturally occurring amino acid
<220>
   <221> misc_feature
   <222> (25) .. (25)
   <223> Xaa can be any naturally occurring amino acid
<220>
   <221> misc_feature
   <222> (30) .. (30)
   <223> Xaa can be any naturally occurring amino acid
<220>
   <221> misc_feature
   <222> (46) .. (46)
   <223> Xaa can be any naturally occurring amino acid
<220>
   <221> misc_feature
   <222> (52) .. (52)
   <223> Xaa can be any naturally occurring amino acid
<220>
   <221> misc_feature
   <222> (55) .. (55)
   <223> Xaa can be any naturally occurring amino acid
<220>
   <221> misc_feature
   <222> (64) .. (64)
   <223> Xaa can be any naturally occurring amino acid
<220>
   <221> misc_feature
   <222> (91) .. (91)
   <223> Xaa can be any naturally occurring amino acid
<220>
   <221> misc_feature
   <222> (95) .. (96)
   <223> Xaa can be any naturally occurring amino acid
<220>
   <221> misc_feature
   <222> (101) .. (101)
   <223> Xaa can be any naturally occurring amino acid
<220>
   <221> misc_feature
   <222> (113) .. (114)
   <223> Xaa can be any naturally occurring amino acid
<220>
   <221> misc_feature
   <222> (119) .. (119)
   <223> Xaa can be any naturally occurring amino acid
<220>
   <221> misc_feature
   <222> (122) .. (123)
   <223> Xaa can be any naturally occurring amino acid
<220>
   <221> misc_feature
   <222> (125) .. (125)
   <223> Xaa can be any naturally occurring amino acid
<220>
   <221> misc_feature
   <222> (127) .. (127)
   <223> Xaa can be any naturally occurring amino acid
<220>
   <221> misc_feature
   <222> (129) .. (130)
   <223> Xaa can be any naturally occurring amino acid
<220>
   <221> misc_feature
   <222> (133) .. (133)
   <223> Xaa can be any naturally occurring amino acid
<220>
   <221> misc_feature
   <222> (136) .. (137)
   <223> Xaa can be any naturally occurring amino acid
<220>
   <221> misc_feature
   <222> (144) .. (144)
   <223> Xaa can be any naturally occurring amino acid
<220>
   <221> misc_feature
   <222> (148) .. (148)
   <223> Xaa can be any naturally occurring amino acid
<220>
   <221> misc_feature
   <222> (152) .. (153)
   <223> Xaa can be any naturally occurring amino acid
<220>
   <221> misc_feature
   <222> (158) .. (158)
   <223> Xaa can be any naturally occurring amino acid
<220>
   <221> misc_feature
   <222> (177) .. (177)
   <223> Xaa can be any naturally occurring amino acid
<220>
   <221> misc_feature
   <222> (179) .. (179)
   <223> Xaa can be any naturally occurring amino acid
<220>
   <221> misc_feature
   <222> (181) .. (181)
   <223> Xaa can be any naturally occurring amino acid
<220>
   <221> misc_feature
   <222> (183) .. (183)
   <223> Xaa can be any naturally occurring amino acid
<220>
   <221> misc_feature
   <222> (195) .. (197)
   <223> Xaa can be any naturally occurring amino acid
<220>
   <221> misc_feature
   <222> (200) .. (202)
   <223> Xaa can be any naturally occurring amino acid
<220>
   <221> misc_feature
   <222> (206) .. (207)
   <223> Xaa can be any naturally occurring amino acid
<220>
   <221> misc_feature
   <222> (217) .. (217)
   <223> Xaa can be any naturally occurring amino acid
<220>
   <221> misc_feature
   <222> (228) .. (228)
   <223> Xaa can be any naturally occurring amino acid
<220>
   <221> misc_feature
   <222> (231) .. (232)
   <223> Xaa can be any naturally occurring amino acid
<220>
   <221> misc_feature
   <222> (240) .. (240)
   <223> Xaa can be any naturally occurring amino acid
<220>
   <221> misc_feature
   <222> (243) .. (243)
   <223> Xaa can be any naturally occurring amino acid
<220>
   <221> misc_feature
   <222> (258) .. (259)
   <223> Xaa can be any naturally occurring amino acid
<220>
   <221> misc_feature
   <222> (264) .. (264)
   <223> Xaa can be any naturally occurring amino acid
<220>
   <221> misc_feature
   <222> (285) .. (285)
   <223> Xaa can be any naturally occurring amino acid
<220>
   <221> misc_feature
   <222> (289) .. (289)
   <223> Xaa can be any naturally occurring amino acid
<220>
   <221> misc_feature
   <222> (293) .. (293)
   <223> Xaa can be any naturally occurring amino acid
<220>
   <221> misc_feature
   <222> (311) .. (311)
   <223> Xaa can be any naturally occurring amino acid
<220>
   <221> misc_feature
   <222> (318) .. (318)
   <223> Xaa can be any naturally occurring amino acid
<220>
   <221> misc_feature
   <222> (320) .. (321)
   <223> Xaa can be any naturally occurring amino acid
<220>
   <221> misc_feature
   <222> (326) .. (326)
   <223> Xaa can be any naturally occurring amino acid
<220>
   <221> misc_feature
   <222> (331) .. (332)
   <223> Xaa can be any naturally occurring amino acid
<220>
   <221> misc_feature
   <222> (338) .. (338)
   <223> Xaa can be any naturally occurring amino acid
<220>
   <221> misc_feature
   <222> (341) .. (342)
   <223> Xaa can be any naturally occurring amino acid
<220>
   <221> misc_feature
   <222> (344) .. (344)
   <223> Xaa can be any naturally occurring amino acid
<220>
   <221> misc_feature
   <222> (347) .. (347)
   <223> Xaa can be any naturally occurring amino acid
<220>
   <221> misc_feature
   <222> (350) .. (351)
   <223> Xaa can be any naturally occurring amino acid
<220>
   <221> misc_feature
   <222> (361) .. (361)
   <223> Xaa can be any naturally occurring amino acid
<220>
   <221> misc_feature
   <222> (377) .. (377)
   <223> Xaa can be any naturally occurring amino acid
<220>
   <221> misc_feature
   <222> (387) .. (388)
   <223> Xaa can be any naturally occurring amino acid
<220>
   <221> misc_feature
   <222> (390) .. (390)
   <223> Xaa can be any naturally occurring amino acid
<220>
   <221> misc_feature
   <222> (401) .. (401)
   <223> Xaa can be any naturally occurring amino acid
<220>
   <221> misc_feature
   <222> (403) .. (404)
   <223> Xaa can be any naturally occurring amino acid
<220>
   <221> misc_feature
   <222> (410) .. (410)
   <223> Xaa can be any naturally occurring amino acid
<220>
   <221> misc_feature
   <222> (412) .. (412)
   <223> Xaa can be any naturally occurring amino acid
<220>
   <221> misc_feature
   <222> (415) .. (415)
   <223> Xaa can be any naturally occurring amino acid
<220>
   <221> misc_feature
   <222> (421) .. (421)
   <223> Xaa can be any naturally occurring amino acid
<220>
   <221> misc_feature
   <222> (430) .. (430)
   <223> Xaa can be any naturally occurring amino acid
<220>
   <221> misc_feature
   <222> (432) .. (432)
   <223> Xaa can be any naturally occurring amino acid
<220>
   <221> misc_feature
   <222> (434) .. (434)
   <223> Xaa can be any naturally occurring amino acid
<220>
   <221> misc_feature
   <222> (438) .. (438)
   <223> Xaa can be any naturally occurring amino acid
<220>
   <221> misc_feature
   <222> (461) .. (461)
   <223> Xaa can be any naturally occurring amino acid
<220>
   <221> misc_feature
   <222> (464) .. (464)
   <223> Xaa can be any naturally occurring amino acid
<220>
   <221> misc_feature
   <222> (468) .. (468)
   <223> Xaa can be any naturally occurring amino acid
<220>
   <221> misc_feature
   <222> (472) .. (472)
   <223> Xaa can be any naturally occurring amino acid
<220>
   <221> misc_feature
   <222> (474) .. (474)
   <223> Xaa can be any naturally occurring amino acid
<220>
   <221> misc_feature
   <222> (478) .. (478)
   <223> Xaa can be any naturally occurring amino acid
<220>
   <221> misc_feature
   <222> (484) .. (484)
   <223> Xaa can be any naturally occurring amino acid
<220>
   <221> misc_feature
   <222> (493) .. (493)
   <223> Xaa can be any naturally occurring amino acid
<400> 7
   Met Ala Ser Thr Leu Gly Xaa Asp Xaa Arg Xaa Glu Ile Val Phe Phe
   1               5                   10                  15
   Asp Leu Glu Thr Ala Val Xaa Thr Xaa Ser Gly Gln Pro Xaa Ala Ile
               20                  25                  30
   Leu Glu Phe Gly Ala Ile Leu Val Cys Pro Met Lys Leu Xaa Glu Leu
           35                  40                  45
   Tyr Ser Tyr Xaa Thr Leu Xaa Arg Pro Thr Asp Leu Ser Leu Ile Xaa
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Xaa Tyr Asp Ile Xaa Xaa
                   85                  90                  95
   Gly Arg Ile Trp Xaa Gly His Asn Ile Lys Arg Phe Asp Cys Val Arg
               100                 105                 110
   Xaa Xaa Asp Ala Phe Ala Xaa Ile Gly Xaa Xaa Pro Xaa Glu Xaa Lys
           115                 120                 125
   Xaa Xaa Ile Asp Xaa Leu Ser Xaa Xaa Ser Gln Lys Phe Gly Lys Xaa
       130                 135                 140
   Ala Gly Asp Xaa Lys Met Ala Xaa Xaa Ala Thr Tyr Phe Xaa Leu Gly
   145                 150                 155                 160
   Asp Gln Ala His Arg Ser Leu Asp Asp Val Arg Met Asn Leu Glu Val
                   165                 170                 175
   Xaa Lys Xaa Cys Xaa Thr Xaa Leu Phe Leu Glu Ser Ser Val Pro Asp
               180                 185                 190
   Ile Leu Xaa Xaa Xaa Ser Trp Xaa Xaa Xaa Arg Lys Ser Xaa Xaa Thr
           195                 200                 205
   Arg Ser Asn Glu Lys Ser Leu Pro Xaa Gly Val Arg Glu Ser Pro Thr
       210                 215                 220
   Ser Ser Ser Xaa Ser Pro Xaa Xaa Asp Pro Ser Ser Ser Ser Val Xaa
   225                 230                 235                 240
   Ala Thr Xaa Val Lys Asn His Pro Ile Ile Ser Leu Leu Thr Glu Cys
                   245                 250                 255
   Ser Xaa Xaa Asp Thr Ser Ser Xaa Glu Ile Asp Pro Ser Asp Ile Thr
               260                 265                 270
   Thr Leu Ile Ser Lys Leu His Ile Gly Thr Leu Lys Xaa Asp Ala Ala
           275                 280                 285
   Xaa Glu Ala Lys Xaa Val Arg Gln Gln Gly Glu Ser Thr Asp Pro Asn
       290                 295                 300
   Ala Lys Asp Glu Ser Phe Xaa Gly Val Asn Glu Val Ser Xaa Ser Xaa
   305                 310                 315                 320
   Xaa Arg Ala Ser Leu Xaa Pro Leu Tyr Arg Xaa Xaa Leu Arg Met Glu
                   325                 330                 335
   Leu Xaa His Asn Xaa Xaa Pro Xaa His Leu Xaa Trp Tyr Xaa Xaa Lys
   340 345 350
   Ile Arg Phe Gly Ile Ser Arg Lys Xaa Val Asp His Val Gly Arg Pro
   355 360 365
   Lys Met Asn Ile Val Val Asp Ile Xaa Pro Asp Leu Cys Lys Ile Leu
   370 375 380
   Asp Ala Xaa Xaa Ala Xaa Ala His Asn Leu Leu Ile Asp Ser Ser Thr
   385 390 395 400
   Xaa Ser Xaa Xaa Arg Pro Thr Val Met Xaa Lys Xaa Gly Phe Xaa Asn
   405 410 415
   Tyr Pro Thr Ala Xaa Leu Gln Ile Ser Ser Glu Ser Asn Xaa Thr Xaa
   420 425 430
   Val Xaa Gln Lys Glu Xaa Pro Leu Gly Thr Asn Gln Lys Leu Asp Phe
   435 440 445
   Ser Ser Asp Asn Phe Glu Lys Leu Glu Ser Ala Leu Xaa Pro Gly Xaa
   450 455 460
   Leu Val Asp Xaa Phe Phe Ser Xaa Glu Xaa Tyr Asp Tyr Xaa Lys Met
   465 470 475 480
   Val Gly Ile Xaa Leu Ala Ala Arg Lys Leu Val Ile Xaa Leu Lys Lys
   485 490 495
<210> 8
   <211> 496
   <212> PRT
   <213> Boechera
<220>
   <221> misc_feature
   <222> (9) .. (9)
   <223> Xaa can be any naturally occurring amino acid
<220>
   <221> misc_feature
   <222> (30) .. (30)
   <223> Xaa can be any naturally occurring amino acid
<220>
   <221> misc_feature
   <222> (96) .. (96)
   <223> Xaa can be any naturally occurring amino acid
<220>
   <221> misc_feature
   <222> (101) .. (101)
   <223> Xaa can be any naturally occurring amino acid
<220>
   <221> misc_feature
   <222> (114) .. (114)
   <223> Xaa can be any naturally occurring amino acid
<220>
   <221> misc_feature
   <222> (119) .. (119)
   <223> Xaa can be any naturally occurring amino acid
<220>
   <221> misc_feature
   <222> (123) .. (123)
   <223> Xaa can be any naturally occurring amino acid
<220>
   <221> misc_feature
   <222> (137) .. (137)
   <223> Xaa can be any naturally occurring amino acid
<220>
   <221> misc_feature
   <222> (153) .. (153)
   <223> Xaa can be any naturally occurring amino acid
<220>
   <221> misc_feature
   <222> (158) .. (158)
   <223> Xaa can be any naturally occurring amino acid
<220>
   <221> misc_feature
   <222> (177) .. (177)
   <223> Xaa can be any naturally occurring amino acid
<220>
   <221> misc_feature
   <222> (179) .. (179)
   <223> Xaa can be any naturally occurring amino acid
<220>
   <221> misc_feature
   <222> (195) .. (197)
   <223> Xaa can be any naturally occurring amino acid
<220>
   <221> misc_feature
   <222> (200) .. (201)
   <223> Xaa can be any naturally occurring amino acid
<220>
   <221> misc_feature
   <222> (207) .. (207)
   <223> Xaa can be any naturally occurring amino acid
<220>
   <221> misc_feature
   <222> (217) .. (217)
   <223> Xaa can be any naturally occurring amino acid
<220>
   <221> misc_feature
   <222> (228) .. (228)
   <223> Xaa can be any naturally occurring amino acid
<220>
   <221> misc_feature
   <222> (231) .. (231)
   <223> Xaa can be any naturally occurring amino acid
<220>
   <221> misc_feature
   <222> (243) .. (243)
   <223> Xaa can be any naturally occurring amino acid
<220>
   <221> misc_feature
   <222> (258) .. (258)
   <223> Xaa can be any naturally occurring amino acid
<220>
   <221> misc_feature
   <222> (285) .. (285)
   <223> Xaa can be any naturally occurring amino acid
<220>
   <221> misc_feature
   <222> (293) .. (293)
   <223> Xaa can be any naturally occurring amino acid
<220>
   <221> misc_feature
   <222> (320) .. (320)
   <223> Xaa can be any naturally occurring amino acid
<220>
   <221> misc_feature
   <222> (331) .. (332)
   <223> Xaa can be any naturally occurring amino acid
<220>
   <221> misc_feature
   <222> (338) .. (338)
   <223> Xaa can be any naturally occurring amino acid
<220>
   <221> misc_feature
   <222> (342) .. (342)
   <223> Xaa can be any naturally occurring amino acid
<220>
   <221> misc_feature
   <222> (344) .. (344)
   <223> Xaa can be any naturally occurring amino acid
<220>
   <221> misc_feature
   <222> (377) .. (377)
   <223> Xaa can be any naturally occurring amino acid
<220>
   <221> misc_feature
   <222> (387) .. (387)
   <223> Xaa can be any naturally occurring amino acid
<220>
   <221> misc_feature
   <222> (401) .. (401)
   <223> Xaa can be any naturally occurring amino acid
<220>
   <221> misc_feature
   <222> (404) .. (404)
   <223> Xaa can be any naturally occurring amino acid
<220>
   <221> misc_feature
   <222> (410) .. (410)
   <223> Xaa can be any naturally occurring amino acid
<220>
   <221> misc_feature
   <222> (412) .. (412)
   <223> Xaa can be any naturally occurring amino acid
<220>
   <221> misc_feature
   <222> (415) .. (415)
   <223> Xaa can be any naturally occurring amino acid
<220>
   <221> misc_feature
   <222> (434) .. (434)
   <223> Xaa can be any naturally occurring amino acid
<220>
   <221> misc_feature
   <222> (468) .. (468)
   <223> Xaa can be any naturally occurring amino acid
<220>
   <221> misc_feature
   <222> (472) .. (472)
   <223> Xaa can be any naturally occurring amino acid
<220>
   <221> misc_feature
   <222> (474) .. (474)
   <223> Xaa can be any naturally occurring amino acid
<220>
   <221> misc_feature
   <222> (484) .. (484)
   <223> Xaa can be any naturally occurring amino acid
<400> 8
   Met Ala Ser Thr Leu Gly Gly Asp Xaa Arg Asn Glu Ile Val Phe Phe
   1               5                   10                  15
   Asp Leu Glu Thr Ala Val Pro Thr Lys Ser Gly Gln Pro Xaa Ala Ile
               20                  25                  30
   Leu Glu Phe Gly Ala Ile Leu Val Cys Pro Met Lys Leu Val Glu Leu
           35                  40                  45
   Tyr Ser Tyr Ser Thr Leu Val Arg Pro Thr Asp Leu Ser Leu Ile Ser
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Val Tyr Asp Ile Leu Xaa
                   85                  90                  95
   Gly Arg Ile Trp Xaa Gly His Asn Ile Lys Arg Phe Asp Cys Val Arg
               100                 105                 110
   Ile Xaa Asp Ala Phe Ala Xaa Ile Gly Leu Xaa Pro Pro Glu Pro Lys
           115                 120                 125
   Ala Thr Ile Asp Ser Leu Ser Leu Xaa Ser Gln Lys Phe Gly Lys Arg
       130                 135                 140
   Ala Gly Asp Met Lys Met Ala Ser Xaa Ala Thr Tyr Phe Xaa Leu Gly
   145                 150                 155                 160
   Asp Gln Ala His Arg Ser Leu Asp Asp Val Arg Met Asn Leu Glu Val
                   165                 170                 175
   Xaa Lys Xaa Cys Ser Thr Val Leu Phe Leu Glu Ser Ser Val Pro Asp
               180                 185                 190
   Ile Leu Xaa Xaa Xaa Ser Trp Xaa Xaa Pro Arg Lys Ser Pro Xaa Thr
           195                 200                 205
   Arg Ser Asn Glu Lys Ser Leu Pro Xaa Gly Val Arg Glu Ser Pro Thr
       210                 215                 220
   Ser Ser Ser Xaa Ser Pro Xaa Thr Asp Pro Ser Ser Ser Ser Val Asp
   225                 230                 235                 240
   Ala Thr Xaa Val Lys Asn His Pro Ile Ile Ser Leu Leu Thr Glu Cys
                   245                 250                 255
   Ser Xaa Ser Asp Thr Ser Ser Cys Glu Ile Asp Pro Ser Asp Ile Thr
               260                 265                 270
   Thr Leu Ile Ser Lys Leu His Ile Gly Thr Leu Lys Xaa Asp Ala Ala
           275                 280                 285
   Asp Glu Ala Lys Xaa Val Arg Gln Gln Gly Glu Ser Thr Asp Pro Asn
               290                 295                 300
   Ala Lys Asp Glu Ser Phe Leu Gly Val Asn Glu Val Ser Val Ser Xaa
   305                 310                 315                 320
   Ile Arg Ala Ser Leu Ile Pro Leu Tyr Arg Xaa Xaa Leu Arg Met Glu
                   325                 330                 335
   Leu Xaa His Asn Asp Xaa Pro Xaa His Leu Cys Trp Tyr Ser Leu Lys
               340                 345                 350
   Ile Arg Phe Gly Ile Ser Arg Lys Tyr Val Asp His Val Gly Arg Pro
           355                 360                 365
   Lys Met Asn Ile Val Val Asp Ile Xaa Pro Asp Leu Cys Lys Ile Leu
       370                 375                 380
   Asp Ala Xaa Asp Ala Ala Ala His Asn Leu Leu Ile Asp Ser Ser Thr
   385                 390                 395                 400
   Xaa Ser Asp Xaa Arg Pro Thr Val Met Xaa Lys Xaa Gly Phe Xaa Asn
                   405                 410                 415
   Tyr Pro Thr Ala Arg Leu Gln Ile Ser Ser Glu Ser Asn Gly Thr Gln
               420                 425                 430
   Val Xaa Gln Lys Glu Glu Pro Leu Gly Thr Asn Gln Lys Leu Asp Phe
           435                 440                 445
   Ser Ser Asp Asn Phe Glu Lys Leu Glu Ser Ala Leu Leu Pro Gly Thr
       450                 455                 460
   Leu Val Asp Xaa Phe Phe Ser Xaa Glu Xaa Tyr Asp Tyr Lys Lys Met
   465                 470                 475                 480
   Val Gly Ile Xaa Leu Ala Ala Arg Lys Leu Val Ile Gln Leu Lys Lys
                   485                 490                 495
<210> 9
   <211> 496
   <212> PRT
   <213> Boechera
<220>
   <221> misc_feature
   <222> (7) .. (7)
   <223> Xaa can be any naturally occurring amino acid
<220>
   <221> misc_feature
   <222> (9) .. (9)
   <223> Xaa can be any naturally occurring amino acid
<220>
   <221> misc_feature
   <222> (11)..(11)
   <223> Xaa can be any naturally occurring amino acid
<220>
   <221> misc_feature
   <222> (23) .. (23)
   <223> Xaa can be any naturally occurring amino acid
<220>
   <221> misc_feature
   <222> (25) .. (25)
   <223> Xaa can be any naturally occurring amino acid
<220>
   <221> misc_feature
   <222> (46)..(46)
   <223> Xaa can be any naturally occurring amino acid
<220>
   <221> misc_feature
   <222> (52)..(52)
   <223> Xaa can be any naturally occurring amino acid
<220>
   <221> misc_feature
   <222> (55)..(55)
   <223> Xaa can be any naturally occurring amino acid
<220>
   <221> misc_feature
   <222> (64)..(64)
   <223> Xaa can be any naturally occurring amino acid
<220>
   <221> misc_feature
   <222> (91)..(91)
   <223> Xaa can be any naturally occurring amino acid
<220>
   <221> misc_feature
   <222> (95)..(95)
   <223> Xaa can be any naturally occurring amino acid
<220>
   <221> misc_feature
   <222> (113)..(114)
   <223> Xaa can be any naturally occurring amino acid
<220>
   <221> misc_feature
   <222> (119)..(119)
   <223> Xaa can be any naturally occurring amino acid
<220>
   <221> misc_feature
   <222> (122)..(123)
   <223> Xaa can be any naturally occurring amino acid
<220>
   <221> misc_feature
   <222> (125)..(125)
   <223> Xaa can be any naturally occurring amino acid
<220>
   <221> misc_feature
   <222> (127)..(127)
   <223> Xaa can be any naturally occurring amino acid
<220>
   <221> misc_feature
   <222> (129)..(130)
   <223> Xaa can be any naturally occurring amino acid
<220>
   <221> misc_feature
   <222> (133)..(133)
   <223> Xaa can be any naturally occurring amino acid
<220>
   <221> misc_feature
   <222> (136)..(137)
   <223> Xaa can be any naturally occurring amino acid
<220>
   <221> misc_feature
   <222> (144)..(144)
   <223> Xaa can be any naturally occurring amino acid
<220>
   <221> misc_feature
   <222> (148)..(148)
   <223> Xaa can be any naturally occurring amino acid
<220>
   <221> misc_feature
   <222> (152)..(153)
   <223> Xaa can be any naturally occurring amino acid
<220>
   <221> misc_feature
   <222> (158)..(158)
   <223> Xaa can be any naturally occurring amino acid
<220>
   <221> misc_feature
   <222> (177)..(177)
   <223> Xaa can be any naturally occurring amino acid
<220>
   <221> misc_feature
   <222> (179)..(179)
   <223> Xaa can be any naturally occurring amino acid
<220>
   <221> misc_feature
   <222> (181)..(181)
   <223> Xaa can be any naturally occurring amino acid
<220>
   <221> misc_feature
   <222> (183)..(183)
   <223> Xaa can be any naturally occurring amino acid
<220>
   <221> misc_feature
   <222> (195)..(196)
   <223> Xaa can be any naturally occurring amino acid
<220>
   <221> misc_feature
   <222> (200)..(202)
   <223> Xaa can be any naturally occurring amino acid
<220>
   <221> misc_feature
   <222> (206)..(206)
   <223> Xaa can be any naturally occurring amino acid
<220>
   <221> misc_feature
   <222> (232)..(232)
   <223> Xaa can be any naturally occurring amino acid
<220>
   <221> misc_feature
   <222> (240)..(240)
   <223> Xaa can be any naturally occurring amino acid
<220>
   <221> misc_feature
   <222> (243)..(243)
   <223> Xaa can be any naturally occurring amino acid
<220>
   <221> misc_feature
   <222> (259) .. (259)
   <223> Xaa can be any naturally occurring amino acid
<220>
   <221> misc_feature
   <222> (264)..(264)
   <223> Xaa can be any naturally occurring amino acid
<220>
   <221> misc_feature
   <222> (285)..(285)
   <223> Xaa can be any naturally occurring amino acid
<220>
   <221> misc_feature
   <222> (289) .. (289)
   <223> Xaa can be any naturally occurring amino acid
<220>
   <221> misc_feature
   <222> (293)..(293)
   <223> Xaa can be any naturally occurring amino acid
<220>
   <221> misc_feature
   <222> (311)..(311)
   <223> Xaa can be any naturally occurring amino acid
<220>
   <221> misc_feature
   <222> (318)..(318)
   <223> Xaa can be any naturally occurring amino acid
<220>
   <221> misc_feature
   <222> (321)..(321)
   <223> Xaa can be any naturally occurring amino acid
<220>
   <221> misc_feature
   <222> (326)..(326)
   <223> Xaa can be any naturally occurring amino acid
<220>
   <221> misc_feature
   <222> (331)..(332)
   <223> Xaa can be any naturally occurring amino acid
<220>
   <221> misc_feature
   <222> (338)..(338)
   <223> Xaa can be any naturally occurring amino acid
<220>
   <221> misc_feature
   <222> (341)..(341)
   <223> Xaa can be any naturally occurring amino acid
<220>
   <221> misc_feature
   <222> (347)..(347)
   <223> Xaa can be any naturally occurring amino acid
<220>
   <221> misc_feature
   <222> (350)..(351)
   <223> Xaa can be any naturally occurring amino acid
<220>
   <221> misc_feature
   <222> (361)..(361)
   <223> Xaa can be any naturally occurring amino acid
<220>
   <221> misc_feature
   <222> (387)..(388)
   <223> Xaa can be any naturally occurring amino acid
<220>
   <221> misc_feature
   <222> (390)..(390)
   <223> Xaa can be any naturally occurring amino acid
<220>
   <221> misc_feature
   <222> (401)..(401)
   <223> Xaa can be any naturally occurring amino acid
<220>
   <221> misc_feature
   <222> (403)..(404)
   <223> Xaa can be any naturally occurring amino acid
<220>
   <221> misc_feature
   <222> (410)..(410)
   <223> Xaa can be any naturally occurring amino acid
<220>
   <221> misc_feature
   <222> (412)..(412)
   <223> Xaa can be any naturally occurring amino acid
<220>
   <221> misc_feature
   <222> (421)..(421)
   <223> Xaa can be any naturally occurring amino acid
<220>
   <221> misc_feature
   <222> (430)..(430)
   <223> Xaa can be any naturally occurring amino acid
<220>
   <221> misc_feature
   <222> (432)..(432)
   <223> Xaa can be any naturally occurring amino acid
<220>
   <221> misc_feature
   <222> (434)..(434)
   <223> Xaa can be any naturally occurring amino acid
<220>
   <221> misc_feature
   <222> (438)..(438)
   <223> Xaa can be any naturally occurring amino acid
<220>
   <221> misc_feature
   <222> (461)..(461)
   <223> Xaa can be any naturally occurring amino acid
<220>
   <221> misc_feature
   <222> (464)..(464)
   <223> Xaa can be any naturally occurring amino acid
<220>
   <221> misc_feature
   <222> (468)..(468)
   <223> Xaa can be any naturally occurring amino acid
<220>
   <221> misc_feature
   <222> (472)..(472)
   <223> Xaa can be any naturally occurring amino acid
<220>
   <221> misc_feature
   <222> (474)..(474)
   <223> Xaa can be any naturally occurring amino acid
<220>
   <221> misc_feature
   <222> (478)..(478)
   <223> Xaa can be any naturally occurring amino acid
<220>
   <221> misc_feature
   <222> (493)..(493)
   <223> Xaa can be any naturally occurring amino acid
<400> 9
   Met Ala Ser Thr Leu Gly Xaa Asp Xaa Arg Xaa Glu Ile Val Phe Phe
   1               5                   10                  15
   Asp Leu Glu Thr Ala Val Xaa Thr Xaa Ser Gly Gln Pro Phe Ala Ile
               20                  25                  30
   Leu Glu Phe Gly Ala Ile Leu Val Cys Pro Met Lys Leu Xaa Glu Leu
           35                  40                  45
   Tyr Ser Tyr Xaa Thr Leu Xaa Arg Pro Thr Asp Leu Ser Leu Ile Xaa
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Xaa Tyr Asp Ile Xaa His
                   85                  90                  95
   Gly Arg Ile Trp Ala Gly His Asn Ile Lys Arg Phe Asp Cys Val Arg
               100                 105                 110
   Xaa Xaa Asp Ala Phe Ala Xaa Ile Gly Xaa Xaa Pro Xaa Glu Xaa Lys
           115                 120                 125
   Xaa Xaa Ile Asp Xaa Leu Ser Xaa Xaa Ser Gln Lys Phe Gly Lys Xaa
       130                 135                 140
   Ala Gly Asp Xaa Lys Met Ala Xaa Xaa Ala Thr Tyr Phe Xaa Leu Gly
   145                 150                 155                 160
   Asp Gln Ala His Arg Ser Leu Asp Asp Val Arg Met Asn Leu Glu Val
                   165                 170                 175
   Xaa Lys Xaa Cys Xaa Thr Xaa Leu Phe Leu Glu Ser Ser Val Pro Asp
               180                 185                 190
   Ile Leu Xaa Xaa Met Ser Trp Xaa Xaa Xaa Arg Lys Ser Xaa Arg Thr
           195                 200                 205
   Arg Ser Asn Glu Lys Ser Leu Pro Asn Gly Val Arg Glu Ser Pro Thr
       210                 215                 220
   Ser Ser Ser Ser Ser Pro Lys Xaa Asp Pro Ser Ser Ser Ser Val Xaa
   225                 230                 235                 240
   Ala Thr Xaa Val Lys Asn His Pro Ile Ile Ser Leu Leu Thr Glu Cys
                   245                 250                 255
   Ser Glu Xaa Asp Thr Ser Ser Xaa Glu Ile Asp Pro Ser Asp Ile Thr
               260                 265                 270
   Thr Leu Ile Ser Lys Leu His Ile Gly Thr Leu Lys Xaa Asp Ala Ala
           275                 280                 285
   Xaa Glu Ala Lys Xaa Val Arg Gln Gln Gly Glu Ser Thr Asp Pro Asn
       290                 295                 300
   Ala Lys Asp Glu Ser Phe Xaa Gly Val Asn Glu Val Ser Xaa Ser Ser
   305                 310                 315                 320
   Xaa Arg Ala Ser Leu Xaa Pro Leu Tyr Arg Xaa Xaa Leu Arg Met Glu
                   325                 330                 335
   Leu Xaa His Asn Xaa Thr Pro Leu His Leu Xaa Trp Tyr Xaa Xaa Lys
               340                 345                 350
   Ile Arg Phe Gly Ile Ser Arg Lys Xaa Val Asp His Val Gly Arg Pro
           355                 360                 365
   Lys Met Asn Ile Val Val Asp Ile Pro Pro Asp Leu Cys Lys Ile Leu
       370                 375                 380
   Asp Ala Xaa Xaa Ala Xaa Ala His Asn Leu Leu Ile Asp Ser Ser Thr
   385                 390                 395                 400
   Xaa Ser Xaa Xaa Arg Pro Thr Val Met Xaa Lys Xaa Gly Phe Ala Asn
                   405                 410                 415
   Tyr Pro Thr Ala Xaa Leu Gln Ile Ser Ser Glu Ser Asn Xaa Thr Xaa
               420                 425                 430
   Val Xaa Gln Lys Glu Xaa Pro Leu Gly Thr Asn Gln Lys Leu Asp Phe
           435                 440                 445
   Ser Ser Asp Asn Phe Glu Lys Leu Glu Ser Ala Leu Xaa Pro Gly Xaa
       450                 455                 460
   Leu Val Asp Xaa Phe Phe Ser Xaa Glu Xaa Tyr Asp Tyr Xaa Lys Met
   465                 470                 475                 480
   Val Gly Ile Arg Leu Ala Ala Arg Lys Leu Val Ile Xaa Leu Lys Lys
                   485                 490                 495
<210> 10
   <211> 165
   <212> PRT
   <213> Boechera
<400> 10
   Val Phe Phe Asp Leu Glu Thr Ala Val Pro Thr Lys Ser Gly Gln Pro
   1               5                   10                  15
   Phe Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
           35                  40                  45
   Leu Ile Ser Thr Leu Thr Lys Arg Arg Ser Gly Ile Thr Arg Asp Gly
       50                  55                  60
   Val Leu Ser Ala Pro Thr Phe Ser Glu Ile Ala Asp Glu Val Tyr Asp
   65                  70                  75                  80
   Ile Leu His Gly Arg Ile Trp Val Gly His Asn Ile Lys Arg Phe Asp
                   85                  90                  95
   Cys Val Arg Ile Arg Asp Ala Phe Ala Glu Ile Gly Leu Pro Pro Pro
               100                 105                 110
   Glu Pro Lys Ala Thr Ile Asp Ser Leu Ser Leu Leu Ser Gln Lys Phe
           115                 120                 125
   Gly Lys Arg Ala Gly Asp Met Lys Met Ala Ser His Ala Thr Tyr Phe
       130                 135                 140
   Gly Leu Gly Asp Gln Ala His Arg Ser Leu Asp Asp Val Arg Met Asn
   145                 150                 155                 160
   Leu Glu Val Ile Lys
                   165
<210> 11
   <211> 165
   <212> PRT
   <213> Boechera
<400> 11
   Val Phe Phe Asp Leu Glu Thr Ala Val Pro Thr Lys Ser Gly Gln Pro
   1               5                   10                  15
   Phe Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
           35                  40                  45
   Leu Ile Ser Thr Leu Thr Lys Arg Arg Ser Gly Ile Thr Arg Asp Gly
       50                  55                  60
   Val Leu Ser Ala Pro Thr Phe Ser Glu Ile Ala Asp Glu Val Tyr Asp
   65                  70                  75                  80
   Ile Leu His Gly Arg Ile Trp Ala Gly His Asn Ile Lys Arg Phe Asp
                   85                  90                  95
   Cys Val Arg Ile Arg Asp Ala Phe Ala Glu Ile Gly Leu Pro Pro Pro
               100                 105                 110
   Glu Pro Lys Ala Thr Ile Asp Ser Leu Ser Leu Leu Ser Gln Lys Phe
           115                 120                 125
   Gly Lys Arg Ala Gly Asp Met Lys Met Ala Ser Leu Ala Thr Tyr Phe
       130                 135                 140
   Gly Leu Gly Asp Gln Ala His Arg Ser Leu Asp Asp Val Arg Met Asn
   145                 150                 155                 160
   Leu Glu Val Ile Lys
                   165
<210> 12
   <211> 165
   <212> PRT
   <213> Boechera
<400> 12
   Val Phe Phe Asp Leu Glu Thr Ala Val Pro Thr Lys Ser Gly Gln Pro
   1               5                   10                  15
   Phe Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
   35 40 45
   Leu Ile Ser Thr Leu Thr Lys Arg Arg Ser Gly Ile Thr Arg Asp Gly
   50 55 60
   Val Leu Ser Ala Pro Thr Phe Ser Glu Ile Ala Asp Glu Val Tyr Asp
   65 70 75 80
   Ile Leu His Gly Arg Ile Trp Ala Gly His Asn Ile Lys Arg Phe Asp
   85 90 95
   Cys Val Arg Ile Arg Asp Ala Phe Ala Glu Ile Gly Leu Pro Pro Pro
   100 105 110
   Glu Pro Lys Ala Thr Ile Asp Ser Leu Ser Leu Leu Ser Gln Lys Phe
   115 120 125
   Gly Lys Arg Ala Gly Asp Met Lys Met Ala Ser Leu Ala Thr Tyr Phe
   130 135 140
   Gly Leu Gly Asp Gln Ala His Arg Ser Leu Asp Asp Val Arg Met Asn
   145 150 155 160
   Leu Glu Val Ile Lys
   165
<210> 13
   <211> 496
   <212> PRT
   <213> Boechera
<400> 13
   Met Ala Ser Thr Leu Gly Gly Asp Glu Arg Asn Glu Ile Val Phe Phe
   1 5 10 15
   Asp Leu Glu Thr Ala Val Pro Thr Lys Ser Gly Gln Pro Phe Ala Ile
   20 25 30
   Leu Glu Phe Gly Ala Ile Leu Val Cys Pro Met Lys Leu Val Glu Leu
   35 40 45
   Tyr Ser Tyr Ser Thr Leu Val Arg Pro Thr Asp Leu Ser Leu Ile Ser
   50 55 60
   Thr Leu Thr Lys Arg Arg Ser Gly Ile Thr Arg Asp Gly Val Leu Ser
   65 70 75 80
   Ala Pro Thr Phe Ser Glu Ile Ala Asp Glu Val Tyr Asp Ile Leu His
   85 90 95
   Gly Arg Ile Trp Val Gly His Asn Ile Lys Arg Phe Asp Cys Val Arg
   100 105 110
   Ile Arg Asp Ala Phe Ala Glu Ile Gly Leu Pro Pro Pro Glu Pro Lys
   115 120 125
   Ala Thr Ile Asp Ser Leu Ser Leu Leu Ser Gln Lys Phe Gly Lys Arg
   130 135 140
   Ala Gly Asp Met Lys Met Ala Ser His Ala Thr Tyr Phe Gly Leu Gly
   145 150 155 160
   Asp Gln Ala His Arg Ser Leu Asp Asp Val Arg Met Asn Leu Glu Val
   165 170 175
   Ile Lys His Cys Ser Thr Val Leu Phe Leu Glu Ser Ser Val Pro Asp
   180 185 190
   Ile Leu Thr Asp Met Ser Trp Leu Phe Pro Arg Lys Ser Pro Arg Thr
   195 200 205
   Arg Ser Asn Glu Lys Ser Leu Pro Asn Gly Val Arg Glu Ser Pro Thr
   210 215 220
   Ser Ser Ser Ser Ser Pro Lys Thr Asp Pro Ser Ser Ser Ser Val Asp
   225 230 235 240
   Ala Thr Ala Val Lys Asn His Pro Ile Ile Ser Leu Leu Thr Glu Cys
   245 250 255
   Ser Glu Ser Asp Thr Ser Ser Cys Glu Ile Asp Pro Ser Asp Ile Thr
   260 265 270
   Thr Leu Ile Ser Lys Leu His Ile Gly Thr Leu Lys Thr Asp Ala Ala
   275 280 285
   Asp Glu Ala Lys Thr Val Arg Gln Gln Gly Glu Ser Thr Asp Pro Asn
   290 295 300
   Ala Lys Asp Glu Ser Phe Leu Gly Val Asn Glu Val Ser Val Ser Ser
   305 310 315 320
   Ile Arg Ala Ser Leu Ile Pro Leu Tyr Arg Arg Ser Leu Arg Met Glu
   325 330 335
   Leu Phe His Asn Asp Thr Pro Leu His Leu Cys Trp Tyr Ser Leu Lys
   340 345 350
   Ile Arg Phe Gly Ile Ser Arg Lys Tyr Val Asp His Val Gly Arg Pro
   355 360 365
   Lys Met Asn Ile Val Val Asp Ile Pro Pro Asp Leu Cys Lys Ile Leu
   370 375 380
   Asp Ala Ser Asp Ala Ala Ala His Asn Leu Leu Ile Asp Ser Ser Thr
   385 390 395 400
   Ser Ser Asp Trp Arg Pro Thr Val Met Arg Lys Lys Gly Phe Ala Asn
   405 410 415
   Tyr Pro Thr Ala Arg Leu Gln Ile Ser Ser Glu Ser Asn Gly Thr Gln
   420 425 430
   Val His Gln Lys Glu Glu Pro Leu Gly Thr Asn Gln Lys Leu Asp Phe
   435 440 445
   Ser Ser Asp Asn Phe Glu Lys Leu Glu Ser Ala Leu Leu Pro Gly Thr
   450 455 460
   Leu Val Asp Ala Phe Phe Ser Leu Glu Pro Tyr Asp Tyr Lys Lys Met
   465 470 475 480
   Val Gly Ile Arg Leu Ala Ala Arg Lys Leu Val Ile His Leu Lys Lys
   485 490 495
<210> 14
   <211> 496
   <212> PRT
   <213> Boechera
<400> 14
   Met Ala Ser Thr Leu Gly Gly Asp Glu Arg Asn Glu Ile Val Phe Phe
   1 5 10 15
   Asp Leu Glu Thr Ala Val Pro Thr Lys Ser Gly Gln Pro Phe Ala Ile
   20 25 30
   Leu Glu Phe Gly Ala Ile Leu Val Cys Pro Met Lys Leu Val Glu Leu
   35 40 45
   Tyr Ser Tyr Ser Thr Leu Val Arg Pro Thr Asp Leu Ser Leu Ile Ser
   50 55 60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Val Tyr Asp Ile Leu His
                   85                  90                  95
   Gly Arg Ile Trp Ala Gly His Asn Ile Lys Arg Phe Asp Cys Val Arg
               100                 105                 110
   Ile Arg Asp Ala Phe Ala Glu Ile Gly Leu Pro Pro Pro Glu Pro Lys
           115                 120                 125
   Ala Thr Ile Asp Ser Leu Ser Leu Leu Ser Gln Lys Phe Gly Lys Arg
       130                 135                 140
   Ala Gly Asp Met Lys Met Ala Ser Leu Ala Thr Tyr Phe Gly Leu Gly
   145                 150                 155                 160
   Asp Gln Ala His Arg Ser Leu Asp Asp Val Arg Met Asn Leu Glu Val
                   165                 170                 175
   Ile Lys His Cys Ser Thr Val Leu Phe Leu Glu Ser Ser Val Pro Asp
               180                 185                 190
   Ile Leu Thr Asp Met Ser Trp Leu Phe Pro Arg Lys Ser Pro Arg Thr
           195                 200                 205
   Arg Ser Asn Glu Lys Ser Leu Pro Asn Gly Val Arg Glu Ser Pro Thr
       210                 215                 220
   Ser Ser Ser Ser Ser Pro Gln Thr Asp Pro Ser Ser Ser Ser Val Asp
   225                 230                 235                 240
   Ala Thr Ala Val Lys Asn His Pro Ile Ile Ser Leu Leu Thr Glu Cys
                   245                 250                 255
   Ser Glu Ser Asp Thr Ser Ser Cys Glu Ile Asp Pro Ser Asp Ile Thr
               260                 265                 270
   Thr Leu Ile Ser Lys Leu His Ile Gly Thr Leu Lys Thr Asp Ala Ala
           275                 280                 285
   Asp Glu Ala Lys Thr Val Arg Gln Gln Gly Glu Ser Thr Asp Pro Asn
       290                 295                 300
   Ala Lys Asp Glu Ser Phe Leu Gly Val Asn Glu Val Ser Val Ser Ser
   305                 310                 315                 320
   Ile Arg Ala Ser Leu Ile Pro Leu Tyr Arg Arg Ser Leu Arg Met Glu
                   325                 330                 335
   Leu Phe His Asn Asp Thr Pro Leu His Leu Cys Trp Tyr Ser Leu Lys
               340                 345                 350
   Ile Arg Phe Gly Ile Ser Arg Lys Tyr Val Asp His Val Gly Arg Pro
           355                 360                 365
   Lys Met Asn Ile Val Val Asp Ile Pro Pro Asp Leu Cys Lys Ile Leu
       370                 375                 380
   Asp Ala Ser Asp Ala Ala Ala His Asn Leu Leu Ile Asp Ser Ser Thr
   385                 390                 395                 400
   Ser Ser Asp Trp Arg Pro Thr Val Met Arg Lys Lys Gly Phe Ala Asn
                   405                 410                 415
   Tyr Pro Thr Ala Arg Leu Gln Ile Ser Ser Glu Ser Asn Gly Thr Gln
               420                 425                 430
   Val Tyr Gln Lys Glu Glu Pro Leu Gly Thr Asn Gln Lys Leu Asp Phe
           435                 440                 445
   Ser Ser Asp Asn Phe Glu Lys Leu Glu Ser Ala Leu Leu Pro Gly Thr
       450                 455                 460
   Leu Val Asp Val Phe Phe Ser Val Glu Pro Tyr Asp Tyr Lys Lys Met
   465                 470                 475                 480
   Val Gly Ile Arg Leu Ala Ala Arg Lys Leu Val Ile Gln Leu Lys Lys
                   485                 490                 495
<210> 15
   <211> 496
   <212> PRT
   <213> Boechera
<400> 15
   Met Ala Ser Thr Leu Gly Gly Asp Glu Arg Asn Glu Ile Val Phe Phe
   1               5                   10                  15
   Asp Leu Glu Thr Ala Val Pro Thr Lys Ser Gly Gln Pro Phe Ala Ile
               20                  25                  30
   Leu Glu Phe Gly Ala Ile Leu Val Cys Pro Met Lys Leu Val Glu Leu
           35                  40                  45
   Tyr Ser Tyr Ser Thr Leu Val Arg Pro Thr Asp Leu Ser Leu Ile Ser
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Val Tyr Asp Ile Leu His
                   85                  90                  95
   Gly Arg Ile Trp Ala Gly His Asn Ile Lys Arg Phe Asp Cys Val Arg
               100                 105                 110
   Ile Arg Asp Ala Phe Ala Glu Ile Gly Leu Pro Pro Pro Glu Pro Lys
           115                 120                 125
   Ala Thr Ile Asp Ser Leu Ser Leu Leu Ser Gln Lys Phe Gly Lys Arg
       130                 135                 140
   Ala Gly Asp Met Lys Met Ala Ser Leu Ala Thr Tyr Phe Gly Leu Gly
   145                 150                 155                 160
   Asp Gln Ala His Arg Ser Leu Asp Asp Val Arg Met Asn Leu Glu Val
                   165                 170                 175
   Ile Lys His Cys Ala Thr Val Leu Phe Leu Glu Ser Ser Val Pro Asp
               180                 185                 190
   Ile Leu Thr Asp Met Ser Trp Leu Phe Pro Arg Lys Ser Pro Arg Thr
           195                 200                 205
   Arg Ser Asn Glu Lys Ser Leu Pro Asn Gly Val Arg Glu Ser Pro Thr
       210                 215                 220
   Ser Ser Ser Ser Ser Pro Lys Thr Asp Pro Ser Ser Ser Ser Val Asp
   225                 230                 235                 240
   Ala Thr Ala Val Lys Asn His Pro Ile Ile Ser Leu Leu Thr Glu Cys
                   245                 250                 255
   Ser Glu Ser Asp Thr Ser Ser Cys Glu Ile Asp Pro Ser Asp Ile Thr
               260                 265                 270
   Thr Leu Ile Ser Lys Leu His Ile Gly Thr Leu Lys Arg Asp Ala Ala
           275                 280                 285
   Asp Glu Ala Lys Ile Val Arg Gln Gln Gly Glu Ser Thr Asp Pro Asn
   290 295 300
   Ala Lys Asp Glu Ser Phe Leu Gly Val Asn Glu Val Ser Val Ser Ser
   305 310 315 320
   Ile Arg Ala Ser Leu Ile Pro Leu Tyr Arg Gly Ser Leu Arg Met Glu
   325 330 335
   Leu Phe His Asn Asp Thr Pro Leu His Leu Cys Trp Tyr Ser Leu Lys
   340 345 350
   Ile Arg Phe Gly Ile Ser Arg Lys Tyr Val Asp His Val Gly Arg Pro
   355 360 365
   Lys Met Asn Ile Val Val Asp Ile Pro Pro Asp Leu Cys Lys Ile Leu
   370 375 380
   Asp Ala Tyr Asp Ala Ala Ala His Asn Leu Leu Ile Asp Ser Ser Thr
   385 390 395 400
   Ser Ser Asp Trp Arg Pro Thr Val Met Arg Lys Glu Gly Phe Ala Asn
   405 410 415
   Tyr Pro Thr Ala Arg Leu Gln Ile Ser Ser Glu Ser Asn Gly Thr Gln
   420 425 430
   Val Tyr Gln Lys Glu Glu Pro Leu Gly Thr Asn Gln Lys Leu Asp Phe
   435 440 445
   Ser Ser Asp Asn Phe Glu Lys Leu Glu Ser Ala Leu Leu Pro Gly Thr
   450 455 460
   Leu Val Asp Ala Phe Phe Ser Pro Glu Ser Tyr Asp Tyr Lys Lys Met
   465 470 475 480
   Val Gly Ile Arg Leu Ala Ala Arg Lys Leu Val Ile His Leu Lys Lys
   485 490 495
<210> 16
   <211> 165
   <212> PRT
   <213> Boechera
<400> 16
   Val Phe Phe Asp Leu Glu Thr Ala Val Pro Thr Lys Ser Gly Gln Pro
   1               5                   10                  15
   Phe Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
           35                  40                  45
   Leu Ile Ser Thr Leu Thr Lys Arg Arg Ser Gly Ile Thr Arg Asp Gly
       50                  55                  60
   Val Leu Ser Ala Pro Thr Phe Ser Glu Ile Ala Asp Glu Val Tyr Asp
   65                  70                  75                  80
   Ile Leu His Gly Arg Ile Trp Ala Gly His Asn Ile Lys Arg Phe Asp
                   85                  90                  95
   Cys Val Arg Ile Arg Asp Ala Phe Ala Gly Ile Gly Val Ser Pro Pro
               100                 105                 110
   Glu Pro Lys Ala Thr Ile Asp Ser Leu Ser Leu Leu Ser Gln Lys Phe
           115                 120                 125
   Gly Lys Arg Ala Gly Asp Met Lys Met Ala Ser Leu Ala Thr Tyr Phe
       130                 135                 140
   Gly Leu Gly Asp Gln Ala His Arg Ser Leu Asp Asp Val Arg Met Asn
   145                 150                 155                 160
   Leu Glu Val Val Lys
                   165
<210> 17
   <211> 165
   <212> PRT
   <213> Boechera
<400> 17
   Val Phe Phe Asp Leu Glu Thr Ala Val Pro Thr Lys Ser Gly Gln Pro
   1               5                   10                  15
   Phe Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
           35                  40                  45
   Leu Ile Ser Thr Leu Thr Lys Arg Arg Ser Gly Ile Thr Arg Asp Gly
       50                  55                  60
   Val Leu Ser Ala Pro Thr Phe Ser Glu Ile Ala Asp Glu Val Tyr Asp
   65                  70                  75                  80
   Ile Leu His Gly Arg Ile Trp Ala Gly His Asn Ile Lys Arg Phe Asp
                   85                  90                  95
   Cys Val Arg Ile Arg Asp Ala Phe Ala Gly Ile Gly Leu Ser Pro Pro
               100                 105                 110
   Glu Pro Lys Ala Thr Ile Asp Ser Leu Ser Leu Leu Ser Gln Lys Phe
           115                 120                 125
   Gly Lys Arg Ala Gly Asp Met Lys Met Ala Ser Leu Ala Thr Tyr Phe
       130                 135                 140
   Gly Leu Gly Asp Gln Ala His Arg Ser Leu Asp Asp Val Arg Met Asn
   145                 150                 155                 160
   Leu Glu Val Val Lys
                   165
<210> 18
   <211> 165
   <212> PRT
   <213> Boechera
<400> 18
   Val Phe Phe Asp Leu Glu Thr Ala Val Pro Thr Lys Ser Gly Gln Pro
   1               5                   10                  15
   Phe Ala Ile Leu Glu Phe Gly Ala Ile Leu Val Cys Pro Met Lys Leu
               20                  25                  30
   Val Glu Leu Tyr Ser Tyr Ser Thr Leu Val Arg Pro Thr Asp Leu Ser
           35                  40                  45
   Leu Ile Ser Thr Leu Thr Lys Arg Arg Ser Gly Ile Thr Arg Asp Gly
       50                  55                  60
   Val Leu Ser Ala Pro Thr Phe Ser Glu Ile Ala Asp Glu Val Tyr Asp
   65                  70                  75                  80
   Ile Leu His Gly Arg Ile Trp Ala Gly His Asn Ile Lys Arg Phe Asp
                   85                  90                  95
   Cys Val Arg Ile Arg Asp Ala Phe Ala Gly Ile Gly Leu Ser Pro Pro
               100                 105                 110
   Glu Pro Lys Ala Thr Ile Asp Ser Leu Ser Leu Leu Ser Gln Lys Phe
           115                 120                 125
   Gly Lys Arg Ala Gly Asp Met Lys Met Ala Ser Leu Ala Thr Tyr Phe
       130                 135                 140
   Gly Leu Gly Asp Gln Ala His Arg Ser Leu Asp Asp Val Arg Met Asn
   145                 150                 155                 160
   Leu Glu Val Val Lys
                   165
<210> 19
   <211> 506
   <212> PRT
   <213> Boechera
<400> 19
   Met Ala Ser Thr Leu Gly Gly Asp Glu Arg Cys Glu Ile Val Phe Phe
   1               5                   10                  15
   Asp Leu Glu Thr Ala Val Pro Thr Lys Ser Gly Gln Pro Phe Ala Ile
               20                  25                  30
   Leu Glu Phe Gly Ala Ile Leu Val Cys Pro Met Lys Leu Val Glu Leu
           35                  40                  45
   Tyr Ser Tyr Ser Thr Leu Val Arg Pro Thr Asp Leu Ser Leu Ile Ser
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Val Tyr Asp Ile Leu His
                   85                  90                  95
   Gly Arg Ile Trp Ala Gly His Asn Ile Lys Arg Phe Asp Cys Val Arg
               100                 105                 110
   Ile Arg Asp Ala Phe Ala Gly Ile Gly Val Ser Pro Pro Glu Pro Lys
           115                 120                 125
   Ala Thr Ile Asp Ser Leu Ser Leu Leu Ser Gln Lys Phe Gly Lys Arg
       130                 135                 140
   Ala Gly Asp Met Lys Met Ala Ser Leu Ala Thr Tyr Phe Gly Leu Gly
   145                 150                 155                 160
   Asp Gln Ala His Arg Ser Leu Asp Asp Val Arg Met Asn Leu Glu Val
                   165                 170                 175
   Val Lys Tyr Cys Ala Thr Val Leu Phe Leu Glu Ser Ser Val Pro Asp
               180                 185                 190
   Ile Leu Lys Asp Met Ser Trp Phe Ser Pro Arg Lys Ser Pro Arg Thr
           195                 200                 205
   Arg Ser Asn Glu Lys Ser Leu Pro Asn Gly Val Arg Glu Ser Pro Thr
       210                 215                 220
   Ser Ser Ser Ser Ser Pro Lys Thr Asp Pro Ser Ser Ser Ser Val Asp
   225                 230                 235                 240
   Ala Thr Thr Val Lys Asn His Pro Ile Ile Ser Leu Leu Thr Glu Cys
                   245                 250                 255
   Ser Glu Ser Asp Thr Ser Ser Cys Glu Ile Asp Pro Ser Asp Ile Thr
               260                 265                 270
   Thr Leu Ile Ser Lys Leu His Ile Gly Thr Leu Lys Arg Asp Ala Ala
           275                 280                 285
   Asp Glu Ala Lys Thr Val Arg Asp Ala Ala Asp Glu Ala Lys Thr Val
       290                 295                 300
   Arg Gln Gln Gly Glu Ser Thr Asp Pro Asn Ala Lys Asp Glu Ser Phe
   305                 310                 315                 320
   Leu Gly Val Asn Glu Val Ser Val Ser Ser Ile Arg Ala Ser Leu Ile
                   325                 330                 335
   Pro Leu Tyr Arg Gly Ser Leu Arg Met Glu Leu Phe His Asn Asp Thr
               340                 345                 350
   Pro Leu His Leu Cys Trp Tyr Ser Leu Lys Ile Arg Phe Gly Ile Ser
           355                 360                 365
   Arg Lys Tyr Val Asp His Val Gly Arg Pro Lys Met Asn Ile Val Val
       370                 375                 380
   Asp Ile Pro Pro Asp Leu Cys Lys Ile Leu Asp Ala Ser Asp Ala Ala
   385                 390                 395                 400
   Ala His Asn Leu Leu Ile Asp Ser Ser Thr Ser Ser Asp Trp Arg Pro
                   405                 410                 415
   Thr Val Met Arg Lys Glu Gly Phe Ala Asn Tyr Pro Thr Ala Arg Leu
               420                 425                 430
   Gln Ile Ser Ser Glu Ser Asn Gly Thr Gln Val His Gln Lys Glu Glu
           435                 440                 445
   Pro Leu Gly Thr Asn Gln Lys Leu Asp Phe Ser Ser Asp Asn Phe Glu
       450                 455                 460
   Lys Leu Glu Ser Ala Leu Leu Pro Gly Thr Leu Val Asp Ala Phe Phe
   465                 470                 475                 480
   Ser Leu Glu Pro Tyr Asp Tyr Lys Lys Met Val Gly Ile Arg Leu Ala
                   485                 490                 495
   Ala Arg Lys Leu Val Ile His Leu Lys Lys
               500                 505
<210> 20
   <211> 496
   <212> PRT
   <213> Boechera
<400> 20
   Met Ala Ser Thr Leu Gly Gly Asp Glu Arg Cys Glu Ile Val Phe Phe
   1               5                   10                  15
   Asp Leu Glu Thr Ala Val Pro Thr Lys Ser Gly Gln Pro Phe Ala Ile
               20                  25                  30
   Leu Glu Phe Gly Ala Ile Leu Val Cys Pro Met Lys Leu Val Glu Leu
           35                  40                  45
   Tyr Ser Tyr Ser Thr Leu Val Arg Pro Thr Asp Leu Ser Leu Ile Ser
       50                  55                  60
   Thr Leu Thr Lys Arg Arg Ser Gly Ile Thr Arg Asp Gly Val Leu Ser
   65                  70                  75                  80
   Ala Pro Thr Phe Ser Glu Ile Ala Asp Glu Val Tyr Asp Ile Leu His
                   85                  90                  95
   Gly Arg Ile Trp Ala Gly His Asn Ile Lys Arg Phe Asp Cys Val Arg
               100                 105                 110
   Ile Arg Asp Ala Phe Ala Gly Ile Gly Leu Ser Pro Pro Glu Pro Lys
           115                 120                 125
   Ala Thr Ile Asp Ser Leu Ser Leu Leu Ser Gln Lys Phe Gly Lys Arg
       130                 135                 140
   Ala Gly Asp Met Lys Met Ala Ser Leu Ala Thr Tyr Phe Gly Leu Gly
   145                 150                 155                 160
   Asp Gln Ala His Arg Ser Leu Asp Asp Val Arg Met Asn Leu Glu Val
                   165                 170                 175
   Val Lys Tyr Cys Ala Thr Val Leu Phe Leu Glu Ser Ser Val Pro Asp
               180                 185                 190
   Ile Leu Lys Asp Met Ser Trp Phe Ser Pro Arg Lys Ser Pro Arg Thr
           195                 200                 205
   Arg Ser Asn Glu Lys Ser Leu Pro Asn Gly Val Arg Glu Ser Pro Thr
       210                 215                 220
   Ser Ser Ser Ser Ser Pro Lys Thr Asp Pro Ser Ser Ser Ser Val Asp
   225                 230                 235                 240
   Ala Thr Thr Val Lys Asn His Pro Ile Ile Ser Leu Leu Thr Glu Cys
                   245                 250                 255
   Ser Glu Ser Asp Thr Ser Ser Cys Glu Ile Asp Pro Ser Asp Ile Thr
               260                 265                 270
   Thr Leu Ile Ser Lys Leu His Ile Gly Thr Leu Lys Arg Asp Ala Ala
           275                 280                 285
   Asp Glu Ala Lys Ile Val Arg Gln Gln Gly Glu Ser Thr Asp Pro Asn
       290                 295                 300
   Ala Lys Asp Glu Ser Phe Leu Gly Val Asn Glu Val Ser Val Ser Ser
   305                 310                 315                 320
   Ile Arg Ala Ser Leu Ile Pro Leu Tyr Arg Gly Ser Leu Arg Met Glu
                   325                 330                 335
   Leu Leu His Asn Asp Thr Pro Leu His Leu Cys Trp Tyr Ser Leu Lys
               340                 345                 350
   Ile Arg Phe Gly Ile Ser Arg Lys Tyr Val Asp His Val Gly Arg Pro
           355                 360                 365
   Lys Met Asn Ile Val Val Asp Ile Pro Pro Asp Leu Cys Lys Ile Leu
       370                 375                 380
   Asp Ala Tyr Asp Ala Ala Ala His Asn Leu Leu Ile Asp Ser Ser Thr
   385 390 395 400
   Ser Ser Asp Trp Arg Pro Thr Val Met Arg Lys Glu Gly Phe Ala Asn
   405 410 415
   Tyr Pro Thr Ala Arg Leu Gln Ile Ser Ser Glu Ser Asn Gly Thr Gln
   420 425 430
   Val Tyr Gln Lys Glu Glu Pro Leu Gly Thr Asn Gln Lys Leu Asp Phe
   435 440 445
   Ser Ser Asp Asn Phe Glu Lys Leu Glu Ser Ala Leu Leu Pro Gly Thr
   450 455 460
   Leu Val Asp Ala Phe Phe Ser Leu Glu Ser Tyr Asp Tyr Lys Lys Met
   465 470 475 480
   Val Gly Ile Arg Leu Ala Ala Arg Lys Leu Val Ile His Leu Lys Lys
   485 490 495
<210> 21
   <211> 496
   <212> PRT
   <213> Boechera
<400> 21
   Met Ala Ser Thr Leu Gly Gly Asp Gly Arg Cys Glu Ile Val Phe Phe
   1 5 10 15
   Asp Leu Glu Thr Ala Val Pro Thr Lys Ser Gly Gln Pro Phe Ala Ile
   20 25 30
   Leu Glu Phe Gly Ala Ile Leu Val Cys Pro Met Lys Leu Val Glu Leu
   35 40 45
   Tyr Ser Tyr Ser Thr Leu Val Arg Pro Thr Asp Leu Ser Leu Ile Ser
   50 55 60
   Thr Leu Thr Lys Arg Arg Ser Gly Ile Thr Arg Asp Gly Val Leu Ser
   65 70 75 80
   Ala Pro Thr Phe Ser Glu Ile Ala Asp Glu Val Tyr Asp Ile Leu His
   85 90 95
   Gly Arg Ile Trp Ala Gly His Asn Ile Lys Arg Phe Asp Cys Val Arg
   100 105 110
   Ile Arg Asp Ala Phe Ala Gly Ile Gly Leu Ser Pro Pro Glu Pro Lys
           115                 120                 125
   Ala Thr Ile Asp Ser Leu Ser Leu Leu Ser Gln Lys Phe Gly Lys Arg
       130                 135                 140
   Ala Gly Asp Met Lys Met Ala Ser Leu Ala Thr Tyr Phe Gly Leu Gly
   145                 150                 155                 160
   Asp Gln Ala His Arg Ser Leu Asp Asp Val Arg Met Asn Leu Glu Val
                   165                 170                 175
   Val Lys Tyr Cys Ala Thr Val Leu Phe Leu Glu Ser Ser Val Pro Asp
               180                 185                 190
   Ile Leu Lys Asp Met Ser Trp Phe Ser Pro Arg Lys Ser Pro Arg Thr
           195                 200                 205
   Arg Ser Asn Glu Lys Ser Leu Pro Asn Gly Val Arg Glu Ser Pro Thr
       210                 215                 220
   Ser Ser Ser Ser Ser Pro Lys Thr Asp Pro Ser Ser Ser Ser Val Asp
   225                 230                 235                 240
   Ala Thr Thr Val Lys Asn His Pro Ile Ile Ser Leu Leu Thr Glu Cys
                   245                 250                 255
   Ser Glu Ser Asp Thr Ser Ser Cys Glu Ile Asp Pro Ser Asp Ile Thr
               260                 265                 270
   Thr Leu Ile Ser Lys Leu His Ile Gly Thr Leu Lys Arg Asp Ala Ala
           275                 280                 285
   Asp Glu Ala Lys Thr Val Arg Gln Gln Gly Glu Ser Thr Asp Pro Asn
       290                 295                 300
   Ala Lys Asp Glu Ser Phe Leu Gly Val Asn Glu Val Ser Val Ser Ser
   305                 310                 315                 320
   Ile Arg Ala Ser Leu Ile Pro Leu Tyr Arg Gly Gly Leu Arg Met Glu
                   325                 330                 335
   Leu Phe His Asn Asp Thr Pro Leu His Leu Arg Trp Tyr Ser Leu Lys
               340                 345                 350
   Ile Arg Phe Gly Ile Ser Arg Lys Tyr Val Asp His Val Gly Arg Pro
           355                 360                 365
   Lys Met Asn Ile Val Val Asp Ile Pro Pro Asp Leu Cys Lys Ile Leu
   370 375 380
   Asp Ala Ser Asp Ala Ala Ala His Asn Leu Leu Ile Asp Ser Ser Thr
   385 390 395 400
   Ser Ser Asp Trp Arg Pro Thr Val Met Arg Lys Glu Gly Phe Ala Asn
   405 410 415
   Tyr Pro Thr Ala Arg Leu Gln Ile Ser Ser Glu Ser Asn Gly Thr Gln
   420 425 430
   Val His Gln Lys Glu Glu Pro Leu Gly Thr Asn Gln Lys Leu Asp Phe
   435 440 445
   Ser Ser Asp Asn Phe Glu Lys Leu Glu Ser Ala Leu Leu Pro Gly Thr
   450 455 460
   Leu Val Asp Ala Phe Phe Ser Leu Glu Pro Tyr Asp Tyr Lys Lys Met
   465 470 475 480
   Val Gly Ile Arg Leu Ala Ala Arg Lys Leu Val Ile His Leu Lys Lys
   485 490 495
<210> 22
   <211> 2423
   <212> DNA
   <213> Boechera
<220>
   <221> misc_feature
   <222> (19)..(19)
   <223> n is a, c, g, or t
<220>
   <221> misc_feature
   <222> (26)..(26)
   <223> n is a, c, g, or t
<220>
   <221> misc_feature
   <222> (30)..(32)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (67)..(67)
   <223> n is a, c, g, or t
<220>
   <221> misc_feature
   <222> (73)..(73)
   <223> n is a, c, g, or t
<220>
   <221> misc_feature
   <222> (81)..(81)
   <223> n is a, c, g, or t
<220>
   <221> misc_feature
   <222> (84)..(84)
   <223> n is a, c, g, or t
<220>
   <221> misc_feature
   <222> (87)..(87)
   <223> n is a, c, g, or t
<220>
   <221> misc_feature
   <222> (89) .. (89)
   <223> n is a, c, g, or t
<220>
   <221> misc_feature
   <222> (108)..(108)
   <223> n is a, c, g, or t
<220>
   <221> misc_feature
   <222> (137)..(137)
   <223> n is a, c, g, or t
<220>
   <221> misc_feature
   <222> (154)..(154)
   <223> n is a, c, g, or t
<220>
   <221> misc_feature
   <222> (159)..(159)
   <223> n is a, c, g, or t
<220>
   <221> misc_feature
   <222> (164)..(164)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (177)..(177)
   <223> n is a, c, g, or t
<220>
   <221> misc_feature
   <222> (183)..(183)
   <223> n is a, c, g, or t
<220>
   <221> misc_feature
   <222> (191)..(191)
   <223> n is a, c, g, or t
<220>
   <221> misc_feature
   <222> (249) .. (249)
   <223> n is a, c, g, or t
<220>
   <221> misc_feature
   <222> (271)..(271)
   <223> n is a, c, g, or t
<220>
   <221> misc_feature
   <222> (279) .. (279)
   <223> n is a, c, g, or t
<220>
   <221> misc_feature
   <222> (284)..(284)
   <223> n is a, c, g, or t
<220>
   <221> misc_feature
   <222> (287)..(287)
   <223> n is a, c, g, or t
<220>
   <221> misc_feature
   <222> (303)..(304)
   <223> n is a, c, g, or t
<220>
   <221> misc_feature
   <222> (312)..(317)
   <223> n is a, c, g, or t
<220>
   <221> misc_feature
   <222> (320)..(321)
   <223> n is a, c, g, or t
<220>
   <221> misc_feature
   <222> (323)..(323)
   <223> n is a, c, g, or t
<220>
   <221> misc_feature
   <222> (325) .. (326)
   <223> n is a, c, g, or t
<220>
   <221> misc_feature
   <222> (334)..(334)
   <223> n is a, c, g, or t
<220>
   <221> misc_feature
   <222> (337)..(337)
   <223> n is a, c, g, or t
<220>
   <221> misc_feature
   <222> (343)..(343)
   <223> n is a, c, g, or t
<220>
   <221> misc_feature
   <222> (346)..(346)
   <223> n is a, c, g, or t
<220>
   <221> misc_feature
   <222> (385)..(386)
   <223> n is a, c, g, or t
<220>
   <221> misc_feature
   <222> (420)..(420)
   <223> n is a, c, g, or t
<220>
   <221> misc_feature
   <222> (423)..(423)
   <223> n is a, c, g, or t
<220>
   <221> misc_feature
   <222> (439)..(439)
   <223> n is a, c, g, or t
<220>
   <221> misc_feature
   <222> (447)..(447)
   <223> n is a, c, g, or t
<220>
   <221> misc_feature
   <222> (450)..(450)
   <223> n is a, c, g, or t
<220>
   <221> misc_feature
   <222> (457)..(458)
   <223> n is a, c, g, or t
<220>
   <221> misc_feature
   <222> (462)..(462)
   <223> n is a, c, g, or t
<220>
   <221> misc_feature
   <222> (464)..(464)
   <223> n is a, c, g, or t
<220>
   <221> misc_feature
   <222> (469)..(469)
   <223> n is a, c, g, or t
<220>
   <221> misc_feature
   <222> (471)..(471)
   <223> n is a, c, g, or t
<220>
   <221> misc_feature
   <222> (480)..(480)
   <223> n is a, c, g, or t
<220>
   <221> misc_feature
   <222> (491)..(493)
   <223> n is a, c, g, or t
<220>
   <221> misc_feature
   <222> (513)..(513)
   <223> n is a, c, g, or t
<220>
   <221> misc_feature
   <222> (521)..(521)
   <223> n is a, c, g, or t
<220>
   <221> misc_feature
   <222> (525)..(525)
   <223> n is a, c, g, or t
<220>
   <221> misc_feature
   <222> (551)..(551)
   <223> n is a, c, g, or t
<220>
   <221> misc_feature
   <222> (572)..(572)
   <223> n is a, c, g, or t
<220>
   <221> misc_feature
   <222> (580)..(580)
   <223> n is a, c, g, or t
<220>
   <221> misc_feature
   <222> (590)..(590)
   <223> n is a, c, g, or t
<220>
   <221> misc_feature
   <222> (608)..(608)
   <223> n is a, c, g, or t
<220>
   <221> misc_feature
   <222> (610)..(610)
   <223> n is a, c, g, or t
<220>
   <221> misc_feature
   <222> (620)..(620)
   <223> n is a, c, g, or t
<220>
   <221> misc_feature
   <222> (638)..(638)
   <223> n is a, c, g, or t
<220>
   <221> misc_feature
   <222> (642)..(642)
   <223> n is a, c, g, or t
<220>
   <221> misc_feature
   <222> (655)..(655)
   <223> n is a, c, g, or t
<220>
   <221> misc_feature
   <222> (658)..(662)
   <223> n is a, c, g, or t
<220>
   <221> misc_feature
   <222> (665)..(665)
   <223> n is a, c, g, or t
<220>
   <221> misc_feature
   <222> (668)..(668)
   <223> n is a, c, g, or t
<220>
   <221> misc_feature
   <222> (678)..(678)
   <223> n is a, c, g, or t
<220>
   <221> misc_feature
   <222> (680)..(680)
   <223> n is a, c, g, or t
<220>
   <221> misc_feature
   <222> (683)..(683)
   <223> n is a, c, g, or t
<220>
   <221> misc_feature
   <222> (690)..(690)
   <223> n is a, c, g, or t
<220>
   <221> misc_feature
   <222> (698)..(698)
   <223> n is a, c, g, or t
<220>
   <221> misc_feature
   <222> (708)..(708)
   <223> n is a, c, g, or t
<220>
   <221> misc_feature
   <222> (711)..(711)
   <223> n is a, c, g, or t
<220>
   <221> misc_feature
   <222> (722)..(722)
   <223> n is a, c, g, or t
<220>
   <221> misc_feature
   <222> (726) .. (727)
   <223> n is a, c, g, or t
<220>
   <221> misc_feature
   <222> (740)..(741)
   <223> n is a, c, g, or t
<220>
   <221> misc_feature
   <222> (760)..(760)
   <223> n is a, c, g, or t
<220>
   <221> misc_feature
   <222> (769)..(769)
   <223> n is a, c, g, or t
<220>
   <221> misc_feature
   <222> (791)..(791)
   <223> n is a, c, g, or t
<220>
   <221> misc_feature
   <222> (794)..(794)
   <223> n is a, c, g, or t
<220>
   <221> misc_feature
   <222> (801)..(801)
   <223> n is a, c, g, or t
<220>
   <221> misc_feature
   <222> (838)..(839)
   <223> n is a, c, g, or t
<220>
   <221> misc_feature
   <222> (845)..(845)
   <223> n is a, c, g, or t
<220>
   <221> misc_feature
   <222> (851)..(851)
   <223> n is a, c, g, or t
<220>
   <221> misc_feature
   <222> (857)..(857)
   <223> n is a, c, g, or t
<220>
   <221> misc_feature
   <222> (862)..(862)
   <223> n is a, c, g, or t
<220>
   <221> misc_feature
   <222> (875)..(875)
   <223> n is a, c, g, or t
<220>
   <221> misc_feature
   <222> (882)..(882)
   <223> n is a, c, g, or t
<220>
   <221> misc_feature
   <222> (902)..(902)
   <223> n is a, c, g, or t
<220>
   <221> misc_feature
   <222> (908)..(908)
   <223> n is a, c, g, or t
<220>
   <221> misc_feature
   <222> (912)..(912)
   <223> n is a, c, g, or t
<220>
   <221> misc_feature
   <222> (915)..(915)
   <223> n is a, c, g, or t
<220>
   <221> misc_feature
   <222> (923)..(923)
   <223> n is a, c, g, or t
<220>
   <221> misc_feature
   <222> (931)..(931)
   <223> n is a, c, g, or t
<220>
   <221> misc_feature
   <222> (934)..(934)
   <223> n is a, c, g, or t
<220>
   <221> misc_feature
   <222> (939)..(940)
   <223> n is a, c, g, or t
<220>
   <221> misc_feature
   <222> (952)..(952)
   <223> n is a, c, g, or t
<220>
   <221> misc_feature
   <222> (961)..(961)
   <223> n is a, c, g, or t
<220>
   <221> misc_feature
   <222> (963)..(963)
   <223> n is a, c, g, or t
<220>
   <221> misc_feature
   <222> (967)..(967)
   <223> n is a, c, g, or t
<220>
   <221> misc_feature
   <222> (970)..(970)
   <223> n is a, c, g, or t
<220>
   <221> misc_feature
   <222> (976)..(977)
   <223> n is a, c, g, or t
<220>
   <221> misc_feature
   <222> (980)..(980)
   <223> n is a, c, g, or t
<220>
   <221> misc_feature
   <222> (983)..(983)
   <223> n is a, c, g, or t
<220>
   <221> misc_feature
   <222> (993)..(993)
   <223> n is a, c, g, or t
<220>
   <221> misc_feature
   <222> (1003)..(1003)
   <223> n is a, c, g, or t
<220>
   <221> misc_feature
   <222> (1025)..(1025)
   <223> n is a, c, g, or t
<220>
   <221> misc_feature
   <222> (1049)..(1050)
   <223> n is a, c, g, or t
<220>
   <221> misc_feature
   <222> (1089)..(1089)
   <223> n is a, c, g, or t
<220>
   <221> misc_feature
   <222> (1095)..(1095)
   <223> n is a, c, g, or t
<220>
   <221> misc_feature
   <222> (1098)..(1098)
   <223> n is a, c, g, or t
<220>
   <221> misc_feature
   <222> (1109)..(1109)
   <223> n is a, c, g, or t
<220>
   <221> misc_feature
   <222> (1111)..(1112)
   <223> n is a, c, g, or t
<220>
   <221> misc_feature
   <222> (1116)..(1116)
   <223> n is a, c, g, or t
<220>
   <221> misc_feature
   <222> (1125)..(1125)
   <223> n is a, c, g, or t
<220>
   <221> misc_feature
   <222> (1127)..(1127)
   <223> n is a, c, g, or t
<220>
   <221> misc_feature
   <222> (1130)..(1130)
   <223> n is a, c, g, or t
<220>
   <221> misc_feature
   <222> (1142)..(1142)
   <223> n is a, c, g, or t
<220>
   <221> misc_feature
   <222> (1144)..(1144)
   <223> n is a, c, g, or t
<220>
   <221> misc_feature
   <222> (1173)..(1174)
   <223> n is a, c, g, or t
<220>
   <221> misc_feature
   <222> (1194)..(1194)
   <223> n is a, c, g, or t
<220>
   <221> misc_feature
   <222> (1209)..(1209)
   <223> n is a, c, g, or t
<220>
   <221> misc_feature
   <222> (1216)..(1216)
   <223> n is a, c, g, or t
<220>
   <221> misc_feature
   <222> (1219)..(1219)
   <223> n is a, c, g, or t
<220>
   <221> misc_feature
   <222> (1224)..(1224)
   <223> n is a, c, g, or t
<220>
   <221> misc_feature
   <222> (1243)..(1243)
   <223> n is a, c, g, or t
<220>
   <221> misc_feature
   <222> (1245)..(1245)
   <223> n is a, c, g, or t
<220>
   <221> misc_feature
   <222> (1248)..(1248)
   <223> n is a, c, g, or t
<220>
   <221> misc_feature
   <222> (1252)..(1252)
   <223> n is a, c, g, or t
<220>
   <221> misc_feature
   <222> (1298)..(1298)
   <223> n is a, c, g, or t
<220>
   <221> misc_feature
   <222> (1301)..(1302)
   <223> n is a, c, g, or t
<220>
   <221> misc_feature
   <222> (1317)..(1317)
   <223> n is a, c, g, or t
<220>
   <221> misc_feature
   <222> (1332)..(1332)
   <223> n is a, c, g, or t
<220>
   <221> misc_feature
   <222> (1379)..(1379)
   <223> n is a, c, g, or t
<220>
   <221> misc_feature
   <222> (1413)..(1413)
   <223> n is a, c, g, or t
<220>
   <221> misc_feature
   <222> (1416)..(1416)
   <223> n is a, c, g, or t
<220>
   <221> misc_feature
   <222> (1420)..(1420)
   <223> n is a, c, g, or t
<220>
   <221> misc_feature
   <222> (1422)..(1422)
   <223> n is a, c, g, or t
<220>
   <221> misc_feature
   <222> (1431)..(1431)
   <223> n is a, c, g, or t
<220>
   <221> misc_feature
   <222> (1433)..(1433)
   <223> n is a, c, g, or t
<220>
   <221> misc_feature
   <222> (1446)..(1446)
   <223> n is a, c, g, or t
<220>
   <221> misc_feature
   <222> (1488)..(1488)
   <223> n is a, c, g, or t
<220>
   <221> misc_feature
   <222> (1500)..(1500)
   <223> n is a, c, g, or t
<220>
   <221> misc_feature
   <222> (1507)..(1507)
   <223> n is a, c, g, or t
<220>
   <221> misc_feature
   <222> (1514)..(1516)
   <223> n is a, c, g, or t
<220>
   <221> misc_feature
   <222> (1531)..(1531)
   <223> n is a, c, g, or t
<220>
   <221> misc_feature
   <222> (1533)..(1533)
   <223> n is a, c, g, or t
<220>
   <221> misc_feature
   <222> (1546)..(1546)
   <223> n is a, c, g, or t
<220>
   <221> misc_feature
   <222> (1549)..(1549)
   <223> n is a, c, g, or t
<220>
   <221> misc_feature
   <222> (1567)..(1567)
   <223> n is a, c, g, or t
<220>
   <221> misc_feature
   <222> (1575)..(1575)
   <223> n is a, c, g, or t
<220>
   <221> misc_feature
   <222> (1578)..(1579)
   <223> n is a, c, g, or t
<220>
   <221> misc_feature
   <222> (1586)..(1586)
   <223> n is a, c, g, or t
<220>
   <221> misc_feature
   <222> (1594)..(1594)
   <223> n is a, c, g, or t
<220>
   <221> misc_feature
   <222> (1596)..(1596)
   <223> n is a, c, g, or t
<220>
   <221> misc_feature
   <222> (1604)..(1604)
   <223> n is a, c, g, or t
<220>
   <221> misc_feature
   <222> (1606)..(1606)
   <223> n is a, c, g, or t
<220>
   <221> misc_feature
   <222> (1629)..(1629)
   <223> n is a, c, g, or t
<220>
   <221> misc_feature
   <222> (1637)..(1637)
   <223> n is a, c, g, or t
<220>
   <221> misc_feature
   <222> (1656)..(1656)
   <223> n is a, c, g, or t
<220>
   <221> misc_feature
   <222> (1674)..(1674)
   <223> n is a, c, g, or t
<220>
   <221> misc_feature
   <222> (1677)..(1677)
   <223> n is a, c, g, or t
<220>
   <221> misc_feature
   <222> (1685)..(1685)
   <223> n is a, c, g, or t
<220>
   <221> misc_feature
   <222> (1715)..(1717)
   <223> n is a, c, g, or t
<220>
   <221> misc_feature
   <222> (1723)..(1723)
   <223> n is a, c, g, or t
<220>
   <221> misc_feature
   <222> (1757)..(1758)
   <223> n is a, c, g, or t
<220>
   <221> misc_feature
   <222> (1764)..(1765)
   <223> n is a, c, g, or t
<220>
   <221> misc_feature
   <222> (1776)..(1776)
   <223> n is a, c, g, or t
<220>
   <221> misc_feature
   <222> (1784)..(1784)
   <223> n is a, c, g, or t
<220>
   <221> misc_feature
   <222> (1789)..(1789)
   <223> n is a, c, g, or t
<220>
   <221> misc_feature
   <222> (1799)..(1799)
   <223> n is a, c, g, or t
<220>
   <221> misc_feature
   <222> (1812)..(1812)
   <223> n is a, c, g, or t
<220>
   <221> misc_feature
   <222> (1816)..(1816)
   <223> n is a, c, g, or t
<220>
   <221> misc_feature
   <222> (1832)..(1832)
   <223> n is a, c, g, or t
<220>
   <221> misc_feature
   <222> (1835)..(1835)
   <223> n is a, c, g, or t
<220>
   <221> misc_feature
   <222> (1847)..(1847)
   <223> n is a, c, g, or t
<220>
   <221> misc_feature
   <222> (1860)..(1860)
   <223> n is a, c, g, or t
<220>
   <221> misc_feature
   <222> (1866)..(1866)
   <223> n is a, c, g, or t
<220>
   <221> misc_feature
   <222> (1871)..(1871)
   <223> n is a, c, g, or t
<220>
   <221> misc_feature
   <222> (1874)..(1874)
   <223> n is a, c, g, or t
<220>
   <221> misc_feature
   <222> (1888)..(1888)
   <223> n is a, c, g, or t
<220>
   <221> misc_feature
   <222> (1901)..(1901)
   <223> n is a, c, g, or t
<220>
   <221> misc_feature
   <222> (1908)..(1909)
   <223> n is a, c, g, or t
<220>
   <221> misc_feature
   <222> (1911)..(1911)
   <223> n is a, c, g, or t
<220>
   <221> misc_feature
   <222> (1918)..(1918)
   <223> n is a, c, g, or t
<220>
   <221> misc_feature
   <222> (1924)..(1924)
   <223> n is a, c, g, or t
<220>
   <221> misc_feature
   <222> (1946)..(1946)
   <223> n is a, c, g, or t
<220>
   <221> misc_feature
   <222> (1951)..(1952)
   <223> n is a, c, g, or t
<220>
   <221> misc_feature
   <222> (1964)..(1966)
   <223> n is a, c, g, or t
<220>
   <221> misc_feature
   <222> (1976)..(1977)
   <223> n is a, c, g, or t
<220>
   <221> misc_feature
   <222> (1999)..(1999)
   <223> n is a, c, g, or t
<220>
   <221> misc_feature
   <222> (2003)..(2003)
   <223> n is a, c, g, or t
<220>
   <221> misc_feature
   <222> (2008)..(2008)
   <223> n is a, c, g, or t
<220>
   <221> misc_feature
   <222> (2020)..(2020)
   <223> n is a, c, g, or t
<220>
   <221> misc_feature
   <222> (2037)..(2037)
   <223> n is a, c, g, or t
<220>
   <221> misc_feature
   <222> (2052)..(2052)
   <223> n is a, c, g, or t
<220>
   <221> misc_feature
   <222> (2064)..(2064)
   <223> n is a, c, g, or t
<220>
   <221> misc_feature
   <222> (2079) .. (2079)
   <223> n is a, c, g, or t
<220>
   <221> misc_feature
   <222> (2088)..(2088)
   <223> n is a, c, g, or t
<220>
   <221> misc_feature
   <222> (2092)..(2092)
   <223> n is a, c, g, or t
<220>
   <221> misc_feature
   <222> (2103)..(2103)
   <223> n is a, c, g, or t
<220>
   <221> misc_feature
   <222> (2109)..(2111)
   <223> n is a, c, g, or t
<220>
   <221> misc_feature
   <222> (2120)..(2121)
   <223> n is a, c, g, or t
<220>
   <221> misc_feature
   <222> (2123)..(2123)
   <223> n is a, c, g, or t
<220>
   <221> misc_feature
   <222> (2125)..(2125)
   <223> n is a, c, g, or t
<220>
   <221> misc_feature
   <222> (2128)..(2128)
   <223> n is a, c, g, or t
<220>
   <221> misc_feature
   <222> (2131)..(2131)
   <223> n is a, c, g, or t
<220>
   <221> misc_feature
   <222> (2133)..(2133)
   <223> n is a, c, g, or t
<220>
   <221> misc_feature
   <222> (2135)..(2135)
   <223> n is a, c, g, or t
<220>
   <221> misc_feature
   <222> (2143)..(2143)
   <223> n is a, c, g, or t
<220>
   <221> misc_feature
   <222> (2155)..(2155)
   <223> n is a, c, g, or t
<220>
   <221> misc_feature
   <222> (2165)..(2165)
   <223> n is a, c, g, or t
<220>
   <221> misc_feature
   <222> (2172)..(2172)
   <223> n is a, c, g, or t
<220>
   <221> misc_feature
   <222> (2177)..(2177)
   <223> n is a, c, g, or t
<220>
   <221> misc_feature
   <222> (2190)..(2190)
   <223> n is a, c, g, or t
<220>
   <221> misc_feature
   <222> (2245)..(2245)
   <223> n is a, c, g, or t
<220>
   <221> misc_feature
   <222> (2257)..(2258)
   <223> n is a, c, g, or t
<220>
   <221> misc_feature
   <222> (2263)..(2263)
   <223> n is a, c, g, or t
<220>
   <221> misc_feature
   <222> (2267)..(2267)
   <223> n is a, c, g, or t
<220>
   <221> misc_feature
   <222> (2278)..(2281)
   <223> n is a, c, g, or t
<220>
   <221> misc_feature
   <222> (2291)..(2292)
   <223> n is a, c, g, or t
<220>
   <221> misc_feature
   <222> (2296)..(2297)
   <223> n is a, c, g, or t
<220>
   <221> misc_feature
   <222> (2310)..(2310)
   <223> n is a, c, g, or t
<220>
   <221> misc_feature
   <222> (2356)..(2356)
   <223> n is a, c, g, or t
<220>
   <221> misc_feature
   <222> (2392)..(2392)
   <223> n is a, c, g, or t
<220>
   <221> misc_feature
   <222> (2402)..(2402)
   <223> n is a, c, g, or t
<220>
   <221> misc_feature
   <222> (2406)..(2406)
   <223> n is a, c, g, or t
<220>
   <221> misc_feature
   <222> (2408)..(2408)
   <223> n is a, c, g, or t
<220>
   <221> misc_feature
   <222> (2415)..(2415)
   <223> n is a, c, g, or t
<220>
   <221> misc_feature
   <222> (2417)..(2417)
   <223> n is a, c, g, or t
<220>
   <221> misc_feature
   <222> (2419) .. (2419)
   <223> n is a, c, g, or t
<220>
   <221> misc_feature
   <222> (2421)..(2423)
   <223> n is a, c, g, or t
<400> 22
   atggcttcga ctctgggcng cgatgngagn nncgagatag tgtttttcga tcttgagacn 60
   gcngttncga ccnaatcggg ncanccntnt gcgattttgg agtttggngc tatcttagtt 120
   tgccctatga agctagngga gctctatagt tacnccacnt tggntcgacc nacnganctt 180
   tcnctcatct ncacgctcac gaagcgacga agcggcatta cgcgcgacgg agttctctct 240
   gcacctacnt tctctgaaat cgctgatgaa ntctacgana ttcnccncgg taagggtttc 300
   tcnntttttt tnnnnnnctn ncncnntctc tctnacncga agntanaagt attgattttg 360
   gtgtttctgt aggacgaatt tgggnnggac ataacataaa gagattcgat tgtgtaagan 420
   tangagatgc atttgcagna attggtntcn ctccccnnga gncnaaagnt ncaattgatn 480
   cactttcgtt nnngtctcag aagtttggga agngagctgg ngacntgaag gtctctcttt 540
   tttcgtcttc ncgatgataa atctcaaagc cnatagcttn cttgttatcn ttatagatat 600
   gaatttcnan gtaacttcan agattcatca ctcatcanag tngctaaaat ttacnctnnn 660
   nnaanaangt agatggcntn gcntgctacn tatttcgngc taggaganca ngctcacagg 720
   tnaaannagt aaacgatacn ntgtgccttt taacgattcn ccagttgtnt caatatggga 780
   ctaaacatgg ntangattca ncaggagctt agatgatgtc cggatgaatc ttgaagtnnt 840
   caagnactgt ncaaccntct tntttctggt attgntgtct tntcatttct tgaataatga 900
   tnaactcnta anttnaaaag gantagatta nagnggttnn gacatatctg anttctgtct 960
   ncngttntgn aaaagnnggn tcnatcttcc ttncagacca canctttgca agccgtaaac 1020
   atggnttgca acttgcaagt atagtttgnn atatcactga gtttaagtac ttggtgtttg 1080
   caggagtcna gtgtnccnga cattcttana nncatnagct ggttntnccn aagaaaaagt 1140
   cngngaacac gaagtaatga gaagtcactg ccnnatggag tcagagaaag cccnacttct 1200
   tcctcttcna gccctnaanc tganccgagt tcgtcttctg tanangcnac anctgtcaaa 1260
   aaccatccca tcatttctct tctgacggaa tgctcagnaa nngatacatc tagttgngaa 1320
   atagatccat cngacataac cactctaata agtaaactac atattggaac tcttaagana 1380
   gatgctgcgg acgaagccaa aactgtgaga gangcngcgn angaagccaa nantgtaaga 1440
   cagcanggtg aatcaaccga tcccaatgcc aaagatgaat catttttngg cgttaatgan 1500
   gtatctnttt ctannntcag ggcaagtctt ntnccgttat atcgtnggng tctgagaatg 1560
   gagctgnttc acaangannc ccctcnacat ctcngntggt atancntgaa aattcggttt 1620
   ggaataagnc ggaagtntgt ggatcatgta ggtcgnccaa agatgaatat tgtngtngac 1680
   atacntcctg atttatgcaa gatcttggac gcatnnnatg ctnctgcgca taacttactg 1740
   attgactcaa gcacaanntc agannggagg cctacngtta tgangaaana aggctttgnc 1800
   aactatccca cngccngact gcagtaagta tncancactc tctctgncct tttacatacn 1860
   agcatnaatc nacnggagag tctctaanac catctccaac nctactcnnt nttcaccncc 1920
   aaantctatt ttggagttaa atcccnccaa nncttgcaaa atannnatct tcaaannttt 1980
   tctccatatt tggagattnt ganttttnaa gtcatgactn cattttggag ttgggtngga 2040
   gaaaaacaca antccaaaat aganttactt cattttggng taaaaaantg angaaatggg 2100
   ttngagatnn nctaacctcn ntnancantc ntntnttgtt ggnagaataa gctcngaatc 2160
   caatngaacc cnggtanacc aaaaagaagn acctttggga accaatcaaa agctcgattt 2220
   cagtagcgat aattttgaaa agctngagtc agcactnntt ccnggtnccc tggttgannn 2280
   nttcttctca nncgannctt acgattatan gaaaatggta gggatacgtc tagcagccag 2340
   aaagttggta atccanctga agaaatgatc tagccaagga aaaatcattc cnctgtctct 2400
   tnctgntnag tcggngngna nnn 2423
<210> 23
   <211> 1521
   <212> DNA
   <213> Boechera
<220>
   <221> misc_feature
   <222> (19)..(19)
   <223> n is a, c, g, or t
<220>
   <221> misc_feature
   <222> (26)..(26)
   <223> n is a, c, g, or t
<220>
   <221> misc_feature
   <222> (30)..(32)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (67)..(67)
   <223> n is a, c, g, or t
<220>
   <221> misc_feature
   <222> (73)..(73)
   <223> n is a, c, g, or t
<220>
   <221> misc_feature
   <222> (81)..(81)
   <223> n is a, c, g, or t
<220>
   <221> misc_feature
   <222> (84)..(84)
   <223> n is a, c, g, or t
<220>
   <221> misc_feature
   <222> (87)..(87)
   <223> n is a, c, g, or t
<220>
   <221> misc_feature
   <222> (89) .. (89)
   <223> n is a, c, g, or t
<220>
   <221> misc_feature
   <222> (108)..(108)
   <223> n is a, c, g, or t
<220>
   <221> misc_feature
   <222> (137)..(137)
   <223> n is a, c, g, or t
<220>
   <221> misc_feature
   <222> (154)..(154)
   <223> n is a, c, g, or t
<220>
   <221> misc_feature
   <222> (159)..(159)
   <223> n is a, c, g, or t
<220>
   <221> misc_feature
   <222> (164)..(164)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (177)..(177)
   <223> n is a, c, g, or t
<220>
   <221> misc_feature
   <222> (183)..(183)
   <223> n is a, c, g, or t
<220>
   <221> misc_feature
   <222> (191)..(191)
   <223> n is a, c, g, or t
<220>
   <221> misc_feature
   <222> (249) .. (249)
   <223> n is a, c, g, or t
<220>
   <221> misc_feature
   <222> (271)..(271)
   <223> n is a, c, g, or t
<220>
   <221> misc_feature
   <222> (279) .. (279)
   <223> n is a, c, g, or t
<220>
   <221> misc_feature
   <222> (284)..(284)
   <223> n is a, c, g, or t
<220>
   <221> misc_feature
   <222> (287)..(287)
   <223> n is a, c, g, or t
<220>
   <221> misc_feature
   <222> (302)..(303)
   <223> n is a, c, g, or t
<220>
   <221> misc_feature
   <222> (337)..(337)
   <223> n is a, c, g, or t
<220>
   <221> misc_feature
   <222> (340)..(340)
   <223> n is a, c, g, or t
<220>
   <221> misc_feature
   <222> (356)..(356)
   <223> n is a, c, g, or t
<220>
   <221> misc_feature
   <222> (364)..(364)
   <223> n is a, c, g, or t
<220>
   <221> misc_feature
   <222> (367)..(367)
   <223> n is a, c, g, or t
<220>
   <221> misc_feature
   <222> (374)..(375)
   <223> n is a, c, g, or t
<220>
   <221> misc_feature
   <222> (379)..(379)
   <223> n is a, c, g, or t
<220>
   <221> misc_feature
   <222> (381)..(381)
   <223> n is a, c, g, or t
<220>
   <221> misc_feature
   <222> (386)..(386)
   <223> n is a, c, g, or t
<220>
   <221> misc_feature
   <222> (388)..(388)
   <223> n is a, c, g, or t
<220>
   <221> misc_feature
   <222> (397)..(397)
   <223> n is a, c, g, or t
<220>
   <221> misc_feature
   <222> (408)..(410)
   <223> n is a, c, g, or t
<220>
   <221> misc_feature
   <222> (430)..(430)
   <223> n is a, c, g, or t
<220>
   <221> misc_feature
   <222> (438)..(438)
   <223> n is a, c, g, or t
<220>
   <221> misc_feature
   <222> (442)..(442)
   <223> n is a, c, g, or t
<220>
   <221> misc_feature
   <222> (453)..(453)
   <223> n is a, c, g, or t
<220>
   <221> misc_feature
   <222> (455)..(455)
   <223> n is a, c, g, or t
<220>
   <221> misc_feature
   <222> (458)..(458)
   <223> n is a, c, g, or t
<220>
   <221> misc_feature
   <222> (465)..(465)
   <223> n is a, c, g, or t
<220>
   <221> misc_feature
   <222> (473)..(473)
   <223> n is a, c, g, or t
<220>
   <221> misc_feature
   <222> (483)..(483)
   <223> n is a, c, g, or t
<220>
   <221> misc_feature
   <222> (486)..(486)
   <223> n is a, c, g, or t
<220>
   <221> misc_feature
   <222> (528) .. (529)
   <223> n is a, c, g, or t
<220>
   <221> misc_feature
   <222> (535)..(535)
   <223> n is a, c, g, or t
<220>
   <221> misc_feature
   <222> (541)..(541)
   <223> n is a, c, g, or t
<220>
   <221> misc_feature
   <222> (547)..(547)
   <223> n is a, c, g, or t
<220>
   <221> misc_feature
   <222> (552)..(552)
   <223> n is a, c, g, or t
<220>
   <221> misc_feature
   <222> (564)..(564)
   <223> n is a, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (584)..(584)
   <223> n is a, c, g, or t
<220>
   <221> misc_feature
   <222> (586)..(587)
   <223> n is a, c, g, or t
<220>
   <221> misc_feature
   <222> (591)..(591)
   <223> n is a, c, g, or t
<220>
   <221> misc_feature
   <222> (600)..(600)
   <223> n is a, c, g, or t
<220>
   <221> misc_feature
   <222> (602)..(602)
   <223> n is a, c, g, or t
<220>
   <221> misc_feature
   <222> (605)..(605)
   <223> n is a, c, g, or t
<220>
   <221> misc_feature
   <222> (617)..(617)
   <223> n is a, c, g, or t
<220>
   <221> misc_feature
   <222> (619)..(619)
   <223> n is a, c, g, or t
<220>
   <221> misc_feature
   <222> (648)..(649)
   <223> n is a, c, g, or t
<220>
   <221> misc_feature
   <222> (669)..(669)
   <223> n is a, c, g, or t
<220>
   <221> misc_feature
   <222> (684)..(684)
   <223> n is a, c, g, or t
<220>
   <221> misc_feature
   <222> (691)..(691)
   <223> n is a, c, g, or t
<220>
   <221> misc_feature
   <222> (694)..(694)
   <223> n is a, c, g, or t
<220>
   <221> misc_feature
   <222> (699)..(699)
   <223> n is a, c, g, or t
<220>
   <221> misc_feature
   <222> (718)..(718)
   <223> n is a, c, g, or t
<220>
   <221> misc_feature
   <222> (720)..(720)
   <223> n is a, c, g, or t
<220>
   <221> misc_feature
   <222> (723)..(723)
   <223> n is a, c, g, or t
<220>
   <221> misc_feature
   <222> (727)..(727)
   <223> n is a, c, g, or t
<220>
   <221> misc_feature
   <222> (773)..(773)
   <223> n is a, c, g, or t
<220>
   <221> misc_feature
   <222> (776)..(777)
   <223> n is a, c, g, or t
<220>
   <221> misc_feature
   <222> (792)..(792)
   <223> n is a, c, g, or t
<220>
   <221> misc_feature
   <222> (807)..(807)
   <223> n is a, c, g, or t
<220>
   <221> misc_feature
   <222> (854)..(854)
   <223> n is a, c, g, or t
<220>
   <221> misc_feature
   <222> (888)..(888)
   <223> n is a, c, g, or t
<220>
   <221> misc_feature
   <222> (891)..(891)
   <223> n is a, c, g, or t
<220>
   <221> misc_feature
   <222> (895)..(895)
   <223> n is a, c, g, or t
<220>
   <221> misc_feature
   <222> (897)..(897)
   <223> n is a, c, g, or t
<220>
   <221> misc_feature
   <222> (906)..(906)
   <223> n is a, c, g, or t
<220>
   <221> misc_feature
   <222> (908)..(908)
   <223> n is a, c, g, or t
<220>
   <221> misc_feature
   <222> (921)..(921)
   <223> n is a, c, g, or t
<220>
   <221> misc_feature
   <222> (963)..(963)
   <223> n is a, c, g, or t
<220>
   <221> misc_feature
   <222> (975)..(975)
   <223> n is a, c, g, or t
<220>
   <221> misc_feature
   <222> (982)..(982)
   <223> n is a, c, g, or t
<220>
   <221> misc_feature
   <222> (989)..(991)
   <223> n is a, c, g, or t
<220>
   <221> misc_feature
   <222> (1006)..(1006)
   <223> n is a, c, g, or t
<220>
   <221> misc_feature
   <222> (1008)..(1008)
   <223> n is a, c, g, or t
<220>
   <221> misc_feature
   <222> (1021)..(1021)
   <223> n is a, c, g, or t
<220>
   <221> misc_feature
   <222> (1024)..(1024)
   <223> n is a, c, g, or t
<220>
   <221> misc_feature
   <222> (1042)..(1042)
   <223> n is a, c, g, or t
<220>
   <221> misc_feature
   <222> (1050)..(1050)
   <223> n is a, c, g, or t
<220>
   <221> misc_feature
   <222> (1053)..(1054)
   <223> n is a, c, g, or t
<220>
   <221> misc_feature
   <222> (1061)..(1061)
   <223> n is a, c, g, or t
<220>
   <221> misc_feature
   <222> (1069)..(1069)
   <223> n is a, c, g, or t
<220>
   <221> misc_feature
   <222> (1071)..(1071)
   <223> n is a, c, g, or t
<220>
   <221> misc_feature
   <222> (1079)..(1079)
   <223> n is a, c, g, or t
<220>
   <221> misc_feature
   <222> (1081)..(1081)
   <223> n is a, c, g, or t
<220>
   <221> misc_feature
   <222> (1104)..(1104)
   <223> n is a, c, g, or t
<220>
   <221> misc_feature
   <222> (1112)..(1112)
   <223> n is a, c, g, or t
<220>
   <221> misc_feature
   <222> (1131)..(1131)
   <223> n is a, c, g, or t
<220>
   <221> misc_feature
   <222> (1149)..(1149)
   <223> n is a, c, g, or t
<220>
   <221> misc_feature
   <222> (1152)..(1152)
   <223> n is a, c, g, or t
<220>
   <221> misc_feature
   <222> (1160)..(1160)
   <223> n is a, c, g, or t
<220>
   <221> misc_feature
   <222> (1190)..(1192)
   <223> n is a, c, g, or t
<220>
   <221> misc_feature
   <222> (1198)..(1198)
   <223> n is a, c, g, or t
<220>
   <221> misc_feature
   <222> (1232)..(1233)
   <223> n is a, c, g, or t
<220>
   <221> misc_feature
   <222> (1239)..(1240)
   <223> n is a, c, g, or t
<220>
   <221> misc_feature
   <222> (1251)..(1251)
   <223> n is a, c, g, or t
<220>
   <221> misc_feature
   <222> (1259)..(1259)
   <223> n is a, c, g, or t
<220>
   <221> misc_feature
   <222> (1264)..(1264)
   <223> n is a, c, g, or t
<220>
   <221> misc_feature
   <222> (1274)..(1274)
   <223> n is a, c, g, or t
<220>
   <221> misc_feature
   <222> (1287)..(1287)
   <223> n is a, c, g, or t
<220>
   <221> misc_feature
   <222> (1291)..(1291)
   <223> n is a, c, g, or t
<220>
   <221> misc_feature
   <222> (1308)..(1308)
   <223> n is a, c, g, or t
<220>
   <221> misc_feature
   <222> (1318)..(1318)
   <223> n is a, c, g, or t
<220>
   <221> misc_feature
   <222> (1325)..(1325)
   <223> n is a, c, g, or t
<220>
   <221> misc_feature
   <222> (1330)..(1330)
   <223> n is a, c, g, or t
<220>
   <221> misc_feature
   <222> (1343)..(1343)
   <223> n is a, c, g, or t
<220>
   <221> misc_feature
   <222> (1398)..(1398)
   <223> n is a, c, g, or t
<220>
   <221> misc_feature
   <222> (1410)..(1411)
   <223> n is a, c, g, or t
<220>
   <221> misc_feature
   <222> (1416)..(1416)
   <223> n is a, c, g, or t
<220>
   <221> misc_feature
   <222> (1420)..(1420)
   <223> n is a, c, g, or t
<220>
   <221> misc_feature
   <222> (1431)..(1434)
   <223> n is a, c, g, or t
<220>
   <221> misc_feature
   <222> (1444)..(1445)
   <223> n is a, c, g, or t
<220>
   <221> misc_feature
   <222> (1449)..(1450)
   <223> n is a, c, g, or t
<220>
   <221> misc_feature
   <222> (1463)..(1463)
   <223> n is a, c, g, or t
<220>
   <221> misc_feature
   <222> (1509)..(1509)
   <223> n is a, c, g, or t
<400> 23
   atggcttcga ctctgggcng cgatgngagn nncgagatag tgtttttcga tcttgagacn      60
   gcngttncga ccnaatcggg ncanccntnt gcgattttgg agtttggngc tatcttagtt     120
   tgccctatga agctagngga gctctatagt tacnccacnt tggntcgacc nacnganctt     180
   tcnctcatct ncacgctcac gaagcgacga agcggcatta cgcgcgacgg agttctctct     240
   gcacctacnt tctctgaaat cgctgatgaa ntctacgana ttcnccncgg acgaatttgg      300
   gnnggacata acataaagag attcgattgt gtaagantan gagatgcatt tgcagnaatt      360
   ggtntcnctc cccnngagnc naaagntnca attgatncac tttcgttnnn gtctcagaag      420
   tttgggaagn gagctggnga cntgaagatg gcntngcntg ctacntattt cgngctagga      480
   gancangctc acaggagctt agatgatgtc cggatgaatc ttgaagtnnt caagnactgt      540
   ncaaccntct tntttctgga gtcnagtgtn ccngacattc ttananncat nagctggttn      600
   tnccnaagaa aaagtcngng aacacgaagt aatgagaagt cactgccnna tggagtcaga      660
   gaaagcccna cttcttcctc ttcnagccct naanctganc cgagttcgtc ttctgtanan      720
   gcnacanctg tcaaaaacca tcccatcatt tctcttctga cggaatgctc agnaanngat      780
   acatctagtt gngaaataga tccatcngac ataaccactc taataagtaa actacatatt      840
   ggaactctta aganagatgc tgcggacgaa gccaaaactg tgagagangc ngcgnangaa      900
   gccaanantg taagacagca nggtgaatca accgatccca atgccaaaga tgaatcattt      960
   ttnggcgtta atgangtatc tntttctann ntcagggcaa gtcttntncc gttatatcgt     1020
   nggngtctga gaatggagct gnttcacaan ganncccctc nacatctcng ntggtatanc     1080
   ntgaaaattc ggtttggaat aagncggaag tntgtggatc atgtaggtcg nccaaagatg     1140
   aatattgtng tngacatacn tcctgattta tgcaagatct tggacgcatn nnatgctnct     1200
   gcgcataact tactgattga ctcaagcaca anntcagann ggaggcctac ngttatgang     1260
   aaanaaggct ttgncaacta tcccacngcc ngactgcaaa taagctcnga atccaatnga     1320
   acccnggtan accaaaaaga agnacctttg ggaaccaatc aaaagctcga tttcagtagc     1380
   gataattttg aaaagctnga gtcagcactn nttccnggtn ccctggttga nnnnttcttc     1440
   tcanncgann cttacgatta tangaaaatg gtagggatac gtctagcagc cagaaagttg     1500
   gtaatccanc tgaagaaatg a                                               1521
<210> 24
   <211> 2393
   <212> DNA
   <213> Boechera
<220>
   <221> misc_feature
   <222> (19)..(19)
   <223> n is a, c, g, or t
<220>
   <221> misc_feature
   <222> (26)..(26)
   <223> n is a, c, g, or t
<220>
   <221> misc_feature
   <222> (30)..(32)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (67)..(67)
   <223> n is a, c, g, or t
<220>
   <221> misc_feature
   <222> (73)..(73)
   <223> n is a, c, g, or t
<220>
   <221> misc_feature
   <222> (81)..(81)
   <223> n is a, c, g, or t
<220>
   <221> misc_feature
   <222> (84)..(84)
   <223> n is a, c, g, or t
<220>
   <221> misc_feature
   <222> (87)..(87)
   <223> n is a, c, g, or t
<220>
   <221> misc_feature
   <222> (89) .. (89)
   <223> n is a, c, g, or t
<220>
   <221> misc_feature
   <222> (108)..(108)
   <223> n is a, c, g, or t
<220>
   <221> misc_feature
   <222> (137)..(137)
   <223> n is a, c, g, or t
<220>
   <221> misc_feature
   <222> (154)..(154)
   <223> n is a, c, g, or t
<220>
   <221> misc_feature
   <222> (159)..(159)
   <223> n is a, c, g, or t
<220>
   <221> misc_feature
   <222> (164)..(164)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (177)..(177)
   <223> n is a, c, g, or t
<220>
   <221> misc_feature
   <222> (183)..(183)
   <223> n is a, c, g, or t
<220>
   <221> misc_feature
   <222> (191)..(191)
   <223> n is a, c, g, or t
<220>
   <221> misc_feature
   <222> (249) .. (249)
   <223> n is a, c, g, or t
<220>
   <221> misc_feature
   <222> (271)..(271)
   <223> n is a, c, g, or t
<220>
   <221> misc_feature
   <222> (279) .. (279)
   <223> n is a, c, g, or t
<220>
   <221> misc_feature
   <222> (284)..(284)
   <223> n is a, c, g, or t
<220>
   <221> misc_feature
   <222> (287)..(287)
   <223> n is a, c, g, or t
<220>
   <221> misc_feature
   <222> (303)..(304)
   <223> n is a, c, g, or t
<220>
   <221> misc_feature
   <222> (312)..(317)
   <223> n is a, c, g, or t
<220>
   <221> misc_feature
   <222> (320)..(321)
   <223> n is a, c, g, or t
<220>
   <221> misc_feature
   <222> (323)..(323)
   <223> n is a, c, g, or t
<220>
   <221> misc_feature
   <222> (325) .. (326)
   <223> n is a, c, g, or t
<220>
   <221> misc_feature
   <222> (334)..(334)
   <223> n is a, c, g, or t
<220>
   <221> misc_feature
   <222> (337)..(337)
   <223> n is a, c, g, or t
<220>
   <221> misc_feature
   <222> (343)..(343)
   <223> n is a, c, g, or t
<220>
   <221> misc_feature
   <222> (346)..(346)
   <223> n is a, c, g, or t
<220>
   <221> misc_feature
   <222> (385)..(386)
   <223> n is a, c, g, or t
<220>
   <221> misc_feature
   <222> (420)..(420)
   <223> n is a, c, g, or t
<220>
   <221> misc_feature
   <222> (423)..(423)
   <223> n is a, c, g, or t
<220>
   <221> misc_feature
   <222> (439)..(439)
   <223> n is a, c, g, or t
<220>
   <221> misc_feature
   <222> (447)..(447)
   <223> n is a, c, g, or t
<220>
   <221> misc_feature
   <222> (450)..(450)
   <223> n is a, c, g, or t
<220>
   <221> misc_feature
   <222> (457)..(458)
   <223> n is a, c, g, or t
<220>
   <221> misc_feature
   <222> (462)..(462)
   <223> n is a, c, g, or t
<220>
   <221> misc_feature
   <222> (464)..(464)
   <223> n is a, c, g, or t
<220>
   <221> misc_feature
   <222> (469)..(469)
   <223> n is a, c, g, or t
<220>
   <221> misc_feature
   <222> (471)..(471)
   <223> n is a, c, g, or t
<220>
   <221> misc_feature
   <222> (480)..(480)
   <223> n is a, c, g, or t
<220>
   <221> misc_feature
   <222> (491)..(493)
   <223> n is a, c, g, or t
<220>
   <221> misc_feature
   <222> (513)..(513)
   <223> n is a, c, g, or t
<220>
   <221> misc_feature
   <222> (521)..(521)
   <223> n is a, c, g, or t
<220>
   <221> misc_feature
   <222> (525)..(525)
   <223> n is a, c, g, or t
<220>
   <221> misc_feature
   <222> (551)..(551)
   <223> n is a, c, g, or t
<220>
   <221> misc_feature
   <222> (572)..(572)
   <223> n is a, c, g, or t
<220>
   <221> misc_feature
   <222> (580)..(580)
   <223> n is a, c, g, or t
<220>
   <221> misc_feature
   <222> (590)..(590)
   <223> n is a, c, g, or t
<220>
   <221> misc_feature
   <222> (608)..(608)
   <223> n is a, c, g, or t
<220>
   <221> misc_feature
   <222> (610)..(610)
   <223> n is a, c, g, or t
<220>
   <221> misc_feature
   <222> (620)..(620)
   <223> n is a, c, g, or t
<220>
   <221> misc_feature
   <222> (638)..(638)
   <223> n is a, c, g, or t
<220>
   <221> misc_feature
   <222> (642)..(642)
   <223> n is a, c, g, or t
<220>
   <221> misc_feature
   <222> (655)..(655)
   <223> n is a, c, g, or t
<220>
   <221> misc_feature
   <222> (658)..(662)
   <223> n is a, c, g, or t
<220>
   <221> misc_feature
   <222> (665)..(665)
   <223> n is a, c, g, or t
<220>
   <221> misc_feature
   <222> (668)..(668)
   <223> n is a, c, g, or t
<220>
   <221> misc_feature
   <222> (678)..(678)
   <223> n is a, c, g, or t
<220>
   <221> misc_feature
   <222> (680)..(680)
   <223> n is a, c, g, or t
<220>
   <221> misc_feature
   <222> (683)..(683)
   <223> n is a, c, g, or t
<220>
   <221> misc_feature
   <222> (690)..(690)
   <223> n is a, c, g, or t
<220>
   <221> misc_feature
   <222> (698)..(698)
   <223> n is a, c, g, or t
<220>
   <221> misc_feature
   <222> (708)..(708)
   <223> n is a, c, g, or t
<220>
   <221> misc_feature
   <222> (711)..(711)
   <223> n is a, c, g, or t
<220>
   <221> misc_feature
   <222> (722)..(722)
   <223> n is a, c, g, or t
<220>
   <221> misc_feature
   <222> (726) .. (727)
   <223> n is a, c, g, or t
<220>
   <221> misc_feature
   <222> (740)..(741)
   <223> n is a, c, g, or t
<220>
   <221> misc_feature
   <222> (760)..(760)
   <223> n is a, c, g, or t
<220>
   <221> misc_feature
   <222> (769)..(769)
   <223> n is a, c, g, or t
<220>
   <221> misc_feature
   <222> (791)..(791)
   <223> n is a, c, g, or t
<220>
   <221> misc_feature
   <222> (794)..(794)
   <223> n is a, c, g, or t
<220>
   <221> misc_feature
   <222> (801)..(801)
   <223> n is a, c, g, or t
<220>
   <221> misc_feature
   <222> (838)..(839)
   <223> n is a, c, g, or t
<220>
   <221> misc_feature
   <222> (845)..(845)
   <223> n is a, c, g, or t
<220>
   <221> misc_feature
   <222> (851)..(851)
   <223> n is a, c, g, or t
<220>
   <221> misc_feature
   <222> (857)..(857)
   <223> n is a, c, g, or t
<220>
   <221> misc_feature
   <222> (862)..(862)
   <223> n is a, c, g, or t
<220>
   <221> misc_feature
   <222> (875)..(875)
   <223> n is a, c, g, or t
<220>
   <221> misc_feature
   <222> (882)..(882)
   <223> n is a, c, g, or t
<220>
   <221> misc_feature
   <222> (902)..(902)
   <223> n is a, c, g, or t
<220>
   <221> misc_feature
   <222> (908)..(908)
   <223> n is a, c, g, or t
<220>
   <221> misc_feature
   <222> (912)..(912)
   <223> n is a, c, g, or t
<220>
   <221> misc_feature
   <222> (915)..(915)
   <223> n is a, c, g, or t
<220>
   <221> misc_feature
   <222> (923)..(923)
   <223> n is a, c, g, or t
<220>
   <221> misc_feature
   <222> (931)..(931)
   <223> n is a, c, g, or t
<220>
   <221> misc_feature
   <222> (934)..(934)
   <223> n is a, c, g, or t
<220>
   <221> misc_feature
   <222> (939)..(940)
   <223> n is a, c, g, or t
<220>
   <221> misc_feature
   <222> (952)..(952)
   <223> n is a, c, g, or t
<220>
   <221> misc_feature
   <222> (961)..(961)
   <223> n is a, c, g, or t
<220>
   <221> misc_feature
   <222> (963)..(963)
   <223> n is a, c, g, or t
<220>
   <221> misc_feature
   <222> (967)..(967)
   <223> n is a, c, g, or t
<220>
   <221> misc_feature
   <222> (970)..(970)
   <223> n is a, c, g, or t
<220>
   <221> misc_feature
   <222> (976)..(977)
   <223> n is a, c, g, or t
<220>
   <221> misc_feature
   <222> (980)..(980)
   <223> n is a, c, g, or t
<220>
   <221> misc_feature
   <222> (983)..(983)
   <223> n is a, c, g, or t
<220>
   <221> misc_feature
   <222> (993)..(993)
   <223> n is a, c, g, or t
<220>
   <221> misc_feature
   <222> (1003)..(1003)
   <223> n is a, c, g, or t
<220>
   <221> misc_feature
   <222> (1025)..(1025)
   <223> n is a, c, g, or t
<220>
   <221> misc_feature
   <222> (1049)..(1050)
   <223> n is a, c, g, or t
<220>
   <221> misc_feature
   <222> (1089)..(1089)
   <223> n is a, c, g, or t
<220>
   <221> misc_feature
   <222> (1095)..(1095)
   <223> n is a, c, g, or t
<220>
   <221> misc_feature
   <222> (1098)..(1098)
   <223> n is a, c, g, or t
<220>
   <221> misc_feature
   <222> (1109)..(1109)
   <223> n is a, c, g, or t
<220>
   <221> misc_feature
   <222> (1111)..(1112)
   <223> n is a, c, g, or t
<220>
   <221> misc_feature
   <222> (1116)..(1116)
   <223> n is a, c, g, or t
<220>
   <221> misc_feature
   <222> (1125)..(1125)
   <223> n is a, c, g, or t
<220>
   <221> misc_feature
   <222> (1127)..(1127)
   <223> n is a, c, g, or t
<220>
   <221> misc_feature
   <222> (1130)..(1130)
   <223> n is a, c, g, or t
<220>
   <221> misc_feature
   <222> (1142)..(1142)
   <223> n is a, c, g, or t
<220>
   <221> misc_feature
   <222> (1144)..(1144)
   <223> n is a, c, g, or t
<220>
   <221> misc_feature
   <222> (1173)..(1174)
   <223> n is a, c, g, or t
<220>
   <221> misc_feature
   <222> (1194)..(1194)
   <223> n is a, c, g, or t
<220>
   <221> misc_feature
   <222> (1209)..(1209)
   <223> n is a, c, g, or t
<220>
   <221> misc_feature
   <222> (1216)..(1216)
   <223> n is a, c, g, or t
<220>
   <221> misc_feature
   <222> (1219)..(1219)
   <223> n is a, c, g, or t
<220>
   <221> misc_feature
   <222> (1224)..(1224)
   <223> n is a, c, g, or t
<220>
   <221> misc_feature
   <222> (1243)..(1243)
   <223> n is a, c, g, or t
<220>
   <221> misc_feature
   <222> (1245)..(1245)
   <223> n is a, c, g, or t
<220>
   <221> misc_feature
   <222> (1248)..(1248)
   <223> n is a, c, g, or t
<220>
   <221> misc_feature
   <222> (1252)..(1252)
   <223> n is a, c, g, or t
<220>
   <221> misc_feature
   <222> (1298)..(1298)
   <223> n is a, c, g, or t
<220>
   <221> misc_feature
   <222> (1301)..(1302)
   <223> n is a, c, g, or t
<220>
   <221> misc_feature
   <222> (1317)..(1317)
   <223> n is a, c, g, or t
<220>
   <221> misc_feature
   <222> (1332)..(1332)
   <223> n is a, c, g, or t
<220>
   <221> misc_feature
   <222> (1379)..(1379)
   <223> n is a, c, g, or t
<220>
   <221> misc_feature
   <222> (1383)..(1383)
   <223> n is a, c, g, or t
<220>
   <221> misc_feature
   <222> (1386)..(1386)
   <223> n is a, c, g, or t
<220>
   <221> misc_feature
   <222> (1390)..(1390)
   <223> n is a, c, g, or t
<220>
   <221> misc_feature
   <222> (1392)..(1392)
   <223> n is a, c, g, or t
<220>
   <221> misc_feature
   <222> (1401)..(1401)
   <223> n is a, c, g, or t
<220>
   <221> misc_feature
   <222> (1403)..(1403)
   <223> n is a, c, g, or t
<220>
   <221> misc_feature
   <222> (1416)..(1416)
   <223> n is a, c, g, or t
<220>
   <221> misc_feature
   <222> (1458)..(1458)
   <223> n is a, c, g, or t
<220>
   <221> misc_feature
   <222> (1470)..(1470)
   <223> n is a, c, g, or t
<220>
   <221> misc_feature
   <222> (1477)..(1477)
   <223> n is a, c, g, or t
<220>
   <221> misc_feature
   <222> (1484)..(1486)
   <223> n is a, c, g, or t
<220>
   <221> misc_feature
   <222> (1501)..(1501)
   <223> n is a, c, g, or t
<220>
   <221> misc_feature
   <222> (1503)..(1503)
   <223> n is a, c, g, or t
<220>
   <221> misc_feature
   <222> (1516)..(1516)
   <223> n is a, c, g, or t
<220>
   <221> misc_feature
   <222> (1519)..(1519)
   <223> n is a, c, g, or t
<220>
   <221> misc_feature
   <222> (1537)..(1537)
   <223> n is a, c, g, or t
<220>
   <221> misc_feature
   <222> (1545)..(1545)
   <223> n is a, c, g, or t
<220>
   <221> misc_feature
   <222> (1548)..(1549)
   <223> n is a, c, g, or t
<220>
   <221> misc_feature
   <222> (1556)..(1556)
   <223> n is a, c, g, or t
<220>
   <221> misc_feature
   <222> (1564)..(1564)
   <223> n is a, c, g, or t
<220>
   <221> misc_feature
   <222> (1566)..(1566)
   <223> n is a, c, g, or t
<220>
   <221> misc_feature
   <222> (1574)..(1574)
   <223> n is a, c, g, or t
<220>
   <221> misc_feature
   <222> (1576)..(1576)
   <223> n is a, c, g, or t
<220>
   <221> misc_feature
   <222> (1599)..(1599)
   <223> n is a, c, g, or t
<220>
   <221> misc_feature
   <222> (1607)..(1607)
   <223> n is a, c, g, or t
<220>
   <221> misc_feature
   <222> (1626)..(1626)
   <223> n is a, c, g, or t
<220>
   <221> misc_feature
   <222> (1644)..(1644)
   <223> n is a, c, g, or t
<220>
   <221> misc_feature
   <222> (1647)..(1647)
   <223> n is a, c, g, or t
<220>
   <221> misc_feature
   <222> (1655)..(1655)
   <223> n is a, c, g, or t
<220>
   <221> misc_feature
   <222> (1685)..(1687)
   <223> n is a, c, g, or t
<220>
   <221> misc_feature
   <222> (1693)..(1693)
   <223> n is a, c, g, or t
<220>
   <221> misc_feature
   <222> (1727)..(1728)
   <223> n is a, c, g, or t
<220>
   <221> misc_feature
   <222> (1734)..(1735)
   <223> n is a, c, g, or t
<220>
   <221> misc_feature
   <222> (1746)..(1746)
   <223> n is a, c, g, or t
<220>
   <221> misc_feature
   <222> (1754)..(1754)
   <223> n is a, c, g, or t
<220>
   <221> misc_feature
   <222> (1759)..(1759)
   <223> n is a, c, g, or t
<220>
   <221> misc_feature
   <222> (1769)..(1769)
   <223> n is a, c, g, or t
<220>
   <221> misc_feature
   <222> (1782)..(1782)
   <223> n is a, c, g, or t
<220>
   <221> misc_feature
   <222> (1786)..(1786)
   <223> n is a, c, g, or t
<220>
   <221> misc_feature
   <222> (1802)..(1802)
   <223> n is a, c, g, or t
<220>
   <221> misc_feature
   <222> (1805)..(1805)
   <223> n is a, c, g, or t
<220>
   <221> misc_feature
   <222> (1817)..(1817)
   <223> n is a, c, g, or t
<220>
   <221> misc_feature
   <222> (1830)..(1830)
   <223> n is a, c, g, or t
<220>
   <221> misc_feature
   <222> (1836)..(1836)
   <223> n is a, c, g, or t
<220>
   <221> misc_feature
   <222> (1841)..(1841)
   <223> n is a, c, g, or t
<220>
   <221> misc_feature
   <222> (1844)..(1844)
   <223> n is a, c, g, or t
<220>
   <221> misc_feature
   <222> (1858)..(1858)
   <223> n is a, c, g, or t
<220>
   <221> misc_feature
   <222> (1871)..(1871)
   <223> n is a, c, g, or t
<220>
   <221> misc_feature
   <222> (1878)..(1879)
   <223> n is a, c, g, or t
<220>
   <221> misc_feature
   <222> (1881)..(1881)
   <223> n is a, c, g, or t
<220>
   <221> misc_feature
   <222> (1888)..(1888)
   <223> n is a, c, g, or t
<220>
   <221> misc_feature
   <222> (1894)..(1894)
   <223> n is a, c, g, or t
<220>
   <221> misc_feature
   <222> (1916)..(1916)
   <223> n is a, c, g, or t
<220>
   <221> misc_feature
   <222> (1921)..(1922)
   <223> n is a, c, g, or t
<220>
   <221> misc_feature
   <222> (1934)..(1936)
   <223> n is a, c, g, or t
<220>
   <221> misc_feature
   <222> (1946)..(1947)
   <223> n is a, c, g, or t
<220>
   <221> misc_feature
   <222> (1969)..(1969)
   <223> n is a, c, g, or t
<220>
   <221> misc_feature
   <222> (1973)..(1973)
   <223> n is a, c, g, or t
<220>
   <221> misc_feature
   <222> (1978)..(1978)
   <223> n is a, c, g, or t
<220>
   <221> misc_feature
   <222> (1990)..(1990)
   <223> n is a, c, g, or t
<220>
   <221> misc_feature
   <222> (2007)..(2007)
   <223> n is a, c, g, or t
<220>
   <221> misc_feature
   <222> (2022)..(2022)
   <223> n is a, c, g, or t
<220>
   <221> misc_feature
   <222> (2034)..(2034)
   <223> n is a, c, g, or t
<220>
   <221> misc_feature
   <222> (2049) .. (2049)
   <223> n is a, c, g, or t
<220>
   <221> misc_feature
   <222> (2058)..(2058)
   <223> n is a, c, g, or t
<220>
   <221> misc_feature
   <222> (2062)..(2062)
   <223> n is a, c, g, or t
<220>
   <221> misc_feature
   <222> (2073)..(2073)
   <223> n is a, c, g, or t
<220>
   <221> misc_feature
   <222> (2079)..(2081)
   <223> n is a, c, g, or t
<220>
   <221> misc_feature
   <222> (2090)..(2091)
   <223> n is a, c, g, or t
<220>
   <221> misc_feature
   <222> (2093)..(2093)
   <223> n is a, c, g, or t
<220>
   <221> misc_feature
   <222> (2095)..(2095)
   <223> n is a, c, g, or t
<220>
   <221> misc_feature
   <222> (2098)..(2098)
   <223> n is a, c, g, or t
<220>
   <221> misc_feature
   <222> (2101)..(2101)
   <223> n is a, c, g, or t
<220>
   <221> misc_feature
   <222> (2103)..(2103)
   <223> n is a, c, g, or t
<220>
   <221> misc_feature
   <222> (2105)..(2105)
   <223> n is a, c, g, or t
<220>
   <221> misc_feature
   <222> (2113)..(2113)
   <223> n is a, c, g, or t
<220>
   <221> misc_feature
   <222> (2125)..(2125)
   <223> n is a, c, g, or t
<220>
   <221> misc_feature
   <222> (2135)..(2135)
   <223> n is a, c, g, or t
<220>
   <221> misc_feature
   <222> (2142)..(2142)
   <223> n is a, c, g, or t
<220>
   <221> misc_feature
   <222> (2147)..(2147)
   <223> n is a, c, g, or t
<220>
   <221> misc_feature
   <222> (2160)..(2160)
   <223> n is a, c, g, or t
<220>
   <221> misc_feature
   <222> (2215)..(2215)
   <223> n is a, c, g, or t
<220>
   <221> misc_feature
   <222> (2227)..(2228)
   <223> n is a, c, g, or t
<220>
   <221> misc_feature
   <222> (2233)..(2233)
   <223> n is a, c, g, or t
<220>
   <221> misc_feature
   <222> (2237)..(2237)
   <223> n is a, c, g, or t
<220>
   <221> misc_feature
   <222> (2248)..(2251)
   <223> n is a, c, g, or t
<220>
   <221> misc_feature
   <222> (2261)..(2262)
   <223> n is a, c, g, or t
<220>
   <221> misc_feature
   <222> (2266)..(2267)
   <223> n is a, c, g, or t
<220>
   <221> misc_feature
   <222> (2280)..(2280)
   <223> n is a, c, g, or t
<220>
   <221> misc_feature
   <222> (2326)..(2326)
   <223> n is a, c, g, or t
<220>
   <221> misc_feature
   <222> (2362)..(2362)
   <223> n is a, c, g, or t
<220>
   <221> misc_feature
   <222> (2372)..(2372)
   <223> n is a, c, g, or t
<220>
   <221> misc_feature
   <222> (2376)..(2376)
   <223> n is a, c, g, or t
<220>
   <221> misc_feature
   <222> (2378)..(2378)
   <223> n is a, c, g, or t
<220>
   <221> misc_feature
   <222> (2385)..(2385)
   <223> n is a, c, g, or t
<220>
   <221> misc_feature
   <222> (2387)..(2387)
   <223> n is a, c, g, or t
<220>
   <221> misc_feature
   <222> (2389) .. (2389)
   <223> n is a, c, g, or t
<220>
   <221> misc_feature
   <222> (2391)..(2393)
   <223> n is a, c, g, or t
<400> 24
   atggcttcga ctctgggcng cgatgngagn nncgagatag tgtttttcga tcttgagacn 60
   gcngttncga ccnaatcggg ncanccntnt gcgattttgg agtttggngc tatcttagtt 120
   tgccctatga agctagngga gctctatagt tacnccacnt tggntcgacc nacnganctt 180
   tcnctcatct ncacgctcac gaagcgacga agcggcatta cgcgcgacgg agttctctct 240
   gcacctacnt tctctgaaat cgctgatgaa ntctacgana ttcnccncgg taagggtttc 300
   tcnntttttt tnnnnnnctn ncncnntctc tctnacncga agntanaagt attgattttg 360
   gtgtttctgt aggacgaatt tgggnnggac ataacataaa gagattcgat tgtgtaagan 420
   tangagatgc atttgcagna attggtntcn ctccccnnga gncnaaagnt ncaattgatn 480
   cactttcgtt nnngtctcag aagtttggga agngagctgg ngacntgaag gtctctcttt 540
   tttcgtcttc ncgatgataa atctcaaagc cnatagcttn cttgttatcn ttatagatat 600
   gaatttcnan gtaacttcan agattcatca ctcatcanag tngctaaaat ttacnctnnn 660
   nnaanaangt agatggcntn gcntgctacn tatttcgngc taggaganca ngctcacagg 720
   tnaaannagt aaacgatacn ntgtgccttt taacgattcn ccagttgtnt caatatggga 780
   ctaaacatgg ntangattca ncaggagctt agatgatgtc cggatgaatc ttgaagtnnt 840
   caagnactgt ncaaccntct tntttctggt attgntgtct tntcatttct tgaataatga 900
   tnaactcnta anttnaaaag gantagatta nagnggttnn gacatatctg anttctgtct 960
   ncngttntgn aaaagnnggn tcnatcttcc ttncagacca canctttgca agccgtaaac 1020
   atggnttgca acttgcaagt atagtttgnn atatcactga gtttaagtac ttggtgtttg 1080
   caggagtcna gtgtnccnga cattcttana nncatnagct ggttntnccn aagaaaaagt 1140
   cngngaacac gaagtaatga gaagtcactg ccnnatggag tcagagaaag cccnacttct 1200
   tcctcttcna gccctnaanc tganccgagt tcgtcttctg tanangcnac anctgtcaaa 1260
   aaccatccca tcatttctct tctgacggaa tgctcagnaa nngatacatc tagttgngaa 1320
   atagatccat cngacataac cactctaata agtaaactac atattggaac tcttaagana 1380
   gangcngcgn angaagccaa nantgtaaga cagcanggtg aatcaaccga tcccaatgcc 1440
   aaagatgaat catttttngg cgttaatgan gtatctnttt ctannntcag ggcaagtctt    1500
   ntnccgttat atcgtnggng tctgagaatg gagctgnttc acaangannc ccctcnacat    1560
   ctcngntggt atancntgaa aattcggttt ggaataagnc ggaagtntgt ggatcatgta 1620
   ggtcgnccaa agatgaatat tgtngtngac atacntcctg atttatgcaa gatcttggac 1680
   gcatnnnatg ctnctgcgca taacttactg attgactcaa gcacaanntc agannggagg 1740
   cctacngtta tgangaaana aggctttgnc aactatccca cngccngact gcagtaagta 1800
   tncancactc tctctgncct tttacatacn agcatnaatc nacnggagag tctctaanac 1860
   catctccaac nctactcnnt nttcaccncc aaantctatt ttggagttaa atcccnccaa 1920
   nncttgcaaa atannnatct tcaaannttt tctccatatt tggagattnt ganttttnaa 1980
   gtcatgactn cattttggag ttgggtngga gaaaaacaca antccaaaat aganttactt 2040
   cattttggng taaaaaantg angaaatggg ttngagatnn nctaacctcn ntnancantc 2100
   ntntnttgtt ggnagaataa gctcngaatc caatngaacc cnggtanacc aaaaagaagn 2160
   acctttggga accaatcaaa agctcgattt cagtagcgat aattttgaaa agctngagtc 2220
   agcactnntt ccnggtnccc tggttgannn nttcttctca nncgannctt acgattatan 2280
   gaaaatggta gggatacgtc tagcagccag aaagttggta atccanctga agaaatgatc 2340
   tagccaagga aaaatcattc cnctgtctct tnctgntnag tcggngngna nnn 2393
<210> 25
   <211> 1491
   <212> DNA
   <213> Boechera
<220>
   <221> misc_feature
   <222> (19)..(19)
   <223> n is a, c, g, or t
<220>
   <221> misc_feature
   <222> (26)..(26)
   <223> n is a, c, g, or t
<220>
   <221> misc_feature
   <222> (30)..(32)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (67)..(67)
   <223> n is a, c, g, or t
<220>
   <221> misc_feature
   <222> (73)..(73)
   <223> n is a, c, g, or t
<220>
   <221> misc_feature
   <222> (81)..(81)
   <223> n is a, c, g, or t
<220>
   <221> misc_feature
   <222> (84)..(84)
   <223> n is a, c, g, or t
<220>
   <221> misc_feature
   <222> (87)..(87)
   <223> n is a, c, g, or t
<220>
   <221> misc_feature
   <222> (89) .. (89)
   <223> n is a, c, g, or t
<220>
   <221> misc_feature
   <222> (108)..(108)
   <223> n is a, c, g, or t
<220>
   <221> misc_feature
   <222> (137)..(137)
   <223> n is a, c, g, or t
<220>
   <221> misc_feature
   <222> (154)..(154)
   <223> n is a, c, g, or t
<220>
   <221> misc_feature
   <222> (159)..(159)
   <223> n is a, c, g, or t
<220>
   <221> misc_feature
   <222> (164)..(164)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (177)..(177)
   <223> n is a, c, g, or t
<220>
   <221> misc_feature
   <222> (183)..(183)
   <223> n is a, c, g, or t
<220>
   <221> misc_feature
   <222> (191)..(191)
   <223> n is a, c, g, or t
<220>
   <221> misc_feature
   <222> (249) .. (249)
   <223> n is a, c, g, or t
<220>
   <221> misc_feature
   <222> (271)..(271)
   <223> n is a, c, g, or t
<220>
   <221> misc_feature
   <222> (279) .. (279)
   <223> n is a, c, g, or t
<220>
   <221> misc_feature
   <222> (284)..(284)
   <223> n is a, c, g, or t
<220>
   <221> misc_feature
   <222> (287)..(287)
   <223> n is a, c, g, or t
<220>
   <221> misc_feature
   <222> (302)..(303)
   <223> n is a, c, g, or t
<220>
   <221> misc_feature
   <222> (337)..(337)
   <223> n is a, c, g, or t
<220>
   <221> misc_feature
   <222> (340)..(340)
   <223> n is a, c, g, or t
<220>
   <221> misc_feature
   <222> (356)..(356)
   <223> n is a, c, g, or t
<220>
   <221> misc_feature
   <222> (364)..(364)
   <223> n is a, c, g, or t
<220>
   <221> misc_feature
   <222> (367)..(367)
   <223> n is a, c, g, or t
<220>
   <221> misc_feature
   <222> (374)..(375)
   <223> n is a, c, g, or t
<220>
   <221> misc_feature
   <222> (379)..(379)
   <223> n is a, c, g, or t
<220>
   <221> misc_feature
   <222> (381)..(381)
   <223> n is a, c, g, or t
<220>
   <221> misc_feature
   <222> (386)..(386)
   <223> n is a, c, g, or t
<220>
   <221> misc_feature
   <222> (388)..(388)
   <223> n is a, c, g, or t
<220>
   <221> misc_feature
   <222> (397)..(397)
   <223> n is a, c, g, or t
<220>
   <221> misc_feature
   <222> (408)..(410)
   <223> n is a, c, g, or t
<220>
   <221> misc_feature
   <222> (430)..(430)
   <223> n is a, c, g, or t
<220>
   <221> misc_feature
   <222> (438)..(438)
   <223> n is a, c, g, or t
<220>
   <221> misc_feature
   <222> (442)..(442)
   <223> n is a, c, g, or t
<220>
   <221> misc_feature
   <222> (453)..(453)
   <223> n is a, c, g, or t
<220>
   <221> misc_feature
   <222> (455)..(455)
   <223> n is a, c, g, or t
<220>
   <221> misc_feature
   <222> (458)..(458)
   <223> n is a, c, g, or t
<220>
   <221> misc_feature
   <222> (465)..(465)
   <223> n is a, c, g, or t
<220>
   <221> misc_feature
   <222> (473)..(473)
   <223> n is a, c, g, or t
<220>
   <221> misc_feature
   <222> (483)..(483)
   <223> n is a, c, g, or t
<220>
   <221> misc_feature
   <222> (486)..(486)
   <223> n is a, c, g, or t
<220>
   <221> misc_feature
   <222> (528) .. (529)
   <223> n is a, c, g, or t
<220>
   <221> misc_feature
   <222> (535)..(535)
   <223> n is a, c, g, or t
<220>
   <221> misc_feature
   <222> (541)..(541)
   <223> n is a, c, g, or t
<220>
   <221> misc_feature
   <222> (547)..(547)
   <223> n is a, c, g, or t
<220>
   <221> misc_feature
   <222> (552)..(552)
   <223> n is a, c, g, or t
<220>
   <221> misc_feature
   <222> (564)..(564)
   <223> n is a, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (584)..(584)
   <223> n is a, c, g, or t
<220>
   <221> misc_feature
   <222> (586)..(587)
   <223> n is a, c, g, or t
<220>
   <221> misc_feature
   <222> (591)..(591)
   <223> n is a, c, g, or t
<220>
   <221> misc_feature
   <222> (600)..(600)
   <223> n is a, c, g, or t
<220>
   <221> misc_feature
   <222> (602)..(602)
   <223> n is a, c, g, or t
<220>
   <221> misc_feature
   <222> (605)..(605)
   <223> n is a, c, g, or t
<220>
   <221> misc_feature
   <222> (617)..(617)
   <223> n is a, c, g, or t
<220>
   <221> misc_feature
   <222> (619)..(619)
   <223> n is a, c, g, or t
<220>
   <221> misc_feature
   <222> (648)..(649)
   <223> n is a, c, g, or t
<220>
   <221> misc_feature
   <222> (669)..(669)
   <223> n is a, c, g, or t
<220>
   <221> misc_feature
   <222> (684)..(684)
   <223> n is a, c, g, or t
<220>
   <221> misc_feature
   <222> (691)..(691)
   <223> n is a, c, g, or t
<220>
   <221> misc_feature
   <222> (694)..(694)
   <223> n is a, c, g, or t
<220>
   <221> misc_feature
   <222> (699)..(699)
   <223> n is a, c, g, or t
<220>
   <221> misc_feature
   <222> (718)..(718)
   <223> n is a, c, g, or t
<220>
   <221> misc_feature
   <222> (720)..(720)
   <223> n is a, c, g, or t
<220>
   <221> misc_feature
   <222> (723)..(723)
   <223> n is a, c, g, or t
<220>
   <221> misc_feature
   <222> (727)..(727)
   <223> n is a, c, g, or t
<220>
   <221> misc_feature
   <222> (773)..(773)
   <223> n is a, c, g, or t
<220>
   <221> misc_feature
   <222> (776)..(777)
   <223> n is a, c, g, or t
<220>
   <221> misc_feature
   <222> (792)..(792)
   <223> n is a, c, g, or t
<220>
   <221> misc_feature
   <222> (807)..(807)
   <223> n is a, c, g, or t
<220>
   <221> misc_feature
   <222> (854)..(854)
   <223> n is a, c, g, or t
<220>
   <221> misc_feature
   <222> (858)..(858)
   <223> n is a, c, g, or t
<220>
   <221> misc_feature
   <222> (861)..(861)
   <223> n is a, c, g, or t
<220>
   <221> misc_feature
   <222> (865)..(865)
   <223> n is a, c, g, or t
<220>
   <221> misc_feature
   <222> (867)..(867)
   <223> n is a, c, g, or t
<220>
   <221> misc_feature
   <222> (876)..(876)
   <223> n is a, c, g, or t
<220>
   <221> misc_feature
   <222> (878)..(878)
   <223> n is a, c, g, or t
<220>
   <221> misc_feature
   <222> (891)..(891)
   <223> n is a, c, g, or t
<220>
   <221> misc_feature
   <222> (933)..(933)
   <223> n is a, c, g, or t
<220>
   <221> misc_feature
   <222> (945)..(945)
   <223> n is a, c, g, or t
<220>
   <221> misc_feature
   <222> (952)..(952)
   <223> n is a, c, g, or t
<220>
   <221> misc_feature
   <222> (959)..(961)
   <223> n is a, c, g, or t
<220>
   <221> misc_feature
   <222> (976) .. (976)
   <223> n is a, c, g, or t
<220>
   <221> misc_feature
   <222> (978)..(978)
   <223> n is a, c, g, or t
<220>
   <221> misc_feature
   <222> (991)..(991)
   <223> n is a, c, g, or t
<220>
   <221> misc_feature
   <222> (994)..(994)
   <223> n is a, c, g, or t
<220>
   <221> misc_feature
   <222> (1012)..(1012)
   <223> n is a, c, g, or t
<220>
   <221> misc_feature
   <222> (1020)..(1020)
   <223> n is a, c, g, or t
<220>
   <221> misc_feature
   <222> (1023)..(1024)
   <223> n is a, c, g, or t
<220>
   <221> misc_feature
   <222> (1031)..(1031)
   <223> n is a, c, g, or t
<220>
   <221> misc_feature
   <222> (1039)..(1039)
   <223> n is a, c, g, or t
<220>
   <221> misc_feature
   <222> (1041)..(1041)
   <223> n is a, c, g, or t
<220>
   <221> misc_feature
   <222> (1049)..(1049)
   <223> n is a, c, g, or t
<220>
   <221> misc_feature
   <222> (1051)..(1051)
   <223> n is a, c, g, or t
<220>
   <221> misc_feature
   <222> (1074)..(1074)
   <223> n is a, c, g, or t
<220>
   <221> misc_feature
   <222> (1082)..(1082)
   <223> n is a, c, g, or t
<220>
   <221> misc_feature
   <222> (1101)..(1101)
   <223> n is a, c, g, or t
<220>
   <221> misc_feature
   <222> (1119)..(1119)
   <223> n is a, c, g, or t
<220>
   <221> misc_feature
   <222> (1122)..(1122)
   <223> n is a, c, g, or t
<220>
   <221> misc_feature
   <222> (1130)..(1130)
   <223> n is a, c, g, or t
<220>
   <221> misc_feature
   <222> (1160)..(1162)
   <223> n is a, c, g, or t
<220>
   <221> misc_feature
   <222> (1168)..(1168)
   <223> n is a, c, g, or t
<220>
   <221> misc_feature
   <222> (1202)..(1203)
   <223> n is a, c, g, or t
<220>
   <221> misc_feature
   <222> (1209)..(1210)
   <223> n is a, c, g, or t
<220>
   <221> misc_feature
   <222> (1221)..(1221)
   <223> n is a, c, g, or t
<220>
   <221> misc_feature
   <222> (1229)..(1229)
   <223> n is a, c, g, or t
<220>
   <221> misc_feature
   <222> (1234)..(1234)
   <223> n is a, c, g, or t
<220>
   <221> misc_feature
   <222> (1244)..(1244)
   <223> n is a, c, g, or t
<220>
   <221> misc_feature
   <222> (1257)..(1257)
   <223> n is a, c, g, or t
<220>
   <221> misc_feature
   <222> (1261)..(1261)
   <223> n is a, c, g, or t
<220>
   <221> misc_feature
   <222> (1278)..(1278)
   <223> n is a, c, g, or t
<220>
   <221> misc_feature
   <222> (1288)..(1288)
   <223> n is a, c, g, or t
<220>
   <221> misc_feature
   <222> (1295)..(1295)
   <223> n is a, c, g, or t
<220>
   <221> misc_feature
   <222> (1300)..(1300)
   <223> n is a, c, g, or t
<220>
   <221> misc_feature
   <222> (1313)..(1313)
   <223> n is a, c, g, or t
<220>
   <221> misc_feature
   <222> (1368)..(1368)
   <223> n is a, c, g, or t
<220>
   <221> misc_feature
   <222> (1380)..(1381)
   <223> n is a, c, g, or t
<220>
   <221> misc_feature
   <222> (1386)..(1386)
   <223> n is a, c, g, or t
<220>
   <221> misc_feature
   <222> (1390)..(1390)
   <223> n is a, c, g, or t
<220>
   <221> misc_feature
   <222> (1401)..(1404)
   <223> n is a, c, g, or t
<220>
   <221> misc_feature
   <222> (1414)..(1415)
   <223> n is a, c, g, or t
<220>
   <221> misc_feature
   <222> (1419)..(1420)
   <223> n is a, c, g, or t
<220>
   <221> misc_feature
   <222> (1433)..(1433)
   <223> n is a, c, g, or t
<220>
   <221> misc_feature
   <222> (1479)..(1479)
   <223> n is a, c, g, or t
<400> 25
   atggcttcga ctctgggcng cgatgngagn nncgagatag tgtttttcga tcttgagacn      60
   gcngttncga ccnaatcggg ncanccntnt gcgattttgg agtttggngc tatcttagtt     120
   tgccctatga agctagngga gctctatagt tacnccacnt tggntcgacc nacnganctt     180
   tcnctcatct ncacgctcac gaagcgacga agcggcatta cgcgcgacgg agttctctct     240
   gcacctacnt tctctgaaat cgctgatgaa ntctacgana ttcnccncgg acgaatttgg     300
   gnnggacata acataaagag attcgattgt gtaagantan gagatgcatt tgcagnaatt     360
   ggtntcnctc cccnngagnc naaagntnca attgatncac tttcgttnnn gtctcagaag     420
   tttgggaagn gagctggnga cntgaagatg gcntngcntg ctacntattt cgngctagga     480
   gancangctc acaggagctt agatgatgtc cggatgaatc ttgaagtnnt caagnactgt     540
   ncaaccntct tntttctgga gtcnagtgtn ccngacattc ttananncat nagctggttn     600
   tnccnaagaa aaagtcngng aacacgaagt aatgagaagt cactgccnna tggagtcaga     660
   gaaagcccna cttcttcctc ttcnagccct naanctganc cgagttcgtc ttctgtanan     720
   gcnacanctg tcaaaaacca tcccatcatt tctcttctga cggaatgctc agnaanngat     780
   acatctagtt gngaaataga tccatcngac ataaccactc taataagtaa actacatatt     840
   ggaactctta aganagangc ngcgnangaa gccaanantg taagacagca nggtgaatca     900
   accgatccca atgccaaaga tgaatcattt ttnggcgtta atgangtatc tntttctann     960
   ntcagggcaa gtcttntncc gttatatcgt nggngtctga gaatggagct gnttcacaan    1020
   ganncccctc nacatctcng ntggtatanc ntgaaaattc ggtttggaat aagncggaag    1080
   tntgtggatc atgtaggtcg nccaaagatg aatattgtng tngacatacn tcctgattta    1140
   tgcaagatct tggacgcatn nnatgctnct gcgcataact tactgattga ctcaagcaca    1200
   anntcagann ggaggcctac ngttatgang aaanaaggct ttgncaacta tcccacngcc    1260
   ngactgcaaa taagctcnga atccaatnga acccnggtan accaaaaaga agnacctttg    1320
   ggaaccaatc aaaagctcga tttcagtagc gataattttg aaaagctnga gtcagcactn    1380
   nttccnggtn ccctggttga nnnnttcttc tcanncgann cttacgatta tangaaaatg    1440
   gtagggatac gtctagcagc cagaaagttg gtaatccanc tgaagaaatg a             1491
<210> 26
   <211> 495
   <212> DNA
   <213> Boechera
<220>
   <221> misc_feature
   <222> (21) .. (21)
   <223> n is a, c, g, or t
<220>
   <221> misc_feature
   <222> (24)..(24)
   <223> n is a, c, g, or t
<220>
   <221> misc_feature
   <222> (28)..(28)
   <223> n is a, c, g, or t
<220>
   <221> misc_feature
   <222> (34)..(34)
   <223> n is a, c, g, or t
<220>
   <221> misc_feature
   <222> (42)..(42)
   <223> n is a, c, g, or t
<220>
   <221> misc_feature
   <222> (45)..(45)
   <223> n is a, c, g, or t
<220>
   <221> misc_feature
   <222> (48)..(48)
   <223> n is a, c, g, or t
<220>
   <221> misc_feature
   <222> (50)..(50)
   <223> n is a, c, g, or t
<220>
   <221> misc_feature
   <222> (69) .. (69)
   <223> n is a, c, g, or t
<220>
   <221> misc_feature
   <222> (98)..(98)
   <223> n is a, c, g, or t
<220>
   <221> misc_feature
   <222> (115)..(115)
   <223> n is a, c, g, or t
<220>
   <221> misc_feature
   <222> (120)..(120)
   <223> n is a, c, g, or t
<220>
   <221> misc_feature
   <222> (125)..(125)
   <223> n is a, c, g, or t
<220>
   <221> misc_feature
   <222> (132)..(132)
   <223> n is a, c, g, or t
<220>
   <221> misc_feature
   <222> (135)..(135)
   <223> n is a, c, g, or t
<220>
   <221> misc_feature
   <222> (138)..(138)
   <223> n is a, c, g, or t
<220>
   <221> misc_feature
   <222> (144)..(144)
   <223> n is a, c, g, or t
<220>
   <221> misc_feature
   <222> (152)..(152)
   <223> n is a, c, g, or t
<220>
   <221> misc_feature
   <222> (210)..(210)
   <223> n is a, c, g, or t
<220>
   <221> misc_feature
   <222> (232) .. (232)
   <223> n is a, c, g, or t
<220>
   <221> misc_feature
   <222> (240)..(240)
   <223> n is a, c, g, or t
<220>
   <221> misc_feature
   <222> (245)..(245)
   <223> n is a, c, g, or t
<220>
   <221> misc_feature
   <222> (248)..(248)
   <223> n is a, c, g, or t
<220>
   <221> misc_feature
   <222> (263) .. (264)
   <223> n is a, c, g, or t
<220>
   <221> misc_feature
   <222> (298) .. (298)
   <223> n is a, c, g, or t
<220>
   <221> misc_feature
   <222> (301)..(301)
   <223> n is a, c, g, or t
<220>
   <221> misc_feature
   <222> (317)..(317)
   <223> n is a, c, g, or t
<220>
   <221> misc_feature
   <222> (325)..(325)
   <223> n is a, c, g, or t
<220>
   <221> misc_feature
   <222> (328)..(328)
   <223> n is a, c, g, or t
<220>
   <221> misc_feature
   <222> (335)..(336)
   <223> n is a, c, g, or t
<220>
   <221> misc_feature
   <222> (340)..(340)
   <223> n is a, c, g, or t
<220>
   <221> misc_feature
   <222> (342)..(342)
   <223> n is a, c, g, or t
<220>
   <221> misc_feature
   <222> (347)..(347)
   <223> n is a, c, g, or t
<220>
   <221> misc_feature
   <222> (349)..(349)
   <223> n is a, c, g, or t
<220>
   <221> misc_feature
   <222> (358)..(358)
   <223> n is a, c, g, or t
<220>
   <221> misc_feature
   <222> (369)..(371)
   <223> n is a, c, g, or t
<220>
   <221> misc_feature
   <222> (391)..(391)
   <223> n is a, c, g, or t
<220>
   <221> misc_feature
   <222> (399)..(399)
   <223> n is a, c, g, or t
<220>
   <221> misc_feature
   <222> (403)..(403)
   <223> n is a, c, g, or t
<220>
   <221> misc_feature
   <222> (414)..(414)
   <223> n is a, c, g, or t
<220>
   <221> misc_feature
   <222> (416)..(416)
   <223> n is a, c, g, or t
<220>
   <221> misc_feature
   <222> (419)..(419)
   <223> n is a, c, g, or t
<220>
   <221> misc_feature
   <222> (426) .. (426)
   <223> n is a, c, g, or t
<220>
   <221> misc_feature
   <222> (434)..(434)
   <223> n is a, c, g, or t
<220>
   <221> misc_feature
   <222> (444)..(444)
   <223> n is a, c, g, or t
<220>
   <221> misc_feature
   <222> (447)..(447)
   <223> n is a, c, g, or t
<220>
   <221> misc_feature
   <222> (489)..(490)
   <223> n is a, c, g, or t
<400> 26
   gtgtttttcg atcttgagac ngcngttncg accnaatcgg gncanccntn tgcgattttg      60
   gagtttggng ctatcttagt ttgccctatg aagctagngg agctctatag ttacnccacn     120
   ttggntcgac cnacnganct ttcnctcatc tncacgctca cgaagcgacg aagcggcatt     180
   acgcgcgacg gagttctctc tgcacctacn ttctctgaaa tcgctgatga antctacgan     240
   attcnccncg gacgaatttg ggnnggacat aacataaaga gattcgattg tgtaaganta     300
   ngagatgcat ttgcagnaat tggtntcnct ccccnngagn cnaaagntnc aattgatnca     360
   ctttcgttnn ngtctcagaa gtttgggaag ngagctggng acntgaagat ggcntngcnt     420
   gctacntatt tcgngctagg agancangct cacaggagct tagatgatgt ccggatgaat     480
   cttgaagtnn tcaag                                                      495
<210> 27
   <211> 2536
   <212> DNA
   <213> Boechera
<220>
   <221> misc_feature
   <222> (107)..(107)
   <223> n is a, c, g, or t
<220>
   <221> misc_feature
   <222> (141)..(141)
   <223> n is a, c, g, or t
<220>
   <221> misc_feature
   <222> (175)..(175)
   <223> n is a, c, g, or t
<220>
   <221> misc_feature
   <222> (199)..(199)
   <223> n is a, c, g, or t
<220>
   <221> misc_feature
   <222> (204)..(204)
   <223> n is a, c, g, or t
<220>
   <221> misc_feature
   <222> (274)..(274)
   <223> n is a, c, g, or t
<220>
   <221> misc_feature
   <222> (286)..(286)
   <223> n is a, c, g, or t
<220>
   <221> misc_feature
   <222> (292)..(292)
   <223> n is a, c, g, or t
<220>
   <221> misc_feature
   <222> (402)..(402)
   <223> n is a, c, g, or t
<220>
   <221> misc_feature
   <222> (427)..(432)
   <223> n is a, c, g, or t
<220>
   <221> misc_feature
   <222> (435)..(436)
   <223> n is a, c, g, or t
<220>
   <221> misc_feature
   <222> (440)..(440)
   <223> n is a, c, g, or t
<220>
   <221> misc_feature
   <222> (449)..(449)
   <223> n is a, c, g, or t
<220>
   <221> misc_feature
   <222> (452)..(452)
   <223> n is a, c, g, or t
<220>
   <221> misc_feature
   <222> (458)..(458)
   <223> n is a, c, g, or t
<220>
   <221> misc_feature
   <222> (500)..(501)
   <223> n is a, c, g, or t
<220>
   <221> misc_feature
   <222> (538)..(538)
   <223> n is a, c, g, or t
<220>
   <221> misc_feature
   <222> (554)..(554)
   <223> n is a, c, g, or t
<220>
   <221> misc_feature
   <222> (565)..(565)
   <223> n is a, c, g, or t
<220>
   <221> misc_feature
   <222> (606)..(607)
   <223> n is a, c, g, or t
<220>
   <221> misc_feature
   <222> (664)..(664)
   <223> n is a, c, g, or t
<220>
   <221> misc_feature
   <222> (687)..(687)
   <223> n is a, c, g, or t
<220>
   <221> misc_feature
   <222> (695)..(695)
   <223> n is a, c, g, or t
<220>
   <221> misc_feature
   <222> (723)..(723)
   <223> n is a, c, g, or t
<220>
   <221> misc_feature
   <222> (735)..(735)
   <223> n is a, c, g, or t
<220>
   <221> misc_feature
   <222> (773)..(777)
   <223> n is a, c, g, or t
<220>
   <221> misc_feature
   <222> (780)..(780)
   <223> n is a, c, g, or t
<220>
   <221> misc_feature
   <222> (783)..(783)
   <223> n is a, c, g, or t
<220>
   <221> misc_feature
   <222> (798)..(798)
   <223> n is a, c, g, or t
<220>
   <221> misc_feature
   <222> (812)..(812)
   <223> n is a, c, g, or t
<220>
   <221> misc_feature
   <222> (826) .. (826)
   <223> n is a, c, g, or t
<220>
   <221> misc_feature
   <222> (837)..(837)
   <223> n is a, c, g, or t
<220>
   <221> misc_feature
   <222> (841)..(842)
   <223> n is a, c, g, or t
<220>
   <221> misc_feature
   <222> (856)..(856)
   <223> n is a, c, g, or t
<220>
   <221> misc_feature
   <222> (906)..(906)
   <223> n is a, c, g, or t
<220>
   <221> misc_feature
   <222> (909)..(909)
   <223> n is a, c, g, or t
<220>
   <221> misc_feature
   <222> (953)..(954)
   <223> n is a, c, g, or t
<220>
   <221> misc_feature
   <222> (960)..(960)
   <223> n is a, c, g, or t
<220>
   <221> misc_feature
   <222> (966)..(966)
   <223> n is a, c, g, or t
<220>
   <221> misc_feature
   <222> (977)..(977)
   <223> n is a, c, g, or t
<220>
   <221> misc_feature
   <222> (990)..(990)
   <223> n is a, c, g, or t
<220>
   <221> misc_feature
   <222> (997)..(997)
   <223> n is a, c, g, or t
<220>
   <221> misc_feature
   <222> (1017)..(1017)
   <223> n is a, c, g, or t
<220>
   <221> misc_feature
   <222> (1026)..(1026)
   <223> n is a, c, g, or t
<220>
   <221> misc_feature
   <222> (1029)..(1029)
   <223> n is a, c, g, or t
<220>
   <221> misc_feature
   <222> (1045)..(1045)
   <223> n is a, c, g, or t
<220>
   <221> misc_feature
   <222> (1052)..(1052)
   <223> n is a, c, g, or t
<220>
   <221> misc_feature
   <222> (1054)..(1054)
   <223> n is a, c, g, or t
<220>
   <221> misc_feature
   <222> (1066)..(1066)
   <223> n is a, c, g, or t
<220>
   <221> misc_feature
   <222> (1077)..(1077)
   <223> n is a, c, g, or t
<220>
   <221> misc_feature
   <222> (1106)..(1106)
   <223> n is a, c, g, or t
<220>
   <221> misc_feature
   <222> (1116)..(1116)
   <223> n is a, c, g, or t
<220>
   <221> misc_feature
   <222> (1138)..(1138)
   <223> n is a, c, g, or t
<220>
   <221> misc_feature
   <222> (1140)..(1145)
   <223> n is a, c, g, or t
<220>
   <221> misc_feature
   <222> (1163)..(1163)
   <223> n is a, c, g, or t
<220>
   <221> misc_feature
   <222> (1202)..(1202)
   <223> n is a, c, g, or t
<220>
   <221> misc_feature
   <222> (1208)..(1208)
   <223> n is a, c, g, or t
<220>
   <221> misc_feature
   <222> (1211)..(1211)
   <223> n is a, c, g, or t
<220>
   <221> misc_feature
   <222> (1222)..(1222)
   <223> n is a, c, g, or t
<220>
   <221> misc_feature
   <222> (1224)..(1225)
   <223> n is a, c, g, or t
<220>
   <221> misc_feature
   <222> (1229)..(1229)
   <223> n is a, c, g, or t
<220>
   <221> misc_feature
   <222> (1238)..(1238)
   <223> n is a, c, g, or t
<220>
   <221> misc_feature
   <222> (1240)..(1240)
   <223> n is a, c, g, or t
<220>
   <221> misc_feature
   <222> (1257)..(1257)
   <223> n is a, c, g, or t
<220>
   <221> misc_feature
   <222> (1287)..(1287)
   <223> n is a, c, g, or t
<220>
   <221> misc_feature
   <222> (1322)..(1322)
   <223> n is a, c, g, or t
<220>
   <221> misc_feature
   <222> (1329)..(1329)
   <223> n is a, c, g, or t
<220>
   <221> misc_feature
   <222> (1337)..(1337)
   <223> n is a, c, g, or t
<220>
   <221> misc_feature
   <222> (1365)..(1365)
   <223> n is a, c, g, or t
<220>
   <221> misc_feature
   <222> (1411)..(1411)
   <223> n is a, c, g, or t
<220>
   <221> misc_feature
   <222> (1415)..(1415)
   <223> n is a, c, g, or t
<220>
   <221> misc_feature
   <222> (1492)..(1492)
   <223> n is a, c, g, or t
<220>
   <221> misc_feature
   <222> (1529)..(1529)
   <223> n is a, c, g, or t
<220>
   <221> misc_feature
   <222> (1535)..(1535)
   <223> n is a, c, g, or t
<220>
   <221> misc_feature
   <222> (1544)..(1544)
   <223> n is a, c, g, or t
<220>
   <221> misc_feature
   <222> (1546)..(1546)
   <223> n is a, c, g, or t
<220>
   <221> misc_feature
   <222> (1627)..(1627)
   <223> n is a, c, g, or t
<220>
   <221> misc_feature
   <222> (1659)..(1659)
   <223> n is a, c, g, or t
<220>
   <221> misc_feature
   <222> (1662)..(1662)
   <223> n is a, c, g, or t
<220>
   <221> misc_feature
   <222> (1680)..(1680)
   <223> n is a, c, g, or t
<220>
   <221> misc_feature
   <222> (1688)..(1688)
   <223> n is a, c, g, or t
<220>
   <221> misc_feature
   <222> (1692)..(1692)
   <223> n is a, c, g, or t
<220>
   <221> misc_feature
   <222> (1699)..(1699)
   <223> n is a, c, g, or t
<220>
   <221> misc_feature
   <222> (1709)..(1709)
   <223> n is a, c, g, or t
<220>
   <221> misc_feature
   <222> (1798)..(1798)
   <223> n is a, c, g, or t
<220>
   <221> misc_feature
   <222> (1828)..(1828)
   <223> n is a, c, g, or t
<220>
   <221> misc_feature
   <222> (1870)..(1871)
   <223> n is a, c, g, or t
<220>
   <221> misc_feature
   <222> (1878)..(1878)
   <223> n is a, c, g, or t
<220>
   <221> misc_feature
   <222> (1889)..(1889)
   <223> n is a, c, g, or t
<220>
   <221> misc_feature
   <222> (1897)..(1897)
   <223> n is a, c, g, or t
<220>
   <221> misc_feature
   <222> (1902)..(1902)
   <223> n is a, c, g, or t
<220>
   <221> misc_feature
   <222> (1912)..(1912)
   <223> n is a, c, g, or t
<220>
   <221> misc_feature
   <222> (1945)..(1945)
   <223> n is a, c, g, or t
<220>
   <221> misc_feature
   <222> (1948)..(1948)
   <223> n is a, c, g, or t
<220>
   <221> misc_feature
   <222> (2001)..(2001)
   <223> n is a, c, g, or t
<220>
   <221> misc_feature
   <222> (2021)..(2022)
   <223> n is a, c, g, or t
<220>
   <221> misc_feature
   <222> (2059) .. (2059)
   <223> n is a, c, g, or t
<220>
   <221> misc_feature
   <222> (2079) .. (2079)
   <223> n is a, c, g, or t
<220>
   <221> misc_feature
   <222> (2089) .. (2090)
   <223> n is a, c, g, or t
<220>
   <221> misc_feature
   <222> (2112)..(2112)
   <223> n is a, c, g, or t
<220>
   <221> misc_feature
   <222> (2116)..(2116)
   <223> n is a, c, g, or t
<220>
   <221> misc_feature
   <222> (2133)..(2133)
   <223> n is a, c, g, or t
<220>
   <221> misc_feature
   <222> (2165)..(2165)
   <223> n is a, c, g, or t
<220>
   <221> misc_feature
   <222> (2177)..(2177)
   <223> n is a, c, g, or t
<220>
   <221> misc_feature
   <222> (2201)..(2201)
   <223> n is a, c, g, or t
<220>
   <221> misc_feature
   <222> (2205)..(2205)
   <223> n is a, c, g, or t
<220>
   <221> misc_feature
   <222> (2216)..(2216)
   <223> n is a, c, g, or t
<220>
   <221> misc_feature
   <222> (2222)..(2224)
   <223> n is a, c, g, or t
<220>
   <221> misc_feature
   <222> (2233)..(2234)
   <223> n is a, c, g, or t
<220>
   <221> misc_feature
   <222> (2246)..(2246)
   <223> n is a, c, g, or t
<220>
   <221> misc_feature
   <222> (2248)..(2248)
   <223> n is a, c, g, or t
<220>
   <221> misc_feature
   <222> (2290)..(2290)
   <223> n is a, c, g, or t
<220>
   <221> misc_feature
   <222> (2370)..(2370)
   <223> n is a, c, g, or t
<220>
   <221> misc_feature
   <222> (2392)..(2394)
   <223> n is a, c, g, or t
<220>
   <221> misc_feature
   <222> (2404)..(2405)
   <223> n is a, c, g, or t
<220>
   <221> misc_feature
   <222> (2409)..(2410)
   <223> n is a, c, g, or t
<220>
   <221> misc_feature
   <222> (2469) .. (2469)
   <223> n is a, c, g, or t
<220>
   <221> misc_feature
   <222> (2505)..(2505)
   <223> n is a, c, g, or t
<220>
   <221> misc_feature
   <222> (2519) .. (2519)
   <223> n is a, c, g, or t
<220>
   <221> misc_feature
   <222> (2534)..(2536)
   <223> n is a, c, g, or t
<400> 27
   tcgtaccgtt gcttctctca agtttagatt tttttccgta aaaagaggag gtggcccgtg        60
   aagtttattc cctttaaaac ccaccaatta gctccttcac tctcagntct caacaatggc       120
   ttcgactctg ggcggcgatg ngagaaacga gatagtgttt ttcgatcttg agacngcggt       180
   tccgaccaaa tcggggcanc cttntgcgat tttggagttt ggggctatct tagtttgccc       240
   tatgaagcta gtggagctct atagttactc cacnttggtt cgaccnaccg anctttctct       300
   catctccacg ctcacgaagc gacgaagcgg cattacgcgc gacggagttc tctctgcacc       360
   tacattctct gaaatcgctg atgaagtcta cgacattctc cncggtaagg gtttctcttt       420
   ttttttnnnn nnctnnctcn atctctctna cncgaagnta caagtattga ttttggtgtt       480
   tctgtaggac gaatttgggn nggacataac ataaagagat tcgattgtgt aagaatanga       540
   gatgcatttg cagnaattgg tctcnctccc ccggagccga aagctacaat tgattcactt       600
   tcgttnntgt ctcagaagtt tgggaagaga gctggtgaca tgaaggtctc tcttttttcg      660
   tctnctcgat gataaatctc aaagccnata gcttncttgt tatctttata gatatgaatt       720
   tcnatgtaac ttcanagatt catcactcat caaagttgct aaaatttact ctnnnnnaan    780
   aangtagatg gcatcgcntg ctacatattt cnggctagga gatcangctc acaggtnaaa    840
   nnagtaaacg ataccntgtg ccttttaacg attcaccagt tgtttcaata tgggactaaa    900
   catggntang attcaccagg agcttagatg atgtccggat gaatcttgaa gtnntcaagn    960
   actgtncaac cgtcttnttt ctggtattgn tgtcttntca tttcttgaat aatgatnaac   1020
   tctaanttna aaaggattag attanagagg tngngacata tctganttct gtctacngtt   1080
   tgcaaaagtt gggtccatct tccttncaga ccacancttt gcaagccgta aacatggntn   1140
   nnnnnttgca agtatagttt gtnatatcac tgagtttaag tacttggtgt ttgcaggagt   1200
   cnagtgtncc ngacattctt ananncatna gctggttntn cccaagaaaa agtccgngaa   1260
   cacgaagtaa tgagaagtca ctgcctnatg gagtcagaga aagcccgact tcttcctctt   1320
   cnagccctna aactganccg agttcgtctt ctgtagatgc cacanctgtc aaaaaccatc   1380
   ccatcatttc tcttctgacg gaatgctcag naagngatac atctagttgt gaaatagatc   1440
   catctgacat aaccactcta ataagtaaac tacatattgg aactcttaag anagatgctg   1500
   cggacgaagc caaaactgtg agagatgcng cggangaagc caanantgta agacagcagg   1560
   gtgaatcaac cgatcccaat gccaaagatg aatcattttt gggcgttaat gaagtatctg   1620
   tttctancat cagggcaagt cttatcccgt tatatcgtng gngtctgaga atggagctgn   1680
   ttcacaanga cncccctcna catctctgnt ggtatagctt gaaaattcgg tttggaataa  1740
   gccggaagta tgtggatcat gtaggtcgtc caaagatgaa tattgttgta gacatacntc   1800
   ctgatttatg caagatcttg gacgcatncg atgctgctgc gcataactta ctgattgact   1860
   caagcacaan ntcagatngg aggcctacng ttatgangaa anaaggcttt gncaactatc   1920
   ccacagccag actgcagtaa gtatncanca ctctctctga ccttttacat acgagcatga   1980
   atccaccgga gagtctctaa naccatctcc aaccctactc nntattcacc tccaaactct   2040
   attttggagt taaatcccnc caacccttgc aaaatagana tcttcaaann ttttctccat   2100
   atttggagat tntgantttt taagtcatga ctncattttg gagttgggtt ggagaaaaac  2160
   acaantccaa aatagantta cttcattttg gagtaaaaaa ntgangaaat gggttngaga   2220
   tnnnctaacc tcnntcacca ttcttntntt gttggcagaa taagctcaga atccaatgga   2280
   acccaggtan accaaaaaga agaacctttg ggaaccaatc aaaagctcga tttcagtagc   2340
   gataattttg aaaagcttga gtcagcactn cttcctggta ccctggttga tnnnttcttc   2400
   tcanncgann cttacgatta taagaaaatg gtagggatac gtctagcagc cagaaagttg   2460
   gtaatccanc tgaagaaatg atctagccaa ggaaaaatca ttccnctgtc tcttcctgnt   2520
   cagtcggtga gcannn                                                   2536
<210> 28
   <211> 1521
   <212> DNA
   <213> Boechera
<220>
   <221> misc_feature
   <222> (26)..(26)
   <223> n is a, c, g, or t
<220>
   <221> misc_feature
   <222> (60)..(60)
   <223> n is a, c, g, or t
<220>
   <221> misc_feature
   <222> (84)..(84)
   <223> n is a, c, g, or t
<220>
   <221> misc_feature
   <222> (89) .. (89)
   <223> n is a, c, g, or t
<220>
   <221> misc_feature
   <222> (159)..(159)
   <223> n is a, c, g, or t
<220>
   <221> misc_feature
   <222> (171)..(171)
   <223> n is a, c, g, or t
<220>
   <221> misc_feature
   <222> (177)..(177)
   <223> n is a, c, g, or t
<220>
   <221> misc_feature
   <222> (287)..(287)
   <223> n is a, c, g, or t
<220>
   <221> misc_feature
   <222> (302)..(303)
   <223> n is a, c, g, or t
<220>
   <221> misc_feature
   <222> (340)..(340)
   <223> n is a, c, g, or t
<220>
   <221> misc_feature
   <222> (356)..(356)
   <223> n is a, c, g, or t
<220>
   <221> misc_feature
   <222> (367)..(367)
   <223> n is a, c, g, or t
<220>
   <221> misc_feature
   <222> (408)..(409)
   <223> n is a, c, g, or t
<220>
   <221> misc_feature
   <222> (458)..(458)
   <223> n is a, c, g, or t
<220>
   <221> misc_feature
   <222> (472)..(472)
   <223> n is a, c, g, or t
<220>
   <221> misc_feature
   <222> (486)..(486)
   <223> n is a, c, g, or t
<220>
   <221> misc_feature
   <222> (528) .. (529)
   <223> n is a, c, g, or t
<220>
   <221> misc_feature
   <222> (535)..(535)
   <223> n is a, c, g, or t
<220>
   <221> misc_feature
   <222> (541)..(541)
   <223> n is a, c, g, or t
<220>
   <221> misc_feature
   <222> (552)..(552)
   <223> n is a, c, g, or t
<220>
   <221> misc_feature
   <222> (564)..(564)
   <223> n is a, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (584)..(584)
   <223> n is a, c, g, or t
<220>
   <221> misc_feature
   <222> (586)..(587)
   <223> n is a, c, g, or t
<220>
   <221> misc_feature
   <222> (591)..(591)
   <223> n is a, c, g, or t
<220>
   <221> misc_feature
   <222> (600)..(600)
   <223> n is a, c, g, or t
<220>
   <221> misc_feature
   <222> (602)..(602)
   <223> n is a, c, g, or t
<220>
   <221> misc_feature
   <222> (619)..(619)
   <223> n is a, c, g, or t
<220>
   <221> misc_feature
   <222> (649) .. (649)
   <223> n is a, c, g, or t
<220>
   <221> misc_feature
   <222> (684)..(684)
   <223> n is a, c, g, or t
<220>
   <221> misc_feature
   <222> (691)..(691)
   <223> n is a, c, g, or t
<220>
   <221> misc_feature
   <222> (699)..(699)
   <223> n is a, c, g, or t
<220>
   <221> misc_feature
   <222> (727)..(727)
   <223> n is a, c, g, or t
<220>
   <221> misc_feature
   <222> (773)..(773)
   <223> n is a, c, g, or t
<220>
   <221> misc_feature
   <222> (777)..(777)
   <223> n is a, c, g, or t
<220>
   <221> misc_feature
   <222> (854)..(854)
   <223> n is a, c, g, or t
<220>
   <221> misc_feature
   <222> (891)..(891)
   <223> n is a, c, g, or t
<220>
   <221> misc_feature
   <222> (897)..(897)
   <223> n is a, c, g, or t
<220>
   <221> misc_feature
   <222> (906)..(906)
   <223> n is a, c, g, or t
<220>
   <221> misc_feature
   <222> (908)..(908)
   <223> n is a, c, g, or t
<220>
   <221> misc_feature
   <222> (989)..(989)
   <223> n is a, c, g, or t
<220>
   <221> misc_feature
   <222> (1021)..(1021)
   <223> n is a, c, g, or t
<220>
   <221> misc_feature
   <222> (1024)..(1024)
   <223> n is a, c, g, or t
<220>
   <221> misc_feature
   <222> (1042)..(1042)
   <223> n is a, c, g, or t
<220>
   <221> misc_feature
   <222> (1050)..(1050)
   <223> n is a, c, g, or t
<220>
   <221> misc_feature
   <222> (1054)..(1054)
   <223> n is a, c, g, or t
<220>
   <221> misc_feature
   <222> (1061)..(1061)
   <223> n is a, c, g, or t
<220>
   <221> misc_feature
   <222> (1071)..(1071)
   <223> n is a, c, g, or t
<220>
   <221> misc_feature
   <222> (1160)..(1160)
   <223> n is a, c, g, or t
<220>
   <221> misc_feature
   <222> (1190)..(1190)
   <223> n is a, c, g, or t
<220>
   <221> misc_feature
   <222> (1232)..(1233)
   <223> n is a, c, g, or t
<220>
   <221> misc_feature
   <222> (1240)..(1240)
   <223> n is a, c, g, or t
<220>
   <221> misc_feature
   <222> (1251)..(1251)
   <223> n is a, c, g, or t
<220>
   <221> misc_feature
   <222> (1259)..(1259)
   <223> n is a, c, g, or t
<220>
   <221> misc_feature
   <222> (1264)..(1264)
   <223> n is a, c, g, or t
<220>
   <221> misc_feature
   <222> (1274)..(1274)
   <223> n is a, c, g, or t
<220>
   <221> misc_feature
   <222> (1330)..(1330)
   <223> n is a, c, g, or t
<220>
   <221> misc_feature
   <222> (1410)..(1410)
   <223> n is a, c, g, or t
<220>
   <221> misc_feature
   <222> (1432)..(1434)
   <223> n is a, c, g, or t
<220>
   <221> misc_feature
   <222> (1444)..(1445)
   <223> n is a, c, g, or t
<220>
   <221> misc_feature
   <222> (1449)..(1450)
   <223> n is a, c, g, or t
<220>
   <221> misc_feature
   <222> (1509)..(1509)
   <223> n is a, c, g, or t
<400> 28
   atggcttcga ctctgggcgg cgatgngaga aacgagatag tgtttttcga tcttgagacn      60
   gcggttccga ccaaatcggg gcanccttnt gcgattttgg agtttggggc tatcttagtt     120
   tgccctatga agctagtgga gctctatagt tactccacnt tggttcgacc naccganctt     180
   tctctcatct ccacgctcac gaagcgacga agcggcatta cgcgcgacgg agttctctct     240
   gcacctacat tctctgaaat cgctgatgaa gtctacgaca ttctccncgg acgaatttgg     300
   gnnggacata acataaagag attcgattgt gtaagaatan gagatgcatt tgcagnaatt     360
   ggtctcnctc ccccggagcc gaaagctaca attgattcac tttcgttnnt gtctcagaag       420
   tttgggaaga gagctggtga catgaagatg gcatcgcntg ctacatattt cnggctagga      480
   gatcangctc acaggagctt agatgatgtc cggatgaatc ttgaagtnnt caagnactgt      540
   ncaaccgtct tntttctgga gtcnagtgtn ccngacattc ttananncat nagctggttn      600
   tncccaagaa aaagtccgng aacacgaagt aatgagaagt cactgcctna tggagtcaga      660
   gaaagcccga cttcttcctc ttcnagccct naaactganc cgagttcgtc ttctgtagat      720
   gccacanctg tcaaaaacca tcccatcatt tctcttctga cggaatgctc agnaagngat      780
   acatctagtt gtgaaataga tccatctgac ataaccactc taataagtaa actacatatt      840
   ggaactctta aganagatgc tgcggacgaa gccaaaactg tgagagatgc ngcggangaa      900
   gccaanantg taagacagca gggtgaatca accgatccca atgccaaaga tgaatcattt      960
   ttgggcgtta atgaagtatc tgtttctanc atcagggcaa gtcttatccc gttatatcgt     1020
   nggngtctga gaatggagct gnttcacaan gacncccctc nacatctctg ntggtatagc     1080
   ttgaaaattc ggtttggaat aagccggaag tatgtggatc atgtaggtcg tccaaagatg     1140
   aatattgttg tagacatacn tcctgattta tgcaagatct tggacgcatn cgatgctgct     1200
   gcgcataact tactgattga ctcaagcaca anntcagatn ggaggcctac ngttatgang     1260
   aaanaaggct ttgncaacta tcccacagcc agactgcaaa taagctcaga atccaatgga     1320
   acccaggtan accaaaaaga agaacctttg ggaaccaatc aaaagctcga tttcagtagc     1380
   gataattttg aaaagcttga gtcagcactn cttcctggta ccctggttga tnnnttcttc     1440
   tcanncgann cttacgatta taagaaaatg gtagggatac gtctagcagc cagaaagttg     1500
   gtaatccanc tgaagaaatg a                                                1521
<210> 29
   <211> 2506
   <212> DNA
   <213> Boechera
<220>
   <221> misc_feature
   <222> (107)..(107)
   <223> n is a, c, g, or t
<220>
   <221> misc_feature
   <222> (141)..(141)
   <223> n is a, c, g, or t
<220>
   <221> misc_feature
   <222> (175)..(175)
   <223> n is a, c, g, or t
<220>
   <221> misc_feature
   <222> (199)..(199)
   <223> n is a, c, g, or t
<220>
   <221> misc_feature
   <222> (204)..(204)
   <223> n is a, c, g, or t
<220>
   <221> misc_feature
   <222> (274)..(274)
   <223> n is a, c, g, or t
<220>
   <221> misc_feature
   <222> (286)..(286)
   <223> n is a, c, g, or t
<220>
   <221> misc_feature
   <222> (292)..(292)
   <223> n is a, c, g, or t
<220>
   <221> misc_feature
   <222> (402)..(402)
   <223> n is a, c, g, or t
<220>
   <221> misc_feature
   <222> (427)..(432)
   <223> n is a, c, g, or t
<220>
   <221> misc_feature
   <222> (435)..(436)
   <223> n is a, c, g, or t
<220>
   <221> misc_feature
   <222> (440)..(440)
   <223> n is a, c, g, or t
<220>
   <221> misc_feature
   <222> (449)..(449)
   <223> n is a, c, g, or t
<220>
   <221> misc_feature
   <222> (452)..(452)
   <223> n is a, c, g, or t
<220>
   <221> misc_feature
   <222> (458)..(458)
   <223> n is a, c, g, or t
<220>
   <221> misc_feature
   <222> (500)..(501)
   <223> n is a, c, g, or t
<220>
   <221> misc_feature
   <222> (538)..(538)
   <223> n is a, c, g, or t
<220>
   <221> misc_feature
   <222> (554)..(554)
   <223> n is a, c, g, or t
<220>
   <221> misc_feature
   <222> (565)..(565)
   <223> n is a, c, g, or t
<220>
   <221> misc_feature
   <222> (606)..(607)
   <223> n is a, c, g, or t
<220>
   <221> misc_feature
   <222> (664)..(664)
   <223> n is a, c, g, or t
<220>
   <221> misc_feature
   <222> (687)..(687)
   <223> n is a, c, g, or t
<220>
   <221> misc_feature
   <222> (695)..(695)
   <223> n is a, c, g, or t
<220>
   <221> misc_feature
   <222> (723)..(723)
   <223> n is a, c, g, or t
<220>
   <221> misc_feature
   <222> (735)..(735)
   <223> n is a, c, g, or t
<220>
   <221> misc_feature
   <222> (773)..(777)
   <223> n is a, c, g, or t
<220>
   <221> misc_feature
   <222> (780)..(780)
   <223> n is a, c, g, or t
<220>
   <221> misc_feature
   <222> (783)..(783)
   <223> n is a, c, g, or t
<220>
   <221> misc_feature
   <222> (798)..(798)
   <223> n is a, c, g, or t
<220>
   <221> misc_feature
   <222> (812)..(812)
   <223> n is a, c, g, or t
<220>
   <221> misc_feature
   <222> (826) .. (826)
   <223> n is a, c, g, or t
<220>
   <221> misc_feature
   <222> (837)..(837)
   <223> n is a, c, g, or t
<220>
   <221> misc_feature
   <222> (841)..(842)
   <223> n is a, c, g, or t
<220>
   <221> misc_feature
   <222> (856)..(856)
   <223> n is a, c, g, or t
<220>
   <221> misc_feature
   <222> (906)..(906)
   <223> n is a, c, g, or t
<220>
   <221> misc_feature
   <222> (909) .. (909)
   <223> n is a, c, g, or t
<220>
   <221> misc_feature
   <222> (953)..(954)
   <223> n is a, c, g, or t
<220>
   <221> misc_feature
   <222> (960)..(960)
   <223> n is a, c, g, or t
<220>
   <221> misc_feature
   <222> (966)..(966)
   <223> n is a, c, g, or t
<220>
   <221> misc_feature
   <222> (977)..(977)
   <223> n is a, c, g, or t
<220>
   <221> misc_feature
   <222> (990)..(990)
   <223> n is a, c, g, or t
<220>
   <221> misc_feature
   <222> (997)..(997)
   <223> n is a, c, g, or t
<220>
   <221> misc_feature
   <222> (1017)..(1017)
   <223> n is a, c, g, or t
<220>
   <221> misc_feature
   <222> (1026)..(1026)
   <223> n is a, c, g, or t
<220>
   <221> misc_feature
   <222> (1029)..(1029)
   <223> n is a, c, g, or t
<220>
   <221> misc_feature
   <222> (1045)..(1045)
   <223> n is a, c, g, or t
<220>
   <221> misc_feature
   <222> (1052)..(1052)
   <223> n is a, c, g, or t
<220>
   <221> misc_feature
   <222> (1054)..(1054)
   <223> n is a, c, g, or t
<220>
   <221> misc_feature
   <222> (1066)..(1066)
   <223> n is a, c, g, or t
<220>
   <221> misc_feature
   <222> (1077)..(1077)
   <223> n is a, c, g, or t
<220>
   <221> misc_feature
   <222> (1106)..(1106)
   <223> n is a, c, g, or t
<220>
   <221> misc_feature
   <222> (1116)..(1116)
   <223> n is a, c, g, or t
<220>
   <221> misc_feature
   <222> (1138)..(1138)
   <223> n is a, c, g, or t
<220>
   <221> misc_feature
   <222> (1140)..(1145)
   <223> n is a, c, g, or t
<220>
   <221> misc_feature
   <222> (1163)..(1163)
   <223> n is a, c, g, or t
<220>
   <221> misc_feature
   <222> (1202)..(1202)
   <223> n is a, c, g, or t
<220>
   <221> misc_feature
   <222> (1208)..(1208)
   <223> n is a, c, g, or t
<220>
   <221> misc_feature
   <222> (1211)..(1211)
   <223> n is a, c, g, or t
<220>
   <221> misc_feature
   <222> (1222)..(1222)
   <223> n is a, c, g, or t
<220>
   <221> misc_feature
   <222> (1224)..(1225)
   <223> n is a, c, g, or t
<220>
   <221> misc_feature
   <222> (1229)..(1229)
   <223> n is a, c, g, or t
<220>
   <221> misc_feature
   <222> (1238)..(1238)
   <223> n is a, c, g, or t
<220>
   <221> misc_feature
   <222> (1240)..(1240)
   <223> n is a, c, g, or t
<220>
   <221> misc_feature
   <222> (1257)..(1257)
   <223> n is a, c, g, or t
<220>
   <221> misc_feature
   <222> (1287)..(1287)
   <223> n is a, c, g, or t
<220>
   <221> misc_feature
   <222> (1322)..(1322)
   <223> n is a, c, g, or t
<220>
   <221> misc_feature
   <222> (1329)..(1329)
   <223> n is a, c, g, or t
<220>
   <221> misc_feature
   <222> (1337)..(1337)
   <223> n is a, c, g, or t
<220>
   <221> misc_feature
   <222> (1365)..(1365)
   <223> n is a, c, g, or t
<220>
   <221> misc_feature
   <222> (1411)..(1411)
   <223> n is a, c, g, or t
<220>
   <221> misc_feature
   <222> (1415)..(1415)
   <223> n is a, c, g, or t
<220>
   <221> misc_feature
   <222> (1492)..(1492)
   <223> n is a, c, g, or t
<220>
   <221> misc_feature
   <222> (1499)..(1499)
   <223> n is a, c, g, or t
<220>
   <221> misc_feature
   <222> (1505)..(1505)
   <223> n is a, c, g, or t
<220>
   <221> misc_feature
   <222> (1514)..(1514)
   <223> n is a, c, g, or t
<220>
   <221> misc_feature
   <222> (1516)..(1516)
   <223> n is a, c, g, or t
<220>
   <221> misc_feature
   <222> (1597)..(1597)
   <223> n is a, c, g, or t
<220>
   <221> misc_feature
   <222> (1629)..(1629)
   <223> n is a, c, g, or t
<220>
   <221> misc_feature
   <222> (1632)..(1632)
   <223> n is a, c, g, or t
<220>
   <221> misc_feature
   <222> (1650)..(1650)
   <223> n is a, c, g, or t
<220>
   <221> misc_feature
   <222> (1658)..(1658)
   <223> n is a, c, g, or t
<220>
   <221> misc_feature
   <222> (1662)..(1662)
   <223> n is a, c, g, or t
<220>
   <221> misc_feature
   <222> (1669)..(1669)
   <223> n is a, c, g, or t
<220>
   <221> misc_feature
   <222> (1679)..(1679)
   <223> n is a, c, g, or t
<220>
   <221> misc_feature
   <222> (1768)..(1768)
   <223> n is a, c, g, or t
<220>
   <221> misc_feature
   <222> (1798)..(1798)
   <223> n is a, c, g, or t
<220>
   <221> misc_feature
   <222> (1840)..(1841)
   <223> n is a, c, g, or t
<220>
   <221> misc_feature
   <222> (1848)..(1848)
   <223> n is a, c, g, or t
<220>
   <221> misc_feature
   <222> (1859)..(1859)
   <223> n is a, c, g, or t
<220>
   <221> misc_feature
   <222> (1867)..(1867)
   <223> n is a, c, g, or t
<220>
   <221> misc_feature
   <222> (1872)..(1872)
   <223> n is a, c, g, or t
<220>
   <221> misc_feature
   <222> (1882)..(1882)
   <223> n is a, c, g, or t
<220>
   <221> misc_feature
   <222> (1915)..(1915)
   <223> n is a, c, g, or t
<220>
   <221> misc_feature
   <222> (1918)..(1918)
   <223> n is a, c, g, or t
<220>
   <221> misc_feature
   <222> (1971)..(1971)
   <223> n is a, c, g, or t
<220>
   <221> misc_feature
   <222> (1991)..(1992)
   <223> n is a, c, g, or t
<220>
   <221> misc_feature
   <222> (2029) .. (2029)
   <223> n is a, c, g, or t
<220>
   <221> misc_feature
   <222> (2049) .. (2049)
   <223> n is a, c, g, or t
<220>
   <221> misc_feature
   <222> (2059)..(2060)
   <223> n is a, c, g, or t
<220>
   <221> misc_feature
   <222> (2082)..(2082)
   <223> n is a, c, g, or t
<220>
   <221> misc_feature
   <222> (2086)..(2086)
   <223> n is a, c, g, or t
<220>
   <221> misc_feature
   <222> (2103)..(2103)
   <223> n is a, c, g, or t
<220>
   <221> misc_feature
   <222> (2135)..(2135)
   <223> n is a, c, g, or t
<220>
   <221> misc_feature
   <222> (2147)..(2147)
   <223> n is a, c, g, or t
<220>
   <221> misc_feature
   <222> (2171)..(2171)
   <223> n is a, c, g, or t
<220>
   <221> misc_feature
   <222> (2175)..(2175)
   <223> n is a, c, g, or t
<220>
   <221> misc_feature
   <222> (2186)..(2186)
   <223> n is a, c, g, or t
<220>
   <221> misc_feature
   <222> (2192)..(2194)
   <223> n is a, c, g, or t
<220>
   <221> misc_feature
   <222> (2203)..(2204)
   <223> n is a, c, g, or t
<220>
   <221> misc_feature
   <222> (2216)..(2216)
   <223> n is a, c, g, or t
<220>
   <221> misc_feature
   <222> (2218)..(2218)
   <223> n is a, c, g, or t
<220>
   <221> misc_feature
   <222> (2260)..(2260)
   <223> n is a, c, g, or t
<220>
   <221> misc_feature
   <222> (2340)..(2340)
   <223> n is a, c, g, or t
<220>
   <221> misc_feature
   <222> (2362)..(2364)
   <223> n is a, c, g, or t
<220>
   <221> misc_feature
   <222> (2374)..(2375)
   <223> n is a, c, g, or t
<220>
   <221> misc_feature
   <222> (2379)..(2380)
   <223> n is a, c, g, or t
<220>
   <221> misc_feature
   <222> (2439) .. (2439)
   <223> n is a, c, g, or t
<220>
   <221> misc_feature
   <222> (2475)..(2475)
   <223> n is a, c, g, or t
<220>
   <221> misc_feature
   <222> (2489) .. (2489)
   <223> n is a, c, g, or t
<220>
   <221> misc_feature
   <222> (2504)..(2506)
   <223> n is a, c, g, or t
<400> 29
   tcgtaccgtt gcttctctca agtttagatt tttttccgta aaaagaggag gtggcccgtg       60
   aagtttattc cctttaaaac ccaccaatta gctccttcac tctcagntct caacaatggc      120
   ttcgactctg ggcggcgatg ngagaaacga gatagtgttt ttcgatcttg agacngcggt      180
   tccgaccaaa tcggggcanc cttntgcgat tttggagttt ggggctatct tagtttgccc      240
   tatgaagcta gtggagctct atagttactc cacnttggtt cgaccnaccg anctttctct      300
   catctccacg ctcacgaagc gacgaagcgg cattacgcgc gacggagttc tctctgcacc      360
   tacattctct gaaatcgctg atgaagtcta cgacattctc cncggtaagg gtttctcttt      420
   ttttttnnnn nnctnnctcn atctctctna cncgaagnta caagtattga ttttggtgtt      480
   tctgtaggac gaatttgggn nggacataac ataaagagat tcgattgtgt aagaatanga      540
   gatgcatttg cagnaattgg tctcnctccc ccggagccga aagctacaat tgattcactt      600
   tcgttnntgt ctcagaagtt tgggaagaga gctggtgaca tgaaggtctc tcttttttcg       660
   tctnctcgat gataaatctc aaagccnata gcttncttgt tatctttata gatatgaatt      720
   tcnatgtaac ttcanagatt catcactcat caaagttgct aaaatttact ctnnnnnaan      780
   aangtagatg gcatcgcntg ctacatattt cnggctagga gatcangctc acaggtnaaa 840
   nnagtaaacg ataccntgtg ccttttaacg attcaccagt tgtttcaata tgggactaaa 900
   catggntang attcaccagg agcttagatg atgtccggat gaatcttgaa gtnntcaagn 960
   actgtncaac cgtcttnttt ctggtattgn tgtcttntca tttcttgaat aatgatnaac 1020
   tctaanttna aaaggattag attanagagg tngngacata tctganttct gtctacngtt 1080
   tgcaaaagtt gggtccatct tccttncaga ccacancttt gcaagccgta aacatggntn     1140
   nnnnnttgca agtatagttt gtnatatcac tgagtttaag tacttggtgt ttgcaggagt 1200
   cnagtgtncc ngacattctt ananncatna gctggttntn cccaagaaaa agtccgngaa 1260
   cacgaagtaa tgagaagtca ctgcctnatg gagtcagaga aagcccgact tcttcctctt 1320
   cnagccctna aactganccg agttcgtctt ctgtagatgc cacanctgtc aaaaaccatc 1380
   ccatcatttc tcttctgacg gaatgctcag naagngatac atctagttgt gaaatagatc 1440
   catctgacat aaccactcta ataagtaaac tacatattgg aactcttaag anagatgcng 1500
   cggangaagc caanantgta agacagcagg gtgaatcaac cgatcccaat gccaaagatg 1560
   aatcattttt gggcgttaat gaagtatctg tttctancat cagggcaagt cttatcccgt 1620
   tatatcgtng gngtctgaga atggagctgn ttcacaanga cncccctcna catctctgnt 1680
   ggtatagctt gaaaattcgg tttggaataa gccggaagta tgtggatcat gtaggtcgtc 1740
   caaagatgaa tattgttgta gacatacntc ctgatttatg caagatcttg gacgcatncg 1800
   atgctgctgc gcataactta ctgattgact caagcacaan ntcagatngg aggcctacng 1860
   ttatgangaa anaaggcttt gncaactatc ccacagccag actgcagtaa gtatncanca 1920
   ctctctctga ccttttacat acgagcatga atccaccgga gagtctctaa naccatctcc 1980
   aaccctactc nntattcacc tccaaactct attttggagt taaatcccnc caacccttgc 2040
   aaaatagana tcttcaaann ttttctccat atttggagat tntgantttt taagtcatga 2100
   ctncattttg gagttgggtt ggagaaaaac acaantccaa aatagantta cttcattttg 2160
   gagtaaaaaa ntgangaaat gggttngaga tnnnctaacc tcnntcacca ttcttntntt 2220
   gttggcagaa taagctcaga atccaatgga acccaggtan accaaaaaga agaacctttg 2280
   ggaaccaatc aaaagctcga tttcagtagc gataattttg aaaagcttga gtcagcactn 2340
   cttcctggta ccctggttga tnnnttcttc tcanncgann cttacgatta taagaaaatg 2400
   gtagggatac gtctagcagc cagaaagttg gtaatccanc tgaagaaatg atctagccaa 2460
   ggaaaaatca ttccnctgtc tcttcctgnt cagtcggtga gcannn 2506
<210> 30
   <211> 1491
   <212> DNA
   <213> Boechera
<220>
   <221> misc_feature
   <222> (26)..(26)
   <223> n is a, c, g, or t
<220>
   <221> misc_feature
   <222> (60)..(60)
   <223> n is a, c, g, or t
<220>
   <221> misc_feature
   <222> (84)..(84)
   <223> n is a, c, g, or t
<220>
   <221> misc_feature
   <222> (89) .. (89)
   <223> n is a, c, g, or t
<220>
   <221> misc_feature
   <222> (159)..(159)
   <223> n is a, c, g, or t
<220>
   <221> misc_feature
   <222> (171)..(171)
   <223> n is a, c, g, or t
<220>
   <221> misc_feature
   <222> (177)..(177)
   <223> n is a, c, g, or t
<220>
   <221> misc_feature
   <222> (287)..(287)
   <223> n is a, c, g, or t
<220>
   <221> misc_feature
   <222> (302)..(303)
   <223> n is a, c, g, or t
<220>
   <221> misc_feature
   <222> (340)..(340)
   <223> n is a, c, g, or t
<220>
   <221> misc_feature
   <222> (356)..(356)
   <223> n is a, c, g, or t
<220>
   <221> misc_feature
   <222> (367)..(367)
   <223> n is a, c, g, or t
<220>
   <221> misc_feature
   <222> (408)..(409)
   <223> n is a, c, g, or t
<220>
   <221> misc_feature
   <222> (458)..(458)
   <223> n is a, c, g, or t
<220>
   <221> misc_feature
   <222> (472)..(472)
   <223> n is a, c, g, or t
<220>
   <221> misc_feature
   <222> (486)..(486)
   <223> n is a, c, g, or t
<220>
   <221> misc_feature
   <222> (528)..(529)
   <223> n is a, c, g, or t
<220>
   <221> misc_feature
   <222> (535)..(535)
   <223> n is a, c, g, or t
<220>
   <221> misc_feature
   <222> (541)..(541)
   <223> n is a, c, g, or t
<220>
   <221> misc_feature
   <222> (552)..(552)
   <223> n is a, c, g, or t
<220>
   <221> misc_feature
   <222> (564)..(564)
   <223> n is a, c, g, or t
<220>
   <221> misc_feature
   <222> (570)..(570)
   <223> n is a, c, g, or t
<220>
   <221> misc_feature
   <222> (573)..(573)
   <223> n is a, c, g, or t
<220>
   <221> misc_feature
   <222> (584)..(584)
   <223> n is a, c, g, or t
<220>
   <221> misc_feature
   <222> (586)..(587)
   <223> n is a, c, g, or t
<220>
   <221> misc_feature
   <222> (591)..(591)
   <223> n is a, c, g, or t
<220>
   <221> misc_feature
   <222> (600)..(600)
   <223> n is a, c, g, or t
<220>
   <221> misc_feature
   <222> (602)..(602)
   <223> n is a, c, g, or t
<220>
   <221> misc_feature
   <222> (619)..(619)
   <223> n is a, c, g, or t
<220>
   <221> misc_feature
   <222> (649)..(649)
   <223> n is a, c, g, or t
<220>
   <221> misc_feature
   <222> (684)..(684)
   <223> n is a, c, g, or t
<220>
   <221> misc_feature
   <222> (691)..(691)
   <223> n is a, c, g, or t
<220>
   <221> misc_feature
   <222> (699)..(699)
   <223> n is a, c, g, or t
<220>
   <221> misc_feature
   <222> (727)..(727)
   <223> n is a, c, g, or t
<220>
   <221> misc_feature
   <222> (773)..(773)
   <223> n is a, c, g, or t
<220>
   <221> misc_feature
   <222> (777)..(777)
   <223> n is a, c, g, or t
<220>
   <221> misc_feature
   <222> (854)..(854)
   <223> n is a, c, g, or t
<220>
   <221> misc_feature
   <222> (861)..(861)
   <223> n is a, c, g, or t
<220>
   <221> misc_feature
   <222> (867)..(867)
   <223> n is a, c, g, or t
<220>
   <221> misc_feature
   <222> (876)..(876)
   <223> n is a, c, g, or t
<220>
   <221> misc_feature
   <222> (878)..(878)
   <223> n is a, c, g, or t
<220>
   <221> misc_feature
   <222> (959)..(959)
   <223> n is a, c, g, or t
<220>
   <221> misc_feature
   <222> (991)..(991)
   <223> n is a, c, g, or t
<220>
   <221> misc_feature
   <222> (994)..(994)
   <223> n is a, c, g, or t
<220>
   <221> misc_feature
   <222> (1012)..(1012)
   <223> n is a, c, g, or t
<220>
   <221> misc_feature
   <222> (1020)..(1020)
   <223> n is a, c, g, or t
<220>
   <221> misc_feature
   <222> (1024)..(1024)
   <223> n is a, c, g, or t
<220>
   <221> misc_feature
   <222> (1031)..(1031)
   <223> n is a, c, g, or t
<220>
   <221> misc_feature
   <222> (1041)..(1041)
   <223> n is a, c, g, or t
<220>
   <221> misc_feature
   <222> (1130)..(1130)
   <223> n is a, c, g, or t
<220>
   <221> misc_feature
   <222> (1160)..(1160)
   <223> n is a, c, g, or t
<220>
   <221> misc_feature
   <222> (1202)..(1203)
   <223> n is a, c, g, or t
<220>
   <221> misc_feature
   <222> (1210)..(1210)
   <223> n is a, c, g, or t
<220>
   <221> misc_feature
   <222> (1221)..(1221)
   <223> n is a, c, g, or t
<220>
   <221> misc_feature
   <222> (1229)..(1229)
   <223> n is a, c, g, or t
<220>
   <221> misc_feature
   <222> (1234)..(1234)
   <223> n is a, c, g, or t
<220>
   <221> misc_feature
   <222> (1244)..(1244)
   <223> n is a, c, g, or t
<220>
   <221> misc_feature
   <222> (1300)..(1300)
   <223> n is a, c, g, or t
<220>
   <221> misc_feature
   <222> (1380)..(1380)
   <223> n is a, c, g, or t
<220>
   <221> misc_feature
   <222> (1402)..(1404)
   <223> n is a, c, g, or t
<220>
   <221> misc_feature
   <222> (1414)..(1415)
   <223> n is a, c, g, or t
<220>
   <221> misc_feature
   <222> (1419)..(1420)
   <223> n is a, c, g, or t
<220>
   <221> misc_feature
   <222> (1479)..(1479)
   <223> n is a, c, g, or t
<400> 30
   atggcttcga ctctgggcgg cgatgngaga aacgagatag tgtttttcga tcttgagacn      60
   gcggttccga ccaaatcggg gcanccttnt gcgattttgg agtttggggc tatcttagtt     120
   tgccctatga agctagtgga gctctatagt tactccacnt tggttcgacc naccganctt     180
   tctctcatct ccacgctcac gaagcgacga agcggcatta cgcgcgacgg agttctctct     240
   gcacctacat tctctgaaat cgctgatgaa gtctacgaca ttctccncgg acgaatttgg     300
   gnnggacata acataaagag attcgattgt gtaagaatan gagatgcatt tgcagnaatt     360
   ggtctcnctc ccccggagcc gaaagctaca attgattcac tttcgttnnt gtctcagaag     420
   tttgggaaga gagctggtga catgaagatg gcatcgcntg ctacatattt cnggctagga     480
   gatcangctc acaggagctt agatgatgtc cggatgaatc ttgaagtnnt caagnactgt     540
   ncaaccgtct tntttctgga gtcnagtgtn ccngacattc ttananncat nagctggttn     600
   tncccaagaa aaagtccgng aacacgaagt aatgagaagt cactgcctna tggagtcaga     660
   gaaagcccga cttcttcctc ttcnagccct naaactganc cgagttcgtc ttctgtagat     720
   gccacanctg tcaaaaacca tcccatcatt tctcttctga cggaatgctc agnaagngat     780
   acatctagtt gtgaaataga tccatctgac ataaccactc taataagtaa actacatatt     840
   ggaactctta aganagatgc ngcggangaa gccaanantg taagacagca gggtgaatca     900
   accgatccca atgccaaaga tgaatcattt ttgggcgtta atgaagtatc tgtttctanc     960
   atcagggcaa gtcttatccc gttatatcgt nggngtctga gaatggagct gnttcacaan    1020
   gacncccctc nacatctctg ntggtatagc ttgaaaattc ggtttggaat aagccggaag    1080
   tatgtggatc atgtaggtcg tccaaagatg aatattgttg tagacatacn tcctgattta    1140
   tgcaagatct tggacgcatn cgatgctgct gcgcataact tactgattga ctcaagcaca    1200
   anntcagatn ggaggcctac ngttatgang aaanaaggct ttgncaacta tcccacagcc    1260
   agactgcaaa taagctcaga atccaatgga acccaggtan accaaaaaga agaacctttg    1320
   ggaaccaatc aaaagctcga tttcagtagc gataattttg aaaagcttga gtcagcactn    1380
   cttcctggta ccctggttga tnnnttcttc tcanncgann cttacgatta taagaaaatg    1440
   gtagggatac gtctagcagc cagaaagttg gtaatccanc tgaagaaatg a             1491
<210> 31
   <211> 495
   <212> DNA
   <213> Boechera
<220>
   <221> misc_feature
   <222> (21) .. (21)
   <223> n is a, c, g, or t
<220>
   <221> misc_feature
   <222> (45)..(45)
   <223> n is a, c, g, or t
<220>
   <221> misc_feature
   <222> (50)..(50)
   <223> n is a, c, g, or t
<220>
   <221> misc_feature
   <222> (120)..(120)
   <223> n is a, c, g, or t
<220>
   <221> misc_feature
   <222> (132)..(132)
   <223> n is a, c, g, or t
<220>
   <221> misc_feature
   <222> (138)..(138)
   <223> n is a, c, g, or t
<220>
   <221> misc_feature
   <222> (248)..(248)
   <223> n is a, c, g, or t
<220>
   <221> misc_feature
   <222> (263)..(264)
   <223> n is a, c, g, or t
<220>
   <221> misc_feature
   <222> (301)..(301)
   <223> n is a, c, g, or t
<220>
   <221> misc_feature
   <222> (317)..(317)
   <223> n is a, c, g, or t
<220>
   <221> misc_feature
   <222> (328)..(328)
   <223> n is a, c, g, or t
<220>
   <221> misc_feature
   <222> (369)..(370)
   <223> n is a, c, g, or t
<220>
   <221> misc_feature
   <222> (419)..(419)
   <223> n is a, c, g, or t
<220>
   <221> misc_feature
   <222> (433)..(433)
   <223> n is a, c, g, or t
<220>
   <221> misc_feature
   <222> (447)..(447)
   <223> n is a, c, g, or t
<220>
   <221> misc_feature
   <222> (489)..(490)
   <223> n is a, c, g, or t
<400> 31
   gtgtttttcg atcttgagac ngcggttccg accaaatcgg ggcanccttn tgcgattttg      60
   gagtttgggg ctatcttagt ttgccctatg aagctagtgg agctctatag ttactccacn     120
   ttggttcgac cnaccganct ttctctcatc tccacgctca cgaagcgacg aagcggcatt     180
   acgcgcgacg gagttctctc tgcacctaca ttctctgaaa tcgctgatga agtctacgac     240
   attctccncg gacgaatttg ggnnggacat aacataaaga gattcgattg tgtaagaata     300
   ngagatgcat ttgcagnaat tggtctcnct cccccggagc cgaaagctac aattgattca     360
   ctttcgttnn tgtctcagaa gtttgggaag agagctggtg acatgaagat ggcatcgcnt     420
   gctacatatt tcnggctagg agatcangct cacaggagct tagatgatgt ccggatgaat     480
   cttgaagtnn tcaag                                                      495
<210> 32
   <211> 2528
   <212> DNA
   <213> Boechera
<220>
   <221> misc_feature
   <222> (1)..(1)
   <223> n is a, c, g, or t
<220>
   <221> misc_feature
   <222> (5)..(6)
   <223> n is a, c, g, or t
<220>
   <221> misc_feature
   <222> (9) .. (9)
   <223> n is a, c, g, or t
<220>
   <221> misc_feature
   <222> (13)..(13)
   <223> n is a, c, g, or t
<220>
   <221> misc_feature
   <222> (23) .. (23)
   <223> n is a, c, g, or t
<220>
   <221> misc_feature
   <222> (32)..(32)
   <223> n is a, c, g, or t
<220>
   <221> misc_feature
   <222> (36)..(38)
   <223> n is a, c, g, or t
<220>
   <221> misc_feature
   <222> (45)..(45)
   <223> n is a, c, g, or t
<220>
   <221> misc_feature
   <222> (55)..(55)
   <223> n is a, c, g, or t
<220>
   <221> misc_feature
   <222> (57)..(57)
   <223> n is a, c, g, or t
<220>
   <221> misc_feature
   <222> (69) .. (69)
   <223> n is a, c, g, or t
<220>
   <221> misc_feature
   <222> (97)..(98)
   <223> n is a, c, g, or t
<220>
   <221> misc_feature
   <222> (101)..(101)
   <223> n is a, c, g, or t
<220>
   <221> misc_feature
   <222> (124)..(124)
   <223> n is a, c, g, or t
<220>
   <221> misc_feature
   <222> (131)..(131)
   <223> n is a, c, g, or t
<220>
   <221> misc_feature
   <222> (135)..(137)
   <223> n is a, c, g, or t
<220>
   <221> misc_feature
   <222> (165)..(165)
   <223> n is a, c, g, or t
<220>
   <221> misc_feature
   <222> (168)..(168)
   <223> n is a, c, g, or t
<220>
   <221> misc_feature
   <222> (172)..(172)
   <223> n is a, c, g, or t
<220>
   <221> misc_feature
   <222> (178)..(178)
   <223> n is a, c, g, or t
<220>
   <221> misc_feature
   <222> (186)..(186)
   <223> n is a, c, g, or t
<220>
   <221> misc_feature
   <222> (192)..(192)
   <223> n is a, c, g, or t
<220>
   <221> misc_feature
   <222> (213)..(213)
   <223> n is a, c, g, or t
<220>
   <221> misc_feature
   <222> (242)..(242)
   <223> n is a, c, g, or t
<220>
   <221> misc_feature
   <222> (259) .. (259)
   <223> n is a, c, g, or t
<220>
   <221> misc_feature
   <222> (264)..(264)
   <223> n is a, c, g, or t
<220>
   <221> misc_feature
   <222> (269) .. (269)
   <223> n is a, c, g, or t
<220>
   <221> misc_feature
   <222> (276)..(276)
   <223> n is a, c, g, or t
<220>
   <221> misc_feature
   <222> (279) .. (279)
   <223> n is a, c, g, or t
<220>
   <221> misc_feature
   <222> (288)..(288)
   <223> n is a, c, g, or t
<220>
   <221> misc_feature
   <222> (296)..(296)
   <223> n is a, c, g, or t
<220>
   <221> misc_feature
   <222> (354)..(354)
   <223> n is a, c, g, or t
<220>
   <221> misc_feature
   <222> (376)..(376)
   <223> n is a, c, g, or t
<220>
   <221> misc_feature
   <222> (384)..(384)
   <223> n is a, c, g, or t
<220>
   <221> misc_feature
   <222> (389)..(389)
   <223> n is a, c, g, or t
<220>
   <221> misc_feature
   <222> (408)..(409)
   <223> n is a, c, g, or t
<220>
   <221> misc_feature
   <222> (417)..(422)
   <223> n is a, c, g, or t
<220>
   <221> misc_feature
   <222> (425)..(426)
   <223> n is a, c, g, or t
<220>
   <221> misc_feature
   <222> (428)..(428)
   <223> n is a, c, g, or t
<220>
   <221> misc_feature
   <222> (430)..(431)
   <223> n is a, c, g, or t
<220>
   <221> misc_feature
   <222> (439)..(439)
   <223> n is a, c, g, or t
<220>
   <221> misc_feature
   <222> (442)..(442)
   <223> n is a, c, g, or t
<220>
   <221> misc_feature
   <222> (448)..(448)
   <223> n is a, c, g, or t
<220>
   <221> misc_feature
   <222> (451)..(451)
   <223> n is a, c, g, or t
<220>
   <221> misc_feature
   <222> (491)..(491)
   <223> n is a, c, g, or t
<220>
   <221> misc_feature
   <222> (525)..(525)
   <223> n is a, c, g, or t
<220>
   <221> misc_feature
   <222> (528)..(528)
   <223> n is a, c, g, or t
<220>
   <221> misc_feature
   <222> (544)..(544)
   <223> n is a, c, g, or t
<220>
   <221> misc_feature
   <222> (552)..(552)
   <223> n is a, c, g, or t
<220>
   <221> misc_feature
   <222> (555)..(555)
   <223> n is a, c, g, or t
<220>
   <221> misc_feature
   <222> (562)..(563)
   <223> n is a, c, g, or t
<220>
   <221> misc_feature
   <222> (567)..(567)
   <223> n is a, c, g, or t
<220>
   <221> misc_feature
   <222> (569)..(569)
   <223> n is a, c, g, or t
<220>
   <221> misc_feature
   <222> (574)..(574)
   <223> n is a, c, g, or t
<220>
   <221> misc_feature
   <222> (576)..(576)
   <223> n is a, c, g, or t
<220>
   <221> misc_feature
   <222> (585)..(585)
   <223> n is a, c, g, or t
<220>
   <221> misc_feature
   <222> (596)..(596)
   <223> n is a, c, g, or t
<220>
   <221> misc_feature
   <222> (598)..(598)
   <223> n is a, c, g, or t
<220>
   <221> misc_feature
   <222> (618)..(618)
   <223> n is a, c, g, or t
<220>
   <221> misc_feature
   <222> (626)..(626)
   <223> n is a, c, g, or t
<220>
   <221> misc_feature
   <222> (630)..(630)
   <223> n is a, c, g, or t
<220>
   <221> misc_feature
   <222> (656)..(656)
   <223> n is a, c, g, or t
<220>
   <221> misc_feature
   <222> (677)..(677)
   <223> n is a, c, g, or t
<220>
   <221> misc_feature
   <222> (695)..(695)
   <223> n is a, c, g, or t
<220>
   <221> misc_feature
   <222> (713)..(713)
   <223> n is a, c, g, or t
<220>
   <221> misc_feature
   <222> (715)..(715)
   <223> n is a, c, g, or t
<220>
   <221> misc_feature
   <222> (743)..(743)
   <223> n is a, c, g, or t
<220>
   <221> misc_feature
   <222> (747)..(747)
   <223> n is a, c, g, or t
<220>
   <221> misc_feature
   <222> (760)..(760)
   <223> n is a, c, g, or t
<220>
   <221> misc_feature
   <222> (763)..(767)
   <223> n is a, c, g, or t
<220>
   <221> misc_feature
   <222> (770)..(770)
   <223> n is a, c, g, or t
<220>
   <221> misc_feature
   <222> (773)..(773)
   <223> n is a, c, g, or t
<220>
   <221> misc_feature
   <222> (783)..(783)
   <223> n is a, c, g, or t
<220>
   <221> misc_feature
   <222> (785)..(785)
   <223> n is a, c, g, or t
<220>
   <221> misc_feature
   <222> (788)..(788)
   <223> n is a, c, g, or t
<220>
   <221> misc_feature
   <222> (795)..(795)
   <223> n is a, c, g, or t
<220>
   <221> misc_feature
   <222> (803)..(803)
   <223> n is a, c, g, or t
<220>
   <221> misc_feature
   <222> (813)..(813)
   <223> n is a, c, g, or t
<220>
   <221> misc_feature
   <222> (816)..(816)
   <223> n is a, c, g, or t
<220>
   <221> misc_feature
   <222> (831)..(832)
   <223> n is a, c, g, or t
<220>
   <221> misc_feature
   <222> (845)..(846)
   <223> n is a, c, g, or t
<220>
   <221> misc_feature
   <222> (865)..(865)
   <223> n is a, c, g, or t
<220>
   <221> misc_feature
   <222> (874)..(874)
   <223> n is a, c, g, or t
<220>
   <221> misc_feature
   <222> (896)..(896)
   <223> n is a, c, g, or t
<220>
   <221> misc_feature
   <222> (906)..(906)
   <223> n is a, c, g, or t
<220>
   <221> misc_feature
   <222> (943)..(944)
   <223> n is a, c, g, or t
<220>
   <221> misc_feature
   <222> (950)..(950)
   <223> n is a, c, g, or t
<220>
   <221> misc_feature
   <222> (956)..(956)
   <223> n is a, c, g, or t
<220>
   <221> misc_feature
   <222> (962)..(962)
   <223> n is a, c, g, or t
<220>
   <221> misc_feature
   <222> (967)..(967)
   <223> n is a, c, g, or t
<220>
   <221> misc_feature
   <222> (980)..(980)
   <223> n is a, c, g, or t
<220>
   <221> misc_feature
   <222> (987)..(987)
   <223> n is a, c, g, or t
<220>
   <221> misc_feature
   <222> (1007)..(1007)
   <223> n is a, c, g, or t
<220>
   <221> misc_feature
   <222> (1013)..(1013)
   <223> n is a, c, g, or t
<220>
   <221> misc_feature
   <222> (1017)..(1017)
   <223> n is a, c, g, or t
<220>
   <221> misc_feature
   <222> (1020)..(1020)
   <223> n is a, c, g, or t
<220>
   <221> misc_feature
   <222> (1028)..(1028)
   <223> n is a, c, g, or t
<220>
   <221> misc_feature
   <222> (1036)..(1036)
   <223> n is a, c, g, or t
<220>
   <221> misc_feature
   <222> (1039)..(1039)
   <223> n is a, c, g, or t
<220>
   <221> misc_feature
   <222> (1044)..(1045)
   <223> n is a, c, g, or t
<220>
   <221> misc_feature
   <222> (1057)..(1057)
   <223> n is a, c, g, or t
<220>
   <221> misc_feature
   <222> (1066)..(1066)
   <223> n is a, c, g, or t
<220>
   <221> misc_feature
   <222> (1072)..(1072)
   <223> n is a, c, g, or t
<220>
   <221> misc_feature
   <222> (1075)..(1075)
   <223> n is a, c, g, or t
<220>
   <221> misc_feature
   <222> (1081)..(1082)
   <223> n is a, c, g, or t
<220>
   <221> misc_feature
   <222> (1085)..(1085)
   <223> n is a, c, g, or t
<220>
   <221> misc_feature
   <222> (1088)..(1088)
   <223> n is a, c, g, or t
<220>
   <221> misc_feature
   <222> (1098)..(1098)
   <223> n is a, c, g, or t
<220>
   <221> misc_feature
   <222> (1130)..(1130)
   <223> n is a, c, g, or t
<220>
   <221> misc_feature
   <222> (1154)..(1154)
   <223> n is a, c, g, or t
<220>
   <221> misc_feature
   <222> (1214)..(1214)
   <223> n is a, c, g, or t
<220>
   <221> misc_feature
   <222> (1216)..(1217)
   <223> n is a, c, g, or t
<220>
   <221> misc_feature
   <222> (1230)..(1230)
   <223> n is a, c, g, or t
<220>
   <221> misc_feature
   <222> (1232)..(1232)
   <223> n is a, c, g, or t
<220>
   <221> misc_feature
   <222> (1235)..(1235)
   <223> n is a, c, g, or t
<220>
   <221> misc_feature
   <222> (1247)..(1247)
   <223> n is a, c, g, or t
<220>
   <221> misc_feature
   <222> (1278)..(1278)
   <223> n is a, c, g, or t
<220>
   <221> misc_feature
   <222> (1299)..(1299)
   <223> n is a, c, g, or t
<220>
   <221> misc_feature
   <222> (1314)..(1314)
   <223> n is a, c, g, or t
<220>
   <221> misc_feature
   <222> (1324)..(1324)
   <223> n is a, c, g, or t
<220>
   <221> misc_feature
   <222> (1348)..(1348)
   <223> n is a, c, g, or t
<220>
   <221> misc_feature
   <222> (1350)..(1350)
   <223> n is a, c, g, or t
<220>
   <221> misc_feature
   <222> (1353)..(1353)
   <223> n is a, c, g, or t
<220>
   <221> misc_feature
   <222> (1357)..(1357)
   <223> n is a, c, g, or t
<220>
   <221> misc_feature
   <222> (1406)..(1407)
   <223> n is a, c, g, or t
<220>
   <221> misc_feature
   <222> (1422)..(1422)
   <223> n is a, c, g, or t
<220>
   <221> misc_feature
   <222> (1437)..(1437)
   <223> n is a, c, g, or t
<220>
   <221> misc_feature
   <222> (1484)..(1484)
   <223> n is a, c, g, or t
<220>
   <221> misc_feature
   <222> (1518)..(1518)
   <223> n is a, c, g, or t
<220>
   <221> misc_feature
   <222> (1525)..(1525)
   <223> n is a, c, g, or t
<220>
   <221> misc_feature
   <222> (1527)..(1527)
   <223> n is a, c, g, or t
<220>
   <221> misc_feature
   <222> (1538)..(1538)
   <223> n is a, c, g, or t
<220>
   <221> misc_feature
   <222> (1551)..(1551)
   <223> n is a, c, g, or t
<220>
   <221> misc_feature
   <222> (1593)..(1593)
   <223> n is a, c, g, or t
<220>
   <221> misc_feature
   <222> (1605)..(1605)
   <223> n is a, c, g, or t
<220>
   <221> misc_feature
   <222> (1612)..(1612)
   <223> n is a, c, g, or t
<220>
   <221> misc_feature
   <222> (1620)..(1621)
   <223> n is a, c, g, or t
<220>
   <221> misc_feature
   <222> (1636)..(1636)
   <223> n is a, c, g, or t
<220>
   <221> misc_feature
   <222> (1638)..(1638)
   <223> n is a, c, g, or t
<220>
   <221> misc_feature
   <222> (1651)..(1651)
   <223> n is a, c, g, or t
<220>
   <221> misc_feature
   <222> (1654)..(1654)
   <223> n is a, c, g, or t
<220>
   <221> misc_feature
   <222> (1672)..(1672)
   <223> n is a, c, g, or t
<220>
   <221> misc_feature
   <222> (1680)..(1680)
   <223> n is a, c, g, or t
<220>
   <221> misc_feature
   <222> (1683)..(1683)
   <223> n is a, c, g, or t
<220>
   <221> misc_feature
   <222> (1699)..(1699)
   <223> n is a, c, g, or t
<220>
   <221> misc_feature
   <222> (1701)..(1701)
   <223> n is a, c, g, or t
<220>
   <221> misc_feature
   <222> (1709)..(1709)
   <223> n is a, c, g, or t
<220>
   <221> misc_feature
   <222> (1711)..(1711)
   <223> n is a, c, g, or t
<220>
   <221> misc_feature
   <222> (1734)..(1734)
   <223> n is a, c, g, or t
<220>
   <221> misc_feature
   <222> (1742)..(1742)
   <223> n is a, c, g, or t
<220>
   <221> misc_feature
   <222> (1761)..(1761)
   <223> n is a, c, g, or t
<220>
   <221> misc_feature
   <222> (1779)..(1779)
   <223> n is a, c, g, or t
<220>
   <221> misc_feature
   <222> (1782)..(1782)
   <223> n is a, c, g, or t
<220>
   <221> misc_feature
   <222> (1820)..(1822)
   <223> n is a, c, g, or t
<220>
   <221> misc_feature
   <222> (1828)..(1828)
   <223> n is a, c, g, or t
<220>
   <221> misc_feature
   <222> (1862)..(1863)
   <223> n is a, c, g, or t
<220>
   <221> misc_feature
   <222> (1869)..(1870)
   <223> n is a, c, g, or t
<220>
   <221> misc_feature
   <222> (1881)..(1881)
   <223> n is a, c, g, or t
<220>
   <221> misc_feature
   <222> (1889)..(1889)
   <223> n is a, c, g, or t
<220>
   <221> misc_feature
   <222> (1894)..(1894)
   <223> n is a, c, g, or t
<220>
   <221> misc_feature
   <222> (1917)..(1917)
   <223> n is a, c, g, or t
<220>
   <221> misc_feature
   <222> (1921)..(1921)
   <223> n is a, c, g, or t
<220>
   <221> misc_feature
   <222> (1940)..(1940)
   <223> n is a, c, g, or t
<220>
   <221> misc_feature
   <222> (1952)..(1952)
   <223> n is a, c, g, or t
<220>
   <221> misc_feature
   <222> (1965)..(1965)
   <223> n is a, c, g, or t
<220>
   <221> misc_feature
   <222> (1971)..(1971)
   <223> n is a, c, g, or t
<220>
   <221> misc_feature
   <222> (1976)..(1976)
   <223> n is a, c, g, or t
<220>
   <221> misc_feature
   <222> (1979)..(1979)
   <223> n is a, c, g, or t
<220>
   <221> misc_feature
   <222> (1993)..(1993)
   <223> n is a, c, g, or t
<220>
   <221> misc_feature
   <222> (2006)..(2006)
   <223> n is a, c, g, or t
<220>
   <221> misc_feature
   <222> (2013)..(2014)
   <223> n is a, c, g, or t
<220>
   <221> misc_feature
   <222> (2016)..(2016)
   <223> n is a, c, g, or t
<220>
   <221> misc_feature
   <222> (2023)..(2023)
   <223> n is a, c, g, or t
<220>
   <221> misc_feature
   <222> (2029) .. (2029)
   <223> n is a, c, g, or t
<220>
   <221> misc_feature
   <222> (2056)..(2057)
   <223> n is a, c, g, or t
<220>
   <221> misc_feature
   <222> (2069)..(2071)
   <223> n is a, c, g, or t
<220>
   <221> misc_feature
   <222> (2081)..(2082)
   <223> n is a, c, g, or t
<220>
   <221> misc_feature
   <222> (2108)..(2108)
   <223> n is a, c, g, or t
<220>
   <221> misc_feature
   <222> (2113)..(2113)
   <223> n is a, c, g, or t
<220>
   <221> misc_feature
   <222> (2142)..(2142)
   <223> n is a, c, g, or t
<220>
   <221> misc_feature
   <222> (2157)..(2157)
   <223> n is a, c, g, or t
<220>
   <221> misc_feature
   <222> (2169) .. (2169)
   <223> n is a, c, g, or t
<220>
   <221> misc_feature
   <222> (2184)..(2184)
   <223> n is a, c, g, or t
<220>
   <221> misc_feature
   <222> (2193)..(2193)
   <223> n is a, c, g, or t
<220>
   <221> misc_feature
   <222> (2208)..(2208)
   <223> n is a, c, g, or t
<220>
   <221> misc_feature
   <222> (2214)..(2216)
   <223> n is a, c, g, or t
<220>
   <221> misc_feature
   <222> (2225)..(2226)
   <223> n is a, c, g, or t
<220>
   <221> misc_feature
   <222> (2228)..(2228)
   <223> n is a, c, g, or t
<220>
   <221> misc_feature
   <222> (2230)..(2230)
   <223> n is a, c, g, or t
<220>
   <221> misc_feature
   <222> (2233)..(2233)
   <223> n is a, c, g, or t
<220>
   <221> misc_feature
   <222> (2236)..(2236)
   <223> n is a, c, g, or t
<220>
   <221> misc_feature
   <222> (2238)..(2238)
   <223> n is a, c, g, or t
<220>
   <221> misc_feature
   <222> (2240)..(2240)
   <223> n is a, c, g, or t
<220>
   <221> misc_feature
   <222> (2248)..(2248)
   <223> n is a, c, g, or t
<220>
   <221> misc_feature
   <222> (2260)..(2260)
   <223> n is a, c, g, or t
<220>
   <221> misc_feature
   <222> (2270)..(2270)
   <223> n is a, c, g, or t
<220>
   <221> misc_feature
   <222> (2277)..(2277)
   <223> n is a, c, g, or t
<220>
   <221> misc_feature
   <222> (2282)..(2282)
   <223> n is a, c, g, or t
<220>
   <221> misc_feature
   <222> (2295)..(2295)
   <223> n is a, c, g, or t
<220>
   <221> misc_feature
   <222> (2350)..(2350)
   <223> n is a, c, g, or t
<220>
   <221> misc_feature
   <222> (2362)..(2363)
   <223> n is a, c, g, or t
<220>
   <221> misc_feature
   <222> (2368)..(2368)
   <223> n is a, c, g, or t
<220>
   <221> misc_feature
   <222> (2372)..(2372)
   <223> n is a, c, g, or t
<220>
   <221> misc_feature
   <222> (2383)..(2386)
   <223> n is a, c, g, or t
<220>
   <221> misc_feature
   <222> (2396)..(2397)
   <223> n is a, c, g, or t
<220>
   <221> misc_feature
   <222> (2401)..(2402)
   <223> n is a, c, g, or t
<220>
   <221> misc_feature
   <222> (2415)..(2415)
   <223> n is a, c, g, or t
<220>
   <221> misc_feature
   <222> (2461)..(2461)
   <223> n is a, c, g, or t
<220>
   <221> misc_feature
   <222> (2507)..(2507)
   <223> n is a, c, g, or t
<220>
   <221> misc_feature
   <222> (2513)..(2513)
   <223> n is a, c, g, or t
<220>
   <221> misc_feature
   <222> (2520)..(2520)
   <223> n is a, c, g, or t
<220>
   <221> misc_feature
   <222> (2522)..(2522)
   <223> n is a, c, g, or t
<220>
   <221> misc_feature
   <222> (2524)..(2524)
   <223> n is a, c, g, or t
<400> 32
   ncgtnncgnt gcntctctca agnttagatt tntttnnncg taaanagagg aggancnatt      60
   gctttaaanc ccaccaatta gctccttcac tctcagnnct naacaatggc ttcgactctg     120
   ggcngcgatg ngagnnncga gatagtgttt ttcgatcttg agacngcngt tncgaccnaa     180
   tcgggncagc cntttgcgat tttggagttt ggngctatct tagtttgccc tatgaagcta     240
   gnggagctct atagttacnc cacnttggnt cgaccnacng atctttcnct catctncacg     300
   ctcacgaagc gacgaagcgg cattacgcgc gacggagttc tctctgcacc tacnttctct     360
   gaaatcgctg atgaantcta cganattcnc cacggtaagg gtttctcnnt ttttttnnnn     420
   nnctnncncn ntctctctna cncgaagnta naagtattga ttttggtgtt tctgtaggac     480
   gaatttgggc nggacataac ataaagagat tcgattgtgt aagantanga gatgcatttg     540
   cagnaattgg tntcnctccc cnngagncna aagntncaat tgatncactt tcgttntngt     600
   ctcagaagtt tgggaagnga gctggngacn tgaaggtctc tcttttttcg tcttcncgat     660
   gataaatctc aaagccnata gcttccttgt tatcnttata gatatgaatt tcnangtaac     720
   ttcaaagatt catcactcat canagtngct aaaatttacn ctnnnnnaan aangtagatg     780
   gcntngcntg ctacntattt cgngctagga gancangctc acaggtaaaa nnagtaaacg     840
   atacnntgtg ccttttaacg attcnccagt tgtntcaata tgggactaaa catggntatg 900
   attcancagg agcttagatg atgtccggat gaatcttgaa gtnntcaagn actgtncaac 960
   cntcttnttt ctggtattgn tgtcttntca tttcttgaat aatgatnaac tcntaanttn 1020
   aaaagganta gattanagng gttnngacat atctganttc tgtctncagt tntgnaaaag 1080
   nnggntcnat cttccttnca gaccacaact ttgcaagccg taaacatggn ttgcaacttg 1140
   caagtatagt ttgntatatc actgagttta agtacttggt gtttgcagga gtccagtgtt 1200
   cctgacattc ttananncat gagctggttn tnccnaagaa aaagtcngag aacacgaagt 1260
   aatgagaagt cactgccnaa tggagtcaga gaaagcccna cttcttcctc ttcnagccct 1320
   aaanctgatc cgagttcgtc ttctgtanan gcnacanctg tcaaaaacca tcccatcatt 1380
   tctcttctga cggaatgctc agaaanngat acatctagtt gngaaataga tccatcngac 1440
   ataaccactc taataagtaa actacatatt ggaactctta aganagatgc tgcggacgaa 1500
   gccaaaactg tgagagangc tgcgnangaa gccaaaantg taagacagca nggtgaatca 1560
   accgatccca atgccaaaga tgaatcattt ttnggcgtta atgangtatc tntttctagn 1620
   ntcagggcaa gtcttntncc gttatatcgt nggngtctga gaatggagct gnttcacaan 1680
   ganacccctc tacatctcng ntggtatanc ntgaaaattc ggtttggaat aagncggaag 1740
   tntgtggatc atgtaggtcg nccaaagatg aatattgtng tngacatacc tcctgattta 1800
   tgcaagatct tggacgcatn nnatgctnct gcgcataact tactgattga ctcaagcaca 1860
   anntcagann ggaggcctac ngttatgang aaanaaggct ttgccaacta tcccacngcc 1920
   ngactgcagt aagtattcan cactctctct gnccttttac atacnagcat naatcnacng 1980
   gagagtctct aanaccatct ccaacnctac tcnntnttca ccnccaaant ctattttgga 2040
   gttaaatccc tccaannctt gcaaaatann natcttcaaa nnttttctcc atatttggag 2100
   attttgantt ttnaagtcat gactccattt tggagttggg tnggagaaaa acacaantcc 2160
   aaaatagant tacttcattt tggngtaaaa aantgaagaa atgggttnga gatnnnctaa 2220
   cctcnntnan cantcntntn ttgttggnag aataagctcn gaatccaatn gaacccnggt 2280
   anaccaaaaa gaagnacctt tgggaaccaa tcaaaagctc gatttcagta gcgataattt 2340
   tgaaaagctn gagtcagcac tnnttccngg tnccctggtt gannnnttct tctcanncga 2400
   nncttacgat tatangaaaa tggtagggat acgtctagca gccagaaagt tggtaatcca 2460
   nctgaagaaa tgatctagcc aaggaaaaat cattcctctg tctcttnctg gtnagtcggn 2520
   gngnactt 2528
<210> 33
   <211> 1521
   <212> DNA
   <213> Boechera
<220>
   <221> misc_feature
   <222> (19)..(19)
   <223> n is a, c, g, or t
<220>
   <221> misc_feature
   <222> (26)..(26)
   <223> n is a, c, g, or t
<220>
   <221> misc_feature
   <222> (30)..(32)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (67)..(67)
   <223> n is a, c, g, or t
<220>
   <221> misc_feature
   <222> (73)..(73)
   <223> n is a, c, g, or t
<220>
   <221> misc_feature
   <222> (81)..(81)
   <223> n is a, c, g, or t
<220>
   <221> misc_feature
   <222> (87)..(87)
   <223> n is a, c, g, or t
<220>
   <221> misc_feature
   <222> (108)..(108)
   <223> n is a, c, g, or t
<220>
   <221> misc_feature
   <222> (137)..(137)
   <223> n is a, c, g, or t
<220>
   <221> misc_feature
   <222> (154)..(154)
   <223> n is a, c, g, or t
<220>
   <221> misc_feature
   <222> (159)..(159)
   <223> n is a, c, g, or t
<220>
   <221> misc_feature
   <222> (164)..(164)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (183)..(183)
   <223> n is a, c, g, or t
<220>
   <221> misc_feature
   <222> (191)..(191)
   <223> n is a, c, g, or t
<220>
   <221> misc_feature
   <222> (249) .. (249)
   <223> n is a, c, g, or t
<220>
   <221> misc_feature
   <222> (271)..(271)
   <223> n is a, c, g, or t
<220>
   <221> misc_feature
   <222> (279) .. (279)
   <223> n is a, c, g, or t
<220>
   <221> misc_feature
   <222> (284)..(284)
   <223> n is a, c, g, or t
<220>
   <221> misc_feature
   <222> (303)..(303)
   <223> n is a, c, g, or t
<220>
   <221> misc_feature
   <222> (337)..(337)
   <223> n is a, c, g, or t
<220>
   <221> misc_feature
   <222> (340)..(340)
   <223> n is a, c, g, or t
<220>
   <221> misc_feature
   <222> (356)..(356)
   <223> n is a, c, g, or t
<220>
   <221> misc_feature
   <222> (364)..(364)
   <223> n is a, c, g, or t
<220>
   <221> misc_feature
   <222> (367)..(367)
   <223> n is a, c, g, or t
<220>
   <221> misc_feature
   <222> (374)..(375)
   <223> n is a, c, g, or t
<220>
   <221> misc_feature
   <222> (379)..(379)
   <223> n is a, c, g, or t
<220>
   <221> misc_feature
   <222> (381)..(381)
   <223> n is a, c, g, or t
<220>
   <221> misc_feature
   <222> (386)..(386)
   <223> n is a, c, g, or t
<220>
   <221> misc_feature
   <222> (388)..(388)
   <223> n is a, c, g, or t
<220>
   <221> misc_feature
   <222> (397)..(397)
   <223> n is a, c, g, or t
<220>
   <221> misc_feature
   <222> (408)..(408)
   <223> n is a, c, g, or t
<220>
   <221> misc_feature
   <222> (410)..(410)
   <223> n is a, c, g, or t
<220>
   <221> misc_feature
   <222> (430)..(430)
   <223> n is a, c, g, or t
<220>
   <221> misc_feature
   <222> (438)..(438)
   <223> n is a, c, g, or t
<220>
   <221> misc_feature
   <222> (442)..(442)
   <223> n is a, c, g, or t
<220>
   <221> misc_feature
   <222> (453)..(453)
   <223> n is a, c, g, or t
<220>
   <221> misc_feature
   <222> (455)..(455)
   <223> n is a, c, g, or t
<220>
   <221> misc_feature
   <222> (458)..(458)
   <223> n is a, c, g, or t
<220>
   <221> misc_feature
   <222> (465)..(465)
   <223> n is a, c, g, or t
<220>
   <221> misc_feature
   <222> (473)..(473)
   <223> n is a, c, g, or t
<220>
   <221> misc_feature
   <222> (483)..(483)
   <223> n is a, c, g, or t
<220>
   <221> misc_feature
   <222> (486)..(486)
   <223> n is a, c, g, or t
<220>
   <221> misc_feature
   <222> (528)..(529)
   <223> n is a, c, g, or t
<220>
   <221> misc_feature
   <222> (535)..(535)
   <223> n is a, c, g, or t
<220>
   <221> misc_feature
   <222> (541)..(541)
   <223> n is a, c, g, or t
<220>
   <221> misc_feature
   <222> (547)..(547)
   <223> n is a, c, g, or t
<220>
   <221> misc_feature
   <222> (552)..(552)
   <223> n is a, c, g, or t
<220>
   <221> misc_feature
   <222> (584)..(584)
   <223> n is a, c, g, or t
<220>
   <221> misc_feature
   <222> (586)..(587)
   <223> n is a, c, g, or t
<220>
   <221> misc_feature
   <222> (600)..(600)
   <223> n is a, c, g, or t
<220>
   <221> misc_feature
   <222> (602)..(602)
   <223> n is a, c, g, or t
<220>
   <221> misc_feature
   <222> (605)..(605)
   <223> n is a, c, g, or t
<220>
   <221> misc_feature
   <222> (617)..(617)
   <223> n is a, c, g, or t
<220>
   <221> misc_feature
   <222> (648)..(648)
   <223> n is a, c, g, or t
<220>
   <221> misc_feature
   <222> (669)..(669)
   <223> n is a, c, g, or t
<220>
   <221> misc_feature
   <222> (684)..(684)
   <223> n is a, c, g, or t
<220>
   <221> misc_feature
   <222> (694)..(694)
   <223> n is a, c, g, or t
<220>
   <221> misc_feature
   <222> (718)..(718)
   <223> n is a, c, g, or t
<220>
   <221> misc_feature
   <222> (720)..(720)
   <223> n is a, c, g, or t
<220>
   <221> misc_feature
   <222> (723)..(723)
   <223> n is a, c, g, or t
<220>
   <221> misc_feature
   <222> (727)..(727)
   <223> n is a, c, g, or t
<220>
   <221> misc_feature
   <222> (776)..(777)
   <223> n is a, c, g, or t
<220>
   <221> misc_feature
   <222> (792)..(792)
   <223> n is a, c, g, or t
<220>
   <221> misc_feature
   <222> (807)..(807)
   <223> n is a, c, g, or t
<220>
   <221> misc_feature
   <222> (854)..(854)
   <223> n is a, c, g, or t
<220>
   <221> misc_feature
   <222> (888)..(888)
   <223> n is a, c, g, or t
<220>
   <221> misc_feature
   <222> (895)..(895)
   <223> n is a, c, g, or t
<220>
   <221> misc_feature
   <222> (897)..(897)
   <223> n is a, c, g, or t
<220>
   <221> misc_feature
   <222> (908)..(908)
   <223> n is a, c, g, or t
<220>
   <221> misc_feature
   <222> (921)..(921)
   <223> n is a, c, g, or t
<220>
   <221> misc_feature
   <222> (963)..(963)
   <223> n is a, c, g, or t
<220>
   <221> misc_feature
   <222> (975)..(975)
   <223> n is a, c, g, or t
<220>
   <221> misc_feature
   <222> (982)..(982)
   <223> n is a, c, g, or t
<220>
   <221> misc_feature
   <222> (990)..(991)
   <223> n is a, c, g, or t
<220>
   <221> misc_feature
   <222> (1006)..(1006)
   <223> n is a, c, g, or t
<220>
   <221> misc_feature
   <222> (1008)..(1008)
   <223> n is a, c, g, or t
<220>
   <221> misc_feature
   <222> (1021)..(1021)
   <223> n is a, c, g, or t
<220>
   <221> misc_feature
   <222> (1024)..(1024)
   <223> n is a, c, g, or t
<220>
   <221> misc_feature
   <222> (1042)..(1042)
   <223> n is a, c, g, or t
<220>
   <221> misc_feature
   <222> (1050)..(1050)
   <223> n is a, c, g, or t
<220>
   <221> misc_feature
   <222> (1053)..(1053)
   <223> n is a, c, g, or t
<220>
   <221> misc_feature
   <222> (1069)..(1069)
   <223> n is a, c, g, or t
<220>
   <221> misc_feature
   <222> (1071)..(1071)
   <223> n is a, c, g, or t
<220>
   <221> misc_feature
   <222> (1079)..(1079)
   <223> n is a, c, g, or t
<220>
   <221> misc_feature
   <222> (1081)..(1081)
   <223> n is a, c, g, or t
<220>
   <221> misc_feature
   <222> (1104)..(1104)
   <223> n is a, c, g, or t
<220>
   <221> misc_feature
   <222> (1112)..(1112)
   <223> n is a, c, g, or t
<220>
   <221> misc_feature
   <222> (1131)..(1131)
   <223> n is a, c, g, or t
<220>
   <221> misc_feature
   <222> (1149)..(1149)
   <223> n is a, c, g, or t
<220>
   <221> misc_feature
   <222> (1152)..(1152)
   <223> n is a, c, g, or t
<220>
   <221> misc_feature
   <222> (1190)..(1192)
   <223> n is a, c, g, or t
<220>
   <221> misc_feature
   <222> (1198)..(1198)
   <223> n is a, c, g, or t
<220>
   <221> misc_feature
   <222> (1232)..(1233)
   <223> n is a, c, g, or t
<220>
   <221> misc_feature
   <222> (1239)..(1240)
   <223> n is a, c, g, or t
<220>
   <221> misc_feature
   <222> (1251)..(1251)
   <223> n is a, c, g, or t
<220>
   <221> misc_feature
   <222> (1259)..(1259)
   <223> n is a, c, g, or t
<220>
   <221> misc_feature
   <222> (1264)..(1264)
   <223> n is a, c, g, or t
<220>
   <221> misc_feature
   <222> (1287)..(1287)
   <223> n is a, c, g, or t
<220>
   <221> misc_feature
   <222> (1291)..(1291)
   <223> n is a, c, g, or t
<220>
   <221> misc_feature
   <222> (1308)..(1308)
   <223> n is a, c, g, or t
<220>
   <221> misc_feature
   <222> (1318)..(1318)
   <223> n is a, c, g, or t
<220>
   <221> misc_feature
   <222> (1325)..(1325)
   <223> n is a, c, g, or t
<220>
   <221> misc_feature
   <222> (1330)..(1330)
   <223> n is a, c, g, or t
<220>
   <221> misc_feature
   <222> (1343)..(1343)
   <223> n is a, c, g, or t
<220>
   <221> misc_feature
   <222> (1398)..(1398)
   <223> n is a, c, g, or t
<220>
   <221> misc_feature
   <222> (1410)..(1411)
   <223> n is a, c, g, or t
<220>
   <221> misc_feature
   <222> (1416)..(1416)
   <223> n is a, c, g, or t
<220>
   <221> misc_feature
   <222> (1420)..(1420)
   <223> n is a, c, g, or t
<220>
   <221> misc_feature
   <222> (1431)..(1434)
   <223> n is a, c, g, or t
<220>
   <221> misc_feature
   <222> (1444)..(1445)
   <223> n is a, c, g, or t
<220>
   <221> misc_feature
   <222> (1449)..(1450)
   <223> n is a, c, g, or t
<220>
   <221> misc_feature
   <222> (1463)..(1463)
   <223> n is a, c, g, or t
<220>
   <221> misc_feature
   <222> (1509)..(1509)
   <223> n is a, c, g, or t
<400> 33
   atggcttcga ctctgggcng cgatgngagn nncgagatag tgtttttcga tcttgagacn      60
   gcngttncga ccnaatcggg ncagccnttt gcgattttgg agtttggngc tatcttagtt     120
   tgccctatga agctagngga gctctatagt tacnccacnt tggntcgacc nacngatctt     180
   tcnctcatct ncacgctcac gaagcgacga agcggcatta cgcgcgacgg agttctctct     240
   gcacctacnt tctctgaaat cgctgatgaa ntctacgana ttcnccacgg acgaatttgg     300
   gcnggacata acataaagag attcgattgt gtaagantan gagatgcatt tgcagnaatt     360
   ggtntcnctc cccnngagnc naaagntnca attgatncac tttcgttntn gtctcagaag     420
   tttgggaagn gagctggnga cntgaagatg gcntngcntg ctacntattt cgngctagga     480
   gancangctc acaggagctt agatgatgtc cggatgaatc ttgaagtnnt caagnactgt     540
   ncaaccntct tntttctgga gtccagtgtt cctgacattc ttananncat gagctggttn     600
   tnccnaagaa aaagtcngag aacacgaagt aatgagaagt cactgccnaa tggagtcaga     660
   gaaagcccna cttcttcctc ttcnagccct aaanctgatc cgagttcgtc ttctgtanan     720
   gcnacanctg tcaaaaacca tcccatcatt tctcttctga cggaatgctc agaaanngat     780
   acatctagtt gngaaataga tccatcngac ataaccactc taataagtaa actacatatt     840
   ggaactctta aganagatgc tgcggacgaa gccaaaactg tgagagangc tgcgnangaa     900
   gccaaaantg taagacagca nggtgaatca accgatccca atgccaaaga tgaatcattt     960
   ttnggcgtta atgangtatc tntttctagn ntcagggcaa gtcttntncc gttatatcgt    1020
   nggngtctga gaatggagct gnttcacaan ganacccctc tacatctcng ntggtatanc    1080
   ntgaaaattc ggtttggaat aagncggaag tntgtggatc atgtaggtcg nccaaagatg    1140
   aatattgtng tngacatacc tcctgattta tgcaagatct tggacgcatn nnatgctnct    1200
   gcgcataact tactgattga ctcaagcaca anntcagann ggaggcctac ngttatgang    1260
   aaanaaggct ttgccaacta tcccacngcc ngactgcaaa taagctcnga atccaatnga    1320
   acccnggtan accaaaaaga agnacctttg ggaaccaatc aaaagctcga tttcagtagc    1380
   gataattttg aaaagctnga gtcagcactn nttccnggtn ccctggttga nnnnttcttc    1440
   tcanncgann cttacgatta tangaaaatg gtagggatac gtctagcagc cagaaagttg    1500
   gtaatccanc tgaagaaatg a                                              1521
<210> 34
   <211> 2498
   <212> DNA
   <213> Boechera
<220>
   <221> misc_feature
   <222> (1)..(1)
   <223> n is a, c, g, or t
<220>
   <221> misc_feature
   <222> (5)..(6)
   <223> n is a, c, g, or t
<220>
   <221> misc_feature
   <222> (9) .. (9)
   <223> n is a, c, g, or t
<220>
   <221> misc_feature
   <222> (13)..(13)
   <223> n is a, c, g, or t
<220>
   <221> misc_feature
   <222> (23)..(23)
   <223> n is a, c, g, or t
<220>
   <221> misc_feature
   <222> (32)..(32)
   <223> n is a, c, g, or t
<220>
   <221> misc_feature
   <222> (36)..(38)
   <223> n is a, c, g, or t
<220>
   <221> misc_feature
   <222> (45)..(45)
   <223> n is a, c, g, or t
<220>
   <221> misc_feature
   <222> (55)..(55)
   <223> n is a, c, g, or t
<220>
   <221> misc_feature
   <222> (57)..(57)
   <223> n is a, c, g, or t
<220>
   <221> misc_feature
   <222> (69) .. (69)
   <223> n is a, c, g, or t
<220>
   <221> misc_feature
   <222> (97)..(98)
   <223> n is a, c, g, or t
<220>
   <221> misc_feature
   <222> (101)..(101)
   <223> n is a, c, g, or t
<220>
   <221> misc_feature
   <222> (124)..(124)
   <223> n is a, c, g, or t
<220>
   <221> misc_feature
   <222> (131)..(131)
   <223> n is a, c, g, or t
<220>
   <221> misc_feature
   <222> (135)..(137)
   <223> n is a, c, g, or t
<220>
   <221> misc_feature
   <222> (165)..(165)
   <223> n is a, c, g, or t
<220>
   <221> misc_feature
   <222> (168)..(168)
   <223> n is a, c, g, or t
<220>
   <221> misc_feature
   <222> (172)..(172)
   <223> n is a, c, g, or t
<220>
   <221> misc_feature
   <222> (178)..(178)
   <223> n is a, c, g, or t
<220>
   <221> misc_feature
   <222> (186)..(186)
   <223> n is a, c, g, or t
<220>
   <221> misc_feature
   <222> (192)..(192)
   <223> n is a, c, g, or t
<220>
   <221> misc_feature
   <222> (213)..(213)
   <223> n is a, c, g, or t
<220>
   <221> misc_feature
   <222> (242)..(242)
   <223> n is a, c, g, or t
<220>
   <221> misc_feature
   <222> (259) .. (259)
   <223> n is a, c, g, or t
<220>
   <221> misc_feature
   <222> (264)..(264)
   <223> n is a, c, g, or t
<220>
   <221> misc_feature
   <222> (269) .. (269)
   <223> n is a, c, g, or t
<220>
   <221> misc_feature
   <222> (276)..(276)
   <223> n is a, c, g, or t
<220>
   <221> misc_feature
   <222> (279)..(279)
   <223> n is a, c, g, or t
<220>
   <221> misc_feature
   <222> (288)..(288)
   <223> n is a, c, g, or t
<220>
   <221> misc_feature
   <222> (296)..(296)
   <223> n is a, c, g, or t
<220>
   <221> misc_feature
   <222> (354)..(354)
   <223> n is a, c, g, or t
<220>
   <221> misc_feature
   <222> (376)..(376)
   <223> n is a, c, g, or t
<220>
   <221> misc_feature
   <222> (384)..(384)
   <223> n is a, c, g, or t
<220>
   <221> misc_feature
   <222> (389)..(389)
   <223> n is a, c, g, or t
<220>
   <221> misc_feature
   <222> (408)..(409)
   <223> n is a, c, g, or t
<220>
   <221> misc_feature
   <222> (417)..(422)
   <223> n is a, c, g, or t
<220>
   <221> misc_feature
   <222> (425)..(426)
   <223> n is a, c, g, or t
<220>
   <221> misc_feature
   <222> (428)..(428)
   <223> n is a, c, g, or t
<220>
   <221> misc_feature
   <222> (430)..(431)
   <223> n is a, c, g, or t
<220>
   <221> misc_feature
   <222> (439)..(439)
   <223> n is a, c, g, or t
<220>
   <221> misc_feature
   <222> (442)..(442)
   <223> n is a, c, g, or t
<220>
   <221> misc_feature
   <222> (448)..(448)
   <223> n is a, c, g, or t
<220>
   <221> misc_feature
   <222> (451)..(451)
   <223> n is a, c, g, or t
<220>
   <221> misc_feature
   <222> (491)..(491)
   <223> n is a, c, g, or t
<220>
   <221> misc_feature
   <222> (525)..(525)
   <223> n is a, c, g, or t
<220>
   <221> misc_feature
   <222> (528)..(528)
   <223> n is a, c, g, or t
<220>
   <221> misc_feature
   <222> (544)..(544)
   <223> n is a, c, g, or t
<220>
   <221> misc_feature
   <222> (552)..(552)
   <223> n is a, c, g, or t
<220>
   <221> misc_feature
   <222> (555)..(555)
   <223> n is a, c, g, or t
<220>
   <221> misc_feature
   <222> (562)..(563)
   <223> n is a, c, g, or t
<220>
   <221> misc_feature
   <222> (567)..(567)
   <223> n is a, c, g, or t
<220>
   <221> misc_feature
   <222> (569)..(569)
   <223> n is a, c, g, or t
<220>
   <221> misc_feature
   <222> (574)..(574)
   <223> n is a, c, g, or t
<220>
   <221> misc_feature
   <222> (576)..(576)
   <223> n is a, c, g, or t
<220>
   <221> misc_feature
   <222> (585)..(585)
   <223> n is a, c, g, or t
<220>
   <221> misc_feature
   <222> (596)..(596)
   <223> n is a, c, g, or t
<220>
   <221> misc_feature
   <222> (598)..(598)
   <223> n is a, c, g, or t
<220>
   <221> misc_feature
   <222> (618)..(618)
   <223> n is a, c, g, or t
<220>
   <221> misc_feature
   <222> (626)..(626)
   <223> n is a, c, g, or t
<220>
   <221> misc_feature
   <222> (630)..(630)
   <223> n is a, c, g, or t
<220>
   <221> misc_feature
   <222> (656)..(656)
   <223> n is a, c, g, or t
<220>
   <221> misc_feature
   <222> (677)..(677)
   <223> n is a, c, g, or t
<220>
   <221> misc_feature
   <222> (695)..(695)
   <223> n is a, c, g, or t
<220>
   <221> misc_feature
   <222> (713)..(713)
   <223> n is a, c, g, or t
<220>
   <221> misc_feature
   <222> (715)..(715)
   <223> n is a, c, g, or t
<220>
   <221> misc_feature
   <222> (743)..(743)
   <223> n is a, c, g, or t
<220>
   <221> misc_feature
   <222> (747)..(747)
   <223> n is a, c, g, or t
<220>
   <221> misc_feature
   <222> (760)..(760)
   <223> n is a, c, g, or t
<220>
   <221> misc_feature
   <222> (763)..(767)
   <223> n is a, c, g, or t
<220>
   <221> misc_feature
   <222> (770)..(770)
   <223> n is a, c, g, or t
<220>
   <221> misc_feature
   <222> (773)..(773)
   <223> n is a, c, g, or t
<220>
   <221> misc_feature
   <222> (783)..(783)
   <223> n is a, c, g, or t
<220>
   <221> misc_feature
   <222> (785)..(785)
   <223> n is a, c, g, or t
<220>
   <221> misc_feature
   <222> (788)..(788)
   <223> n is a, c, g, or t
<220>
   <221> misc_feature
   <222> (795)..(795)
   <223> n is a, c, g, or t
<220>
   <221> misc_feature
   <222> (803)..(803)
   <223> n is a, c, g, or t
<220>
   <221> misc_feature
   <222> (813)..(813)
   <223> n is a, c, g, or t
<220>
   <221> misc_feature
   <222> (816)..(816)
   <223> n is a, c, g, or t
<220>
   <221> misc_feature
   <222> (831)..(832)
   <223> n is a, c, g, or t
<220>
   <221> misc_feature
   <222> (845)..(846)
   <223> n is a, c, g, or t
<220>
   <221> misc_feature
   <222> (865)..(865)
   <223> n is a, c, g, or t
<220>
   <221> misc_feature
   <222> (874)..(874)
   <223> n is a, c, g, or t
<220>
   <221> misc_feature
   <222> (896)..(896)
   <223> n is a, c, g, or t
<220>
   <221> misc_feature
   <222> (906)..(906)
   <223> n is a, c, g, or t
<220>
   <221> misc_feature
   <222> (943)..(944)
   <223> n is a, c, g, or t
<220>
   <221> misc_feature
   <222> (950)..(950)
   <223> n is a, c, g, or t
<220>
   <221> misc_feature
   <222> (956)..(956)
   <223> n is a, c, g, or t
<220>
   <221> misc_feature
   <222> (962)..(962)
   <223> n is a, c, g, or t
<220>
   <221> misc_feature
   <222> (967)..(967)
   <223> n is a, c, g, or t
<220>
   <221> misc_feature
   <222> (980)..(980)
   <223> n is a, c, g, or t
<220>
   <221> misc_feature
   <222> (987)..(987)
   <223> n is a, c, g, or t
<220>
   <221> misc_feature
   <222> (1007)..(1007)
   <223> n is a, c, g, or t
<220>
   <221> misc_feature
   <222> (1013)..(1013)
   <223> n is a, c, g, or t
<220>
   <221> misc_feature
   <222> (1017)..(1017)
   <223> n is a, c, g, or t
<220>
   <221> misc_feature
   <222> (1020)..(1020)
   <223> n is a, c, g, or t
<220>
   <221> misc_feature
   <222> (1028)..(1028)
   <223> n is a, c, g, or t
<220>
   <221> misc_feature
   <222> (1036)..(1036)
   <223> n is a, c, g, or t
<220>
   <221> misc_feature
   <222> (1039)..(1039)
   <223> n is a, c, g, or t
<220>
   <221> misc_feature
   <222> (1044)..(1045)
   <223> n is a, c, g, or t
<220>
   <221> misc_feature
   <222> (1057)..(1057)
   <223> n is a, c, g, or t
<220>
   <221> misc_feature
   <222> (1066)..(1066)
   <223> n is a, c, g, or t
<220>
   <221> misc_feature
   <222> (1072)..(1072)
   <223> n is a, c, g, or t
<220>
   <221> misc_feature
   <222> (1075)..(1075)
   <223> n is a, c, g, or t
<220>
   <221> misc_feature
   <222> (1081)..(1082)
   <223> n is a, c, g, or t
<220>
   <221> misc_feature
   <222> (1085)..(1085)
   <223> n is a, c, g, or t
<220>
   <221> misc_feature
   <222> (1088)..(1088)
   <223> n is a, c, g, or t
<220>
   <221> misc_feature
   <222> (1098)..(1098)
   <223> n is a, c, g, or t
<220>
   <221> misc_feature
   <222> (1130)..(1130)
   <223> n is a, c, g, or t
<220>
   <221> misc_feature
   <222> (1154)..(1154)
   <223> n is a, c, g, or t
<220>
   <221> misc_feature
   <222> (1214)..(1214)
   <223> n is a, c, g, or t
<220>
   <221> misc_feature
   <222> (1216)..(1217)
   <223> n is a, c, g, or t
<220>
   <221> misc_feature
   <222> (1230)..(1230)
   <223> n is a, c, g, or t
<220>
   <221> misc_feature
   <222> (1232)..(1232)
   <223> n is a, c, g, or t
<220>
   <221> misc_feature
   <222> (1235)..(1235)
   <223> n is a, c, g, or t
<220>
   <221> misc_feature
   <222> (1247)..(1247)
   <223> n is a, c, g, or t
<220>
   <221> misc_feature
   <222> (1278)..(1278)
   <223> n is a, c, g, or t
<220>
   <221> misc_feature
   <222> (1299)..(1299)
   <223> n is a, c, g, or t
<220>
   <221> misc_feature
   <222> (1314)..(1314)
   <223> n is a, c, g, or t
<220>
   <221> misc_feature
   <222> (1324)..(1324)
   <223> n is a, c, g, or t
<220>
   <221> misc_feature
   <222> (1348)..(1348)
   <223> n is a, c, g, or t
<220>
   <221> misc_feature
   <222> (1350)..(1350)
   <223> n is a, c, g, or t
<220>
   <221> misc_feature
   <222> (1353)..(1353)
   <223> n is a, c, g, or t
<220>
   <221> misc_feature
   <222> (1357)..(1357)
   <223> n is a, c, g, or t
<220>
   <221> misc_feature
   <222> (1406)..(1407)
   <223> n is a, c, g, or t
<220>
   <221> misc_feature
   <222> (1422)..(1422)
   <223> n is a, c, g, or t
<220>
   <221> misc_feature
   <222> (1437)..(1437)
   <223> n is a, c, g, or t
<220>
   <221> misc_feature
   <222> (1484)..(1484)
   <223> n is a, c, g, or t
<220>
   <221> misc_feature
   <222> (1488)..(1488)
   <223> n is a, c, g, or t
<220>
   <221> misc_feature
   <222> (1495)..(1495)
   <223> n is a, c, g, or t
<220>
   <221> misc_feature
   <222> (1497)..(1497)
   <223> n is a, c, g, or t
<220>
   <221> misc_feature
   <222> (1508)..(1508)
   <223> n is a, c, g, or t
<220>
   <221> misc_feature
   <222> (1521)..(1521)
   <223> n is a, c, g, or t
<220>
   <221> misc_feature
   <222> (1563)..(1563)
   <223> n is a, c, g, or t
<220>
   <221> misc_feature
   <222> (1575)..(1575)
   <223> n is a, c, g, or t
<220>
   <221> misc_feature
   <222> (1582)..(1582)
   <223> n is a, c, g, or t
<220>
   <221> misc_feature
   <222> (1590)..(1591)
   <223> n is a, c, g, or t
<220>
   <221> misc_feature
   <222> (1606)..(1606)
   <223> n is a, c, g, or t
<220>
   <221> misc_feature
   <222> (1608)..(1608)
   <223> n is a, c, g, or t
<220>
   <221> misc_feature
   <222> (1621)..(1621)
   <223> n is a, c, g, or t
<220>
   <221> misc_feature
   <222> (1624)..(1624)
   <223> n is a, c, g, or t
<220>
   <221> misc_feature
   <222> (1642)..(1642)
   <223> n is a, c, g, or t
<220>
   <221> misc_feature
   <222> (1650)..(1650)
   <223> n is a, c, g, or t
<220>
   <221> misc_feature
   <222> (1653)..(1653)
   <223> n is a, c, g, or t
<220>
   <221> misc_feature
   <222> (1669)..(1669)
   <223> n is a, c, g, or t
<220>
   <221> misc_feature
   <222> (1671)..(1671)
   <223> n is a, c, g, or t
<220>
   <221> misc_feature
   <222> (1679)..(1679)
   <223> n is a, c, g, or t
<220>
   <221> misc_feature
   <222> (1681)..(1681)
   <223> n is a, c, g, or t
<220>
   <221> misc_feature
   <222> (1704)..(1704)
   <223> n is a, c, g, or t
<220>
   <221> misc_feature
   <222> (1712)..(1712)
   <223> n is a, c, g, or t
<220>
   <221> misc_feature
   <222> (1731)..(1731)
   <223> n is a, c, g, or t
<220>
   <221> misc_feature
   <222> (1749)..(1749)
   <223> n is a, c, g, or t
<220>
   <221> misc_feature
   <222> (1752)..(1752)
   <223> n is a, c, g, or t
<220>
   <221> misc_feature
   <222> (1790)..(1792)
   <223> n is a, c, g, or t
<220>
   <221> misc_feature
   <222> (1798)..(1798)
   <223> n is a, c, g, or t
<220>
   <221> misc_feature
   <222> (1832)..(1833)
   <223> n is a, c, g, or t
<220>
   <221> misc_feature
   <222> (1839)..(1840)
   <223> n is a, c, g, or t
<220>
   <221> misc_feature
   <222> (1851)..(1851)
   <223> n is a, c, g, or t
<220>
   <221> misc_feature
   <222> (1859)..(1859)
   <223> n is a, c, g, or t
<220>
   <221> misc_feature
   <222> (1864)..(1864)
   <223> n is a, c, g, or t
<220>
   <221> misc_feature
   <222> (1887)..(1887)
   <223> n is a, c, g, or t
<220>
   <221> misc_feature
   <222> (1891)..(1891)
   <223> n is a, c, g, or t
<220>
   <221> misc_feature
   <222> (1910)..(1910)
   <223> n is a, c, g, or t
<220>
   <221> misc_feature
   <222> (1922)..(1922)
   <223> n is a, c, g, or t
<220>
   <221> misc_feature
   <222> (1935)..(1935)
   <223> n is a, c, g, or t
<220>
   <221> misc_feature
   <222> (1941)..(1941)
   <223> n is a, c, g, or t
<220>
   <221> misc_feature
   <222> (1946)..(1946)
   <223> n is a, c, g, or t
<220>
   <221> misc_feature
   <222> (1949)..(1949)
   <223> n is a, c, g, or t
<220>
   <221> misc_feature
   <222> (1963)..(1963)
   <223> n is a, c, g, or t
<220>
   <221> misc_feature
   <222> (1976)..(1976)
   <223> n is a, c, g, or t
<220>
   <221> misc_feature
   <222> (1983)..(1984)
   <223> n is a, c, g, or t
<220>
   <221> misc_feature
   <222> (1986)..(1986)
   <223> n is a, c, g, or t
<220>
   <221> misc_feature
   <222> (1993)..(1993)
   <223> n is a, c, g, or t
<220>
   <221> misc_feature
   <222> (1999)..(1999)
   <223> n is a, c, g, or t
<220>
   <221> misc_feature
   <222> (2026) .. (2027)
   <223> n is a, c, g, or t
<220>
   <221> misc_feature
   <222> (2039)..(2041)
   <223> n is a, c, g, or t
<220>
   <221> misc_feature
   <222> (2051)..(2052)
   <223> n is a, c, g, or t
<220>
   <221> misc_feature
   <222> (2078)..(2078)
   <223> n is a, c, g, or t
<220>
   <221> misc_feature
   <222> (2083)..(2083)
   <223> n is a, c, g, or t
<220>
   <221> misc_feature
   <222> (2112)..(2112)
   <223> n is a, c, g, or t
<220>
   <221> misc_feature
   <222> (2127)..(2127)
   <223> n is a, c, g, or t
<220>
   <221> misc_feature
   <222> (2139) .. (2139)
   <223> n is a, c, g, or t
<220>
   <221> misc_feature
   <222> (2154)..(2154)
   <223> n is a, c, g, or t
<220>
   <221> misc_feature
   <222> (2163)..(2163)
   <223> n is a, c, g, or t
<220>
   <221> misc_feature
   <222> (2178)..(2178)
   <223> n is a, c, g, or t
<220>
   <221> misc_feature
   <222> (2184)..(2186)
   <223> n is a, c, g, or t
<220>
   <221> misc_feature
   <222> (2195)..(2196)
   <223> n is a, c, g, or t
<220>
   <221> misc_feature
   <222> (2198)..(2198)
   <223> n is a, c, g, or t
<220>
   <221> misc_feature
   <222> (2200)..(2200)
   <223> n is a, c, g, or t
<220>
   <221> misc_feature
   <222> (2203)..(2203)
   <223> n is a, c, g, or t
<220>
   <221> misc_feature
   <222> (2206)..(2206)
   <223> n is a, c, g, or t
<220>
   <221> misc_feature
   <222> (2208)..(2208)
   <223> n is a, c, g, or t
<220>
   <221> misc_feature
   <222> (2210)..(2210)
   <223> n is a, c, g, or t
<220>
   <221> misc_feature
   <222> (2218)..(2218)
   <223> n is a, c, g, or t
<220>
   <221> misc_feature
   <222> (2230)..(2230)
   <223> n is a, c, g, or t
<220>
   <221> misc_feature
   <222> (2240)..(2240)
   <223> n is a, c, g, or t
<220>
   <221> misc_feature
   <222> (2247)..(2247)
   <223> n is a, c, g, or t
<220>
   <221> misc_feature
   <222> (2252)..(2252)
   <223> n is a, c, g, or t
<220>
   <221> misc_feature
   <222> (2265)..(2265)
   <223> n is a, c, g, or t
<220>
   <221> misc_feature
   <222> (2320)..(2320)
   <223> n is a, c, g, or t
<220>
   <221> misc_feature
   <222> (2332)..(2333)
   <223> n is a, c, g, or t
<220>
   <221> misc_feature
   <222> (2338)..(2338)
   <223> n is a, c, g, or t
<220>
   <221> misc_feature
   <222> (2342)..(2342)
   <223> n is a, c, g, or t
<220>
   <221> misc_feature
   <222> (2353)..(2356)
   <223> n is a, c, g, or t
<220>
   <221> misc_feature
   <222> (2366) .. (2367)
   <223> n is a, c, g, or t
<220>
   <221> misc_feature
   <222> (2371)..(2372)
   <223> n is a, c, g, or t
<220>
   <221> misc_feature
   <222> (2385)..(2385)
   <223> n is a, c, g, or t
<220>
   <221> misc_feature
   <222> (2431)..(2431)
   <223> n is a, c, g, or t
<220>
   <221> misc_feature
   <222> (2477)..(2477)
   <223> n is a, c, g, or t
<220>
   <221> misc_feature
   <222> (2483)..(2483)
   <223> n is a, c, g, or t
<220>
   <221> misc_feature
   <222> (2490)..(2490)
   <223> n is a, c, g, or t
<220>
   <221> misc_feature
   <222> (2492)..(2492)
   <223> n is a, c, g, or t
<220>
   <221> misc_feature
   <222> (2494)..(2494)
   <223> n is a, c, g, or t
<400> 34
   ncgtnncgnt gcntctctca agnttagatt tntttnnncg taaanagagg aggancnatt 60
   gctttaaanc ccaccaatta gctccttcac tctcagnnct naacaatggc ttcgactctg 120
   ggcngcgatg ngagnnncga gatagtgttt ttcgatcttg agacngcngt tncgaccnaa 180
   tcgggncagc cntttgcgat tttggagttt ggngctatct tagtttgccc tatgaagcta 240
   gnggagctct atagttacnc cacnttggnt cgaccnacng atctttcnct catctncacg 300
   ctcacgaagc gacgaagcgg cattacgcgc gacggagttc tctctgcacc tacnttctct 360
   gaaatcgctg atgaantcta cganattcnc cacggtaagg gtttctcnnt ttttttnnnn 420
   nnctnncncn ntctctctna cncgaagnta naagtattga ttttggtgtt tctgtaggac 480
   gaatttgggc nggacataac ataaagagat tcgattgtgt aagantanga gatgcatttg 540
   cagnaattgg tntcnctccc cnngagncna aagntncaat tgatncactt tcgttntngt 600
   ctcagaagtt tgggaagnga gctggngacn tgaaggtctc tcttttttcg tcttcncgat 660
   gataaatctc aaagccnata gcttccttgt tatcnttata gatatgaatt tcnangtaac 720
   ttcaaagatt catcactcat canagtngct aaaatttacn ctnnnnnaan aangtagatg 780
   gcntngcntg ctacntattt cgngctagga gancangctc acaggtaaaa nnagtaaacg 840
   atacnntgtg ccttttaacg attcnccagt tgtntcaata tgggactaaa catggntatg 900
   attcancagg agcttagatg atgtccggat gaatcttgaa gtnntcaagn actgtncaac 960
   cntcttnttt ctggtattgn tgtcttntca tttcttgaat aatgatnaac tcntaanttn 1020
   aaaagganta gattanagng gttnngacat atctganttc tgtctncagt tntgnaaaag 1080
   nnggntcnat cttccttnca gaccacaact ttgcaagccg taaacatggn ttgcaacttg 1140
   caagtatagt ttgntatatc actgagttta agtacttggt gtttgcagga gtccagtgtt 1200
   cctgacattc ttananncat gagctggttn tnccnaagaa aaagtcngag aacacgaagt 1260
   aatgagaagt cactgccnaa tggagtcaga gaaagcccna cttcttcctc ttcnagccct 1320
   aaanctgatc cgagttcgtc ttctgtanan gcnacanctg tcaaaaacca tcccatcatt 1380
   tctcttctga cggaatgctc agaaanngat acatctagtt gngaaataga tccatcngac 1440
   ataaccactc taataagtaa actacatatt ggaactctta aganagangc tgcgnangaa 1500
   gccaaaantg taagacagca nggtgaatca accgatccca atgccaaaga tgaatcattt     1560
   ttnggcgtta atgangtatc tntttctagn ntcagggcaa gtcttntncc gttatatcgt     1620
   nggngtctga gaatggagct gnttcacaan ganacccctc tacatctcng ntggtatanc     1680
   ntgaaaattc ggtttggaat aagncggaag tntgtggatc atgtaggtcg nccaaagatg     1740
   aatattgtng tngacatacc tcctgattta tgcaagatct tggacgcatn nnatgctnct     1800
   gcgcataact tactgattga ctcaagcaca anntcagann ggaggcctac ngttatgang     1860
   aaanaaggct ttgccaacta tcccacngcc ngactgcagt aagtattcan cactctctct     1920
   gnccttttac atacnagcat naatcnacng gagagtctct aanaccatct ccaacnctac     1980
   tcnntnttca ccnccaaant ctattttgga gttaaatccc tccaannctt gcaaaatann     2040
   natcttcaaa nnttttctcc atatttggag attttgantt ttnaagtcat gactccattt     2100
   tggagttggg tnggagaaaa acacaantcc aaaatagant tacttcattt tggngtaaaa     2160
   aantgaagaa atgggttnga gatnnnctaa cctcnntnan cantcntntn ttgttggnag     2220
   aataagctcn gaatccaatn gaacccnggt anaccaaaaa gaagnacctt tgggaaccaa     2280
   tcaaaagctc gatttcagta gcgataattt tgaaaagctn gagtcagcac tnnttccngg     2340
   tnccctggtt gannnnttct tctcanncga nncttacgat tatangaaaa tggtagggat     2400
   acgtctagca gccagaaagt tggtaatcca nctgaagaaa tgatctagcc aaggaaaaat     2460
   cattcctctg tctcttnctg gtnagtcggn gngnactt                             2498
<210> 35
   <211> 1491
   <212> DNA
   <213> Boechera
<220>
   <221> misc_feature
   <222> (19)..(19)
   <223> n is a, c, g, or t
<220>
   <221> misc_feature
   <222> (26)..(26)
   <223> n is a, c, g, or t
<220>
   <221> misc_feature
   <222> (30)..(32)
   <223> n is a, c, g, or t
<220>
   <221> misc_feature
   <222> (60)..(60)
   <223> n is a, c, g, or t
<220>
   <221> misc_feature
   <222> (63)..(63)
   <223> n is a, c, g, or t
<220>
   <221> misc_feature
   <222> (67)..(67)
   <223> n is a, c, g, or t
<220>
   <221> misc_feature
   <222> (73)..(73)
   <223> n is a, c, g, or t
<220>
   <221> misc_feature
   <222> (81)..(81)
   <223> n is a, c, g, or t
<220>
   <221> misc_feature
   <222> (87)..(87)
   <223> n is a, c, g, or t
<220>
   <221> misc_feature
   <222> (108)..(108)
   <223> n is a, c, g, or t
<220>
   <221> misc_feature
   <222> (137)..(137)
   <223> n is a, c, g, or t
<220>
   <221> misc_feature
   <222> (154)..(154)
   <223> n is a, c, g, or t
<220>
   <221> misc_feature
   <222> (159)..(159)
   <223> n is a, c, g, or t
<220>
   <221> misc_feature
   <222> (164)..(164)
   <223> n is a, c, g, or t
<220>
   <221> misc_feature
   <222> (171)..(171)
   <223> n is a, c, g, or t
<220>
   <221> misc_feature
   <222> (174)..(174)
   <223> n is a, c, g, or t
<220>
   <221> misc_feature
   <222> (183)..(183)
   <223> n is a, c, g, or t
<220>
   <221> misc_feature
   <222> (191)..(191)
   <223> n is a, c, g, or t
<220>
   <221> misc_feature
   <222> (249) .. (249)
   <223> n is a, c, g, or t
<220>
   <221> misc_feature
   <222> (271)..(271)
   <223> n is a, c, g, or t
<220>
   <221> misc_feature
   <222> (279)..(279)
   <223> n is a, c, g, or t
<220>
   <221> misc_feature
   <222> (284)..(284)
   <223> n is a, c, g, or t
<220>
   <221> misc_feature
   <222> (303)..(303)
   <223> n is a, c, g, or t
<220>
   <221> misc_feature
   <222> (337)..(337)
   <223> n is a, c, g, or t
<220>
   <221> misc_feature
   <222> (340)..(340)
   <223> n is a, c, g, or t
<220>
   <221> misc_feature
   <222> (356)..(356)
   <223> n is a, c, g, or t
<220>
   <221> misc_feature
   <222> (364)..(364)
   <223> n is a, c, g, or t
<220>
   <221> misc_feature
   <222> (367)..(367)
   <223> n is a, c, g, or t
<220>
   <221> misc_feature
   <222> (374)..(375)
   <223> n is a, c, g, or t
<220>
   <221> misc_feature
   <222> (379)..(379)
   <223> n is a, c, g, or t
<220>
   <221> misc_feature
   <222> (381)..(381)
   <223> n is a, c, g, or t
<220>
   <221> misc_feature
   <222> (386)..(386)
   <223> n is a, c, g, or t
<220>
   <221> misc_feature
   <222> (388)..(388)
   <223> n is a, c, g, or t
<220>
   <221> misc_feature
   <222> (397)..(397)
   <223> n is a, c, g, or t
<220>
   <221> misc_feature
   <222> (408)..(408)
   <223> n is a, c, g, or t
<220>
   <221> misc_feature
   <222> (410)..(410)
   <223> n is a, c, g, or t
<220>
   <221> misc_feature
   <222> (430)..(430)
   <223> n is a, c, g, or t
<220>
   <221> misc_feature
   <222> (438)..(438)
   <223> n is a, c, g, or t
<220>
   <221> misc_feature
   <222> (442)..(442)
   <223> n is a, c, g, or t
<220>
   <221> misc_feature
   <222> (453)..(453)
   <223> n is a, c, g, or t
<220>
   <221> misc_feature
   <222> (455)..(455)
   <223> n is a, c, g, or t
<220>
   <221> misc_feature
   <222> (458)..(458)
   <223> n is a, c, g, or t
<220>
   <221> misc_feature
   <222> (465)..(465)
   <223> n is a, c, g, or t
<220>
   <221> misc_feature
   <222> (473)..(473)
   <223> n is a, c, g, or t
<220>
   <221> misc_feature
   <222> (483)..(483)
   <223> n is a, c, g, or t
<220>
   <221> misc_feature
   <222> (486)..(486)
   <223> n is a, c, g, or t
<220>
   <221> misc_feature
   <222> (528)..(529)
   <223> n is a, c, g, or t
<220>
   <221> misc_feature
   <222> (535)..(535)
   <223> n is a, c, g, or t
<220>
   <221> misc_feature
   <222> (541)..(541)
   <223> n is a, c, g, or t
<220>
   <221> misc_feature
   <222> (547)..(547)
   <223> n is a, c, g, or t
<220>
   <221> misc_feature
   <222> (552)..(552)
   <223> n is a, c, g, or t
<220>
   <221> misc_feature
   <222> (584)..(584)
   <223> n is a, c, g, or t
<220>
   <221> misc_feature
   <222> (586)..(587)
   <223> n is a, c, g, or t
<220>
   <221> misc_feature
   <222> (600)..(600)
   <223> n is a, c, g, or t
<220>
   <221> misc_feature
   <222> (602)..(602)
   <223> n is a, c, g, or t
<220>
   <221> misc_feature
   <222> (605)..(605)
   <223> n is a, c, g, or t
<220>
   <221> misc_feature
   <222> (617)..(617)
   <223> n is a, c, g, or t
<220>
   <221> misc_feature
   <222> (648)..(648)
   <223> n is a, c, g, or t
<220>
   <221> misc_feature
   <222> (669)..(669)
   <223> n is a, c, g, or t
<220>
   <221> misc_feature
   <222> (684)..(684)
   <223> n is a, c, g, or t
<220>
   <221> misc_feature
   <222> (694)..(694)
   <223> n is a, c, g, or t
<220>
   <221> misc_feature
   <222> (718)..(718)
   <223> n is a, c, g, or t
<220>
   <221> misc_feature
   <222> (720)..(720)
   <223> n is a, c, g, or t
<220>
   <221> misc_feature
   <222> (723)..(723)
   <223> n is a, c, g, or t
<220>
   <221> misc_feature
   <222> (727)..(727)
   <223> n is a, c, g, or t
<220>
   <221> misc_feature
   <222> (776)..(777)
   <223> n is a, c, g, or t
<220>
   <221> misc_feature
   <222> (792)..(792)
   <223> n is a, c, g, or t
<220>
   <221> misc_feature
   <222> (807)..(807)
   <223> n is a, c, g, or t
<220>
   <221> misc_feature
   <222> (854)..(854)
   <223> n is a, c, g, or t
<220>
   <221> misc_feature
   <222> (858)..(858)
   <223> n is a, c, g, or t
<220>
   <221> misc_feature
   <222> (865)..(865)
   <223> n is a, c, g, or t
<220>
   <221> misc_feature
   <222> (867)..(867)
   <223> n is a, c, g, or t
<220>
   <221> misc_feature
   <222> (878)..(878)
   <223> n is a, c, g, or t
<220>
   <221> misc_feature
   <222> (891)..(891)
   <223> n is a, c, g, or t
<220>
   <221> misc_feature
   <222> (933)..(933)
   <223> n is a, c, g, or t
<220>
   <221> misc_feature
   <222> (945)..(945)
   <223> n is a, c, g, or t
<220>
   <221> misc_feature
   <222> (952)..(952)
   <223> n is a, c, g, or t
<220>
   <221> misc_feature
   <222> (960)..(961)
   <223> n is a, c, g, or t
<220>
   <221> misc_feature
   <222> (976)..(976)
   <223> n is a, c, g, or t
<220>
   <221> misc_feature
   <222> (978)..(978)
   <223> n is a, c, g, or t
<220>
   <221> misc_feature
   <222> (991)..(991)
   <223> n is a, c, g, or t
<220>
   <221> misc_feature
   <222> (994)..(994)
   <223> n is a, c, g, or t
<220>
   <221> misc_feature
   <222> (1012)..(1012)
   <223> n is a, c, g, or t
<220>
   <221> misc_feature
   <222> (1020)..(1020)
   <223> n is a, c, g, or t
<220>
   <221> misc_feature
   <222> (1023)..(1023)
   <223> n is a, c, g, or t
<220>
   <221> misc_feature
   <222> (1039)..(1039)
   <223> n is a, c, g, or t
<220>
   <221> misc_feature
   <222> (1041)..(1041)
   <223> n is a, c, g, or t
<220>
   <221> misc_feature
   <222> (1049)..(1049)
   <223> n is a, c, g, or t
<220>
   <221> misc_feature
   <222> (1051)..(1051)
   <223> n is a, c, g, or t
<220>
   <221> misc_feature
   <222> (1074)..(1074)
   <223> n is a, c, g, or t
<220>
   <221> misc_feature
   <222> (1082)..(1082)
   <223> n is a, c, g, or t
<220>
   <221> misc_feature
   <222> (1101)..(1101)
   <223> n is a, c, g, or t
<220>
   <221> misc_feature
   <222> (1119)..(1119)
   <223> n is a, c, g, or t
<220>
   <221> misc_feature
   <222> (1122)..(1122)
   <223> n is a, c, g, or t
<220>
   <221> misc_feature
   <222> (1160)..(1162)
   <223> n is a, c, g, or t
<220>
   <221> misc_feature
   <222> (1168)..(1168)
   <223> n is a, c, g, or t
<220>
   <221> misc_feature
   <222> (1202)..(1203)
   <223> n is a, c, g, or t
<220>
   <221> misc_feature
   <222> (1209)..(1210)
   <223> n is a, c, g, or t
<220>
   <221> misc_feature
   <222> (1221)..(1221)
   <223> n is a, c, g, or t
<220>
   <221> misc_feature
   <222> (1229)..(1229)
   <223> n is a, c, g, or t
<220>
   <221> misc_feature
   <222> (1234)..(1234)
   <223> n is a, c, g, or t
<220>
   <221> misc_feature
   <222> (1257)..(1257)
   <223> n is a, c, g, or t
<220>
   <221> misc_feature
   <222> (1261)..(1261)
   <223> n is a, c, g, or t
<220>
   <221> misc_feature
   <222> (1278)..(1278)
   <223> n is a, c, g, or t
<220>
   <221> misc_feature
   <222> (1288)..(1288)
   <223> n is a, c, g, or t
<220>
   <221> misc_feature
   <222> (1295)..(1295)
   <223> n is a, c, g, or t
<220>
   <221> misc_feature
   <222> (1300)..(1300)
   <223> n is a, c, g, or t
<220>
   <221> misc_feature
   <222> (1313)..(1313)
   <223> n is a, c, g, or t
<220>
   <221> misc_feature
   <222> (1368)..(1368)
   <223> n is a, c, g, or t
<220>
   <221> misc_feature
   <222> (1380)..(1381)
   <223> n is a, c, g, or t
<220>
   <221> misc_feature
   <222> (1386)..(1386)
   <223> n is a, c, g, or t
<220>
   <221> misc_feature
   <222> (1390)..(1390)
   <223> n is a, c, g, or t
<220>
   <221> misc_feature
   <222> (1401)..(1404)
   <223> n is a, c, g, or t
<220>
   <221> misc_feature
   <222> (1414)..(1415)
   <223> n is a, c, g, or t
<220>
   <221> misc_feature
   <222> (1419)..(1420)
   <223> n is a, c, g, or t
<220>
   <221> misc_feature
   <222> (1433)..(1433)
   <223> n is a, c, g, or t
<220>
   <221> misc_feature
   <222> (1479)..(1479)
   <223> n is a, c, g, or t
<400> 35
   atggcttcga ctctgggcng cgatgngagn nncgagatag tgtttttcga tcttgagacn 60
   gcngttncga ccnaatcggg ncagccnttt gcgattttgg agtttggngc tatcttagtt 120
   tgccctatga agctagngga gctctatagt tacnccacnt tggntcgacc nacngatctt      180
   tcnctcatct ncacgctcac gaagcgacga agcggcatta cgcgcgacgg agttctctct      240
   gcacctacnt tctctgaaat cgctgatgaa ntctacgana ttcnccacgg acgaatttgg      300
   gcnggacata acataaagag attcgattgt gtaagantan gagatgcatt tgcagnaatt      360
   ggtntcnctc cccnngagnc naaagntnca attgatncac tttcgttntn gtctcagaag      420
   tttgggaagn gagctggnga cntgaagatg gcntngcntg ctacntattt cgngctagga      480
   gancangctc acaggagctt agatgatgtc cggatgaatc ttgaagtnnt caagnactgt      540
   ncaaccntct tntttctgga gtccagtgtt cctgacattc ttananncat gagctggttn      600
   tnccnaagaa aaagtcngag aacacgaagt aatgagaagt cactgccnaa tggagtcaga      660
   gaaagcccna cttcttcctc ttcnagccct aaanctgatc cgagttcgtc ttctgtanan      720
   gcnacanctg tcaaaaacca tcccatcatt tctcttctga cggaatgctc agaaanngat      780
   acatctagtt gngaaataga tccatcngac ataaccactc taataagtaa actacatatt      840
   ggaactctta aganagangc tgcgnangaa gccaaaantg taagacagca nggtgaatca      900
   accgatccca atgccaaaga tgaatcattt ttnggcgtta atgangtatc tntttctagn      960
   ntcagggcaa gtcttntncc gttatatcgt nggngtctga gaatggagct gnttcacaan     1020
   ganacccctc tacatctcng ntggtatanc ntgaaaattc ggtttggaat aagncggaag     1080
   tntgtggatc atgtaggtcg nccaaagatg aatattgtng tngacatacc tcctgattta     1140
   tgcaagatct tggacgcatn nnatgctnct gcgcataact tactgattga ctcaagcaca     1200
   anntcagann ggaggcctac ngttatgang aaanaaggct ttgccaacta tcccacngcc     1260
   ngactgcaaa taagctcnga atccaatnga acccnggtan accaaaaaga agnacctttg     1320
   ggaaccaatc aaaagctcga tttcagtagc gataattttg aaaagctnga gtcagcactn     1380
   nttccnggtn ccctggttga nnnnttcttc tcanncgann cttacgatta tangaaaatg     1440
   gtagggatac gtctagcagc cagaaagttg gtaatccanc tgaagaaatg a              1491
<210> 36
   <211> 495
   <212> DNA
   <213> Boechera
<220>
   <221> misc_feature
   <222> (21)..(21)
   <223> n is a, c, g, or t
<220>
   <221> misc_feature
   <222> (24)..(24)
   <223> n is a, c, g, or t
<220>
   <221> misc_feature
   <222> (28)..(28)
   <223> n is a, c, g, or t
<220>
   <221> misc_feature
   <222> (34)..(34)
   <223> n is a, c, g, or t
<220>
   <221> misc_feature
   <222> (42)..(42)
   <223> n is a, c, g, or t
<220>
   <221> misc_feature
   <222> (48)..(48)
   <223> n is a, c, g, or t
<220>
   <221> misc_feature
   <222> (69) .. (69)
   <223> n is a, c, g, or t
<220>
   <221> misc_feature
   <222> (98)..(98)
   <223> n is a, c, g, or t
<220>
   <221> misc_feature
   <222> (115)..(115)
   <223> n is a, c, g, or t
<220>
   <221> misc_feature
   <222> (120)..(120)
   <223> n is a, c, g, or t
<220>
   <221> misc_feature
   <222> (125)..(125)
   <223> n is a, c, g, or t
<220>
   <221> misc_feature
   <222> (132)..(132)
   <223> n is a, c, g, or t
<220>
   <221> misc_feature
   <222> (135)..(135)
   <223> n is a, c, g, or t
<220>
   <221> misc_feature
   <222> (144)..(144)
   <223> n is a, c, g, or t
<220>
   <221> misc_feature
   <222> (152)..(152)
   <223> n is a, c, g, or t
<220>
   <221> misc_feature
   <222> (210)..(210)
   <223> n is a, c, g, or t
<220>
   <221> misc_feature
   <222> (232)..(232)
   <223> n is a, c, g, or t
<220>
   <221> misc_feature
   <222> (240)..(240)
   <223> n is a, c, g, or t
<220>
   <221> misc_feature
   <222> (245)..(245)
   <223> n is a, c, g, or t
<220>
   <221> misc_feature
   <222> (264)..(264)
   <223> n is a, c, g, or t
<220>
   <221> misc_feature
   <222> (298)..(298)
   <223> n is a, c, g, or t
<220>
   <221> misc_feature
   <222> (301)..(301)
   <223> n is a, c, g, or t
<220>
   <221> misc_feature
   <222> (317)..(317)
   <223> n is a, c, g, or t
<220>
   <221> misc_feature
   <222> (325)..(325)
   <223> n is a, c, g, or t
<220>
   <221> misc_feature
   <222> (328)..(328)
   <223> n is a, c, g, or t
<220>
   <221> misc_feature
   <222> (335)..(336)
   <223> n is a, c, g, or t
<220>
   <221> misc_feature
   <222> (340)..(340)
   <223> n is a, c, g, or t
<220>
   <221> misc_feature
   <222> (342)..(342)
   <223> n is a, c, g, or t
<220>
   <221> misc_feature
   <222> (347)..(347)
   <223> n is a, c, g, or t
<220>
   <221> misc_feature
   <222> (349)..(349)
   <223> n is a, c, g, or t
<220>
   <221> misc_feature
   <222> (358)..(358)
   <223> n is a, c, g, or t
<220>
   <221> misc_feature
   <222> (369)..(369)
   <223> n is a, c, g, or t
<220>
   <221> misc_feature
   <222> (371)..(371)
   <223> n is a, c, g, or t
<220>
   <221> misc_feature
   <222> (391)..(391)
   <223> n is a, c, g, or t
<220>
   <221> misc_feature
   <222> (399)..(399)
   <223> n is a, c, g, or t
<220>
   <221> misc_feature
   <222> (403)..(403)
   <223> n is a, c, g, or t
<220>
   <221> misc_feature
   <222> (414)..(414)
   <223> n is a, c, g, or t
<220>
   <221> misc_feature
   <222> (416)..(416)
   <223> n is a, c, g, or t
<220>
   <221> misc_feature
   <222> (419)..(419)
   <223> n is a, c, g, or t
<220>
   <221> misc_feature
   <222> (426)..(426)
   <223> n is a, c, g, or t
<220>
   <221> misc_feature
   <222> (434)..(434)
   <223> n is a, c, g, or t
<220>
   <221> misc_feature
   <222> (444)..(444)
   <223> n is a, c, g, or t
<220>
   <221> misc_feature
   <222> (447)..(447)
   <223> n is a, c, g, or t
<220>
   <221> misc_feature
   <222> (489)..(490)
   <223> n is a, c, g, or t
<400> 36
   gtgtttttcg atcttgagac ngcngttncg accnaatcgg gncagccntt tgcgattttg        60
   gagtttggng ctatcttagt ttgccctatg aagctagngg agctctatag ttacnccacn       120
   ttggntcgac cnacngatct ttcnctcatc tncacgctca cgaagcgacg aagcggcatt       180
   acgcgcgacg gagttctctc tgcacctacn ttctctgaaa tcgctgatga antctacgan       240
   attcnccacg gacgaatttg ggcnggacat aacataaaga gattcgattg tgtaaganta       300
   ngagatgcat ttgcagnaat tggtntcnct ccccnngagn cnaaagntnc aattgatnca       360
   ctttcgttnt ngtctcagaa gtttgggaag ngagctggng acntgaagat ggcntngcnt       420
   gctacntatt tcgngctagg agancangct cacaggagct tagatgatgt ccggatgaat       480
   cttgaagtnn tcaag                                                         495
<210> 37
   <211> 2483
   <212> DNA
   <213> Boechera
<400> 37
   tcgtaccgtt gcttctctca agtttagatt tttttccgta aaaagaggag gtggcccgtg 60
   aagtttattc cctttaaaac ccaccaatta gctccttcac tctcagttct caacaatggc 120
   ttcgactctg ggcggcgatg agagaaacga gatagtgttt ttcgatcttg agactgcggt 180
   tccgaccaaa tcggggcagc cttttgcgat tttggagttt ggggctatct tagtttgccc 240
   tatgaagcta gtggagctct atagttactc cactttggtt cgacctaccg atctttctct 300
   catctccacg ctcacgaagc gacgaagcgg cattacgcgc gacggagttc tctctgcacc 360
   tacattctct gaaatcgctg atgaagtcta cgacattctc cacggtaagg gtttctcttt 420
   ttttttttct ttctcaatct ctctcacgcg aagctacaag tattgatttt ggtgtttctg 480
   taggacgaat ttgggtggga cataacataa agagattcga ttgtgtaaga ataagagatg 540
   catttgcaga aattggtctc cctcccccgg agccgaaagc tacaattgat tcactttcgt 600
   tgttgtctca gaagtttggg aagagagctg gtgacatgaa ggtctctctt ttttcgtctt 660
   ctcgatgata aatctcaaag cctatagctt ccttgttatc tttatagata tgaatttcaa 720
   tgtaacttca aagattcatc actcatcaaa gttgctaaaa tttactctaa ataatgtaga 780
   tggcatcgca tgctacatat ttcgggctag gagatcaagc tcacaggtaa aagagtaaac 840
   gataccctgt gccttttaac gattcaccag ttgtttcaat atgggactaa acatggatat 900
   gattcaccag gagcttagat gatgtccgga tgaatcttga agttatcaag cactgttcaa 960
   ccgtcttgtt tctggtattg ttgtcttctc atttcttgaa taatgattaa ctctaactta 1020
   aaaggattag attaaagagg ttgagacata tctgacttct gtctacagtt tgcaaaagtt 1080
   gggtccatct tccttccaga ccacaacttt gcaagccgta aacatggttt gcaagtatag 1140
   tttgtcatat cactgagttt aagtacttgg tgtttgcagg agtccagtgt tcctgacatt 1200
   cttacagaca tgagctggtt attcccaaga aaaagtccga gaacacgaag taatgagaag 1260
   tcactgccta atggagtcag agaaagcccg acttcttcct cttcgagccc taaaactgat 1320
   ccgagttcgt cttctgtaga tgccacagct gtcaaaaacc atcccatcat ttctcttctg 1380
   acggaatgct cagaaagtga tacatctagt tgtgaaatag atccatctga cataaccact 1440
   ctaataagta aactacatat tggaactctt aagacagatg ctgcggacga agccaaaact 1500
   gtaagacagc agggtgaatc aaccgatccc aatgccaaag atgaatcatt tttgggcgtt 1560
   aatgaagtat ctgtttctag catcagggca agtcttatcc cgttatatcg taggagtctg 1620
   agaatggagc tgtttcacaa cgacacccct ctacatctct gttggtatag cttgaaaatt 1680
   cggtttggaa taagccggaa gtatgtggat catgtaggtc gtccaaagat gaatattgtt 1740
   gtagacatac ctcctgattt atgcaagatc ttggacgcat ccgatgctgc tgcgcataac 1800
   ttactgattg actcaagcac aagctcagat tggaggccta ccgttatgag gaaaaaaggc 1860
   tttgccaact atcccacagc cagactgcag taagtattca acactctctc tgacctttta 1920
   catacgagca tgaatccacc ggagagtctc taagaccatc tccaacccta ctctattcac 1980
   ctccaaactc tattttggag ttaaatccct ccaacccttg caaaatagac atcttcaaaa 2040
   ttttctccat atttggagat tttgattttt taagtcatga ctccattttg gagttgggtt 2100
   ggagaaaaac acaactccaa aatagattta cttcattttg gagtaaaaaa tgaagaaatg 2160
   ggttggagat actaacctct gtcaccattc ttatgttgtt ggcagaataa gctcagaatc 2220
   caatggaacc caggtacacc aaaaagaaga acctttggga accaatcaaa agctcgattt 2280
   cagtagcgat aattttgaaa agcttgagtc agcacttctt cctggtaccc tggttgatgc 2340
   attcttctca ctcgagcctt acgattataa gaaaatggta gggatacgtc tagcagccag 2400
   aaagttggta atccacctga agaaatgatc tagccaagga aaaatcattc ctctgtctct 2460
   tcctggtcag tcggtgagca ctt 2483
<210> 38
   <211> 1491
   <212> DNA
   <213> Boechera
<400> 38
   atggcttcga ctctgggcgg cgatgagaga aacgagatag tgtttttcga tcttgagact      60
   gcggttccga ccaaatcggg gcagcctttt gcgattttgg agtttggggc tatcttagtt     120
   tgccctatga agctagtgga gctctatagt tactccactt tggttcgacc taccgatctt     180
   tctctcatct ccacgctcac gaagcgacga agcggcatta cgcgcgacgg agttctctct     240
   gcacctacat tctctgaaat cgctgatgaa gtctacgaca ttctccacgg acgaatttgg     300
   gtgggacata acataaagag attcgattgt gtaagaataa gagatgcatt tgcagaaatt     360
   ggtctccctc ccccggagcc gaaagctaca attgattcac tttcgttgtt gtctcagaag     420
   tttgggaaga gagctggtga catgaagatg gcatcgcatg ctacatattt cgggctagga     480
   gatcaagctc acaggagctt agatgatgtc cggatgaatc ttgaagttat caagcactgt     540
   tcaaccgtct tgtttctgga gtccagtgtt cctgacattc ttacagacat gagctggtta     600
   ttcccaagaa aaagtccgag aacacgaagt aatgagaagt cactgcctaa tggagtcaga     660
   gaaagcccga cttcttcctc ttcgagccct aaaactgatc cgagttcgtc ttctgtagat     720
   gccacagctg tcaaaaacca tcccatcatt tctcttctga cggaatgctc agaaagtgat     780
   acatctagtt gtgaaataga tccatctgac ataaccactc taataagtaa actacatatt     840
   ggaactctta agacagatgc tgcggacgaa gccaaaactg taagacagca gggtgaatca     900
   accgatccca atgccaaaga tgaatcattt ttgggcgtta atgaagtatc tgtttctagc     960
   atcagggcaa gtcttatccc gttatatcgt aggagtctga gaatggagct gtttcacaac    1020
   gacacccctc tacatctctg ttggtatagc ttgaaaattc ggtttggaat aagccggaag    1080
   tatgtggatc atgtaggtcg tccaaagatg aatattgttg tagacatacc tcctgattta    1140
   tgcaagatct tggacgcatc cgatgctgct gcgcataact tactgattga ctcaagcaca    1200
   agctcagatt ggaggcctac cgttatgagg aaaaaaggct ttgccaacta tcccacagcc    1260
   agactgcaaa taagctcaga atccaatgga acccaggtac accaaaaaga agaacctttg    1320
   ggaaccaatc aaaagctcga tttcagtagc gataattttg aaaagcttga gtcagcactt    1380
   cttcctggta ccctggttga tgcattcttc tcactcgagc cttacgatta taagaaaatg    1440
   gtagggatac gtctagcagc cagaaagttg gtaatccacc tgaagaaatg a             1491
<210> 39
   <211> 495
   <212> DNA
   <213> Boechera
<400> 39
   gtgtttttcg atcttgagac tgcggttccg accaaatcgg ggcagccttt tgcgattttg        60
   gagtttgggg ctatcttagt ttgccctatg aagctagtgg agctctatag ttactccact       120
   ttggttcgac ctaccgatct ttctctcatc tccacgctca cgaagcgacg aagcggcatt       180
   acgcgcgacg gagttctctc tgcacctaca ttctctgaaa tcgctgatga agtctacgac       240
   attctccacg gacgaatttg ggtgggacat aacataaaga gattcgattg tgtaagaata       300
   agagatgcat ttgcagaaat tggtctccct cccccggagc cgaaagctac aattgattca       360
   ctttcgttgt tgtctcagaa gtttgggaag agagctggtg acatgaagat ggcatcgcat       420
   gctacatatt tcgggctagg agatcaagct cacaggagct tagatgatgt ccggatgaat       480
   cttgaagtta tcaag                                                        495
<210> 40
   <211> 2483
   <212> DNA
   <213> Boechera
<400> 40
   tcgtaccgtt gcttctctca agtttagatt tttttccgta aaaagaggag gtggcccgtg        60
   aagtttattc cctttaaaac ccaccaatta gctccttcac tctcagttct caacaatggc       120
   ttcgactctg ggcggcgatg agagaaacga gatagtgttt ttcgatcttg agactgcggt       180
   tccgaccaaa tcggggcagc cttttgcgat tttggagttt ggggctatct tagtttgccc       240
   tatgaagcta gtggagctct atagttactc cactttggtt cgacctaccg atctttctct       300
   catctccacg ctcacgaagc gacgaagcgg cattacgcgc gacggagttc tctctgcacc       360
   tacattctct gaaatcgctg atgaagtcta cgacattctc cacggtaagg gtttctcttt       420
   tttttttctt tctcaatctc tctcacgcga agctacaagt attgattttg gtgtttctgt       480
   aggacgaatt tgggcgggac ataacataaa gagattcgat tgtgtaagaa taagagatgc       540
   atttgcagaa attggtctcc ctcccccgga gccgaaagct acaattgatt cactttcgtt       600
   gttgtctcag aagtttggga agagagctgg tgacatgaag gtctctcttt tttcgtcttc       660
   tcgatgataa atctcaaagc ctatagcttc cttgttatct ttatagatat gaatttcaat       720
   gtaacttcaa agattcatca ctcatcaaag ttgctaaaat ttactctaaa taatgtagat       780
   ggcatcgctt gctacatatt tcgggctagg agatcaagct cacaggtaaa agagtaaacg       840
   ataccctgtg ccttttaacg attcaccagt tgtttcaata tgggactaaa catggatatg       900
   attcaccagg agcttagatg atgtccggat gaatcttgaa gttatcaagc actgttcaac       960
   cgtcttgttt ctggtattgt tgtcttctca tttcttgaat aatgattaac tctaatttaa 1020
   aaggattaga ttaaagaggt tgagacatat ctgacttctg tctacagttt gcaaaagttg 1080
   ggtccatctt ccttccagac cacagctttg caagccgtaa acatggtttg caagtatagt 1140
   ttgtcatatc actgagttta agtacttggt gtttgcagga gtccagtgtt cctgacattc 1200
   ttacagacat gagctggtta ttcccaagaa aaagtccgag aacacgaagt aatgagaagt 1260
   cactgcctaa tggagtcaga gaaagcccga cttcttcctc ttcgagccct caaactgatc 1320
   cgagttcgtc ttctgtagat gccacagctg tcaaaaacca tcccatcatt tctcttctga 1380
   cggaatgctc agaaagtgat acatctagtt gtgaaataga tccatctgac ataaccactc 1440
   taataagtaa actacatatt ggaactctta agacagatgc tgcggacgaa gccaaaactg 1500
   taagacagca gggtgaatca accgatccca atgccaaaga tgaatcattt ttgggcgtta 1560
   atgaagtatc tgtttctagc atcagggcaa gtcttatccc gttatatcgt aggagtctga 1620
   gaatggagct gtttcacaac gacacccctc tacatctctg ttggtatagc ttgaaaattc 1680
   ggtttggaat aagccggaag tatgtggatc atgtaggtcg tccaaagatg aatattgttg 1740
   tagacatacc tcctgattta tgcaagatct tggacgcatc cgatgctgct gcgcataact 1800
   tactgattga ctcaagcaca agctcagatt ggaggcctac cgttatgagg aaaaaaggct 1860
   ttgccaacta tcccacagcc agactgcagt aagtattcaa cactctctct gaccttttac 1920
   atacgagcat gaatccaccg gagagtctct aagaccatct ccaaccctac tctattcacc 1980
   tccaaactct attttggagt taaatccctc caacccttgc aaaatagaga tcttcaaatt 2040
   tttctccata tttggagatt ttgattttta agtcatgact ccattttgga gttgggttgg 2100
   agaaaaacac aattccaaaa tagagttact tcattttgga gtaaaaaatg aagaaatggg 2160
   ttcgagatgc tctaacctct gtcaccattc ttatcttgtt ggcagaataa gctcagaatc 2220
   caatggaacc caggtatacc aaaaagaaga acctttggga accaatcaaa agctcgattt 2280
   cagtagcgat aattttgaaa agcttgagtc agcactactt cctggtaccc tggttgatgt 2340
   attcttctca gtcgagcctt acgattataa gaaaatggta gggatacgtc tagcagccag 2400
   aaagttggta atccagctga agaaatgatc tagccaagga aaaatcattc ctctgtctct 2460
   tcctgttcag tcggtgagca ctt 2483
<210> 41
   <211> 1491
   <212> DNA
   <213> Boechera
<400> 41
   atggcttcga ctctgggcgg cgatgagaga aacgagatag tgtttttcga tcttgagact 60
   gcggttccga ccaaatcggg gcagcctttt gcgattttgg agtttggggc tatcttagtt 120
   tgccctatga agctagtgga gctctatagt tactccactt tggttcgacc taccgatctt 180
   tctctcatct ccacgctcac gaagcgacga agcggcatta cgcgcgacgg agttctctct 240
   gcacctacat tctctgaaat cgctgatgaa gtctacgaca ttctccacgg acgaatttgg 300
   gcgggacata acataaagag attcgattgt gtaagaataa gagatgcatt tgcagaaatt 360
   ggtctccctc ccccggagcc gaaagctaca attgattcac tttcgttgtt gtctcagaag 420
   tttgggaaga gagctggtga catgaagatg gcatcgcttg ctacatattt cgggctagga 480
   gatcaagctc acaggagctt agatgatgtc cggatgaatc ttgaagttat caagcactgt 540
   tcaaccgtct tgtttctgga gtccagtgtt cctgacattc ttacagacat gagctggtta 600
   ttcccaagaa aaagtccgag aacacgaagt aatgagaagt cactgcctaa tggagtcaga 660
   gaaagcccga cttcttcctc ttcgagccct caaactgatc cgagttcgtc ttctgtagat 720
   gccacagctg tcaaaaacca tcccatcatt tctcttctga cggaatgctc agaaagtgat 780
   acatctagtt gtgaaataga tccatctgac ataaccactc taataagtaa actacatatt 840
   ggaactctta agacagatgc tgcggacgaa gccaaaactg taagacagca gggtgaatca 900
   accgatccca atgccaaaga tgaatcattt ttgggcgtta atgaagtatc tgtttctagc 960
   atcagggcaa gtcttatccc gttatatcgt aggagtctga gaatggagct gtttcacaac 1020
   gacacccctc tacatctctg ttggtatagc ttgaaaattc ggtttggaat aagccggaag 1080
   tatgtggatc atgtaggtcg tccaaagatg aatattgttg tagacatacc tcctgattta 1140
   tgcaagatct tggacgcatc cgatgctgct gcgcataact tactgattga ctcaagcaca 1200
   agctcagatt ggaggcctac cgttatgagg aaaaaaggct ttgccaacta tcccacagcc 1260
   agactgcaaa taagctcaga atccaatgga acccaggtat accaaaaaga agaacctttg 1320
   ggaaccaatc aaaagctcga tttcagtagc gataattttg aaaagcttga gtcagcacta 1380
   cttcctggta ccctggttga tgtattcttc tcagtcgagc cttacgatta taagaaaatg 1440
   gtagggatac gtctagcagc cagaaagttg gtaatccagc tgaagaaatg a 1491
<210> 42
   <211> 495
   <212> DNA
   <213> Boechera
<400> 42
   gtgtttttcg atcttgagac tgcggttccg accaaatcgg ggcagccttt tgcgattttg        60
   gagtttgggg ctatcttagt ttgccctatg aagctagtgg agctctatag ttactccact       120
   ttggttcgac ctaccgatct ttctctcatc tccacgctca cgaagcgacg aagcggcatt       180
   acgcgcgacg gagttctctc tgcacctaca ttctctgaaa tcgctgatga agtctacgac       240
   attctccacg gacgaatttg ggcgggacat aacataaaga gattcgattg tgtaagaata       300
   agagatgcat ttgcagaaat tggtctccct cccccggagc cgaaagctac aattgattca       360
   ctttcgttgt tgtctcagaa gtttgggaag agagctggtg acatgaagat ggcatcgctt       420
   gctacatatt tcgggctagg agatcaagct cacaggagct tagatgatgt ccggatgaat       480
   cttgaagtta tcaag                                                        495
<210> 43
   <211> 2488
   <212> DNA
   <213> Boechera
<400> 43
   tcgtaccgtt gcttctctca agtttagatt tttttccgta aaaagaggag gtggcccgtg      60
   aagtttattc cctttaaaac ccaccaatta gctccttcac tctcagttct caacaatggc     120
   ttcgactctg ggcggcgatg agagaaacga gatagtgttt ttcgatcttg agactgcggt     180
   tccgaccaaa tcggggcagc cttttgcgat tttggagttt ggggctatct tagtttgccc     240
   tatgaagcta gtggagctct atagttactc cactttggtt cgacctaccg atctttctct     300
   catctccacg ctcacgaagc gacgaagcgg cattacgcgc gacggagttc tctctgcacc     360
   tacattctct gaaatcgctg atgaagtcta cgacattctc cacggtaagg gtttctcttt     420
   ttttttttct ttctcaatct ctctcacgcg aagctacaag tattgatttt ggtgtttctg     480
   taggacgaat ttgggcggga cataacataa agagattcga ttgtgtaaga ataagagatg     540
   catttgcaga aattggtctc cctcccccgg agccgaaagc tacaattgat tcactttcgt     600
   tgttgtctca gaagtttggg aagagagctg gtgacatgaa ggtctctctt ttttcgtctt     660
   ctcgatgata aatctcaaag cctatagctt ccttgttatc tttatagata tgaatttcaa     720
   tgtaacttca aagattcatc actcatcaaa gttgctaaaa tttactctaa ataatgtaga     780
   tggcatcgct tgctacatat ttcgggctag gagatcaagc tcacaggtaa aacagtaaac     840
   gataccctgt gccttttaac gattcaccag ttgtttcaat atgggactaa acatggatat     900
   gattcaccag gagcttagat gatgtccgga tgaatcttga agttatcaag cactgtgcaa     960
   ccgtcttgtt tctggtattg ttgtcttctc atttcttgaa taatgattaa ctctaactta    1020
   aaaggattag attaaagagg ttgagacata tctgacttct gtctacagtt tgcaaaagtt    1080
   gggtccatct tccttccaga ccacaacttt gcaagccgta aacatggttt gcaagtatag    1140
   tttgtcatat cactgagttt aagtacttgg tgtttgcagg agtccagtgt tcctgacatt    1200
   cttacagaca tgagctggtt attcccaaga aaaagtccga gaacacgaag taatgagaag    1260
   tcactgccta atggagtcag agaaagcccg acttcttcct cttcgagccc taaaactgat    1320
   ccgagttcgt cttctgtaga tgccacagct gtcaaaaacc atcccatcat ttctcttctg    1380
   acggaatgct cagaaagtga tacatctagt tgtgaaatag atccatctga cataaccact    1440
   ctaataagta aactacatat tggaactctt aagagagatg ctgcggacga agccaaaatt 1500
   gtaagacagc agggtgaatc aaccgatccc aatgccaaag atgaatcatt tttgggcgtt 1560
   aatgaagtat ctgtttctag catcagggca agtcttatcc cgttatatcg tgggagtctg 1620
   agaatggagc tgtttcacaa tgacacccct ctacatctct gttggtatag cttgaaaatt 1680
   cggtttggaa taagccggaa gtatgtggat catgtaggtc gtccaaagat gaatattgtt 1740
   gtagacatac ctcctgattt atgcaagatc ttggacgcat acgatgctgc tgcgcataac 1800
   ttactgattg actcaagcac aagctcagat tggaggccta ctgttatgag gaaagaaggc 1860
   tttgccaact atcccacagc cagactgcag taagtattca acactctctc tgacctttta 1920
   catacgagca tgaatccacc ggagagtctc taagaccatc tccaacccta ctccgtattc 1980
   acctccaaac tctattttgg agttaaatcc ctccaaccct tgcaaaatag atatcttcaa 2040
   aattttctcc atatttggag attttgattt tttaagtcat gactccattt tggagttggg 2100
   ttggagaaaa acacaactcc aaaatagagt tacttcattt tggagtaaaa aaatgaagaa 2160
   atgggttgga gatactctaa cctctttcac cattcttatg ttgttggcag aataagctca 2220
   gaatccaatg gaacccaggt ataccaaaaa gaagaacctt tgggaaccaa tcaaaagctc 2280
   gatttcagta gcgataattt tgaaaagctt gagtcagcac tacttcctgg taccctggtt 2340
   gatgcattct tctcacccga atcttacgat tataagaaaa tggtagggat acgtctagca 2400
   gccagaaagt tggtaatcca cctgaagaaa tgatctagcc aaggaaaaat cattcctctg 2460
   tctcttcctg gtcagtcggt gagcactt 2488
<210> 44
   <211> 1491
   <212> DNA
   <213> Boechera
<400> 44
   atggcttcga ctctgggcgg cgatgagaga aacgagatag tgtttttcga tcttgagact      60
   gcggttccga ccaaatcggg gcagcctttt gcgattttgg agtttggggc tatcttagtt     120
   tgccctatga agctagtgga gctctatagt tactccactt tggttcgacc taccgatctt     180
   tctctcatct ccacgctcac gaagcgacga agcggcatta cgcgcgacgg agttctctct     240
   gcacctacat tctctgaaat cgctgatgaa gtctacgaca ttctccacgg acgaatttgg     300
   gcgggacata acataaagag attcgattgt gtaagaataa gagatgcatt tgcagaaatt     360
   ggtctccctc ccccggagcc gaaagctaca attgattcac tttcgttgtt gtctcagaag     420
   tttgggaaga gagctggtga catgaagatg gcatcgcttg ctacatattt cgggctagga     480
   gatcaagctc acaggagctt agatgatgtc cggatgaatc ttgaagttat caagcactgt     540
   gcaaccgtct tgtttctgga gtccagtgtt cctgacattc ttacagacat gagctggtta     600
   ttcccaagaa aaagtccgag aacacgaagt aatgagaagt cactgcctaa tggagtcaga     660
   gaaagcccga cttcttcctc ttcgagccct aaaactgatc cgagttcgtc ttctgtagat     720
   gccacagctg tcaaaaacca tcccatcatt tctcttctga cggaatgctc agaaagtgat     780
   acatctagtt gtgaaataga tccatctgac ataaccactc taataagtaa actacatatt     840
   ggaactctta agagagatgc tgcggacgaa gccaaaattg taagacagca gggtgaatca     900
   accgatccca atgccaaaga tgaatcattt ttgggcgtta atgaagtatc tgtttctagc     960
   atcagggcaa gtcttatccc gttatatcgt gggagtctga gaatggagct gtttcacaat    1020
   gacacccctc tacatctctg ttggtatagc ttgaaaattc ggtttggaat aagccggaag    1080
   tatgtggatc atgtaggtcg tccaaagatg aatattgttg tagacatacc tcctgattta    1140
   tgcaagatct tggacgcata cgatgctgct gcgcataact tactgattga ctcaagcaca    1200
   agctcagatt ggaggcctac tgttatgagg aaagaaggct ttgccaacta tcccacagcc    1260
   agactgcaaa taagctcaga atccaatgga acccaggtat accaaaaaga agaacctttg    1320
   ggaaccaatc aaaagctcga tttcagtagc gataattttg aaaagcttga gtcagcacta    1380
   cttcctggta ccctggttga tgcattcttc tcacccgaat cttacgatta taagaaaatg    1440
   gtagggatac gtctagcagc cagaaagttg gtaatccacc tgaagaaatg a             1491
<210> 45
   <211> 495
   <212> DNA
   <213> Boechera
<400> 45
   gtgtttttcg atcttgagac tgcggttccg accaaatcgg ggcagccttt tgcgattttg 60
   gagtttgggg ctatcttagt ttgccctatg aagctagtgg agctctatag ttactccact 120
   ttggttcgac ctaccgatct ttctctcatc tccacgctca cgaagcgacg aagcggcatt 180
   acgcgcgacg gagttctctc tgcacctaca ttctctgaaa tcgctgatga agtctacgac 240
   attctccacg gacgaatttg ggcgggacat aacataaaga gattcgattg tgtaagaata 300
   agagatgcat ttgcagaaat tggtctccct cccccggagc cgaaagctac aattgattca 360
   ctttcgttgt tgtctcagaa gtttgggaag agagctggtg acatgaagat ggcatcgctt 420
   gctacatatt tcgggctagg agatcaagct cacaggagct tagatgatgt ccggatgaat 480
   cttgaagtta tcaag 495
<210> 46
   <211> 2519
   <212> DNA
   <213> Boechera
<400> 46
   tcgtaccgtt gcttctctca agtttagatt ttttttccgt aaatagagga ggatcaattg      60
   ctttaaaacc caccaattag ctccttcact ctcagttctc aacaatggct tcgactctgg     120
   gcggcgatga gagatgcgag atagtgtttt tcgatcttga gacggcggtt ccgaccaaat     180
   cggggcagcc ttttgcgatt ttggagtttg gggctatctt agtttgccct atgaagctag     240
   tggagctcta tagttactcc actttggttc gacccaccga tctttctctc atctccacgc     300
   tcacgaagcg acgaagcggc attacgcgcg acggagttct ctctgcacct acattctctg     360
   aaatcgctga tgaagtctac gacattctcc acggtaaggg tttctctttt ttttttttct      420
   ttctcaatct ctctgacacg aagctacaag tattgatttt ggtgtttctg taggacgaat     480
   ttgggcggga cataacataa agagattcga ttgtgtaaga ataagagatg catttgcagg     540
   aattggtgtc tctcccccgg agccgaaagc tacaattgat tcactttcgt tgttgtctca     600
   gaagtttggg aagagagctg gtgacatgaa ggtctctctt ttttcgtctt ctcgatgata     660
   aatctcaaag cctatagctt ccttgttatc tttatagata tgaatttcca tgtaacttca     720
   aagattcatc actcatcaga gttgctaaaa tttactcttt ttaaaaaatg tagatggcat     780
   cgcttgctac atatttcggg ctaggagatc aagctcacag gtaaaaagag taaacgatac     840
   catgtgcctt ttaacgattc accagttgtt tcaatatggg actaaacatg gttatgattc     900
   accaggagct tagatgatgt ccggatgaat cttgaagtag tcaagtactg tgcaaccgtc     960
   ttgtttctgg tattgctgtc ttttcatttc ttgaataatg attaactcta acttaaaagg    1020
   attagattag agaggttgag acatatctga cttctgtcta cagtttgcaa aagttgggtc    1080
   catcttcctt tcagaccaca actttgcaag ccgtaaacat gggttgcaac ttgcaagtat    1140
   agtttgtcat atcactgagt ttaagtactt ggtgtttgca ggagtccagt gttcctgaca    1200
   ttcttaaaga catgagctgg ttttccccaa gaaaaagtcc gagaacacga agtaatgaga    1260
   agtcactgcc taatggagtc agagaaagcc cgacttcttc ctcttcaagc cctaaaactg    1320
   atccgagttc gtcttctgta gatgccacaa ctgtcaaaaa ccatcccatc atttctcttc    1380
   tgacggaatg ctcagaaagt gatacatcta gttgtgaaat agatccatct gacataacca    1440
   ctctaataag taaactacat attggaactc ttaagagaga tgctgcggac gaagccaaaa    1500
   ctgtgagaga tgctgcggac gaagccaaaa ctgtaagaca gcagggtgaa tcaaccgatc    1560
   ccaatgccaa agatgaatca tttttgggcg ttaatgaagt atctgtttct agcatcaggg    1620
   caagtcttat cccgttatat cgtgggagtc tgagaatgga gctgtttcac aatgacaccc    1680
   ctctacatct ctgttggtat agcttgaaaa ttcggtttgg aataagccgg aagtatgtgg    1740
   atcatgtagg tcgtccaaag atgaatattg ttgtagacat acctcctgat ttatgcaaga    1800
   tcttggacgc atccgatgct gctgcgcata acttactgat tgactcaagc acaagctcag    1860
   attggaggcc tactgttatg aggaaagaag gctttgccaa ctatcccaca gccagactgc    1920
   agtaagtatt caacactctc tctgaccttt tacatacgag catgaatcca ccggagagtc 1980
   tctaagacca tctccaaccc tactccgtat tcacctccaa actctatttt ggagttaaat 2040
   ccctccaacc cttgcaaaat agatatcttc aaaattttct ccatatttgg agattttgaa 2100
   tttttaagtc atgactccat tttggagttg ggttggagaa aaacacaact ccaaaataga 2160
   gttacttcat tttggagtaa aaaatgaaga aatgggttgg agatactcta acctctgtca 2220
   ccattcttat gttgttggca gaataagctc agaatccaat ggaacccagg tacaccaaaa 2280
   agaagaacct ttgggaacca atcaaaagct cgatttcagt agcgataatt ttgaaaagct 2340
   tgagtcagca ctacttcctg gtaccctggt tgatgcattc ttctcactcg agccttacga 2400
   ttataagaaa atggtaggga tacgtctagc agccagaaag ttggtaatcc acctgaagaa 2460
   atgatctagc caaggaaaaa tcattcctct gtctcttgct ggtcagtcgg tgagcactt 2519
<210> 47
   <211> 1521
   <212> DNA
   <213> Boechera
<400> 47
   atggcttcga ctctgggcgg cgatgagaga tgcgagatag tgtttttcga tcttgagacg      60
   gcggttccga ccaaatcggg gcagcctttt gcgattttgg agtttggggc tatcttagtt     120
   tgccctatga agctagtgga gctctatagt tactccactt tggttcgacc caccgatctt     180
   tctctcatct ccacgctcac gaagcgacga agcggcatta cgcgcgacgg agttctctct     240
   gcacctacat tctctgaaat cgctgatgaa gtctacgaca ttctccacgg acgaatttgg     300
   gcgggacata acataaagag attcgattgt gtaagaataa gagatgcatt tgcaggaatt     360
   ggtgtctctc ccccggagcc gaaagctaca attgattcac tttcgttgtt gtctcagaag     420
   tttgggaaga gagctggtga catgaagatg gcatcgcttg ctacatattt cgggctagga     480
   gatcaagctc acaggagctt agatgatgtc cggatgaatc ttgaagtagt caagtactgt     540
   gcaaccgtct tgtttctgga gtccagtgtt cctgacattc ttaaagacat gagctggttt     600
   tccccaagaa aaagtccgag aacacgaagt aatgagaagt cactgcctaa tggagtcaga     660
   gaaagcccga cttcttcctc ttcaagccct aaaactgatc cgagttcgtc ttctgtagat     720
   gccacaactg tcaaaaacca tcccatcatt tctcttctga cggaatgctc agaaagtgat     780
   acatctagtt gtgaaataga tccatctgac ataaccactc taataagtaa actacatatt     840
   ggaactctta agagagatgc tgcggacgaa gccaaaactg tgagagatgc tgcggacgaa     900
   gccaaaactg taagacagca gggtgaatca accgatccca atgccaaaga tgaatcattt     960
   ttgggcgtta atgaagtatc tgtttctagc atcagggcaa gtcttatccc gttatatcgt    1020
   gggagtctga gaatggagct gtttcacaat gacacccctc tacatctctg ttggtatagc    1080
   ttgaaaattc ggtttggaat aagccggaag tatgtggatc atgtaggtcg tccaaagatg    1140
   aatattgttg tagacatacc tcctgattta tgcaagatct tggacgcatc cgatgctgct    1200
   gcgcataact tactgattga ctcaagcaca agctcagatt ggaggcctac tgttatgagg    1260
   aaagaaggct ttgccaacta tcccacagcc agactgcaaa taagctcaga atccaatgga    1320
   acccaggtac accaaaaaga agaacctttg ggaaccaatc aaaagctcga tttcagtagc    1380
   gataattttg aaaagcttga gtcagcacta cttcctggta ccctggttga tgcattcttc    1440
   tcactcgagc cttacgatta taagaaaatg gtagggatac gtctagcagc cagaaagttg    1500
   gtaatccacc tgaagaaatg a                                               1521
<210> 48
   <211> 495
   <212> DNA
   <213> Boechera
<400> 48
   gtgtttttcg atcttgagac ggcggttccg accaaatcgg ggcagccttt tgcgattttg 60
   gagtttgggg ctatcttagt ttgccctatg aagctagtgg agctctatag ttactccact 120
   ttggttcgac ccaccgatct ttctctcatc tccacgctca cgaagcgacg aagcggcatt 180
   acgcgcgacg gagttctctc tgcacctaca ttctctgaaa tcgctgatga agtctacgac 240
   attctccacg gacgaatttg ggcgggacat aacataaaga gattcgattg tgtaagaata 300
   agagatgcat ttgcaggaat tggtgtctct cccccggagc cgaaagctac aattgattca 360
   ctttcgttgt tgtctcagaa gtttgggaag agagctggtg acatgaagat ggcatcgctt 420
   gctacatatt tcgggctagg agatcaagct cacaggagct tagatgatgt ccggatgaat 480
   cttgaagtag tcaag 495
<210> 49
   <211> 2487
   <212> DNA
   <213> Boechera
<400> 49
   tcgtaccgtt gcttctctca aggttagatt ttttttccgt aaaaagagga ggatcgattg 60
   ctttaaaacc caccaattag ctccttcact ctcagtcctt aacaatggct tcgactctgg 120
   gcggcgatga gagatgcgag atagtgtttt tcgatcttga gacggcagtt ccgaccaaat 180
   cggggcagcc ttttgcgatt ttggagtttg gggctatctt agtttgccct atgaagctag 240
   tggagctcta tagttactcc accttggttc gacccacaga tctttctctc atctccacgc 300
   tcacgaagcg acgaagcggc attacgcgcg acggagttct ctctgcacct acattctctg 360
   aaatcgctga tgaagtctac gacattctcc acggtaaggg tttctctttt ttttttctct 420
   ccatctctct cacacgaagg tacaagtatt gattttggtg tttctgtagg acgaatttgg 480
   gcgggacata acataaagag attcgattgt gtaagaataa gagatgcatt tgcaggaatt 540
   ggtctctctc ccccggagcc gaaagctaca attgattcac tttcgttgtt gtctcagaag 600
   tttgggaaga gagctggtga catgaaggtc tctctttttt cgtcttctcg atgataaatc 660
   tcaaagccaa tagcttcctt gttatcttta tagatatgaa tttccatgta acttcaaaga 720
   ttcatcactc atcagagttg ctaaaattta ctctttttca ataacgtaga tggcatcgct 780
   tgctacatat ttcgggctag gagatcaagc tcacaggtaa aaagagtaaa cgataccctg 840
   tgccttttaa cgattcacca gttgtttcaa tatgggacta aacatggtta tgattcacca 900
   ggagcttaga tgatgtccgg atgaatcttg aagtagtcaa gtactgtgca accgtcttat 960
   ttctggtatt gctgtcttct catttcttga ataatgatca actctaactt aaaaaggatt 1020
   agattagaga ggttgagaca tatctgactt ctgtctacag tttgcaaaag ttgggtccat 1080
   cttcctttca gaccacaact ttgcaagccg taaacatggg ttgcaacttg caagtatagt 1140
   ttgtcatatc actgagttta agtacttggt gtttgcagga gtccagtgtt cctgacattc 1200
   ttaaagacat gagctggttt tccccaagaa aaagtccgag aacacgaagt aatgagaagt 1260
   cactgcctaa tggagtcaga gaaagcccga cttcttcctc ttcaagccct aaaactgatc 1320
   cgagttcgtc ttctgtagat gccacaactg tcaaaaacca tcccatcatt tctcttctga 1380
   cggaatgctc agaaagtgat acatctagtt gtgaaataga tccatctgac ataaccactc 1440
   taataagtaa actacatatt ggaactctta agagagatgc tgcggatgaa gccaaaattg 1500
   taagacagca gggtgaatca accgatccca atgccaaaga tgaatcattt ttgggcgtta 1560
   atgaagtatc tgtttctagc atcagggcaa gtcttatccc gttatatcgt gggagtctga 1620
   gaatggagct gcttcacaat gacacccctc tacatctctg ttggtatagc ttgaaaattc 1680
   ggtttggaat aagccggaag tatgtggatc atgtaggtcg tccaaagatg aatattgttg 1740
   tagacatacc tcctgattta tgcaagatct tggacgcata cgatgctgct gcgcataact 1800
   tactgattga ctcaagcaca agctcagatt ggaggcctac tgttatgagg aaagaaggct 1860
   ttgccaacta tcccacagcc agactgcagt aagtattcaa cactctctct gaccttttac 1920
   atacgagcat gaatccaccg gagagtctct aaaaccatct ccaaccctac tccgtattca 1980
   cctccaaact ctattttgga gttaaatccc tccaaccctt gcaaaataga tatcttcaaa 2040
   attttctcca tatttggaga ttttgatttt ttaagtcatg actccatttt ggagttgggt 2100
   tggagaaaaa cacaactcca aaatagagtt acttcatttt ggagtaaaaa aatgaagaaa 2160
   tgggttggag atactctaac ctctgtcacc attcttatgt tgttggcaga ataagctcag 2220
   aatccaatgg aacccaggta taccaaaaag aagaaccttt gggaaccaat caaaagctcg 2280
   atttcagtag cgataatttt gaaaagcttg agtcagcact acttcctggt accctggttg 2340
   atgcattctt ctcactcgaa tcttacgatt ataagaaaat ggtagggata cgtctagcag 2400
   ccagaaagtt ggtaatccac ctgaagaaat gatctagcca aggaaaaatc attcctctgt 2460
   ctcttcctgg tcagtcggtg agcactt 2487
<210> 50
   <211> 1491
   <212> DNA
   <213> Boechera
<400> 50
   atggcttcga ctctgggcgg cgatgagaga tgcgagatag tgtttttcga tcttgagacg      60
   gcagttccga ccaaatcggg gcagcctttt gcgattttgg agtttggggc tatcttagtt     120
   tgccctatga agctagtgga gctctatagt tactccacct tggttcgacc cacagatctt     180
   tctctcatct ccacgctcac gaagcgacga agcggcatta cgcgcgacgg agttctctct     240
   gcacctacat tctctgaaat cgctgatgaa gtctacgaca ttctccacgg acgaatttgg     300
   gcgggacata acataaagag attcgattgt gtaagaataa gagatgcatt tgcaggaatt     360
   ggtctctctc ccccggagcc gaaagctaca attgattcac tttcgttgtt gtctcagaag     420
   tttgggaaga gagctggtga catgaagatg gcatcgcttg ctacatattt cgggctagga     480
   gatcaagctc acaggagctt agatgatgtc cggatgaatc ttgaagtagt caagtactgt     540
   gcaaccgtct tatttctgga gtccagtgtt cctgacattc ttaaagacat gagctggttt     600
   tccccaagaa aaagtccgag aacacgaagt aatgagaagt cactgcctaa tggagtcaga     660
   gaaagcccga cttcttcctc ttcaagccct aaaactgatc cgagttcgtc ttctgtagat     720
   gccacaactg tcaaaaacca tcccatcatt tctcttctga cggaatgctc agaaagtgat     780
   acatctagtt gtgaaataga tccatctgac ataaccactc taataagtaa actacatatt     840
   ggaactctta agagagatgc tgcggatgaa gccaaaattg taagacagca gggtgaatca     900
   accgatccca atgccaaaga tgaatcattt ttgggcgtta atgaagtatc tgtttctagc     960
   atcagggcaa gtcttatccc gttatatcgt gggagtctga gaatggagct gcttcacaat    1020
   gacacccctc tacatctctg ttggtatagc ttgaaaattc ggtttggaat aagccggaag    1080
   tatgtggatc atgtaggtcg tccaaagatg aatattgttg tagacatacc tcctgattta    1140
   tgcaagatct tggacgcata cgatgctgct gcgcataact tactgattga ctcaagcaca    1200
   agctcagatt ggaggcctac tgttatgagg aaagaaggct ttgccaacta tcccacagcc    1260
   agactgcaaa taagctcaga atccaatgga acccaggtat accaaaaaga agaacctttg    1320
   ggaaccaatc aaaagctcga tttcagtagc gataattttg aaaagcttga gtcagcacta    1380
   cttcctggta ccctggttga tgcattcttc tcactcgaat cttacgatta taagaaaatg    1440
   gtagggatac gtctagcagc cagaaagttg gtaatccacc tgaagaaatg a              1491
<210> 51
   <211> 495
   <212> DNA
   <213> Boechera
<400> 51
   gtgtttttcg atcttgagac ggcagttccg accaaatcgg ggcagccttt tgcgattttg 60
   gagtttgggg ctatcttagt ttgccctatg aagctagtgg agctctatag ttactccacc 120
   ttggttcgac ccacagatct ttctctcatc tccacgctca cgaagcgacg aagcggcatt 180
   acgcgcgacg gagttctctc tgcacctaca ttctctgaaa tcgctgatga agtctacgac 240
   attctccacg gacgaatttg ggcgggacat aacataaaga gattcgattg tgtaagaata 300
   agagatgcat ttgcaggaat tggtctctct cccccggagc cgaaagctac aattgattca 360
   ctttcgttgt tgtctcagaa gtttgggaag agagctggtg acatgaagat ggcatcgctt 420
   gctacatatt tcgggctagg agatcaagct cacaggagct tagatgatgt ccggatgaat 480
   cttgaagtag tcaag 495
<210> 52
   <211> 2486
   <212> DNA
   <213> Boechera
<400> 52
   tcgtaccgtt gcttctctca agtttagatt ttttttccgt aaatagagga ggatcaattg 60
   ctttaaaacc caccaattag ctccttcact ctcagttctc aacaatggct tcgactctgg 120
   gcggcgatgg gagatgcgag atagtgtttt tcgatcttga gacggcggtt ccgaccaaat 180
   cggggcagcc ttttgcgatt ttggagtttg gggctatctt agtttgccct atgaagctag 240
   tggagctcta tagttactcc actttggttc gacccaccga tctttctctc atctccacgc 300
   tcacgaagcg acgaagcggc attacgcgcg acggagttct ctctgcacct acattctctg 360
   aaatcgctga tgaagtctac gacattctcc acggtaaggg tttctctttt tttttttctt 420
   tctcaatctc tctgacacga agctacaagt attgattttg gtgtttctgt aggacgaatt 480
   tgggcgggac ataacataaa gagattcgat tgtgtaagaa tacgagatgc atttgcagga 540
   attggtctct ctcccccgga gccgaaagct acaattgatt cactttcgtt attgtctcag 600
   aagtttggga agagagctgg tgacatgaag gtctctcttt tttcgtcttc tcgatgataa 660
   atctcaaagc ctatagcttc cttgttatct ttatagatat gaatttccat gtaacttcaa 720
   agattcatca ctcatcagag ttgctaaaat ttactctttt taaaaaatgt agatggcatc 780
   gcttgctaca tatttcgggc taggagatca ggctcacagg taaaaagagt aaacgatacc 840
   atgtgccttt taacgattca ccagttgttt caatatggga ctaaacatgg ttatgattca 900
   ccaggagctt agatgatgtc cggatgaatc ttgaagtagt caagtactgt gcaaccgtct 960
   tgtttctggt attgctgtct tttcatttct tgaataatga ttaactctaa cttaaaagga 1020
   ttagattaga gaggttgaga catatctgat ttctgtctac agtttgcaaa agttggttcc 1080
   atcttccttt cagaccacaa ctttgcaagc cgtaaacatg ggttgcaact tgcaagtata 1140
   gtttgttata tcactgagtt taagtacttg gtgtttgcag gagtccagtg ttcctgacat 1200
   tcttaaagac atgagctggt tttccccaag aaaaagtccg agaacacgaa gtaatgagaa 1260
   gtcactgcct aatggagtca gagaaagccc gacttcttcc tcttcaagcc ctaaaactga 1320
   tccgagttcg tcttctgtag atgccacaac tgtcaaaaac catcccatca tttctcttct 1380
   gacggaatgc tcagaaagtg atacatctag ttgtgaaata gatccatctg acataaccac 1440
   tctaataagt aaactacata ttggaactct taagagagat gctgcggacg aagccaaaac 1500
   tgtaagacag cagggtgaat caaccgatcc caatgccaaa gatgaatcat ttttgggcgt 1560
   taatgaagta tctgtttcta gcatcagggc aagtcttatc ccgttatatc gtgggggtct 1620
   gagaatggag ctgtttcaca atgacacccc tctacatctc cgttggtata gcttgaaaat 1680
   tcggtttgga ataagccgga agtatgtgga tcatgtaggt cgtccaaaga tgaatattgt 1740
   cgtagacata cctcctgatt tatgcaagat cttggacgca tccgatgctg ctgcgcataa 1800
   cttactgatt gactcaagca caagctcaga ttggaggcct actgttatga ggaaagaagg 1860
   ctttgccaac tatcccacag ccagactgca gtaagtattc agcactctct ctgacctttt 1920
   acatacgagc atgaatccac cggagagtct ctaagaccat ctccaaccct actccgtatt 1980
   cacctccaaa ctctattttg gagttaaatc cctccaaccc ttgcaaaata gacatcttca 2040
   aaattttctc catatttgga gattttgatt ttttaagtca tgactccatt ttggagttgg 2100
   gttggagaaa aacacaactc caaaatagat ttacttcatt ttggagtaaa aaatgaagaa 2160
   atgggttgga gatactaacc tctgtcacca ttcttatgtt gttggcagaa taagctcaga 2220
   atccaatgga acccaggtac accaaaaaga agaacctttg ggaaccaatc aaaagctcga 2280
   tttcagtagc gataattttg aaaagcttga gtcagcactt cttcctggta ccctggttga 2340
   tgcattcttc tcactcgagc cttacgatta taagaaaatg gtagggatac gtctagcagc 2400
   cagaaagttg gtaatccacc tgaagaaatg atctagccaa ggaaaaatca ttcctctgtc 2460
   tcttcctggt cagtcggtga gcactt 2486
<210> 53
   <211> 1491
   <212> DNA
   <213> Boechera
<400> 53
   atggcttcga ctctgggcgg cgatgggaga tgcgagatag tgtttttcga tcttgagacg        60
   gcggttccga ccaaatcggg gcagcctttt gcgattttgg agtttggggc tatcttagtt       120
   tgccctatga agctagtgga gctctatagt tactccactt tggttcgacc caccgatctt       180
   tctctcatct ccacgctcac gaagcgacga agcggcatta cgcgcgacgg agttctctct       240
   gcacctacat tctctgaaat cgctgatgaa gtctacgaca ttctccacgg acgaatttgg       300
   gcgggacata acataaagag attcgattgt gtaagaatac gagatgcatt tgcaggaatt       360
   ggtctctctc ccccggagcc gaaagctaca attgattcac tttcgttatt gtctcagaag       420
   tttgggaaga gagctggtga catgaagatg gcatcgcttg ctacatattt cgggctagga       480
   gatcaggctc acaggagctt agatgatgtc cggatgaatc ttgaagtagt caagtactgt       540
   gcaaccgtct tgtttctgga gtccagtgtt cctgacattc ttaaagacat gagctggttt       600
   tccccaagaa aaagtccgag aacacgaagt aatgagaagt cactgcctaa tggagtcaga       660
   gaaagcccga cttcttcctc ttcaagccct aaaactgatc cgagttcgtc ttctgtagat       720
   gccacaactg tcaaaaacca tcccatcatt tctcttctga cggaatgctc agaaagtgat       780
   acatctagtt gtgaaataga tccatctgac ataaccactc taataagtaa actacatatt       840
   ggaactctta agagagatgc tgcggacgaa gccaaaactg taagacagca gggtgaatca       900
   accgatccca atgccaaaga tgaatcattt ttgggcgtta atgaagtatc tgtttctagc       960
   atcagggcaa gtcttatccc gttatatcgt gggggtctga gaatggagct gtttcacaat      1020
   gacacccctc tacatctccg ttggtatagc ttgaaaattc ggtttggaat aagccggaag      1080
   tatgtggatc atgtaggtcg tccaaagatg aatattgtcg tagacatacc tcctgattta      1140
   tgcaagatct tggacgcatc cgatgctgct gcgcataact tactgattga ctcaagcaca      1200
   agctcagatt ggaggcctac tgttatgagg aaagaaggct ttgccaacta tcccacagcc      1260
   agactgcaaa taagctcaga atccaatgga acccaggtac accaaaaaga agaacctttg      1320
   ggaaccaatc aaaagctcga tttcagtagc gataattttg aaaagcttga gtcagcactt      1380
   cttcctggta ccctggttga tgcattcttc tcactcgagc cttacgatta taagaaaatg      1440
   gtagggatac gtctagcagc cagaaagttg gtaatccacc tgaagaaatg a                1491
<210> 54
   <211> 495
   <212> DNA
   <213> Boechera
<400> 54
   gtgtttttcg atcttgagac ggcggttccg accaaatcgg ggcagccttt tgcgattttg        60
   gagtttgggg ctatcttagt ttgccctatg aagctagtgg agctctatag ttactccact       120
   ttggttcgac ccaccgatct ttctctcatc tccacgctca cgaagcgacg aagcggcatt       180
   acgcgcgacg gagttctctc tgcacctaca ttctctgaaa tcgctgatga agtctacgac       240
   attctccacg gacgaatttg ggcgggacat aacataaaga gattcgattg tgtaagaata       300
   cgagatgcat ttgcaggaat tggtctctct cccccggagc cgaaagctac aattgattca       360
   ctttcgttat tgtctcagaa gtttgggaag agagctggtg acatgaagat ggcatcgctt       420
   gctacatatt tcgggctagg agatcaggct cacaggagct tagatgatgt ccggatgaat       480
   cttgaagtag tcaag                                                        495
<210> 55
   <211> 115
   <212> DNA
   <213> Boechera
<220>
   <221> misc_feature
   <222> (107)..(107)
   <223> n is a, c, g, or t
<400> 55
   tcgtaccgtt gcttctctca agtttagatt tttttccgta aaaagaggag gtggcccgtg        60
   aagtttattc cctttaaaac ccaccaatta gctccttcac tctcagntct caaca           115
<210> 56
   <211> 105
   <212> DNA
   <213> Boechera
<220>
   <221> misc_feature
   <222> (1)..(1)
   <223> n is a, c, g, or t
<220>
   <221> misc_feature
   <222> (5)..(6)
   <223> n is a, c, g, or t
<220>
   <221> misc_feature
   <222> (9) .. (9)
   <223> n is a, c, g, or t
<220>
   <221> misc_feature
   <222> (13)..(13)
   <223> n is a, c, g, or t
<220>
   <221> misc_feature
   <222> (23)..(23)
   <223> n is a, c, g, or t
<220>
   <221> misc_feature
   <222> (32)..(32)
   <223> n is a, c, g, or t
<220>
   <221> misc_feature
   <222> (36)..(38)
   <223> n is a, c, g, or t
<220>
   <221> misc_feature
   <222> (45)..(45)
   <223> n is a, c, g, or t
<220>
   <221> misc_feature
   <222> (55)..(55)
   <223> n is a, c, g, or t
<220>
   <221> misc_feature
   <222> (57)..(57)
   <223> n is a, c, g, or t
<220>
   <221> misc_feature
   <222> (69) .. (69)
   <223> n is a, c, g, or t
<220>
   <221> misc_feature
   <222> (97)..(98)
   <223> n is a, c, g, or t
<220>
   <221> misc_feature
   <222> (101)..(101)
   <223> n is a, c, g, or t
<400> 56
   ncgtnncgnt gcntctctca agnttagatt tntttnnncg taaanagagg aggancnatt 60
   gctttaaanc ccaccaatta gctccttcac tctcagnnct naaca 105
<210> 57
   <211> 115
   <212> DNA
   <213> Boechera
<400> 57
   tcgtaccgtt gcttctctca agtttagatt tttttccgta aaaagaggag gtggcccgtg        60
   aagtttattc cctttaaaac ccaccaatta gctccttcac tctcagttct caaca            115
<210> 58
   <211> 115
   <212> DNA
   <213> Boechera
<400> 58
   tcgtaccgtt gcttctctca agtttagatt tttttccgta aaaagaggag gtggcccgtg        60
   aagtttattc cctttaaaac ccaccaatta gctccttcac tctcagttct caaca            115
<210> 59
   <211> 115
   <212> DNA
   <213> Boechera
<400> 59
   tcgtaccgtt gcttctctca agtttagatt tttttccgta aaaagaggag gtggcccgtg        60
   aagtttattc cctttaaaac ccaccaatta gctccttcac tctcagttct caaca            115
<210> 60
   <211> 104
   <212> DNA
   <213> Boechera
<400> 60
   tcgtaccgtt gcttctctca agtttagatt ttttttccgt aaatagagga ggatcaattg        60
   ctttaaaacc caccaattag ctccttcact ctcagttctc aaca                        104
<210> 61
   <211> 104
   <212> DNA
   <213> Boechera
<400> 61
   tcgtaccgtt gcttctctca aggttagatt ttttttccgt aaaaagagga ggatcgattg        60
   ctttaaaacc caccaattag ctccttcact ctcagtcctt aaca                        104
<210> 62
   <211> 104
   <212> DNA
   <213> Boechera
<400> 62
   tcgtaccgtt gcttctctca agtttagatt ttttttccgt aaatagagga ggatcaattg       60
   ctttaaaacc caccaattag ctccttcact ctcagttctc aaca                        104
<210> 63
   <211> 10
   <212> PRT
   <213> Boechera
<400> 63
   Asp Ala Ala Asp Glu Ala Lys Thr Val Arg
   1               5                   10
<210> 64
   <211> 30
   <212> DNA
   <213> Boechera
<400> 64
   agatgctgcg gacgaagcca aaactgtgag 30
<210> 65
   <211> 20
   <212> DNA
   <213> Boechera
<400> 65
   tggcccgtga agtttattcc 20

## Description

The present invention relates to nucleic acid molecules for use in inducing apomixis in a plant, transgenic cells, in particular transgenic plant cells, comprising said nucleic acid molecule, transgenic plants, in particular plant seeds, comprising said nucleic acid molecule, methods for inducing apomixis in a plant, methods for the production of apomictic plants and uses thereof.

Naturally occurring vegetative, non-sexual reproduction in plants through seeds, also called apomixis, is a genetically controlled reproductive mechanism of plants primarily found in some polyploid non-cultivated species. Various types of apomixis, inter alia gametophytic and sporophytic, can be distinguished. In sporophytic apomixis also called adventitive embryony, a somatic embryo develops not from the gametophyte but directly from the cells of the nucellus, ovary wall or integuments. Somatic embryos from surrounding cells invade the sexual ovary, one of the somatic embryos out-competes the other somatic embryos and the sexual embryo, and utilizes the produced endosperm.

Gametophytic apomixis is a naturally-occurring type of asexual seed formation whereby progeny, which are clonal to the maternal genotype, are produced from meiotically-unreduced embryo sacs, i. e. the female gametophyte. Most gametophytic apomictic species are found in the Asteraceae, Rosaceae and Poaceae, where they have arisen independently and recurrently. Polyploidy, facultative apomixis (both sexual and apomictic seed production within one individual), and faster development of the apomeiotic ovule relative to the sexual one are traits which are shared among most of these taxa.

Apomixis is derived from sex, and three independent developmental steps must be acquired for a sexual plant to produce seeds apomictically: the formation of an unreduced megaspore, that means the formation of an embryo sac having the same ploidy as the somatic cells of the mother plant from a meiotically-unreduced megaspore (diplospory, apomeiosis) or from nucellar cell (apospory), the subsequent development of an embryo from an unreduced egg in the absence of fertilization (parthenogenesis) and fertilization of the binu-cleate central cell to form a functional endosperm (pseudogamy). The term "apomeiosis" covers both apospory and diplospory. The apomeiotically-derived embryo thus receives its entire genome through the female line. As these components are under separate genetic control, it has been difficult to envision how all three could evolve in unison in a sexual ancestor considering random mutations, since the expression of any single step would decrease the fitness of its sexual carrier. It is widely accepted that apomictic seed development results from deregulation of the sexual development pathway, which would be manifested at multiple loci simultaneously. In wild apomictic taxa, this coordinated deregulation is hypothesized to be influenced by global regulatory changes resulting from hybridization and/or polyploidy (Grossniklaus, 2001, From sexuality to apomixis: Molecular and genetic approaches, In: The flowering of apomixis: From Mechanisms to Genetic Engineering, 168-211).

Recent reports analyse the gene expression of apomeiosis, that means unreduced gamete formation, in microdissected ovules of Boechera, and were able to identify quite a large number of differentially expressed alleles between sexual and apomeiotic ovules in a particular stage of the development, namely the megaspore mother cell (MMC) stage. Further studies focussed on heterochrony of gene expression patterns over a series of developmental stages in sexual and apomeiotic ovules (Sharbel et al., 2009, The Plant Journal, 58, 870-882, Sharbel et al., 2010, The Plant Cell, 22, 655-671). However, although the state of the art expectedly show that apomictic and sexual ovules are characterised by specific molecular signatures, it does not provide any clue on how to induce apomixis in a desired plant in a reliable and foreseeable manner, in particular by means of conventional gene transfer techniques.

In fact, one of the main difficulties in identifying the molecular genetic mechanisms controlling apomixis is that the genomes of virtually all apomicts are both polyploidy and hybrid in nature. Although considerable efforts, including in-depth functional molecular analyses, have been undertaken to analyse the molecular framework underlying apomictic phenomena, so far it still remains a challenge to control separately for the influences of either effect, both of which can have diverse regulatory consequences.

Engineering apomixis to a controllable, more reproducible trait would provide many advantages in plant improvement and cultivar development. Apomixis would provide for true-breeding, seed propagated hybrids. Harnessing apomixis would, thus, greatly facilitate and accelerate the ability of plant breeders to fix and faithfully propagate genetic heterozygosity and associated hybrid vigour in crop plants. Moreover, apomixis could shorten and simplify conventional breeding processes so that selfing and progeny testing to produce or stabilize a desirable gene combination could be eliminated.

The controlled use of apomixis would therefore certainly simplify commercial hybrid seed production. In particular, the need for physical isolation of commercial hybrid production fields would be eliminated, available land could be used to grow hybrid seed instead of dividing space between pollinators and male sterile lines and finally the need to maintain parental line seed stocks would be eliminated.

Apomixis would provide for the use as cultivars of genotypes with unique gene combinations since apomictic genotypes breed true irrespective of heterozygosity. Genes or groups of genes could thus be fixed in super genotypes. Every superior apomictic genotype from a sexual-apomictic cross would have the potential to be a cultivar. Apomixis would therefore allow plant breeders to develop cultivars with specific stable traits for such characters as height, seed and forage quality and maturity.

Thus, the application of apomixis in agriculture is considered an important enabling technology that would greatly facilitate the fixation and faithful propagation of genetic heterozygosity and associated hybrid vigor in crop plants (Spillane, 2004, Nat Biotech 22(6), 687-691).

All these potential benefits which rely on the production of seed via apomixis are presently, however, unrealized, to a large extent because of the problem of engineering apomictic capacity into plants of interest.

US 2002/0069433 A1 discloses methods for increasing the probability of vegetative reproduction of a new plant generation wherein a gene which encodes a protein acting in the signal transduction cascade triggered by the somatic embryogenesis receptor kinase is transgenically expressed. US 2008/0155712 A1 discloses processes for identifying in a plant, in particular maize, sequences responsible for apomictic development, in particular by genome mapping. WO 99/35258 A1 discloses nucleic acid markers for an apospory specific genomic region from the genus Pennisetum. US 7,541,514 B2 discloses methods for producing apomictic plants from sexual plants by selecting, collecting and breeding specific plant lines.

None of said disclosures provide means, in particular particular polynucleotides, which can easily be used in gene transfer methods to obtain in a controllable and inexpensive way apomixis in plants.

The technical problem underlying the present invention is therefore to provide means and methods to overcome the above-identified problems, in particular to provide means and methods to introduce apomixis into a plant for instance by means of recombinant gene technology, in particular by means of recombinant DNA transfer technology, in particular to provide means and methods to induce apomixis in plants, in particular in a controllable, foreseeable, reliable, easy and cost-effective way.

The present invention solves its underlying problem by the provision of the teaching of the independent claims, in particular by the provision of nucleic acid molecules, in particular isolated nucleic acid molecules, useful for inducing apomixis in plants, plant cells and plant parts containing said sequence as well as methods to induce apomixis in plants, methods to produce apomictic plants and uses thereof. In particular, the present invention solves its underlying technical problem by the provision of the following:
- A method for inducing apomixis in a transgenic plant, wherein an exogenous nucleotide sequence encoding a protein capable of inducing apomixis in a plant is introduced into the plant and expressed in the ovule of said plant, and wherein said nucleotide sequence comprises a polynucleotide, which codes for a protein with DEDD 3' to 5' exonuclease activity, which polynucleotide is selected from the group consisting of
   a) the polynucleotide defined in any one of SEQ ID No. 22 to 25, 27 to 30, 32 to 35, 37, 38, 40, 41, 43, 44, 46, 47, 49, 50, 52, or 53 or a fully complementary strand thereof,
   b) a polynucleotide encoding a polypeptide with the amino acid sequence defined in any one of SEQ ID No. 4 to 9, 13 to 15, 19 to 21 or a fully complementary strand thereof, and
   c) a polynucleotide variant having a degree of sequence identity of more than 70% to the nucleic acid sequence defined in a) or b), or a fully complementary strand thereof;
- A method for the production of an apomictic plant, wherein a plant cell is transformed with a nucleic acid molecule capable of inducing the expression of a nucleotide sequence encoding a protein capable of inducing apomixis in a plant and regenerating the transformed plant cell into a transformed plant that contains the transformed nucleic acid molecule so as to express a protein with DEDD 3' to 5' exonuclease activity in a plant ovule and to induce apomixis in the plant, wherein the nucleotide sequence encoding the protein capable of inducing apomixis in the plant is a polynucleotide, which codes for a protein with DEDD 3' to 5' exonuclease activity, which polynucleotide is selected from the group consisting of
   a) the polynucleotide defined in any one of SEQ ID No. 22 to 25, 27 to 30, 32 to 35, 37, 38, 40, 41, 43, 44, 46, 47, 49, 50, 52, or 53 or a fully complementary strand thereof,
   b) a polynucleotide encoding a polypeptide with the amino acid sequence defined in any one of SEQ ID No. 4 to 9, 13 to 15 or 19 to 21 or a fully complementary strand thereof, and
   c) a polynucleotide variant having a degree of sequence identity of more than 70% to the nucleic acid sequence defined in a) or b), or a fully complementary strand thereof.
- A method for isolating an apomixis inducing nucleic acid molecule from a plant, wherein an isolated nucleic acid molecule comprising a polynucleotide which codes for a protein with DEDD 3' to 5' exonuclease activity, which polynucleotide is selected from the group consisting of
   a) the polynucleotide defined in any one of SEQ ID No. 22 to 54 or a fully complementary strand thereof,
   b) a polynucleotide encoding a polypeptide with the amino acid sequence defined in any one of SEQ ID No. 1 to 21 or a fully complementary strand thereof, and
   c) a polynucleotide variant having a degree of sequence identity of more than 70% to the nucleic acid sequence defined in a) or b), or a fully complementary strand thereof, or the isolated nucleic acid molecule of claim 3, or the vector of claim 5, is used to screen and isolate nucleic acid sequences derived from the plant;
- A transgenic plant, plant cell or plant material comprising a cell according to claim 6, that is produced according to a method according to any one of claims 9 or 10, or progeny thereof that contain the vector according to claim 5 or that contain the polynucleotide which codes for a protein with DEDD 3' to 5' exonuclease activity according to any one of claims 1, 2, 4 or 10, wherein the polynucleotide is arranged in an order or orientation, or in a genomic position, that is not normally found in the plant, plant cell, or plant material to which it is transferred and wherein the protein with DEDD 3' to 5' exonuclease activity is expressed in a plant ovule;
- The use of an isolated nucleic acid molecule in inducing apomixis in a transgenic plant, wherein an exogenous nucleotide sequence encoding a protein capable of inducing apomixis in a plant is introduced into the plant and expressed in the ovule of said plant, and wherein said nucleotide sequence comprises a polynucleotide coding for a protein with DEDD 3' to 5' exonuclease activity which is selected from the group consisting of a) the polynucleotide defined in any one of SEQ ID No. 22, 23, 24, 25, 27, 28, 29, 30, 32, 33, 34, 35, 37, 38, 40, 41, 43, 44, 46, 47, 49, 50, 52, 53, or a fully complementary strand thereof, b) a polynucleotide encoding a polypeptide with the amino acid sequence defined in any one of SEQ ID No. 4, 5, 6, 7, 8, 9, 13, 14, 15, 19, 20, 21, or a fully complementary strand thereof, and c) a polynucleotide variant having a degree of sequence identity of more than 90 % to the nucleic acid sequence defined in a) or b), or a fully complementary strand thereof, wherein the protein with DEDD 3' to 5' exonuclease activity is expressed in a plant ovule. The sequence identity is preferably based on the entire sequence. Preferably, the sequence identity is determined by BLAST analysis, preferably in the NCBI database, in particular by GAP analysis using Gap Weight of 50 and Length Weight of 3.

An isolated nucleic acid molecule is disclosed herein which comprises a polynucleotide coding for a protein capable of inducing apomixis in a plant, preferably in a plant ovule, preferably exhibiting an exonuclease activity in a plant ovule, which is selected from the group consisting of a1) the polynucleotide defined in any one of SEQ ID No. 22 to 62, in particular 23, 25, 27, 28, 29, 30, 33, 35, 37, 38, 40, 41, 43, 44, 47, 50 or 53, or a fully complementary strand thereof, b1) a polynucleotide encoding a polypeptide with the amino acid sequence defined in any one of SEQ ID No. 1 to 21, preferably SEQ ID No. 4 to 9, SEQ ID No. 13 to 15 or SEQ ID No. 19 to 21, or a fully complementary strand thereof, and c1) a polynucleotide variant having a degree of sequence identity of more than 30%, 40%, 50% or, preferably 70 % to the nucleic acid sequence defined in a1) or b1), or a fully complementary strand thereof, preferably wherein the sequence identity is based on the entire sequence. Preferably, the sequence identity is determined by BLAST analysis, preferably in the NCBI database, in particular by GAP analysis using Gap Weight of 50 and Length Weight of 3 or any other suitable analysis.

The disclosed nucleic acid molecules represent the so-called apollo gene, which means "Apomixis linked locus", or are essential and specific parts thereof. Said gene, in particular its coding sequence, codes for the apollo protein which upon expression in the plant ovule leads to the production of apomictic seed.

The above-identified protein-coding polynucleotide disclosed herein is in particular characterised by the presence of at least one specific duplicated marker sequence in an exon, namely the fifth exon, of said sequence and which represents a nucleotide stretch duplication. Preferably, said duplicated marker nucleotide sequence is given in SEQ ID No. 64 and its corresponding amino acid sequence in SEQ ID No. 63.

Accordingly, the method for inducing apomixis in a transgenic plant according to the present invention also relates to the use of an isolated nucleic acid molecule, which comprises a polynucleotide coding for a protein capable of inducing apomixis in a plant, preferably in a plant ovule, preferably exhibiting an exonuclease activity in a plant ovule, wherein the polynucleotide comprises a nucleic acid sequence selected from the group consisting of a2) the polynucleotide defined in any one of SEQ ID No. 22, 23, 27, 28, 32 or 33, preferably 23, 28 or 33, or a fully complementary strand thereof, b2) a polynucleotide encoding a polypeptide with the amino acid sequence defined in any one of SEQ ID No. 4, 5 or 6 or a fully complementary strand thereof, and c2) a polynucleotide variant having a degree of sequence identity of more than 70 % to the nucleic acid sequence defined in a2) or b2), or a fully complementary strand thereof. The sequence identity can be based on the entire sequence. Preferably, the sequence identity is determined by BLAST analysis, preferably in the NCBI database, in particular by GAP analysis using Gap Weight of 50 and Length Weight of 3 or any other suitable analysis.

Polynucleotides, in particular polynucleotides coding for a protein capable of inducing apomixis in a plant, namely the apollo protein, and polynucleotides capable of functioning as regulatory elements for said coding sequence, in isolated and purified form are disclosed herein. Furthermore, the teaching is disclosed that plants, in particular their genome, comprise endogenously nucleotide sequences, hereinafter also called "polynucleotide" or "polynucleotide sequence", coding said apollo protein capable of inducing apomixis and its regulatory elements, hereinafter also called "endogenously present polynucleotide coding a protein capable of inducing apomixis in a plant". Thus, both the coding and the regulatory sequences as specified for instance in SEQ ID No. 37, 40, 43, 46, 49 or 52 are usually endogenously present in various allelic states in their natural and original genome environment in a plant, particularly in Brassica-ceae, preferably Boechera, and are responsible for the development of a sexual or apomictic phenotype in the plant. According to the findings of the present invention in the naturally occurring sexually propagating plant, said nucleotide sequences in their sexual allelic state, such as in SEQ ID No. 46, 49 or 52, however, are in the ovule of said plant repressed, that means not expressed, thereby preventing apomixis. In contrast, said polynucleotide in its apomictic allelic state, such as in SEQ ID No. 37, 40 or 43 is induced, that means is expressed in the ovule of a plant propagating asexually, that means an apomictic plant.

In particular, in a plant ovule of a sexually propagating plant the endogenously present gene coding for the apollo protein with an apomixis-inducing capacity is suppressed or inactivated in said tissue and therefore needs to be activated in order to produce an apomictic plant. Both in sexually and apomictic plants the coding regions of the apollo gene in its apomictic and sexual allelic form, are functionally equivalent. Differences in their expression are due to their different regulatory elements preferably as specified in SEQ ID No. 57 to 62 and 65. In particular, apomictic regulatory elements, preferably those as identified in SEQ ID No. 55, 57, 58 and 59, are in particular characterised by the presence of a 20 base pair promoter insertion, in particular that of SEQ ID No. 65, which leads to an ovule expression, i.e. expression in the ovule, of a coding element linked to said regulatory element. A sexual regulatory element disclosed herein is in particular characterised by the absence of such a promoter insert of SEQ ID No. 65 and is represented in particular by a regulatory element as given in SEQ ID No. 56, 60, 61 or 62 and provides a somatic gene expression, but not an expression in the ovule, possibly due to being suppressed in said tissue.

In particular, the invention therefore provides the teaching to modify, in particular activate or induce, that means to get said sequences expressed in order to achieve a plant of a desired phenotype, in particular an apomictic phenotype. This is achieved by either transforming a plant with expressible coding sequences for the apollo protein as defined in the claims for its expression in the plant ovule, so as to provide the apomictic phenotype to said plant and its progeny. This aim of providing an apomictic plant is achieved by transforming a plant with any nucleotide sequence, in particular any DNA molecule, which structurally interferes with the repressed regulatory element of an endogenously present apollo gene. According to the invention, it is achieved by transforming a plant with a polynucleotide sequence, capable of expressing a protein capable of inducing apomixis in the plant as defined in the claims, thereby derepressing said apollo gene and allowing its expression in a plant ovule so as to produce an apomictic plant.

Thus, the present invention foresees to introduce an exogenous polynucleotide, in particular transgenic, coding sequence for the apollo protein into a plant as defined in the claims, so as to express said coding sequence in the plant ovule.

In the context of the present invention, the term "inducing the expression of a gene - or polynucleotide - coding for protein capable of inducing apomixis" therefore refers to the activation, hereinafter also termed derepression, of a regulatory element governing the expression of said coding sequence, that means refers to the activation of expression allowing the production of a functional apollo protein in the plant ovule.

Thus, the present invention provides advantageous means and methods as claimed to induce apomixis in a plant. The polynucleotides used according to the the present invention, which code for a protein capable of inducing apomixis, can be transformed in a plant cell so as to produce a plant which comprises said exogenously introduced polynucleotide, expresses said polynucleotide in a plant ovule and thereby produces an apomictic phenotype and apomictic plant. This is achieved by using the polynucleotides of the present invention, defined in any one of SEQ ID No. 22, 23, 25, 27, 28, 29, 30, 33, 35, 37, 38, 40, 41, 43, 44, 47, 50 or 53, in particular 23, 25, 28, 30, 33, 35, 38, 41, 44, 47, 50 or 53, coding for a protein capable of inducing apomixis in a plant ovule. Said protein is disclosed in any one of SEQ ID No. 4 to 21, and according to the invention it is SEQ ID No. 4 to 9, SEQ ID No. 13 to 15 or SEQ ID No. 19 to 21, under control of a constitutively expressing promoter or a promoter providing an ovule-specific expression in the ovule.

Thus, disclosed isolated nucleic acid molecules comprise polynucleotides, in particular polynucleotides as specifically disclosed herein or polynucleotide variants, for use in inducing apomixis, which code for a protein capable of inducing apomixis in a plant, in particular in a plant ovule, in particular code for a protein with a specific exonuclease activity capable of inducing apomixis, in particular apomeiosis, in a plant ovule, and wherein said specific polynucleotides or variants thereof can advantageously be used to be transferred into a plant, in particular plant cell, be stably integrated in its genome and can preferably be expressed, in particular and most preferably in a constitutive manner, in the ovule of the obtained transformed plant in order to produce a transgenic apomictic plant, in particular transgenic plant, which produces apomictic seed. It is disclosed to transfer a polynucleotide encoding a protein capable of inducing apomixis in a plant and being specified in any one of the consensus SEQ ID No. 1 to 9. According to the invention it is provided to transfer a polynucleotide encoding a protein capable of inducing apomixis in a plant and being specified in any one of SEQ ID No. 4 to 9, SEQ ID No. 13 to 15 or 19 to 21, into a plant so as to allow expression of said polynucleotide, preferably being under control of a constitutively or ovule-specific promoter, thereby producing the desired apollo protein in the ovule.

Polynucleotides are also disclosed which are capable of functioning as a regulatory element and which can be used to transform plant cells and whereby said polynucleotides capable of functioning as regulatory elements structurally modify the regulatory elements of the endogenously present genes which code for proteins capable of inducing apomixis so as to derepress, that means activate, the endogenously present regulatory elements of said genes thereby allowing the expression of the protein capable of inducing apomixis and producing plants with an apomictic phenotype. This particular approach is based on the findings of the present invention that the gene coding for the protein capable of inducing apomixis is present also in wild type plants, but is, however, not activated, that means is not induced and therefore is not expressed in the ovule of a sexually propagating plant. Without being bound by theory, in wild type sexually propagating plants the expression of the endogenously present gene coding for a protein capable of inducing apomixis is suppressed or inactivated, most likely due to suppressed regulatory elements of the protein-coding regions. Thus, it is disclosed the introduction of regulatory elements which structurally interfere with the endogenously present and suppressed regulatory elements of a nucleotide sequence region coding for a protein capable of inducing apomixis in a plant ovule allows the reversion of the suppression of the regulatory elements and induces the expression of the coding sequence.

Accordingly, a polynucleotide is disclosed, in particular a specifically disclosed polynucleotide or polynucleotide variant, in particular a regulatory element as specified in any one of SEQ ID No. 55 to 62 or 65, that is transformed into a plant so as to modify the endogenously present regulatory element having a sequence as given in the sexual promoter given in any one of SEQ ID No. 56, 60, 61 or 62 of an endogenously present gene encoding the apollo protein capable of inducing apomixis in a plant so as to enable the expression of the endogenously present polynucleotide encoding the polypeptide capable of inducing apomixis in the plant, in particular the ovule.

Accordingly, the present invention provides isolated nucleic acid molecules are disclosed, which comprise polynucleotides, that means the polynucleotides specifically disclosed herein or polynucleotide variants, for use in inducing apomixis, wherein the specific polynucleotides or polynucleotide variants are regulatory elements and are useful for inducing apomixis in a plant in so far as they allow a regulatable expression of coding sequences operably linked thereto in the plant ovule, in particular during ovule development in a plant. Thus, these regulatory elements provide an ovule non-suppressability to a coding sequence and provide the advantage of being capable to direct expression of coding sequences in the ovule of plants.

Thus, an induced mutation is disclosed, for instance a recombination, duplication, deletion, insertion or inversion, of all or part of the endogenously present regulatory element for the coding sequence of the polypeptide capable of inducing apomixis in a plant ovule that allows the expression of said polynucleotide consequently leading to apomixis in the plant.

It is disclosed herein the induction of apomixis in a plant by modifying, in particular inducing, hereinafter also called activating, the expression of the endogenously present regulatory elements of the endogenously present nucleotide sequence encoding a protein capable of inducing apomixis in a plant by structurally modifying said endogenously present regulatory elements for instance by mutating, in particular by insertion, deletion, duplication or inversion of said regulatory element. Said structural modification may preferably be achieved by any means for mutation, for instance radiation, use of chemical agents or of nucleotide sequences, in particular a DNA molecule, introduced into a plant cell, which means, in particular sequence, is capable of structurally interfering with said regulatory element and which sequence may be a transposon or any other sequence being able to interfere, for instance recombine or insert into said regulatory element in the ovule of a sexually propagating plant.

Herein disclosed are specific polynucleotides and polynucleotide variants which are capable of acting as regulatory elements, in particular promoters, which very specifically act in a regulatory manner in the ovule. In particular such a regulatory element, hereinafter also called sexual promoter, is capable of being expressed in all somatic tissue of a transformed transgenic plant, but specifically not in the ovule of said plant. In another aspect of the disclosure such a regulatory element, hereinafter also called apo-promoter, said regulatory element is expressed in the somatic tissue of a transformed transgenic plant and is also expressed in the ovule of said plant. Thus, polynucleotides are provided which allow a somatic gene expression excluding the ovule tissue, or allow an ovule gene expression. Namely the ovule expressing embodiment, being specified in any one of SEQ ID No. 55, 57, 58 or 59 is primarily characterised by a nucleotide sequence comprising a regulatory insert of twenty nucleotides with SEQ ID No. 65 in comparison to the firstly mentioned embodiment, namely the non-ovule expressing embodiment, lacking said insert and being specified in SEQ ID No. 56, 60, 61 or 62. Thus, the regulatory element allowing expression in somatic tissue, but not in the ovule, that means the sexual promoter, is characterised by any one of SEQ ID No. 56, 60, 61 or 62. Furthermore the regulatory element capable of being expressed in the ovule, in particular by being not suppressible or not suppressed, that means the apo-promoter, is characterised by SEQ ID No. 55, 57, 58 or 59.

Thus, disclosed herein is an isolated nucleic acid molecule, which comprises a polynucleotide, which polynucleotide is able to act as a regulatory element and is selected from the group consisting of a3) the polynucleotide defined in any one of SEQ ID No. 55 to 62 or 65, or a fully complementary strand thereof and b3) a polynucleotide variant having a degree of sequence identity of more than 30%, 40%, 50%, 60%, preferably 70 % to the nucleic acid sequence defined in a3), or a fully complementary strand thereof, preferably wherein the sequence identity is based on the entire sequence. Preferably, the sequence identity is determined by BLAST analysis, preferably in the NCBI database, in particular by GAP analysis using Gap Weight of 50 and Length Weight of 3 or any other suitable analysis.

Thus, it is disclosed the vegetative production of seed identical to the parent. In particular, the present nucleotide acid molecules can be transformed into a desired plant, for instance high yielding hybrids, in order to change their reproductive mode into apomictic seed production. Thus, high yielding hybrids can according to the present invention be used in seed production to multiply identical copies of said high yielding hybrid seed which would greatly reduce the cost for the seed production and in turn increases the number of genotypes which could commercially be offered. Further on, genes can be evaluated directly in commercial hybrids, since the progeny would not segregate saving the cumbersome backcrossing procedures. Apomixis can be used to stabilise desirable phenotypes even with complex traits such as hybrid vigor. Such traits can be maintained very easily and be multiplied via apomixis indefinitive. Further, it is disclosed the possibility to combine it with male sterility, advantageously preventing genetically engineered stabilised traits from being hybridised with undesired relatives.

The present invention provides a solution as defined in the claims to the above-identified technical problem by providing specific isolated nucleic acid molecules which can be used for inducing apomixis in a plant, in particular in a plant ovule, preferably for inducing apomeiosis and/or parthenogenesis in a plant, preferably in a plant ovule.

These nucleic acid molecules used in the present invention comprise in one preferred embodiment specific polynucleotides characterised by their ability to induce apomixis in a plant and by the presence of specific consensus nucleotide sequence patterns according to any one of SEQ ID No. 27, 28, 29, 30, in particular 27, 28, 29, 30, preferably 27 or 29, which represent nucleotide patterns present in all specifically disclosed apomixis-inducing alleles for use in the present invention.

In a further preferred embodiment the specific polynucleotides are the various apomixis-inducing alleles, which are specifically identified, isolated and characterised according to the present invention and are characterised in any one of SEQ ID No. 37, 38, 40, 41, 43, 44.

Disclosed herein are polynucleotides and polypeptides in specific and in consensus forms. The consensus forms are generalised sequence motifs, that means patterns, being in one embodiment found in all of the polymorphic apollo genes identified and isolated according to the present invention, in particular are common to the coding sequence of all the different polymorphic forms including the apomictic and sexual forms. The consensus sequences are also given as generalised sequence motifs solely found in the apomictic polymorphic alleles or, are solely found in the sexual polymorphic allelic forms isolated according to the present invention. The apomictic and sexual alleles can be classified by different consensus sequences for their regulatory elements and share the same consensus sequence for their coding regions. In the consensus sequence "Xaa" stands for any naturally occurring amino acid and "n" for any one of the nucleotides a, t, g or c.

The specific polynucleotides and polypeptides used in the present invention are specifically isolated and analysed and display the consensus sequence pattern in exemplified form.

In a particularly preferred embodiment the present invention therefore relates to the use of consensus and specific polynucleotides and polypeptides characterised in the following tables I and II.

**Table I**

| Apollo-amino acid sequences (polypeptides) | | | | |
|---|---|---|---|---|
| **SEQ ID No.** | **type** | **subtype** | **characterisation** | **coded by SEQ ID No.** |
| 4 | consensus | Global | protein with duplication | 22, 23 |
| 5 | consensus | Apo | protein with duplication | 27, 28 |
| 6 | consensus | Sex | protein with duplication | 32, 33 |
| 7 | consensus | Global | protein without duplication | 24, 25 |
| 8 | consensus | Apo | protein without duplication | 29, 30 |
| 9 | consensus | Sex | protein without duplication | 34, 35 |
| 13 | specific | Apo | A011a Protein | 37, 38 |
| 14 | specific | Apo | A043a Protein | 40, 41 |
| 15 | specific | Apo | A081a Protein | 43, 44 |
| 19 | specific | Sex | S011a Protein | 46, 47 |
| 20 | specific | Sex | S355a Protein | 49, 50 |
| 21 | specific | Sex | S390a Protein | 52, 53 |

| | | | | |
|---|---|---|---|---|
| legend: A011a, A043a, A081a: apomictic Boechera holboellii alleles; S011a, S355a, S390a: sexual Boechera holboellii alleles "consensus" means consensus sequence, that means a general sequence motif present in more than one specific allele of the apollo gene with specifically identified positions for observed sequence deviations, namely nucleotide/amino acid polymorphisms. In amino acid sequences "Xaa" can be any naturally occurring amino acid. In nucleotide sequences "n" can be any of a, g, t or c, in introns "n" can additionally designate a missing nucleotide. "specific" means a specifically isolated polymorphic allele with sequenced or deduced nucleotide and amino acid sequence. "Global" means a consensus sequence both for apomictic and sexual apollo gene or protein. "Apo" means apomictic apollo gene or protein. "Sex" means sexual apollo gene or protein. "protein" means apollo protein. "Exonuclease domain" means the fragment of the apollo protein in which the specific biologically active DEDDh 3'-5' exonuclease activity is located. "duplication" means a duplicated marker sequence optionally present in the coding region of the apomictic and sexual allele of the apollo gene and specified in SEQ ID No. 63 (amino acid) and 64 (nucleotide). | | | | |

**Table II**

| Apollo-protein coding polynucleotides | | | |
|---|---|---|---|
| **SEQ ID No.** | **type** | **subtype** | **characterisation** |
| 22 | consensus | Global | genomic with duplication |
| 23 | consensus | Global | coding with duplication |
| 24 | consensus | Global | genomic without duplication |
| 25 | consensus | Global | coding without duplication |
| 27 | consensus | Apo | genomic with duplication |
| 28 | consensus | Apo | coding with duplication |
| 29 | consensus | Apo | genomic without duplication |
| 30 | consensus | Apo | coding without duplication |
| 32 | consensus | Sex | genomic with duplication |
| 33 | consensus | Sex | coding with duplication |
| 34 | consensus | Sex | genomic without duplication |
| 35 | consensus | Sex | coding without duplication |
| 37 | specific | Apo | A011a genomic |
| 38 | specific | Apo | A011a coding |
| 40 | specific | Apo | A043a genomic |
| 41 | specific | Apo | A043a coding |
| 43 | specific | Apo | A081a genomic |
| 44 | specific | Apo | A081a coding |
| 46 | specific | Sex | S011a genomic |
| 47 | specific | Sex | S011a coding |
| 49 | specific | Sex | S355a genomic |
| 50 | specific | Sex | S355a coding |
| 52 | specific | Sex | S390a genomic |
| 53 | specific | Sex | S390a coding |

| | | | |
|---|---|---|---|
| legend: see table I; "genomic" means genomic DNA sequence, preferably including regulatory elements, exons and introns. "coding" means solely the coding DNA sequence which codes the full length apollo protein. legend: see table I; "promoter insert": regulatory insertion of 20 bp found in apo-promoters | | | |

The present invention provides in one embodiment the use in the claimed methods and means of global consensus genomic sequences, in particular those of SEQ ID No. 22 and 24 which represent nucleotide sequence patterns found in the apomictic and sexual alleles of the present invention in so far as the nucleotide sequences given are to be found in both types of alleles.

Thus, the present invention polynucleotides coding for the apollo protein are provided which are characterised by any one of the polynucleotide sequences given in SEQ ID No. 22, 23, 24, 25, 27, 28, 29, 30, 32, 33, 34, 35, 37, 38, 40, 41, 43, 44, 46, 47, 49, 50, 52, 53 which are consensus and specific sequences found in apomictic and sexual alleles and which code for the consensus or specific apollo protein of any one of SEQ ID No. 4 to 9, 13 to 15 or 19 to 21. The polynucleotides are identified in Table I coding for the consensus apollo proteins or essential parts thereof, namely any one of SEQ ID No. 4, 5, 6, 7, 8, 9, 13, 14, 15, 19, 20 or 21. Other disclosed polynucleotides comprise any one of SEQ ID No. 23 or 25, 26, 27, 28, 29, 30, 31, 33, 35, 36, 37, 38, 39, 40, 41, 42, 43, 44, 45, 47, 48, 50, 51, 53 or 54 all of them comprising coding sequences for the apollo protein, but no sexual-specific regulatory elements being suppressible in a plant ovule. Thus, these sequences do not comprise the sexual promoters with SEQ ID No. 56 or any one of 60, 61 and 62, which are in particular lacking the promoter insert of SEQ ID No. 65.

Herein disclosed are the polynucleotide sequences comprising sexual regulatory elements such as the polynucleotides of SEQ ID No. 32, 34, 46, 49, 52, 56, 60, 61 or 62 comprising regulatory elements useful for providing suppressibility in plant ovule expression or for mutating endogenously present apollo genes so as to induce apomixis. These polynucleotides can be modified, in particular to contain the apomictic promoter insert of SEQ ID No. 65 thereby resulting in a regulatory element being expressed in the ovule thereby not being suppressed anymore in the ovule of a plant.

Functionally equivalent polynucleotides are disclosed for use in inducing apomixis in a plant, in particular in a plant ovule, preferably for inducing apomeiosis and/or parthenogenesis in a plant, preferably in a plant ovule, which do not exactly show the specific nucleotide sequence of said specific nucleotide sequence patterns or apomixis-inducing alleles and in particular given in the sequence identity protocols given herein, but which do exhibit slight deviations therefrom and which are termed "polynucleotide variants". Such polynucleotide variants are allelic, polymorphic, mutated, truncated or prolonged variants of the polynucleotides defined in the present sequence identity protocols and which therefore show deletions, insertions, inversions or additions of nucleotides in comparison to the polynucleotides defined in the present sequence identity protocol. Thus, polynucleotide or polypeptide variants, hereinafter also termed "functional equivalents" of a polynucleotide or polypeptide, have a structure and a sufficient length to provide the same biological activity, that means the same capability to induce apomixis in the plant as the specifically disclosed polynucleotides or polypeptides.

A polypeptide coded by a polynucleotide variant is - in case its amino acid sequence is altered in comparison to the amino acid sequence of the polypeptide coded by the polynucleotide of the present invention - termed a polypeptide variant. However, due to the degeneracy of the genetic code a polynucleotide variant not necessarily codes in any case for a polypeptide variant but may also code a polypeptide as used in the present invention.

The term "variant" refers to a substantially similar sequence of the specifically disclosed polynucleotides or polypeptides for use in the present invention. Polynucleotide variants for use in the invention will have at least 70%, 90%, in particular those representing the present apomixis-inducing alleles, in particular its coding sequence, wherein the % sequence identity is based on the entire sequence. Preferably, the sequence identity is determined by BLAST analysis, preferably in the NCBI database, in particular by GAP analysis using Gap Weight of 50 and Length Weight of 3 or any other suitable analysis.

Disclosed polypeptide sequence variants will have at least about 30%, 40%, 50%, 55%, 60%, 65%, 70%, 75% or 80%, preferably at least about 85% or 90%, and more preferably at least about 91%, preferably at least 92%, preferably at least 93%, preferably at least 94%, preferably at least 95%, preferably at least 96%, preferably at least 97% and most preferably at least 98% or at least 99% sequence identity to the present protein capable of inducing apomixis, wherein the % sequence identity is based on the entire sequence. Preferably, the sequence identity is determined by BLAST analysis, preferably in the NCBI database, in particular by GAP analysis using Gap Weight of 12 and Length Weight of 4 or any other suitable analysis.

A number of amino acids of the present polypeptides can be replaced, inserted or deleted without altering a protein's function. The relationship between proteins is reflected by the degree of sequence identity between aligned amino acid sequences of individual proteins or aligned component sequences thereof.

For sequence alignments and the determination of sequence identities in the context of the present invention various programs and algorithms can be used, such as the Wilbur-Lipman (Wilbur WJ, Lipman DJ, (1983), Rapid similarity searches of nucleic acid and protein data banks. Proc Natl Acad Sci USA 80:726-730), the Lipman-Pearson (Lipman DJ, Pearson WR (1985),Rapid and sensitive protein similarity searches. Science 227:1435-1441), the Martinez-NW (Needleman-Wunsch) algorithms (Martinez H (1983), An efficient method for finding repeats in molecular sequences. Nucleic Acids Res 11:4629-4634; Needleman SB and Wunsch CD (1970), A general method applicable to the search for similarities in the amino acid sequences of two proteins. J Mol Biol 48:444-453) or a combination thereof. The Wilbur-Lipman method is preferably used with the default ones provided by the program (ktuple = 3; Gap Penalty = 3; window = 20). As the instructions of the program MegAlign describes, the Wilbur-Lipman method constructs tables of K-tuples to find regions of similarity between two DNA sequence pairs using the method of Wilbur and Lipman (1983). This method reads the sequences, builds case structures of the K-tuples, finds the diagonals and matches, and creates the finished alignment. The method of Martinez-NW uses two alignment methods in succession. An approach described by Martinez (Martinez H (1983), An efficient method for finding repeats in molecular sequences. Nucleic Acids Res 11:4629-4634) identifies regions of perfect match. The Needleman-Wunsch (Needleman SB and Wunsch CD (1970), A general method applicable to the search for similarities in the amino acid sequences of two proteins. J Mol Biol 48:444-453) method then optimizes the fit in between perfect matches. The conditions of the alignment were the default ones provided by the program (Minimum Match = 9; Gap Penalty = 1.10; Gap Length Penalty = 0.33). The program preferably used for calculating the algorithms can be MegAlign (DNASTAR La-sergene version 9 Core Suite (DNASTAR, Inc., 3801 Regent Street, Madison, WI 53705, USA).

Dynamic programming algorithms yield different kinds of alignments. Algorithms as proposed by Needleman and Wunsch and by Sellers align the entire length of two sequences providing a global alignment of the sequences. The Smith-Waterman algorithm yields local alignments. A local alignment aligns the pair of regions within the sequences that are most similar given the choice of scoring matrix and gap penalties. This allows a database search to focus on the most highly conserved regions of the sequences. It also allows similar domains within sequences to be identified. To speed up alignments using the Smith-Waterman algorithm both BLAST (Basic Local Alignment Search Tool) and FASTA place additional restrictions on the alignments.

Within the context of the present invention alignments can be performed using BLAST, a set of similarity search programs designed to explore all of the available sequence databases regardless of whether the query is protein or DNA. Version BLAST 2.2 (Gapped BLAST) of this search tool has been made publicly available (currently http://www.ncbi.nlm.nih.gov/BLAST or http://blast.ncbi.nlm.nih.gov/BLAST.cgi). It uses a heuristic algorithm which seeks local as opposed to global alignments and is therefore able to detect relationships among sequences which share only isolated regions. The scores assigned in a BLAST search have a well-defined statistical interpretation. Particularly useful within the scope of the present invention are the blastp program allowing for the introduction of gaps in the local sequence alignments and the PSI-BLAST program, both programs comparing an amino acid query sequence against a protein sequence database, as well as a blastp variant program allowing local alignment of two sequences only.

Sequence alignments, preferably using BLAST, can also take into account whether the substitution of one amino acid for another is likely to conserve the physical and chemical properties necessary to maintain the structure and function of a protein or is more likely to disrupt essential structural and functional features. For example non-conservative replacements may occur at a low frequency and conservative replacements may be made between amino acids within the following groups: (i) serine and threonine; (ii) glutamic acid and aspartic acid; (iii) arginine and lysine; (iv) asparagine and glutamine; (v) isoleucine, leucine, valine and methionine; (vi) phenylalanine, tyrosine and tryptophan (vii) alanine and glycine.

Such sequence similarity is quantified in terms of percentage of positive amino acids, as compared to the percentage of identical amino acids.

The polynucleotide or polypeptide variants used in the present invention, however, are in spite of their structural deviations also capable of exhibiting the same or essentially the same biological activity as the polynucleotides or polypeptides defined in the sequence identity protocols.

In the context of the present invention the term "biological activity" refers to the capability of the polynucleotide or polypeptide of the present invention or their variants to induce apomixis in a plant. The term "to induce apomixis in a plant" refers to the capability of a polynucleotide or polypeptide or variant thereof to induce an asexual production of viable seed in a plant, in particular in the ovule of a plant, in particular the capability to induce apomeiosis or parthenogenesis or both apomeiosis and parthenogenesis in a plant ovule, in particular by coding or exerting an exonuclease activity in the ovule.

It is disclosed a polynucleotide able to induce apomixis in a plant ovule by activating or derepressing, in particular by structurally changing, a regulatory element of an endogenously present gene coding for a protein with an ovule exonuclease activity, preferably ovule-specific exonuclease activity, capable of inducing apomixis in a plant. Such a gene is in particular characterised by having a polynucleotide sequence allowing, upon derepression, that means induction, the expression of said endogenously coded protein with an ovule exonuclease activity, preferably an ovule-specific exonuclease activity, capable of inducing apomixis in the plant.

In a particularly preferred embodiment the biological activity exerted by a polypeptide used in the present invention, that means a protein capable of inducing apomixis in a plant, is a specific exonuclease activity characterised by expression at least in the ovule, preferably by an ovule specificity, in so far as its expression is activated in the ovule, preferably specifically in the ovule, of an apomictic plant and repressed or inactivated in a sexual plant.

The present protein, namely the apollo protein, which is capable of inducing apomixis in a plant, in particular a plant ovule and having a specific exonuclease activity appears to be, without being bound by theory, a DEDD 3'→ 5' exonuclease, also termed a DNA Q protein, which preferably is characterised by four acidic residues, namely three aspartats (D) and glutamate (E) distributed in three separate sequence segments, namely exo I, exo II and exo III (Moser et al., Nucl. Acids. Res 25 (1997), 5110-5118). Furthermore, these proteins are characterised by either a tyrosine (y) or histidine (h) amino acid located at its active side determinative for being a DEDDy or DEDDh protein. The present polypeptide capable of inducing apomixis in a plant ovule may be a DEDDh exonuclease, preferably comprising the amino acid sequence as given in any one of SEQ ID No. 1 to 3, 10 to 12 or 16 to 18, preferably catalysing the excision of nucleoside monophosphates at the DNA or RNA termini in the 3'-5' direction. It is disclosed the exonuclease is a plant DEDDh exonuclease.

Herein disclosed is the specific biological activity performed by the polypeptide capable of inducing apomixis in the plant ovule in said plant ovule, that means the apollo protein, that appears to be a meiosis-modifying, in particular meiosis-altering, changing or varying activity, in particular is a meiosis-inhibiting activity thereby preventing the reduction of chromosome number in the germ cells.

The isolated nucleic acid molecules used in the present invention may be present in isolated form. The isolated nucleic acid molecules may, however, also be combined with other nucleic acid molecules, for instance regulatory elements or vectors, thereby forming another molecule comprising not solely the nucleic acid molecule used in the present invention. In this case the "nucleic acid molecule "is also termed a "nucleic acid sequence".

In the context of the present invention the term "comprising" is understood to have the meaning of "including" or "containing" which means that one first entity contains a second entity, wherein said first entity may in addition to the second entity further contain a third entity. Thus, in particular, the term "a nucleic acid molecule comprising a polynucleotide" means that the nucleic acid molecule used in the present invention contains a polynucleotide or a polynucleotide variant, but may in addition contain other nucleotides or polynucleotides. In a particular preferred embodiment the term "comprising" as used herein is also understood to mean "consisting of" thereby excluding the presence of other elements besides the explicitly mentioned element. Thus, the present invention also relates to the use of nucleic acid molecules which consist of polynucleotides or polynucleotide variants as defined in the claims, meaning that the nucleic acid molecule is only composed of the polynucleotide or polynucleotide variant as defined in the claims and does not comprise any further nucleotides, polynucleotides or other elements. According to this embodiment, the nucleic acid molecule used in the present invention is the polynucleotide or polynucleotide variant herein defined.

Both, the nucleic acid molecule used in the present invention and the polynucleotide comprised therein do exhibit the desired biological activity of being capable of inducing apomixis.

The term "apomixis" refers to the replacement of the normal sexual reproduction by asexual reproduction, that means preferably reproduction without fertilisation of the egg cell, in particular that means only fertilisation of the central cell which is a pseudogamous event, in particular without any fertilisation, in particular the term refers to asexual reproduction through seeds, leading to apomictically produced offsprings or progeny genetically identical to the parent plant, in particular the female plant.

The term "gene" refers to a coding nucleotide sequence and associated regulatory nucleotide sequences. The coding sequence is transcribed into RNA, which depending on the specific gene, will be mRNA, rRNA, tRNA, snRNA, sense RNA or antisense RNA. Examples of regulatory sequences, hereinafter also termed regulatory elements, are promoter sequences, 5' and 3' untranslated sequences and termination sequences. Further elements that may be present are, for example, introns or enhancers. A structural gene may constitute an uninterrupted coding region or it may include one or more introns bounded by appropriate splice junctions. The structural gene may be a composite of segments derived from different sources, naturally occurring or synthetic.

The gene to be expressed may be modified in that known mRNA instability motifs or polyadenylation signals are removed or codons which are preferred by the plant into which the sequence is to be inserted may be used.

Disclosed herein are the present nucleic acid molecules, in particular a polynucleotide or polynucleotide variant, in particular a DNA sequence, wherein said nucleic acid molecule or sequence encodes a polypeptide capable of inducing apomixis, in particular in a plant, preferably plant ovule, and having, preferably comprising, the amino acid sequence depicted in SEQ ID No. 1, 2, 3, 10, 11, 12, 16, 17 or 18, or a polypeptide variant thereof, that means a functional equivalent of a polypeptide, preferably a polypeptide being in terms of biological activity similar thereto. The disclosure also provides a polypeptide variant, in particular having a length of at least 150, at least 200, at least 250, at least 300, at least 350, at least 400, at least 450, at least 500 amino acids which after alignment reveals at least 30% or 40% and preferably at least 50%, at least 60 %, at least 70 %, at least 80 %, at least 90 %, at least 95 %, at least 99 % or more sequence identity with the, preferably full-length, polypeptide, in particular as characterised in any one of SEQ ID No. 1 to 21, preferably 4, 5, 6, 7, 8, 9, 13, 14, 15, 19, 20 or 21.

The terms "protein" and "polypeptide" are used interchangeably and refer to a molecule with a particular amino acid sequence comprising at least 20, 30, 40, 50 or 60 amino acid residues.

The term "polypeptide" thus means proteins used in the present invention and variants thereof, in particular protein fragments, modified proteins, amino acid sequences and synthetic amino acid sequences. The polypeptide can be glycosylated or not.

A polypeptide variant which is truncated is also termed a "fragment". Thus, the term "fragment" refers to a portion of a polynucleotide sequence or a portion of a polypeptide, that means an amino acid sequence and hence polypeptide encoded thereby. Fragments of a polynucleotide sequence such as SEQ ID No. 26, 31, 36, 39, 42, 45, 48, 51 or 54, may encode polypeptide fragments that retain the biological activity of the polypeptide, such as given in any one of SEQ ID No. 1, 2, 3, 10, 11, 12, 16, 17 or 18. Alternatively, fragments of a polynucleotide sequence that are useful as hybridization probes generally do not encode fragments of a polypeptide retaining biological activity. Fragments of a polynucleotide sequence are generally greater than 20, 30, 50, 100, 150, 200 or 300 nucleotides and up to the entire nucleotide sequence encoding the polypeptide. Generally, the fragments have a length of less than 1000 nucleotides and preferably less than 500 nucleotides. Fragments include antisense sequences used to decrease expression of the present polynucleotides. Such antisense fragments may vary in length ranging from at least 20 nucleotides, 50 nucleotides, 100 nucleotides, up to and including the entire coding sequence.

The term "regulatory element" refers to a sequence, preferably a nucleotide sequence, located upstream (5'), within and/or downstream (3') to a nucleotide sequence, preferably a coding sequence, whose transcription and expression is controlled by the regulatory element, potentially in conjunction with the protein biosynthetic apparatus of the cell. "Regulation" or "regulate" refer to the modulation of the gene expression induced by DNA sequence elements located primarily, but not exclusively upstream (5') from the transcription start of the gene of interest. Regulation may result in an all or none response to a stimulation, or it may result in variations in the level of gene expression.

A regulatory element, in particular DNA sequence, such as a promoter is said to be "operably linked to" or "associated with" a DNA sequence that codes for a RNA or a protein, if the two sequences are situated and orientated such that the regulatory DNA sequence effects expression of the coding DNA sequence.

A "promoter" is a DNA sequence initiating transcription of an associated DNA sequence, in particular being located upstream (5') from the start of transcription and being involved in recognition and being of the RNA-polymerase. Depending on the specific promoter region it may also include elements that act as regulators of gene expression such as activators, enhancers, and/or repressors.

A "3' regulatory element" (or "3' end") refers to that portion of a gene comprising a DNA segment, excluding the 5' sequence which drives the initiation of transcription and the structural portion of the gene, that determines the correct termination site and contains a polyadenylation signal and any other regulatory signals capable of effecting messenger RNA (mRNA) processing or gene expression. The polyadenylation signal is usually characterised by effecting the addition of polyadenylic acid tracts to the 3' end of the mRNA precursor. Polyadenylation signals are often recognised by the presence of homology to the canonical form 5'-AATAAA-3'.

The term "coding sequence" refers to that portion of a gene encoding a protein, polypeptide, or a portion thereof, and excluding the regulatory sequences which drive the initiation or termination of transcription.

The gene, coding sequence or the regulatory element may be one normally found in the cell, in which case it is called "autologous", or it may be one not normally found in a cellular location, in which case it is termed "heterologous" or "transgenic".

A "heterologous" gene, coding sequence or regulatory element may also be autologous to the cell but is, however, arranged in an order and/or orientation or in a genomic position or environment not normally found or occurring in the cell in which it is transferred.

The term "vector" refers to a recombinant DNA construct which may be a plasmid, virus, autonomously replicating sequence, an artificial chromosome, such as the bacterial artificial chromosome BAC, phage or other nucleotide sequence, in which at least two nucleotide sequences, at least one of which is a nucleic acid molecule used in the present invention, have been joined or recombined. A vector may be linear or circular. A vector may be composed of a single or double stranded DNA or RNA. A vector may be derived from any source. Such a vector is preferably capable of introducing the regulatory element, for instance a promoter fragment, and the nucleic acid molecule used in the present invention, preferably a DNA sequence for inducing apomixis, in a plant, in sense or antisense orientation along with appropriate 3' untranslated sequence into a cell, in particular a plant cell.

The term "expression" refers to the transcription and/or translation of an endogenous gene or a transgene in plants.

"Marker genes" usually encode a selectable or screenable trait. Thus, expression of a "selectable marker gene" gives the cell a selective advantage which may be due to their ability to grow in the presence of a negative selective agent, such as an antibiotic or a herbicide compared to the growth of non-transformed cells. The selective advantage possessed by the transformed cells, compared to non-transformed cells, may also be due to their enhanced or novel capacity to utilize an added compound as a nutrient, growth factor or energy source. Selectable marker gene also refers to a gene or a combination of genes whose expression in a plant cell gives the cell both, a negative and a positive selective advantage. On the other hand a "screenable marker gene" does not confer a selective advantage to a transformed cell, but its expression makes the transformed cell phenotypically distinct from untransformed cells.

The term "expression in the vicinity of the embryo sac" refers to expression in carpel, integuments, ovule, ovule primordium, ovary wall, chalaza, nucellus, funicle or placenta. The term "integuments" refers to tissues which are derived therefrom, such as endothelium. The term "embryogenic" refers to the capability of cells to develop into an embryo under permissive conditions.

The term "plant" refers to any plant, but particularly seed plants.

The term "transgenic plant" or "transgenic plant cell" or "transgenic plant material" refers to a plant, plant cell or plant material which is characterised by the presence of a polynucleotide or polynucleotide variant of the present invention, which may - in case it is autologous to the plant - either be located at another place or in another orientation than usually found in the plant, plant cell or plant material or which is heterologous to the plant, plant cell or plant material. Preferably, the transgenic plant, plant cell or plant material expresses the polynucleotide or its variants such as to induce apomixis.

A transgenic plant, transgenic plant cell or transgenic plant material can be identified at the phenotypical level, for instance by observation of apomictic seed production, or at protein level, for instance by immunodetection or at the DNA or RNA level, for instance with polymerase chain reaction (PCR). Even in case the transgene in the transgenic plant, transgenic plant cell or transgenic plant material has a natural homologue therein with a very high similarity, PCR can be used to discriminate such a transgene by at least one nucleotide difference. In particular, SNP (single nucleotide polymorphism) existing between host alleles and transforming alleles can be used to detect transformed plants simply by PCR.

The term "plant cell" describes the structural and physiological unit of the plant, and comprises a protoplast and a cell wall. The plant cell may be in form of an isolated single cell, such as a stomatal guard cells or a cultured cell, or as a part of a higher organized unit such as, for example, a plant tissue, or a plant organ.

The term "plant material" includes plant parts, in particular plant cells, plant tissue, in particular plant propagation material, preferably leaves, stems, roots, emerged radicles, flowers or flower parts, petals, fruits, pollen, pollen tubes, anther filaments, ovules, embryo sacs, egg cells, ovaries, zygotes, embryos, zygotic embryos per se, somatic embryos, hypocotyl sections, apical meristems, vascular bundles, pericycles, seeds, roots, cuttings, cell or tissue cultures, or any other part or product of a plant.

Thus, the present invention also provides plant propagation material of the transgenic plants of the present invention. Said "plant propagation material" is understood to be any plant material that may be propagated sexually or asexually in vivo or in vitro. Particularly preferred within the scope of the present invention are protoplasts, cells, calli, tissues, organs, seeds, embryos, pollen, egg cells, zygotes, together with any other propagating material obtained from transgenic plants. Parts of plants, such as for example flowers, stems, fruits, leaves, roots originating in transgenic plants or their progeny previously transformed by means of the methods of the present invention and therefore consisting at least in part of transgenic cells, are also an object of the present invention. Especially preferred plant materials, in particular plant propagation materials, are apomictic seeds.

Particularly preferred plants are monocotyledonous or dicotyledonous plants. Particularly preferred are crop or agricultural plants, such as sunflower, peanut, corn, potato, sweet potato, bean, pea, chicory, lettuce, endive, cabbage, cauliflower, broccoli, turnip, radish, spinach, onion, garlic, eggplant, celery, carrot, squash, pumpkin, zucchini, cucumber, apple, pear, melon, strawberry, grape, raspberry, pineapple, soybean, Cannabis, Humulus (hop), tomato, sorghum, sugar cane, and non-fruit bearing trees such as poplar, rubber, Paulownia, pine, elm, Lolium, Festuca, Dactylis, alfalfa, safflower, tobacco, cassava, coffee, coconut, pineapple, citrus trees, cocoa, tea, banana, avocado, fig, guava, mango, olive, papaya, cashew, macada-mia, almond, green beans, lima beans, peas, fir, hemlock, spruce, redwood, in particular maize, wheat, barley, sorghum, rye, oats, turf and forage grasses, millet, rice and sugar cane. Especially preferred are maize, wheat, sorghum, rye, oats, turf grasses and rice.

Particularly preferred are also ornamental plants such as ornamental flowers and ornamental crops, for instance Begonia, Carnation, Chrysanthemum, Dahlia, Gardenia, Asparagus, Geranium, Daisy, Gladiolus, Petunia, Gypsophila, Lilium, Hyacinth, Orchid, Rose, Tulip, Aphelandra, Aspidistra, Aralia, Clivia, Coleus, Cordyline, Cyclamen, Dracaena, Dieffnbachia, Ficus, Philodendron, Poinsettia, Fern, Ivy, Hydrangea, Limonium, Monstera, Palm, Date-palm, Potho, Singonio, Violet, Daffodil, Lavender, Lily, Narcissus, Crocus, Iris, Peonies, Zephyranthes, Anthurium, Gloxinia, Azalea, Ageratum, Bamboo, Camellia, Dianthus, Impatien, Lobelia, Pelargonium, Lilac, Lily of the Valley, Stephanotis, Hydrangea, Sunflower, Gerber daisy, Oxalis, Marigold and Hibiscus.

Among the dicotyledonous plants Arabidopsis, Boechera, soybean, cotton, sugar beet, oilseed rape, tobacco, pepper, melon, lettuce, Brassica vegetables, in particular Brassica napus, sugar beet, oilseed rape and sunflower are more preferred herein.

"Transformation", "transforming" and "transferring" refers to methods to transfer nucleic acid molecules, in particular DNA, into cells including, but not limited to, biolistic approaches such as particle bombardment, microinjection, permeabilising the cell membrane with various physical, for instance electroporation, or chemical treatments, for instance polyethylene glycol or PEG, treatments; the fusion of protoplasts or Agrobacterium tumefaciens or rhizogenes mediated trans-formation. For the injection and electroporation of DNA in plant cells there are no specific requirements for the plasmids used. Plasmids such as pUC derivatives can be used. If whole plants are to be regenerated from such transformed cells, the use of a selectable marker is preferred. Depending upon the method for the introduction of desired genes into the plant cell, further DNA sequences may be necessary; if, for example, the Ti or Ri plasmid is used for the transformation of the plant cell, at least the right border, often, however, the right and left border of the Ti and Ri plasmid T-DNA have to be linked as flanking region to the genes to be introduced. Preferably, the transferred nucleic acid molecules are stably integrated in the genome or plastome of the recipient plant.

The expression "progeny" or "offspring" refers to both, "asexually" and "sexually" generated progeny of transgenic plants. This definition is also meant to include all mutants and variants obtainable by means of known processes, such as for example cell fusion or mutant selection and which still exhibit the characteristic properties of the initial transformed plant according to the present invention, together with all crossing and fusion products of the transformed plant material. This also includes progeny plants that result from a backcrossing, as long as the said progeny plants still contain the polynucleotide and/or polypeptide used according to the present invention.

The isolated nucleic acid molecule used in the present invention may be a DNA, preferably a DNA from a plant, preferably from Brassicaceae, in particular Boechera, in particular Boechera holboellii, Boechera divaricarpa or Boechera stricta, in a particular genomic or cDNA sequence molecule. It may, however, also be a RNA, in particular mRNA.

The present invention also provides in a preferred embodiment a vector comprising the nucleic acid sequence according to the claimed invention. Both, the specific polynucleotide or the polynucleotide variant as defined in the claims can be contained in the vector in sense or antisense orientation to a regulatory element.

The vector of the present invention comprises the nucleic acid sequence, in particular the specific polynucleotide or its variant coding the apomixis-inducing protein of the present invention, operably linked to at least one regulatory element, for instance a promoter, enhancer and/or polyadenylation signal.

Said promoter may be an inducible or constitutive promoter. The promoter may be a regulatable promoter. The promoter may also be an ovule-specific promoter, which is a promoter allowing the expression of an operably linked coding sequence in the plant ovule of a plant, but not in other plant tissues. The promoter may be the Ubiquitin-, ocs-, mas-, actin-, ADH-, NOS- or CaMV355-promoter. In order to obtain expression of the present nucleic acid molecule in a regenerated plant, in particular the ovule thereof, in a tissue specific manner the polynucleotide or polynucleotide variant is preferably under expression control a regulatory element, for instance of an inducible or developmentally regulated promoter.

Herein disclosed is a polynucleotide, in particular the specific polynucleotide or polynucleotide variant, coding for a protein with exonuclease activity is operably linked to a polynucleotide or polynucleotide variant of the present invention which is able to act as a regulatory element, in particular a promoter.

Herein disclosed is a vector comprises a polynucleotide, in particular the specific polynucleotide or polynucleotide variant of the present invention capable of acting as a regulatory element operably linked to a protein coding nucleic acid sequence desired to be expressed in a plant, in particular a plant ovule.

The present invention also provides in a preferred embodiment a host cell containing the vector of the present invention. Preferably, the host cell is not a human cell, preferably not a human stem cell, germinal cell or embryogenic cell.

The present invention also provides a transgenic plant, plant cell, plant material, in particular plant seed comprising at least one nucleic acid molecule used according to the present invention or the vector of the present invention. The present invention also provides in a preferred embodiment a cell culture, preferably a plant cell culture comprising a cell according to the present invention.

In a particularly preferred embodiment the present invention provides a transgenic plant, plant cell, plant material, in particular plant seed, wherein the polynucleotide, the polypeptide or the variant thereof exhibit its biological function. In the present invention a plant or plant seed is provided which comprises the polynucleotide, polypeptide or variants thereof as defined in the claims and which show due to the presence of said polynucleotide or polypeptide or variant thereof apomixis.

Also disclosed are proteins, in particular polypeptides or polypeptides variants, that means functional equivalents to polypeptides of the present invention, that means polypeptides capable of inducing apomixis in a plant or in vitro, which are coded by the nucleic acid molecules of the present invention.

Thus, the present invention the present proteins capable of inducing apomixis in a plant are apollo proteins, that means comprise an amino acid sequence as characterised by any one of SEQ ID No. 4, 5, 6, 7, 8, 9, 13, 14, 15, 19, 20 or 21, preferably 4, 5, 6.. Also disclosed are proteins capable of inducing apomixis in a plant have, preferably comprise, an amino acid sequence as set forth in any one of SEQ ID No. 10, 11, 12, 13, 14, 15, 16, 17, 18, 19, 20 or 21, preferably 13, 14, 15, 19, 20 or 21. Disclosed are also proteins comprising the amino acid sequence as given in SEQ ID No. 1, 2 or 3.

The present invention also provides a method for inducing apomixis in a plant, wherein the expression of a nucleotide sequence encoding a protein capable of inducing apomixis in the plant, in particular the apollo protein, in particular in the ovule of the plant, is induced in said ovule wherein said protein has the amino acid sequence as specified in any one of SEQ ID No. 4 to 9, 13 to 15 or 19 to 21, preferably 4, 5, 6, 7.

Thus, the present invention foresees a method according to which in a plant ovule the expression of the polynucleotides used in the present invention, in particular the presence of the protein of the present invention capable of inducing apomixis, is provided in order to induce apomixis and whereby said polynucleotides have been transformed in said plant in an expressible status, that means in a form capable of inducing apomixisAccording to the disclosure said polynucleotides are alternatively endogenously present and are activated, in particular their regulatory elements, by mutation, for instance by radiation, chemical agents or exogenously transformed polynucleotides. The present invention provides the teaching to induce the expression of polynucleotides used in the present invention in the plant ovule so as to allow the induction of apomixis in the plant ovule.

In a particularly preferred embodiment of the present invention the present invention therefore foresees to induce expression of polynucleotides encoding polypeptides capable of inducing apomixis in a plant ovule by transforming a plant with polynucleotides as defined in the claims being under appropriate regulatory control, in particular under control of a promoter, which polynucleotide codes a protein capable of inducing apomixis in a plant, so as to allow and induce expression of the transformed polynucleotide in the plant ovule thereby inducing apomixis in the plant.

The present invention also provides a method as defined in the claims for inducing apomixis in a plant by transforming a plant cell with the isolated nucleic acid molecule ad defined according to the present invention or the vector according to the present invention and regenerating the transformed plant cell into a transformed plant that contains, in particular contains and expresses, the at least one nucleic acid sequence so as to induce apomixis in the plant.

It is also disclosed a method for inducing apomixis in a plant, wherein the regulation of endogenously present polynucleotides having the same DNA sequence as the presently isolated polynucleotides of the present invention are induced to be expressed in a plant ovule. Thus, it is disclosed to induce the expression of endogenously present polynucleotides encoding proteins capable of inducing apomixis in a plant ovule, in particular by structurally altering the regulatory elements of said endogenously present polynucleotide sequence, in particular its promoter, so as to allow expression therefrom.

Disclosed herein is structurally altering of the regulatory elements of said endogenously present polynucleotide sequence coding a protein capable of inducing apomixis in the plant by transforming the plant with either any DNA sequence capable of structurally modifying the endogenously present regulatory elements of said polynucleotide capable of expressing a protein capable of inducing apomixis in a plant or by transforming the specific regulatory elements of the present invention so as to induce apomixis in the plant.

Thus, the present invention also relates to a method as claimed for the production of an apomictic plant by transforming a plant cell with the isolated nucleic acid molecule or the vector as defined in the claims and regenerating the transformed plant cell into a transformed plant that contains, in particular contains and expresses, the at least one nucleic acid sequence so as to induce apomixis in the plant.

Disclosed herein is a method of inducing vegetative reproduction via seeds in a plant generation comprising transforming a plant cell with the isolated nucleic acid molecule according to the present invention or the vector according to the present invention and regenerating the transformed plant cell into a transformed plant which contains, in particular contains and expresses, the at least one nucleic acid sequence so as to induce apomixis in the plant.

Disclosed herein is a method for inducing apomixis by inducing vegetative reproduction of a new or further plant generation, comprising transgenically expressing a nucleic acid molecule, in particular nucleic acid sequence of the present invention, in particular in a plant or plant cell.

According to the present invention the transgenic polynucleotide or polynucleotide variantas defined in the claims, is transgenically expressed in the ovule, in particular in the vicinity of the embryo sac.

The present invention also provides in a preferred embodiment a method as claimed for isolating an apomixis-inducing nucleic acid molecule from a plant wherein the isolated nucleic acid molecule used in the present invention is used to screen and isolate nucleic acid molecules derived from the plant. Thus, the present invention provides the teaching on the identity of a nucleic acid molecule for use in inducing apomixis in plants which allows the skilled person to design on the basis of said nucleic acid molecules one or more primer to identify similar sequence by PCR in a genome or a part thereof.

Disclosed herein is a method for identifying, in particular screening, for an effector of apomixis, in particular an apomictic phenotype, wherein a transgenic plant, plant cell or plant material according to the present invention is used, in particular cultivated, preferably cultivated and analysed.

Apomixis effectors can be detected by different technologies, preferably depending upon the initial information available, for instance by protein or immunodetection.

Thus, it is disclosed means and methods to identify and obtain further substances, in particular proteins or nucleic acid sequences, which are involved in the development of an apomictic phenotype, in particular which are associated, in particular relate to the development of an apomictic phenotype.

Disclosed herein are:
1: A method for inducing apomixis in a plant, wherein a nucleotide sequence encoding a protein capable of inducing apomixis in a plant is induced to be expressed in the ovule of said plant and wherein said nucleotide sequence comprises a polynucleotide, which codes for a protein with exonuclease activity, which polynucleotide is selected from the group consisting of
   xa) the polynucleotide defined in any one of SEQ ID No. 22 to 54 or a fully complementary strand thereof,
   xb) a polynucleotide encoding a polypeptide with the amino acid sequence defined in any one of SEQ ID No. 1 to 21 or a fully complementary strand thereof, and
   xc) a polynucleotide variant having a degree of sequence identity of more than 30%, 40%, 50% or preferably 70 % to the nucleic acid sequence defined in xa) or xb), or a fully complementary strand thereof.
2: The method of 1, wherein the polynucleotide is selected from the group consisting of
   xa1) the polynucleotide defined in any one of SEQ ID No. 26, 31, 36, 39, 42, 45, 48, 51, 54 or a fully complementary strand thereof,
   xb1) a polynucleotide encoding a polypeptide with the amino acid sequence defined in any one of SEQ ID No. 1, 2, 3, 10, 11, 12, 16, 17, 18 or a fully complementary strand thereof, and
   xc1) a polynucleotide variant having a degree of sequence identity of more than 30%, 40%, 50% or, preferably 70 % to the nucleic acid sequence defined in xa1) or xb1), or a fully complementary strand thereof.
3: The method of 1, wherein the polynucleotide is selected from the group consisting of
   xa2) the polynucleotide defined in any one of SEQ ID No. 22, 23, 27, 28, 32, 33 or a fully complementary strand thereof,
   xb2) a polynucleotide encoding a polypeptide with the amino acid sequence defined in any one of SEQ ID No. 4, 5, 6 or a fully complementary strand thereof, and
   xc2) a polynucleotide variant having a degree of sequence identity of more than 70 % to the nucleic acid sequence defined in xa2) or xb2), or a fully complementary strand thereof.
4: An isolated nucleic acid molecule for use in inducing apomixis in a plant, which comprises a polynucleotide which polynucleotide is able to act as a regulatory element and is selected from the group consisting of
   a3) the polynucleotide defined in any one of SEQ ID No. 55 to 62 or 65 or a fully complementary strand thereof and
   b3) a polynucleotide variant having a degree of sequence identity of more than 70 % to the nucleic acid sequence defined in a3), or a fully complementary strand thereof.
5: An isolated nucleic acid molecule for use in inducing apomixis in a plant, which comprises a polynucleotide coding for a protein with exonuclease activity, which polynucleotide is selected from the group consisting of
   xa4) the polynucleotide defined in any one of SEQ ID No. 26, 31, 36, 39, 42, 45, 48, 51, 54 or a fully complementary strand thereof,
   xb4) a polynucleotide encoding a polypeptide with the amino acid sequence defined in any one of SEQ ID No. 1, 2, 3, 10, 11, 12, 16, 17, 18 or a fully complementary strand thereof, and
   xc4) a polynucleotide variant having a degree of sequence identity of more than 98 % to the nucleic acid sequence defined in xa4) or xb4), or a fully complementary strand thereof.
6: An isolated nucleic acid molecule for use in inducing apomixis in a plant, which comprises a polynucleotide coding for a protein with exonuclease activity, which polynucleotide is selected from the group consisting of
   xa5) the polynucleotide defined in any one of SEQ ID No. 22, 23, 24, 25, 27, 28, 29, 30, 32, 33, 34, 35, 37, 38, 40, 41, 43, 44, 46, 47, 49, 50, 52, 53 or a fully complementary strand thereof,
   xb5) a polynucleotide encoding a polypeptide with the amino acid sequence defined in any one of SEQ ID No. 4, 5, 6, 7, 8, 9, 13, 14, 15, 19, 20, 21 or a fully complementary strand thereof, and
   xc5) a polynucleotide variant having a degree of sequence identity of more than 90 % to the nucleic acid sequence defined in xa5) or xb5), or a fully complementary strand thereof.
7: A vector comprising the nucleic acid molecule of any one of 4 to 6.
8: A host cell containing the vector of 7.
9: A protein encoded by a nucleotide acid sequence according to anyone of 5 or 6.
10: A transgenic plant, plant cell or plant material comprising at least one transgenic nucleic acid molecule of any one of embodiments 4 to 6 or the vector of 7.
11: A cell culture, preferably a plant cell culture comprising a cell according to 8.
12: The method for inducing apomixis in a plant according to any one of 1 to 3, wherein the expression is induced by transforming a plant cell with an isolated nucleic acid molecule comprising a polynucleotide which codes for a protein with exonuclease activity as defined in any one of 1 to 3, 5 or 6, with the isolated nucleic acid molecule of 4 or with the vector according to 7 and regenerating the transformed plant cell into a transformed plant that contains the transformed at least one nucleic acid sequence so as to induce apomixis in the plant.
13: A method for the production of an apomictic plant, wherein a plant cell is transformed with a nucleic acid molecule capable of inducing the expression of a nucleotide sequence encoding a protein capable of inducing apomixis in a plant and regenerating the transformed plant cell into a transformed plant that contains the transformed nucleic acid molecule so as to induce apomixis in the plant, wherein the nucleotide sequence encoding the protein capable of inducing apomixis in the plant is a polynucleotide, which codes for a protein with exonuclease activity, which polynucleotide is selected from the group consisting of
   xa) the polynucleotide defined in any one of SEQ ID No. 22 to 54 or a fully complementary strand thereof,
   xb) a polynucleotide encoding a polypeptide with the amino acid sequence defined in any one of SEQ ID No. 1 to 21 or a fully complementary strand thereof, and
   xc) a polynucleotide variant having a degree of sequence identity of more than 70 % to the nucleic acid sequence defined in xa) or xb), or a fully complementary strand thereof.
14: The method for the production of an apomictic plant by transforming according to 13, wherein the plant cell is transformed with an isolated nucleic acid molecule comprising a polynucleotide, which codes for a protein with exonuclease activity, which polynucleotide is selected from the group consisting of
   xa) the polynucleotide defined in any one of SEQ ID No. 22 to 54 or a fully complementary strand thereof,
   xb) a polynucleotide encoding a polypeptide with the amino acid sequence defined in any one of SEQ ID No. 1 to 21 or a fully complementary strand thereof, and
   xc) a polynucleotide variant having a degree of sequence identity of more than 70 % to the nucleic acid sequence defined in xa) or xb), or a fully complementary strand thereof
   or with the isolated nucleic acid molecule of embodiment 4 or with the vector of embodiment 7 and the transformed plant cell is regenerated into a transformed plant that contains the at least one nucleic acid sequence so as to induce apomixis in the plant.
15: A method for isolating an apomixis inducing nucleic acid molecule from a plant, wherein an isolated nucleic acid molecule comprising a polynucleotide, which codes for a protein with exonuclease activity, which polynucleotide is selected from the group consisting of
   xa) the polynucleotide defined in any one of SEQ ID No. 22 to 54 or a fully complementary strand thereof,
   xb) a polynucleotide encoding a polypeptide with the amino acid sequence defined in any one of SEQ ID No. 1 to 21 or a fully complementary strand thereof, and
   xc) a polynucleotide variant having a degree of sequence identity of more than 70 % to the nucleic acid sequence defined in xa) or xb), or a fully complementary strand thereof
   or the isolated nucleic acid molecule of 4 or the vector of 7 is used to screen and isolate nucleic acid sequences derived from the plant.
16: A transgenic plant, plant cell or plant material comprising a cell according to any one of embodiment 8 or produced according to a method according to any one of 13 or 14 or progeny thereof.
17: The transgenic plant, plant cell or plant material according to 16 transgenically expressing the nucleotide acid sequence of any one of 5 or 6.
18: A method for identifying an effector for apomixis in a plant, wherein the transgenic plant, plant cell or plant material according to any one of 16 or 17 is cultivated.

The invention will now be illustrated by way of example.

### Example 1: Screening and isolation of apomixis-inducing gene (apollo gene)

### 1.a) Plant material and seed screen analysis

Plants were grown from seedlings onwards in a phytotron under controlled environmental conditions. The flow cytometric seed screen was used to analyse reproductive variability in 18 Boechera accessions (Table IV).

**Table IV. Boechera accessions used in Microarrays and RT-PCR analyses.**

| Table IV - Boechera accessions used in Microarrays and RT-PCR analyses. | | |
|---|---|---|
| Accession | Apomeiosis frequency | Collection locality |
| B08-1 | 1 | Birch Creek, Montana |
| B08-11 | 1 | Sliderock, Ranch Creek, Granite, Montana |
| B08-33 | 1 | Mule Ranch, Montana |
| B08-111 | 1 | Morgan Switch Back, Idaho |
| B08-81 | 1 | Vipond Park, Beaverhead, Montana |
| B08-168 | 1 | Vipond Park, Beaverhead, Montana |

| B08-43 | 1 | Mule Ranch, Montana |
|---|---|---|
| B08-66 | 1 | Highwood Mtns, Montana |
| B08-104 | 1 | Lost Trail Meadow |
| B08-215 | 1 | Blue Lakes road, California |
| B08-369 | 0 | Twin Saddle, Idaho |
| B08-376 | 0 | Sagebrush Meadow, Montana |
| B08-380 | 0 | Buffalo Pass, Colorado |
| B08-355 | 0 | Gold Creek, Colorado |
| B08-329 | 0 | Big Hole Pass, Montana |
| B08-385 | 0 | Parker Meadow, Idaho |
| B08-344 | 0 | Bandy Ranch, Montana |
| B08-390 | 0 | Panther Creek |

Single seeds were ground individually with three 2.3 mm stainless steel beads in each well of 96-well plate (PP-Master-block 128.0/85MM, 1.0ml 96 well plate by Greiner bio-one, www.gbo.com) containing 50µl extraction-nuclei isolation buffer (see below) using a Geno-Grinder 2000 (SPEX Certi-Prep) at rate of 150 strokes/minute for 90 seconds.

A two-step procedure consisting of an isolation and staining buffer was used: (a) isolation buffer I - 0.1M Citric acid monohydrate and 0.5% v/v Tween 20 dissolved in H₂O and adjusted to pH 2.5); and (b) staining buffer II - 0.4M Na₂HPO₄.12H₂0 dissolved in H₂O plus 4 µg/ml 4',6-Diamidinophenyl-indole (DAPI) and adjusted to pH 8.5. 50 µl of isolation buffer I was added to each seed per well in a 96-well plate before grinding, and a further 160 µl buffer I was added after grinding to recover enough volume through filtration (using Partec 30 µm mesh-width nylon filters). 100 µl of staining buffer II was then added to 50 µl of the resultant suspension (isolated nuclei), and incubated on ice for 10 minutes before flow cytometric analysis. To avoid sample degradation over the 2-hour period required for the analysis of 96 samples, the sample plate was sealed with aluminum sealing tape.

All sample plates were analysed on a 4° C cooled Robby-Well autosampler hooked up to a Partec PAII flow Cytometer (Partec GmbH, Munster, Germany). Two single seeds from SAD 12, a known sexual self-fertile Boechera were always included as an external reference at well positions 1 and 96 in order to normalize other peaks and correct peak shifts over the analysis period. SAD 12 seeds were composed exclusively of 2C embryo to 3C endosperm ratio, which reflected an embryo composition of C (C denotes monoploid DNA content) maternal (Cm) genomes + C paternal (Cp) = 2C genomes, and an endosperm composition of 2Cm + Cp = 3C.

Based upon the present high-throughput flow-cytometric seed screen data, all apomictic accessions were shown to be characterized by 100% apomictic seed production.

### 1.b) Ovule micro-dissection

Ovules at megasporogenesis between stages 2-II to 2-IV were selected where megaspore mother cell is differentiated, inner and outer integument initiated in order to examine changes in gene expression associated with meiosis and apomeiosis. The gynoecia of sexual and apomictic Boechera were dissected out from non-pollinated flowers at the stage of megasporogenesis in 0.55 M sterile mannitol solution, at a standardized time (between 8 and 9 a.m.) over multiple days. Microdissections were done in a sterile laminar air flow cabinet using a stereoscopic Microscope (1000 Stemi, Carl Zeiss, Jena, Germany) under 2X magnification. The gynoecium was held with forceps while a sterile scalpel was used to cut longitudinally such that the halves of the silique along with the ovules were immediately exposed to the mannitol. Individual live ovules were subsequently collected under an inverted Microscope (Axiovert 200M, Carl Zeiss) in sterile conditions, using sterile glass needles (self-made using a Narishige PC-10 puller, and bent to an angle of about 100°) to isolate the ovules from placental tissue. Using a glass capillary (with an opening of 150 µm interior diameter) interfaced to an Eppendorf Cell Tram Vario, the ovules were collected in sterile Eppendorf tubes containing 100 µl of RNA stabilizing buffer (RNA later, Sigma). Between 20 and 40 ovules per accession were collected in this way, frozen directly in liquid nitrogen and stored at -80° C.

### 1.c) Ovule RNA isolation

Total RNA extractions were carried out using PicoPure RNA isolation kit (Arcturus Bioscience, CA). RNA integrity and quantity was verified on an Agilent 2100 Bioanalyzer using the RNA Pico chips (Agilent Technologies, Palo Alto, CA).

### 1.d) Microarray

### 1.d.i) Microarray design

The 454 (FLX) technology was used to sequence the complete transcriptomes of 3 sexual and 3 apomictic Boechera accessions, as a first step in the design of high-density Boechera-specific microarrays for use in comparisons of gene expression and copy number variation. The goal of transcriptome sequencing was thus to identify all genes which can be expressed during flower development, followed by the spotting of all identified genes onto an (Agilent) microarray.

This was accomplished by pooling flowers at multiple developmental stages separately for sexual and apomictic plants, followed by a cDNA normalization procedure in order to balance out transcript levels to increase the chance that all observable mRNA species are sequenced. Furthermore, a 3'-UTR (untranslated region) anchored 454 procedure was employed such that mRNA sequences were biased towards their 3'-UTRs, regions which demonstrate relatively high (but not random) levels of variability, to enable the identification of allelic variation.

The 454 sequences were assembled using the CLC Genomics workbench using standard assembly parameters for long-read high-throughput sequences, after trimming of all reads using internal sequence quality scores. In doing so, 36 289 contig sequences and 154 468 non-assembled singleton sequences were obtained. This data was provided to ImaGenes (GmbH, Germany) for microarray development using their Pre-selection strategy (PSS) service.

The PSS service worked as follows: 14 different oligonucleotides (each 60 bp in length) per contig and 8 oligonucleotides per singleton, including the "anti-sense" sequence of each oligo, were bioinformatically designed and spotted onto two 1 million-spot test arrays. These test-arrays were probed using (1) a "complex cRNA mixture" (obtained by pooling tissues and harvesting all RNA from them), and (2) genomic DNA extracted from leaf tissue pooled from a sexual and an apomictic individual. Based upon the separate hybridization results from the cRNA and genomic DNA samples, and after all quality tests, a final 2 X 105 000 spot array was designed. This array should contain multiple oligonucleotides (i.e. technical replicates) of every gene expressed during Boechera flower development.

### 1.d.ii) Hybridization

cRNA was prepared and labelled using the Quick-Amp One-Color Labeling Kit (Agilent Technologies, CA) and hybridized to the Agilent custom Boechera arrays (8 and 10 biological replicates were hybridized for sexual and apomictic genotypes respectively).

### 1.d.iii) Statistical analysis

Analyses were performed using GeneSpring GX Software (version 10) and candidate probes significantly differentially expressed (p ≤ 0.05) between apomictic and sexual plants were selected based on the following parameters: (a) percentile shift 75 normalization, median as baseline, reproductive mode (apomictic or sexual) as interpretation (1st level), T-test unpaired as statistical analysis and Bonferroni FWER multiple test corrections. Using the highest level of significance cutoff led to the identification of 4 different spots on the microarray (p < 0.01 for the first three and p < 0.05 for the fourth). Importantly, when the oligonucleotide sequences of these 4 spots were BLASTed to a 454 cDNA sequence database, all 4 blasted to the same Boechera transcript. Thus, not only has the present experiment been corrected for biological noise, furthermore a single differentially-expressed transcript between the microdissected ovules of all sexual and apomictic genotypes, with 4 technical replicates for the specific gene on the microarray was detected. This gene is expressed to a similar fashion when comparing both diploid and triploid apomictic ovules to those of sexuals, and hence its expression behavior is apparently not influenced by ploidy. Finally, a search for homologues to this Boechera transcript demonstrated that it is involved with the cell cycle in other species, thus supporting evidence regarding deregulation of the sexual pathway as a means to produce apomixis.

### Example 2: Characterisation of apomixis-inducing gene

### 2.a) Candidate gene characterization

### 2.a.i) Genome level

### 2.a.i.1) Cloning

The full-length transcript from all 18 accessions was cloned and sequenced (TOPO-TA Cloning kit, Invitrogen) using proofreading polymerase (Accuprime). The transcript is highly polymorphic, and is characterized by comparable levels of single nucleotide polymorphisms between sexual and apomicts. Nevertheless, a single "apomixis polymorphism" is found in all 10 apomictic accessions, but not in any sexual accession. SEQ ID No. 46 to 54 show the genomic and the coding sequence of three sexual alleles, namely S011a, S355a and S390a. SEQ ID No. 37 to 45 show the genomic and the coding sequence of three apomictic alleles, namely A011a, A043a and A081a. Considering that the geographic collection points of all accessions range from California to the American mid-west (i.e. 1000's of kilometers), the sharing of this polymorphism in all apomicts is highly significant. Finally, the SNP polymorphism spectrum surrounding the "apomixis polymorphism" reflects that found in all other alleles in both sexual and apomictic accessions. Hence the "apomixis polymorphism" appears to have undergone recombination during the evolution of Boechera, but which is nonetheless shared by all apomicts, regardless of different genetic, ploidy or geographic backgrounds.

### 2.a.i.2) BAC

Pooled DNA of all tissues accessions was used as a template for hybridization probes generation. Two probes of different size (1.6 and 2.3 kb) were prepared by PCR amplification using two pairs of specific primers of the candidate gene genomic sequence. Both probes were labeled and used for hybridization on a apomictic Boechera BAC library. There were 8 positive hybridizations. The respective isolated BACs (PureLink Plasmid DNA Purification kit) were named 1, 2a, 2b, 3, 4, 5, 6 and 7. Selected BACs were retested using specific primers for the candidate gene. All BACs were confirmed except the BAC-3. The other seven BACs were fingerprinted by restriction enzyme digestion. BAC-1 and BAC-2a seemed to be redundant with the other BACs. The BACs: 2b, 4, 5, 6 and 7 were sequenced.

BAC sequences could be assembled together for the pairs 2b_4 and 5_7, whereas BAC-6 remained alone.

BAC sequences were characterized by comparison with other plant sequences.

### 2.a.ii) Transcriptome level

RACE experiments (SMARTer RACE cDNA Amplification Kit) were performed.

The results revealed that mRNA corresponding to apomictic accessions has a truncated 5' extreme upstream the "apomixis polymorphism" whereas sexual accessions have ∼200 pb of additional length.

Once 5' and 3' mRNA extremes were known, further PCRs over all tissues cDNA were performed for complete splicing profile characterization.

### 2.b) Validation

### 2.b.i) QRT-PCR

An allele-specific qRT-PCR analysis of the candidate gene on the microdissected live ovules (megaspore mother cell stage) from 6 sexual and 10 diploid apomictic Boechera accessions (3 technical replicates per accession) was completed. Using two different forward PCR primers which spanned the apomixis-specific polymorphism which was identified from the gene sequences, it was possible to measure transcript abundance for both the sexual and apomictic alleles separately.

cDNA was prepared using RevertAid H Minus reverse transcriptase.

For the real-time PCR reactions the SYBR® Green PCR Master Mix (Applied Biosystems, Foster City, CA) was used. QRT-PCR amplifications were carried out in a 7900HT Fast RT-PCR System machine (Applied Biosystems) with the following temperature profile for SYBRgreen assays: initial denaturation at 90°C for 10 min, followed by 40 cycles of 95°C for 15 sec. and 60°C for 1 min. For checking amplicon quality, a melting curve gradient was obtained from the product at the end of the amplification. The Ct, defined as the PCR cycle at which a statistically significant increase of reporter fluorescence is first detected, was used as a measure for the starting copy numbers of the target gene. The mean expression level and standard deviation for each set of three technical replicates for each cDNA was calculated. Relative quantitation and normalization of the amplified targets were performed by the comparative ΔΔCt method using a calibrator sample in reference to the expression levels of the house-keeping gene UBQ10.

The results are conclusive: the apomictic allele is exclusively expressed in the microdissected ovules of all apomictic accessions, while the sexual allele is never expressed in any, which means sexual or apomictic, ovule. Both alleles are expressed in other tissues, namely somatic tissue. Hence, it appears very reasonable to assume that the sexual allele is inactive/silenced during normal sexual ovule development, while the expression of the apomictic allele is correlated with apomeiotic ovule development.

### Example 3: Construction of transformation vectors and transformation of Arabidopsis thaliana with apomixis-inducing gene

### Plant transformation

Transformations of Arabidopsis thaliana (sex) (hybrids F1) and Boechera (sex) with the gene of the present invention are able to show a change of their reproductive mode into apomictic seed production. For this, the complete genomic allele (including complete promoter) has been cloned in pNOS-ABM.

In addition, different constructs are used to characterize the role of the present regulatory elements, in particular the promoter of the present invention, in its expression. For this, both apo and sex promoters have been exactly connected to the ATG in front of gus in pGUS-ABM.

Complete BAC-4 is as well used for transformations.

## Claims

1. A method for inducing apomixis in a transgenic plant, wherein an exogenous nucleotide sequence encoding a protein capable of inducing apomixis in a plant is introduced into the plant and expressed in the ovule of said plant, and wherein said nucleotide sequence comprises a polynucleotide, which codes for a protein with DEDD 3' to 5' exonuclease activity, which polynucleotide is selected from the group consisting of
a) the polynucleotide defined in any one of SEQ ID No. 22 to 25, 27 to 30, 32 to 35, 37, 38, 40, 41, 43, 44, 46, 47, 49, 50, 52, or 53 or a fully complementary strand thereof,
b) a polynucleotide encoding a polypeptide with the amino acid sequence defined in any one of SEQ ID No. 4 to 9, 13 to 15, 19 to 21 or a fully complementary strand thereof, and
c) a polynucleotide variant having a degree of sequence identity of more than 70% to the nucleic acid sequence defined in a) or b), or a fully complementary strand thereof.

2. The method of claim 1, wherein the polynucleotide is selected from the group consisting of
a) the polynucleotide defined in any one of SEQ ID No. 22, 23, 27, 28, 32, 33 or a fully complementary strand thereof,
b) a polynucleotide encoding a polypeptide with the amino acid sequence defined in any one of SEQ ID No. 4, 5, 6 or a fully complementary strand thereof, and
c) a polynucleotide variant having a degree of sequence identity of more than 70% to the nucleic acid sequence defined in a) or b), or a fully complementary strand thereof.

3. Use of an isolated nucleic acid molecule for inducing apomixis in a plant, which comprises transforming said nucleic acid into a plant cell to produce said plant, wherein said nucleic acid comprises a polynucleotide which codes for a protein with DEDD 3' to 5' exonuclease activity and is a polynucleotide encoding a polypeptide with the amino acid sequence defined in any one of SEQ ID No. 4 to 9, 13 to 15, or 19 to 21, wherein the polynucleotide is under the control of a constitutive or an ovule-specific promoter and the protein is expressed in the ovule.

4. Use of an isolated nucleic acid molecule for inducing apomixis in a plant, which comprises a polynucleotide coding for a protein with DEDD 3' to 5' exonuclease activity, which polynucleotide is selected from the group consisting of
a) the polynucleotide defined in any one of SEQ ID No. 22, 23, 24, 25, 27, 28, 29, 30, 32, 33, 34, 35, 37, 38, 40, 41, 43, 44, 46, 47, 49, 50, 52, 53 or a fully complementary strand thereof,
b) a polynucleotide encoding a polypeptide with the amino acid sequence defined in any one of SEQ ID No. 4, 5, 6, 7, 8, 9, 13, 14, 15, 19, 20, 21 or a fully complementary strand thereof, and
c) a polynucleotide variant having a degree of sequence identity of more than 90% to the nucleic acid sequence defined in a) or b), or a fully complementary strand thereof, wherein the protein with DEDD 3' to 5' exonuclease activity is expressed in a plant ovule.

5. A vector for inducing apomixis in a plant comprising a first polynucleotide which is able to act as a regulatory element that leads to expression in the ovule of an operably linked polynucleotide and is a constitutive or ovule-specific promoter,
wherein the first polynucleotide is operably linked to a second polynucleotide which codes for a protein with DEDD 3' to 5' exonuclease activity and is selected from the group consisting of:
i) the polynucleotide defined in any one of SEQ ID No. 22 to 25, 27 to 30, 32 to 35, 37, 38, 40, 41, 43, 44, 46, 47, 49, 50, 52, or 53 or a fully complementary strand thereof,
ii) a polynucleotide encoding a polypeptide with the amino acid sequence defined in any one of SEQ ID No. 4 to 9, 13 to 15, 19 to 21 or a fully complementary strand thereof, and
iii) a polynucleotide variant having a degree of sequence identity of more than 70% to the nucleic acid sequence defined in i) or ii), or a fully complementary strand thereof.

6. A host cell containing the vector of claim 5.

7. A transgenic plant, plant cell or plant material comprising at least one transgenic nucleic acid molecule as defined in any one of claims 3 or 4 wherein the nucleic acid molecule is arranged in an order or orientation, or in a genomic position, that is not normally found in the plant, plant cell, or plant material to which it is transferred or the vector of claim 5.

8. A cell culture, preferably a plant cell culture, comprising a cell according to claim 6.

9. The method for inducing apomixis in a plant according to any one of claims 1 or 2, wherein the expression is induced by transforming a plant cell with an isolated nucleic acid molecule comprising a polynucleotide which codes for the protein with DEDD 3' to 5' exonuclease activity as defined in any one of claims 1, 2 or 4, with the isolated nucleic acid molecule of claim 3 or with the vector according to claim 5 and regenerating the transformed plant cell into a transformed plant that contains the transformed nucleic acid sequence so as to express the protein with DEDD 3' to 5' exonuclease activity in a plant ovule and to induce apomixis in the plant.

10. A method for the production of an apomictic plant, wherein a plant cell is transformed with a nucleic acid molecule capable of inducing the expression of a nucleotide sequence encoding a protein capable of inducing apomixis in a plant and regenerating the transformed plant cell into a transformed plant that contains the transformed nucleic acid molecule so as to express a protein with DEDD 3' to 5' exonuclease activity in a plant ovule and to induce apomixis in the plant, wherein the nucleotide sequence encoding the protein capable of inducing apomixis in the plant is a polynucleotide, which codes for a protein with DEDD 3' to 5' exonuclease activity, which polynucleotide is selected from the group consisting of
a) the polynucleotide defined in any one of SEQ ID No. 22 to 25, 27 to 30, 32 to 35, 37, 38, 40, 41, 43, 44, 46, 47, 49, 50, 52, or 53 or a fully complementary strand thereof,
b) a polynucleotide encoding a polypeptide with the amino acid sequence defined in any one of SEQ ID No. 4 to 9, 13 to 15 or 19 to 21 or a fully complementary strand thereof, and
c) a polynucleotide variant having a degree of sequence identity of more than 70% to the nucleic acid sequence defined in a) or b), or a fully complementary strand thereof.

11. The method for the production of an apomictic plant by transforming a plant cell according to claim 10, wherein the plant cell is transformed with an isolated nucleic acid molecule comprising a polynucleotide, which codes for a protein with DEDD 3' to 5' exonuclease activity, which polynucleotide is selected from the group consisting of
a) the polynucleotide defined in any one of SEQ ID No. 22, 23, 27, 28, 32, or 33 or a fully complementary strand thereof,
b) a polynucleotide encoding a polypeptide with the amino acid sequence defined in any one of SEQ ID No. 4, 5, or 6 or a fully complementary strand thereof, and
c) a polynucleotide variant having a degree of sequence identity of more than 70% to the nucleic acid sequence defined in a) or b), or a fully complementary strand thereof
or with the isolated nucleic acid molecule of claim 3 or with the vector of claim 5 and the transformed plant cell is regenerated into a transformed plant that contains the at least one nucleic acid sequence so as to express the protein with DEDD 3' to 5' exonuclease activity in a plant ovule and to induce apomixis in the plant.

12. A method for isolating an apomixis inducing nucleic acid molecule from a plant, wherein an isolated nucleic acid molecule comprising a polynucleotide which codes for a protein with DEDD 3' to 5' exonuclease activity, which polynucleotide is selected from the group consisting of
a) the polynucleotide defined in any one of SEQ ID No. 22 to 54 or a fully complementary strand thereof,
b) a polynucleotide encoding a polypeptide with the amino acid sequence defined in any one of SEQ ID No. 1 to 21 or a fully complementary strand thereof, and
c) a polynucleotide variant having a degree of sequence identity of more than 70% to the nucleic acid sequence defined in a) or b), or a fully complementary strand thereof,
or the isolated nucleic acid molecule of claim 3, or the vector of claim 5, is used to screen and isolate nucleic acid sequences derived from the plant.

13. A transgenic plant, plant cell or plant material comprising a cell according to claim 6, that is produced according to a method according to any one of claims 9 or 10, or progeny thereof that contain the vector according to claim 5 or that contain the polynucleotide which codes for a protein with DEDD 3' to 5' exonuclease activity according to any one of claims 1, 2, 4 or 10, wherein the polynucleotide is arranged in an order or orientation, or in a genomic position, that is not normally found in the plant, plant cell, or plant material to which it is transferred and wherein the protein with DEDD 3' to 5' exonuclease activity is expressed in a plant ovule.

14. The transgenic plant, plant cell or plant material according to claim 13, transgenically expressing the polynucleotide sequence of claim 4.

15. Use of the transgenic plant, plant cell or plant material according to any one of claims 13 or 14 to screen for an effector of apomixis.

## Patentansprüche

1. Verfahren zum Induzieren von Apomixis in einer transgenen Pflanze, wobei eine exogene Nukleotidsequenz, die für ein Protein kodiert, das zum Induzieren von Apomixis in einer Pflanze geeignet ist, in die Pflanze eingeführt und in der Eizelle der Pflanze exprimiert wird, und wobei die Nukleotidsequenz ein Polynukleotid umfasst, das für ein Protein mit DEDD 3' bis 5'-Exonukleaseaktivität kodiert, wobei das Polynukleotid ausgewählt ist aus der Gruppe bestehend aus
a) dem in einer der SEQ ID No. 22 bis 25, 27 bis 30, 32 bis 35, 37, 38, 40, 41, 43, 44, 46, 47, 49, 50, 52 oder 53 definierten Polynukleotid oder einem vollständig komplementären Strang davon,
b) einem Polynukleotid, das für ein Polypeptid mit der in einer der SEQ ID No. 4 bis 9, 13 bis 15, 19 bis 21 definierten Aminosäuresequenz kodiert, oder einem vollständig komplementären Strang davon, und
c) einer Polynukleotidvariante mit einem Grad an Sequenzidentität von mehr als 70 % zur in a) oder b) definierten Nukleinsäuresequenz oder einem vollständig komplementären Strang davon.

2. Verfahren nach Anspruch 1, wobei das Polynukleotid ausgewählt ist aus der Gruppe bestehend aus
a) dem in einer der SEQ ID No. 22, 23, 27, 28, 32, 33 definierten Polynukleotid oder einem vollständig komplementären Strang davon,
b) einem Polynukleotid, das für ein Polypeptid mit der in einer der SEQ ID No. 4, 5, 6 definierten Aminosäuresequenz kodiert, oder einem vollständig komplementären Strang davon, und
c) einer Polynukleotidvariante mit einem Grad an Sequenzidentität von mehr als 70 % zur in a) oder b) definierten Nukleinsäuresequenz oder einem vollständig komplementären Strang davon.

3. Verwendung eines isolierten Nukleinsäuremoleküls zum Induzieren von Apomixis in einer Pflanze, die eine Transformation der Nukleinsäure in eine Pflanzenzelle zum Ausbilden dieser Pflanze umfasst, wobei die Nukleinsäure ein Polynukleotid umfasst, das für ein Protein mit DEDD 3' bis 5'-Exonukleaseaktivität kodiert, und ein Polynukleotid ist, das für ein Polypeptid mit der in einer der SEQ ID No. 4 bis 9, 13 bis 15 oder 19 bis 21 definierten Aminosäuresequenz kodiert, wobei das Polynukleotid unter der Kontrolle eines konstitutiven oder eines eizellen-spezifischen Promotors steht und das Protein in der Eizelle exprimiert wird.

4. Verwendung eines isolierten Nukleinsäuremoleküls zum Induzieren von Apomixis in einer Pflanze, das ein Polynukleotid umfasst, das für ein Protein mit DEDD 3' bis 5'-Exonukleaseaktivität kodiert, wobei das Polynukleotid ausgewählt ist aus der Gruppe bestehend aus
a) dem in einer der SEQ ID No. 22, 23, 24, 25, 27, 28, 29, 30, 32, 33, 34, 35, 37, 38, 40, 41, 43, 44, 46, 47, 49, 50, 52, 53 definierten Polynukleotid oder einem vollständig komplementären Strang davon,
b) einem Polynukleotid, das für ein Polypeptid mit der in einer der SEQ ID No. 4, 5, 6, 7, 8, 9, 13, 14, 15, 19, 20, 21 definierten Aminosäuresequenz kodiert, oder einem vollständig komplementären Strang davon, und
c) einer Polynukleotidvariante mit einem Grad an Sequenzidentität von mehr als 90 % zur in a) oder b) definierten Nukleinsäuresequenz oder einem vollständig komplementären Strang davon, wobei das Protein mit DEDD 3' bis 5'-Exonukleaseaktivität in einer Pflanzeneizelle exprimiert wird.

5. Vektor zum Induzieren von Apomixis in einer Pflanze, umfassend ein erstes Polynukleotid, das als regulatorisches Element wirksam sein kann, das zur Expression eines operativ verknüpften Polynukleotids in der Eizelle führt und einen konstitutiven oder eizellen-spezifischen Promotor darstellt,
wobei das erste Polynukleotid operativ mit einem zweiten Polynukleotid verknüpft ist, das für ein Protein mit DEDD 3' bis 5'-Exonukleaseaktivität kodiert und ausgewählt ist aus der Gruppe bestehend aus:
i) dem in einer der SEQ ID No. 22 bis 25, 27 bis 30, 32 bis 35, 37, 38, 40, 41, 43, 44, 46, 47, 49, 50, 52 oder 53 definierten Polynukleotid oder einem vollständig komplementären Strang davon,
ii) einem Polynukleotid, das für ein Polypeptid mit der in einer der SEQ ID No. 4 bis 9, 13 bis 15, 19 bis 21 definierten Aminosäuresequenz kodiert, oder einem vollständig komplementären Strang davon, und
iii) einer Polynukleotidvariante mit einem Grad an Sequenzidentität von mehr als 70 % zur in i) oder ii) definierten Nukleinsäuresequenz oder einem vollständig komplementären Strang davon.

6. Wirtszelle, die den Vektor nach Anspruch 5 enthält.

7. Transgene Pflanze, Pflanzenzelle oder transgenes Pflanzenmaterial umfassend mindestens ein transgenes Nukleinsäuremolekül wie in einem der Ansprüche 3 oder 4 definiert, wobei das Nukleinsäuremolekül in einer Reihenfolge oder Orientierung oder in einer Position im Genom angeordnet ist, die in der Pflanze, Pflanzenzelle oder dem Pflanzenmaterial, auf die es übertragen wurde, oder dem Vektor nach Anspruch 5 normalerweise nicht gefunden wird.

8. Zellkultur, bevorzugt Pflanzenzellkultur, umfassend eine Zelle nach Anspruch 6.

9. Verfahren zum Induzieren von Apomixis in einer Pflanze nach einem der Ansprüche 1 oder 2, wobei die Expression induziert wird durch Transformation einer Pflanzenzelle mit einem isolierten Nukleinsäuremolekül, das ein Polynukleotid umfasst, das für das Protein mit DEDD 3' bis 5'-Exonukleaseaktivität kodiert, wie in einem der Ansprüche 1, 2 oder 4 definiert, mit dem isolierten Nukleinsäuremolekül nach Anspruch 3 oder mit dem Vektor nach Anspruch 5 und Regenerieren der transformierten Pflanzenzelle in eine transformierte Pflanze, die die transformierte Nukleinsäuresequenz enthält, so dass das Protein mit DEDD 3' bis 5'-Exonukleaseaktivität in einer Pflanzeneizelle exprimiert wird und Apomixis in der Pflanze induziert wird.

10. Verfahren zur Herstellung einer apomiktischen Pflanze, wobei eine Pflanzenzelle transformiert wird mit einem Nukleinsäuremolekül, das zum Induzieren der Expression einer Nukleotidsequenz geeignet ist, die für ein Protein kodiert, das zum Induzieren von Apomixis in einer Pflanze geeignet ist, und Regenerieren der transformierten Pflanzenzelle in eine transformierte Pflanze, die das transformierte Nukleinsäuremolekül enthält, so dass ein Protein mit DEDD 3' bis 5'-Exonukleaseaktivität in einer Pflanzeneizelle exprimiert wird und Apomixis in der Pflanze induziert wird, wobei die Nukleotidsequenz, die für das Protein kodiert, das zum Induzieren von Apomixis in der Pflanze geeignet ist, ein Polynukleotid ist, das für ein Protein mit DEDD 3' bis 5'-Exonukleaseaktivität kodiert, wobei das Polynukleotid ausgewählt ist aus der Gruppe bestehend aus
a) dem in einer der SEQ ID No. 22 bis 25, 27 bis 30, 32 bis 35, 37, 38, 40, 41, 43, 44, 46, 47, 49, 50, 52 oder 53 definierten Polynukleotid oder einem vollständig komplementären Strang davon,
b) einem Polynukleotid, das für ein Polypeptid mit der in einer der SEQ ID No. 4 bis 9, 13 bis 15 oder 19 bis 21 definierten Aminosäuresequenz kodiert, oder einem vollständig komplementären Strang davon, und
c) einer Polynukleotidvariante mit einem Grad an Sequenzidentität von mehr als 70 % zur in a) oder b) definierten Nukleinsäuresequenz oder einem vollständig komplementären Strang davon.

11. Verfahren zur Herstellung einer apomiktischen Pflanze durch Transformation einer Pflanzenzelle nach Anspruch 10, wobei die Pflanzenzelle transformiert wird mit einem isolierten Nukleinsäuremolekül, das ein Polynukleotid umfasst, das für ein Protein mit DEDD 3' bis 5'-Exonukleaseaktivität kodiert, wobei das Polynukleotid ausgewählt ist aus der Gruppe bestehend aus
a) dem in einer der SEQ ID No. 22, 23, 27, 28, 32 oder 33 definierten Polynukleotid oder einem vollständig komplementären Strang davon,
b) einem Polynukleotid, das für ein Polypeptid mit der in einer der SEQ ID No. 4, 5 oder 6 definierten Aminosäuresequenz kodiert, oder einem vollständig komplementären Strang davon, und
c) einer Polynukleotidvariante mit einem Grad an Sequenzidentität von mehr als 70 % zur in a) oder b) definierten Nukleinsäuresequenz oder einem vollständig komplementären Strang davon
oder mit dem isolierten Nukleinsäuremolekül nach Anspruch 3 oder mit dem Vektor nach Anspruch 5 und die transformierte Pflanzenzelle in eine transformierte Pflanze regeneriert wird, die mindestens eine Nukleinsäuresequenz enthält, so dass das Protein mit DEDD 3' bis 5'-Exonukleaseaktivität in einer Pflanzeneizelle exprimiert wird und Apomixis in der Pflanze induziert wird.

12. Verfahren zum Isolieren eines Apomixis induzierenden Nukleinsäuremoleküls aus einer Pflanze, wobei ein isoliertes Nukleinsäuremolekül, das ein Polynukleotid umfasst, das für ein Protein mit DEDD 3' bis 5'-Exonukleaseaktivität kodiert, wobei das Polynukleotid ausgewählt ist aus der Gruppe bestehend aus
a) dem in einer der SEQ ID No. 22 bis 54 definierten Polynukleotid oder einem vollständig komplementären Strang davon,
b) einem Polynukleotid, das für ein Polypeptid mit der in einer der SEQ ID No. 1 bis 21 definierten Aminosäuresequenz kodiert, oder einem vollständig komplementären Strang davon, und
c) einer Polynukleotidvariante mit einem Grad an Sequenzidentität von mehr als 70 % zur in a) oder b) definierten Nukleinsäuresequenz oder einem vollständig komplementären Strang davon,
oder das isolierte Nukleinsäuremolekül nach Anspruch 3 oder der Vektor nach Anspruch 5 zum Screenen und Isolieren der aus der Pflanze gewonnenen Nukleinsäuresequenzen verwendet wird.

13. Transgene Pflanze, Pflanzenzelle oder transgenes Pflanzenmaterial umfassend eine Zelle nach Anspruch 6, die gemäß einem Verfahren nach einem der Ansprüche 9 oder 10 hergestellt ist, oder Abkömmlinge davon, die den Vektor nach Anspruch 5 enthalten oder das Polynukleotid enthalten, das für ein Protein mit DEDD 3' bis 5'-Exonukleaseaktivität kodiert, nach einem der Ansprüche 1, 2, 4 oder 10, wobei das Polynukleotid in einer Reihenfolge oder Orientierung oder einer Position im Genom angeordnet ist, die in der Pflanze, Pflanzenzelle oder dem Pflanzenmaterial, auf die es übertragen wurde, normalerweise nicht gefunden wird, und wobei das Protein mit DEDD 3' bis 5'-Exonukleaseaktivität in einer Pflanzeneizelle exprimiert wird.

14. Transgene Pflanze, Pflanzenzelle oder transgenes Pflanzenmaterial nach Anspruch 13 zur transgenen Expression der Polynukleotidsequenz nach Anspruch 4.

15. Verwendung der transgenen Pflanze, Pflanzenzelle oder des transgenen Pflanzenmaterials nach einem der Ansprüche 13 oder 14 zum Screenen auf einen Effektor der Apomixis.

## Revendications

1. Procédé pour induire une apomixie dans une plante transgénique, dans lequel une séquence nucléotidique exogène codant pour une protéine apte à induire une apomixie dans une plante est introduite dans la plante et exprimée dans l'ovule de ladite plante, et dans lequel ladite séquence nucléotidique comprend un polynucléotide, qui code pour une protéine ayant une activité DEDD exonucléase de 3' vers 5', lequel polynucléotide est choisi dans le groupe constitué par
a) le polynucléotide défini selon l'une quelconque de SEQ ID No 22 à 25, 27 à 30, 32 à 35, 37, 38, 40, 41, 43, 44, 46, 47, 49, 50, 52 ou 53, ou un brin entièrement complémentaire de celui-ci,
b) un polynucléotide codant pour un polypeptide ayant la séquence d'acides aminés définie selon l'une quelconque de SEQ ID No 4 à 9, 13 à 15, 19 à 21 ou un brin entièrement complémentaire de celui-ci, et
c) un variant polynucléotidique ayant un degré d'identité de séquence supérieur à 70 % avec la séquence d'acide nucléique définie en a) ou b), ou un brin entièrement complémentaire de celui-ci.

2. Procédé selon la revendication 1, dans lequel le polynucléotide est choisi dans le groupe constitué par
a) le polynucléotide défini selon l'une quelconque de SEQ ID No 22, 23, 27, 28, 32, 33 ou un brin entièrement complémentaire de celui-ci,
b) un polynucléotide codant pour un polypeptide ayant la séquence d'acides aminés définie selon l'une quelconque de SEQ ID No 4, 5, 6 ou un brin entièrement complémentaire de celui-ci, et
c) un variant polynucléotidique ayant un degré d'identité de séquence supérieur à 70 % avec la séquence d'acide nucléique définie en a) ou b), ou un brin entièrement complémentaire de celui-ci.

3. Utilisation d'une molécule d'acide nucléique isolée pour induire une apomixie dans une plante, qui comprend la transformation dudit acide nucléique en une cellule végétale pour produire ladite plante, dans laquelle ledit acide nucléique comprend un polynucléotide qui code pour une protéine ayant une activité DEDD exonucléase de 3' vers 5' et est un polynucléotide codant pour un polypeptide ayant la séquence d'acides aminés définie selon l'une quelconque de SEQ ID No 4 à 9, 13 à 15, ou 19 à 21, dans laquelle le polynucléotide est sous le contrôle d'un promoteur constitutif ou spécifique de l'ovule et la protéine est exprimée dans l'ovule.

4. Utilisation d'une molécule d'acide nucléique isolée pour induire une apomixie dans une plante, qui comprend un polynucléotide codant pour une protéine ayant une activité DEDD exonucléase de 3' vers 5', lequel polynucléotide est choisi dans le groupe constitué par
a) le polynucléotide défini selon l'une quelconque de SEQ ID No 22, 23, 24, 25, 27, 28, 29, 30, 32, 33, 34, 35, 37, 38, 40, 41, 43, 44, 46, 47, 49, 50, 52, 53 ou un brin entièrement complémentaire de celui-ci,
b) un polynucléotide codant pour un polypeptide ayant la séquence d'acides aminés définie selon l'une quelconque de SEQ ID No 4, 5, 6, 7, 8, 9, 13, 14, 15, 19, 20, 21 ou un brin entièrement complémentaire de celui-ci, et
c) un variant polynucléotidique ayant un degré d'identité de séquence supérieur à 90 % avec la séquence d'acide nucléique définie en a) ou b), ou un brin entièrement complémentaire de celui-ci, dans lequel la protéine ayant une activité DEDD exonucléase de 3' vers 5' est exprimée dans un ovule de plante.

5. Vecteur pour induire une apomixie dans une plante comprenant un premier polynucléotide quoi est apte à agir comme un élément régulateur qui conduit à l'expression dans l'ovule d'un polynucléotide lié de manière fonctionnelle et est un promoteur constitutif ou spécifique de l'ovule,
dans lequel le premier polynucléotide est lié de manière fonctionnelle à un deuxième polynucléotide qui code pour une protéine ayant une activité DEDD exonucléase de 3' vers 5' et est choisi dans le groupe constitué par :
i) le polynucléotide défini selon l'une quelconque de SEQ ID No 22 à 25, 27 à 30, 32 à 35, 37, 38, 40, 41, 43, 44, 46, 47, 49, 50, 52 ou 53 ou un brin entièrement complémentaire de celui-ci,
ii) un polynucléotide codant pour un polypeptide ayant la séquence d'acides aminés définie selon l'une quelconque de SEQ ID No 4 à 9, 13 à 15, 19 à 21 ou un brin entièrement complémentaire de celui-ci, et
iii) un variant polynucléotidique ayant un degré d'identité de séquence supérieur à 70 % avec la séquence d'acide nucléique définie en i) ou ii), ou un brin entièrement complémentaire de celui-ci.

6. Cellule hôte contenant le vecteur selon la revendication 5.

7. Plante transgénique, cellule végétale ou matériel végétal comprenant au moins une molécule d'acide nucléique transgénique telle que définie selon l'une quelconque des revendications 3 ou 4, dans laquelle la molécule d'acide nucléique est agencée dans un ordre ou une orientation, ou dans une position génomique qui n'est pas trouvée normalement dans la plante, la cellule végétale ou le matériel végétal auquel elle est transférée ou le vecteur selon la revendication 5.

8. Culture de cellules, de préférence une culture de cellules végétales, comprenant une cellule selon la revendication 6.

9. Procédé pour induire une apomixie dans une plante selon l'une quelconque des revendications 1 ou 2, dans lequel l'expression est induite par transformation d'une cellule végétale avec une molécule d'acide nucléique isolée comprenant un polynucléotide qui code pour la protéine ayant une activité DEDD exonucléase de 3' vers 5' telle que définie selon l'une quelconque des revendications 1, 2 ou 4, avec la molécule d'acide nucléique isolée selon la revendication 3 ou avec le vecteur selon la revendication 5 et la régénération de la cellule végétale transformée en une plante transformée qui contient la séquence d'acide nucléique transformée de manière à exprimer la protéine ayant une activité DEDD exonucléase de 3' vers 5' dans un ovule de plante et à induire une apomixie dans la plante.

10. Procédé pour la production d'une plante apomictique, dans lequel une cellule végétale est transformée avec une molécule d'acide nucléique apte à induire l'expression d'une séquence nucléotidique codant pour une protéine apte à induire une apomixie dans une plante et la régénération de la cellule végétale transformée en une plante transformée qui contient la molécule d'acide nucléique transformée de manière à exprimer une protéine ayant une activité DEDD exonucléase de 3' vers 5' dans un ovule de plante et à induire une apomixie dans la plante, dans lequel la séquence nucléotidique codant pour la protéine apte à induire une apomixie dans la plante est un polynucléotide, qui code pour une protéine ayant une activité DEDD exonucléase de 3' vers 5', lequel polynucléotide est choisi dans le groupe constitué par
a) le polynucléotide défini selon l'une quelconque de SEQ ID No 22 à 25, 27 à 30, 32 à 35, 37, 38, 40, 41, 43, 44, 46, 47, 49, 50, 52 ou 53, ou un brin entièrement complémentaire de celui-ci,
b) un polynucléotide codant pour un polypeptide ayant la séquence d'acides aminés définie selon l'une quelconque de SEQ ID No 4 à 9, 13 à 15, 19 à 21 ou un brin entièrement complémentaire de celui-ci, et
c) un variant polynucléotidique ayant un degré d'identité de séquence supérieur à 70 % avec la séquence d'acide nucléique définie en a) ou b), ou un brin entièrement complémentaire de celui-ci.

11. Procédé pour la production d'une plante apomictique par transformation d'une cellule végétale selon la revendication 10, dans lequel la cellule végétale est transformée avec une molécule d'acide nucléique isolée comprenant un polynucléotide, qui code pour une protéine ayant une activité DEDD exonucléase de 3' vers 5', lequel polynucléotide est choisi dans le groupe constitué par
a) le polynucléotide défini selon l'une quelconque de SEQ ID No 22, 23, 27, 28, 32 ou 33, ou un brin entièrement complémentaire de celui-ci,
b) un polynucléotide codant pour un polypeptide ayant la séquence d'acides aminés définie selon l'une quelconque de SEQ ID No 4, 5 ou 6, ou un brin entièrement complémentaire de celui-ci, et
c) un variant polynucléotidique ayant un degré d'identité de séquence supérieur à 70 % avec la séquence d'acide nucléique définie en a) ou b), ou un brin entièrement complémentaire de celui-ci
ou avec la molécule d'acide nucléique isolée selon la revendication 3 ou avec le vecteur selon la revendication 5 et la cellule transformée est régénérée en une plante transformée qui contient la ou les séquences d'acide nucléique de sorte à exprimer la protéine ayant une activité DEDD exonucléase de 3' vers 5' dans un ovule de la plante et à induire une apomixie dans la plante.

12. Procédé pour isoler une molécule d'acide nucléique induisant une apomixie à partir d'une plante, dans lequel une molécule d'acide nucléique isolée comprenant un polynucléotide qui code pour une protéine ayant une activité DEDD exonucléase de 3' vers 5', lequel polynucléotide est choisi dans le groupe constitué par
a) le polynucléotide défini selon l'une quelconque de SEQ ID No 22 à 54 ou un brin entièrement complémentaire de celui-ci,
b) un polynucléotide codant un polypeptide ayant la séquence d'acides aminés définie selon l'une quelconque de SEQ ID No 1 à 21 ou un brin entièrement complémentaire de celui-ci, et
c) un variant polynucléotidique ayant un degré d'identité de séquence supérieur à 70 % avec la séquence d'acide nucléique définie en a) ou b), ou un brin entièrement complémentaire de celui-ci, ou la molécule d'acide nucléique isolée selon la revendication 3, ou le vecteur selon la revendication 5, est utilisé pour cribler et isoler des séquences d'acide nucléique dérivées de la plante.

13. Plante transgénique, cellule végétale ou matériel végétal comprenant une cellule selon la revendication 6, qui est produite selon un procédé selon l'une quelconque des revendications 9 ou 10, ou la descendance de celle-ci qui contient le vecteur selon la revendication 5, ou qui contient le polynucléotide qui code pour une protéine ayant une activité DEDD exonucléase de 3' vers 5' selon l'une quelconque des revendications 1, 2, 4 ou 10, dans laquelle le polynucléotide est agencé dans un ordre ou une orientation, ou dans une position génomique, qui n'est pas trouvée normalement dans la plante, la cellule végétale ou le matériel végétal auquel il est transféré, et dans laquelle la protéine ayant une activité DEDD exonucléase de 3' vers 5' est exprimée dans un ovule de la plante.

14. Plante transgénique, cellule végétale ou matériel végétal selon la revendication 13, exprimant de manière transgénique la séquence polynucléotidique selon la revendication 4.

15. Utilisation de la plante transgénique, de la cellule végétale ou du matériel végétal selon l'une quelconque des revendications 13 ou 14 pour cribler un effecteur d'apomixie.
